# EUROPÄISCHE PATENTSCHRIFT

(11) **EP 0 818 448 B1**
(45) Veröffentlichungstag und Bekanntmachung des Hinweises auf die Patenterteilung: **12.11.2003**
(21) Anmeldenummer: 97110275.1
(22) Anmeldetag: 24.06.1997
(51) Int. Cl.: C07D 215/20, C07D 221/04, A61K 31/435

(54) **Cycloalkano-pyridine als CETP-Inhibitoren**
Cycloalkano-pyridine as CETP inhibitors
Cycloalkano-pyridines comme inhibiteurs de CETP

(30) Priorität: 08.07.1996 DE 19627419; 24.02.1997 DE 19707199
(43) Veröffentlichungstag der Anmeldung: 14.01.1998
(73) Patentinhaber: BAYER AG, 51368 Leverkusen (DE)
(72) Erfinder: Schmidt, Gunter, Dr., 42115 Wuppertal (DE); Brandes, Arndt, Dr., 42115 Wuppertal (DE); Angerbauer, Rolf, Dr., Higashinada-ku, Kobe-shi (JP); Lögers, Michael, Dr., 42327 Wuppertal (DE); Müller-Gliemann, Matthias, Dr., 42719 Solingen (DE); Schmeck, Carsten, Dr., 42113 Wuppertal (DE); Bremm, Klaus-Dieter, Dr., 45661 Recklinghausen (DE); Bischoff, Hilmar, Dr., 42113 Wuppertal (DE); Schmidt, Delf, Dr., 42113 Wuppertal (DE); Schuhmacher, Joachim, Dr., 42113 Wuppertal (DE); Giera, Henry, Dr., 51429 Bergisch Gladbach (DE); Paulsen, Holger, Dr., 42115 Wuppertal (DE); Naab, Paul, Dr., 42287 Wuppertal (DE); Conrad, Michael, Dr., 42327 Wuppertal (DE); Stoltefuss, Jürgen, 42781 Haan (DE)

(56) Entgegenhaltungen:
- EP-A- 0 304 063
- EP-A- 0 325 130
- CHEMICAL ABSTRACTS, vol. 93, no. 19, 10.November 1980 Columbus, Ohio, US; abstract no. 186126, DREIMANE, A. ET AL: "Competing reactions of.beta.-dicarbonyl and.beta.- aminovinylcarbonyl compounds with aldehydes in the synthesis of hexahydroquinolines" XP002043484 & KHIM. GETEROTSIKL. SOEDIN. (1980), (6), 791-5 CODEN: KGSSAQ;ISSN: 0453-8234, 1980,

## Beschreibung

Die vorliegende Erfindung betrifft Cycloalkano-pyridine, Verfahren zu ihrer Herstellung und ihre Verwendung in Arzneimitteln.

Aus der Publikation US-5 169 857-A2 sind 7-(polysubstituierte Pyridyl)-6-heptenoate zur Behandlung der Arteriosklerose, Lipoproteinaemia und Hyperproteinaemia bekannt. Außerdem wird die Herstellung von 7-(4-Aryl-3-pyridyl)-3,5-dihydroxy-6-heptenoate in der Publikation EP-325 130-A2 beschrieben. Ferner ist die Verbindung 5(6H)-Quinolone,3-benzyl-7,8-dihydro-2,7,7-trimethyl-4-phenyl, aus der Publikation Khim. Geterotsikl. Soedin. (1967), (6), 1118-1120 bekannt.

Die vorliegende Erfindung betrifft Cycloalkano-pyridine der allgemeinen Formel (I), 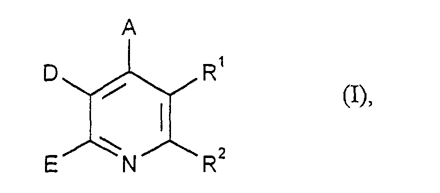 in welcher
- A: für Aryl mit 6 bis 10 Kohlenstoffatomen steht, das gegebenenfalls bis zu 5- fach gleich oder verschieden durch Halogen, Nitro, Hydroxy, Trifluormethyl, Trifluormethoxy oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert ist,
worin
R³ und R⁴ gleich oder verschieden sind und Wasserstoff, Phenyl oder geradkettiges oder verzweigtes Alkyl mit bis zu 6 Kohlenstoffatomen bedeuten,
- D: für Aryl mit 6 bis 10 Kohlenstoffatomen steht, das gegebenenfalls durch Phenyl, Nitro, Halogen, Trifluormethyl oder Trifluormethoxy substituiert ist, oder
für einen Rest der Formel

R⁵―L―,

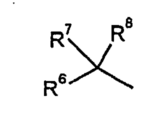

oder

R⁹―T―V―X―

steht,
worin
R⁵, R⁶ und R⁹ unabhängig voneinander Cycloalkyl mit 3 bis 6 Kohlenstoffatomen bedeuten, oder
Aryl mit 6 bis 10 Kohlenstoffatomen bedeuten oder einen 5- bis 7- gliedrigen, gegebenenfalls benzokondensierten, gesättigten oder ungesättigten, mono-, bi- oder tricyclischen Heterocyclus mit bis zu 4 Heteroatomen aus der Reihe S, N und/oder O bedeuten,
wobei die Cyclen, gegebenenfalls, im Fall der stickstoffhaltigen Ringe auch über die N-Funktion, bis zu 5-fach gleich oder verschieden durch Halogen, Trifluormethyl, Nitro, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 6 Kohlenstoffatomen, durch Aryl oder Trifluormethyl substituiertes Aryl mit jeweils 6 bis 10 Kohlenstoffatomen oder durch einen, gegebenenfalls benzokondensierten, aromatischen 5- bis 7-gliedrigen Heterocyclus mit bis zu 3 Heteroatomen aus der Reihe S, N und/oder O substituiert sind,
und/oder durch eine Gruppe der Formel -OR¹⁰, -SR¹¹, -SO₂R¹² oder -NR¹³R¹⁴ substituiert sind,
worin
R¹⁰, R¹¹ und R¹² unabhängig voneinander Aryl mit 6 bis 10 Kohlenstoffatomen bedeuten, das seinerseits gegebenenfalls bis zu 2-fach gleich oder verschieden durch Phenyl, Halogen oder durch geradkettiges oder verzweigtes Alkyl mit bis zu 6 Kohlenstoffatomen substituiert ist,
R¹³ und R¹⁴ gleich oder verschieden sind und die oben angegebene Bedeutung von R³ und R⁴ haben, oder
R⁵ und/oder R⁶ einen Rest der Formel 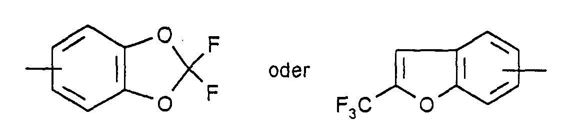 bedeuten,
R⁷ Wasserstoff oder Halogen bedeutet, und
R⁸ Wasserstoff, Halogen, Azido, Trifluormethyl, Hydroxy, Trifluormethoxy, geradkettiges oder verzweigtes Alkoxy oder Alkyl mit jeweils bis zu 6 Kohlenstoffatomen oder einen Rest der Formel -NR¹⁵R¹⁶ bedeutet,
worin
R¹⁵ und R¹⁶ gleich oder verschieden sind und die oben angegebene Bedeutung von R³ und R⁴ haben, oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 6 Kohlenstoffatomen bedeutet,
L eine geradkettige oder verzweigte Alkylen- oder Alkenylen-Kette mit jeweils bis zu 8 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 8 Kohlenstoffatomen bedeuten, oder
T oder X eine Bindung bedeutet,
V für ein Sauerstoff- oder Schwefelatom oder für eine -NR¹⁸-Gruppe steht,
worin
R¹⁸ Wasserstoff oder geradkettiges oder verzweigtes Alkyl mit bis zu 6 Kohlenstoffatomen oder Phenyl bedeutet,
E für Cycloalkyl mit 3 bis 8 Kohlenstoffatomen steht, oder für geradkettiges oder verzweigtes Alkyl mit bis zu 8 Kohlenstoffatomen steht, das gegebenenfalls durch Cycloalkyl mit 3 bis 8 Kohlenstoffatomen oder Hydroxy substituiert ist, oder für Phenyl steht, das gegebenenfalls durch Halogen oder Trifluormethyl substituiert ist,
- R¹ und R²: gemeinsam eine geradkettige oder verzweigte Alkylenkette mit bis zu 7 Kohlenstoffatomen bilden, die durch eine Oxogruppe und/oder durch einen Rest der Formel 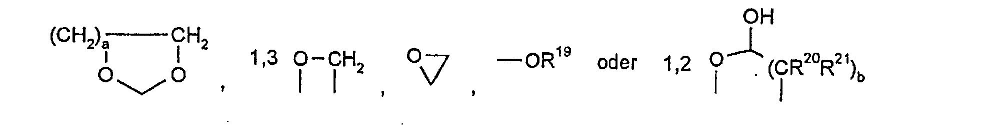 substituiert sein muß,
worin
a und b gleich oder verschieden sind und eine Zahl 1, 2 oder 3 bedeuten,
R¹⁹ Wasserstoff, Cycloalkyl mit 3 bis 7 Kohlenstoffatomen, geradkettiges oder verzweigtes Silylalkyl mit bis zu 8 Kohlenstoffatomen oder geradkettiges oder verzweigtes Alkyl mit bis zu 8 Kohlenstoffatomen bedeutet, das gegebenenfalls durch Hydroxy, geradkettiges oder verzweigtes Alkoxy mit bis zu 6 Kohlenstoffatomen oder durch Phenyl substituiert ist, das seinerseits durch Halogen, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 4 Kohlenstoffatomen oder Benzyl bedeutet, oder
R¹⁹ geradkettiges oder verzweigtes Acyl mit bis zu 20 Kohlenstoffatomen oder Benzoyl bedeutet, das gegebenenfalls durch Halogen, Trifluormethyl, Nitro oder Trifluormethoxy substituiert ist, oder geradkettiges oder verzweigtes Fluoracyl mit bis zu 8 Kohlenstoffatomen und 9 Fluoratomen bedeutet,
R²⁰ und R²¹ gleich oder verschieden sind, Wasserstoff, Phenyl oder geradkettiges oder verzweigtes Alkyl mit bis zu 6 Kohlenstoffatomen bedeuten, oder
R²⁰ und R²¹ gemeinsam einen 3 bis 6-gliedrigen Carbocyclus bilden, und, gegebenenfalls auch geminal, die gebildeten Carbocyclen gegebenenfalls bis zu 6-fach gleich oder verschieden durch Trifluormethyl, Hydroxy, Nitril, Halogen, Carboxyl, Nitro, Azido, Cyano, Cycloalkyl oder Cycloalkyloxy mit jeweils 3 bis 7 Kohlenstoffatomen, durch geradkettiges oder verzweigtes Alkoxycarbonyl, Alkoxy oder Alkylthio mit jeweils bis zu 6 Kohlenstoffatomen oder durch geradkettiges oder verzweigtes Alkyl mit bis zu 6 Kohlenstoffatomen substituiert sind, das seinerseits bis zu 2-fach gleich oder verschieden durch Hydroxyl, Benzyloxy, Trifluormethyl, Benzoyl, geradkettiges oder verzweigtes Alkoxy, Oxyacyl oder Carboxyl mit jeweils bis zu 4 Kohlenstoffatomen und/oder Phenyl substituiert ist, das seinerseits durch Halogen, Trifluormethyl oder Trifluormethoxy substituiert sein kann,
und/oder die gebildeten Carbocyclen, auch geminal, gegebenenfalls bis zu 5-fach gleich oder verschieden durch Phenyl, Benzoyl, Thiophenyl oder Sulfonylbenzyl substituiert sind, die ihrerseits gegebenenfalls durch Halogen, Trifluormethyl, Trifluormethoxy oder Nitro substituiert sind,
und/oder gegebenenfalls durch einen Rest der Formel

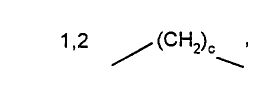

-SO₂-C₆H₅,

-(CO)_{d}-NR²³R²⁴

oder=O
substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cycloalkyl mit 3 bis 6 Kohlenstoffatomen, geradkettiges oder verzweigtes Alkyl mit bis zu 6 Kohlenstoffatomen, Benzyl oder Phenyl bedeuten, das gegebenenfalls bis zu 2-fach gleich oder verschieden durch Halogen, Trifluormethyl, Cyano, Phenyl oder Nitro substituiert ist, und/oder die gebildeten Carbocyclen gegebenenfalls durch einen spiro-verknüpften Rest der Formel 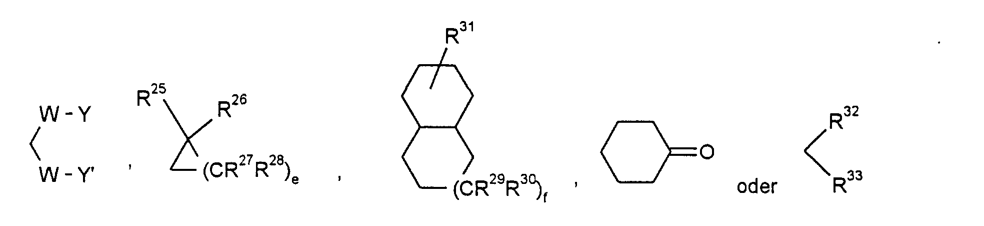 substituiert sind,
worin
W entweder ein Sauerstoff oder ein Schwefelatom bedeutet,
Y und Y' gemeinsam eine 2- bis 6-gliedrige geradkettige oder verzweigte Alkylenkette bilden,
e eine Zahl 1,2,3, 4, 5, 6 oder 7 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Halogen oder geradkettiges oder verzweigtes Alkyl oder Alkoxy mit jeweils bis zu 6 Kohlenstoffatomen bedeuten, oder
R²⁵ und R²⁶ oder R²⁷ und R²⁸ jeweils gemeinsam eine geradkettige oder verzweigte Alkylkette mit bis zu 6 Kohlenstoffatomen bilden, oder
R²⁵ und R²⁶ oder R²⁷ und R²⁸ jeweils gemeinsam einen Rest der Formel 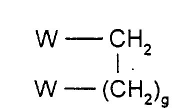 bilden,
worin
W die oben angegebene Bedeutung hat,
g eine Zahl 1, 2, 3, 4, 5, 6 oder 7 bedeutet,
R³² und R³³ gemeinsam einen 3- bis 7-gliedrigen Heterocyclus bilden, der ein Sauerstoff- oder Schwefelatom oder eine Gruppe der Formel SO, SO₂ oder -NR³⁴ enthält,
worin
R³⁴ Wasserstoff, Phenyl, Benzyl oder geradkettiges oder verzweigtes Alkyl mit bis zu 4 Kohlenstoffatomen bedeutet, und deren Salze und N-Oxide, mit Ausnahme von 5(6H)-Quinolone, 3-benzoyl-7,8-dihydro-2,7,7-trimethyl-4-phenyl.

Die erfindungsgemäßen Alkanopyridine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di-bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren sowie deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Indolyl, Isochinolyl, Chinolyl, Benzo[b]thiophen, Benzo[b]furanyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind die erfindungsgemäßen Verbindungen der allgemeinen Formel (I),
in welcher
- A: für Naphthyl oder Phenyl steht, die gegebenenfalls bis zu 2-fach gleich oder verschieden durch Fluor, Chlor, Brom, Amino, Hydroxy, Trifluormethyl, Trifluormethoxy oder durch geradkettiges oder verzweigtes Alkyl, oder Alkoxy mit jeweils bis zu 6 Kohlenstoffatomen substituiert sind,
- D: für Phenyl steht, das gegebenenfalls durch Nitro, Fluor, Chlor, Brom, Phenyl, Trifluormethyl oder Trifluormethoxy substituiert ist, oder für einen Rest der Formel

R⁵―L―

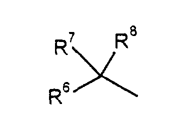

oder

R⁹―T―V―X―

steht,
worin
R⁵, R⁶ und R⁹ unabhängig voneinander Cyclopropyl, Cyclopentyl oder Cyclohexyl bedeuten, oder
Phenyl, Naphthyl, Pyridyl, Tetrazolyl, Pyrimidyl, Pyrazinyl, Pyrrolidinyl, Indolyl, Morpholinyl, Imidazolyl, Benzothiazolyl, Phenoxathiin-2-yl, Benzoxazolyl, Furyl, Chinolyl oder Purin-8-yl bedeuten, wobei die Cyclen, gegebenenfalls bis zu 3-fach im Fall der stickstoffhaltigen Ringe auch über die N-Funktion, gleich oder verschieden durch Fluor, Chlor, Brom, Trifluormethyl, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 4 Kohlenstoffatomen, Triazolyl, Tetrazolyl, Benzoxathiazolyl, oder Trifluormethyl substituiertes Phenyl oder Phenyl substituiert sind,
und/oder durch eine Gruppe der Formel -OR¹⁰, -SR¹¹ oder -SO₂R¹² substituiert sind,
worin
R¹⁰, R¹¹ und R¹² gleich oder verschieden sind und Phenyl bedeuten, das seinerseits gegebenenfalls bis zu 2-fach gleich oder verschieden durch Phenyl, Fluor, Chlor oder durch geradkettiges oder verzweigtes Alkyl mit bis zu 4 Kohlenstoffatomen substituiert ist, oder
R⁵ und/oder R⁶ einen Rest der Formel 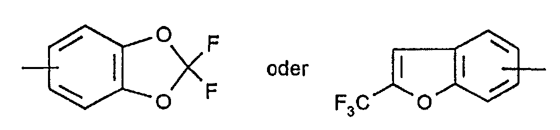 bedeuten,
R⁷ Wasserstoff, Fluor, Chlor oder Brom bedeutet, und
R⁸ Wasserstoff, Fluor, Chlor, Brom, Azido, Trifluormethyl, Hydroxy, Trifluormethoxy, geradkettiges oder verzweigtes Alkoxy oder Alkyl mit bis zu jeweils 5 Kohlenstoffatomen oder einen Rest der Formel -NR¹⁵R¹⁶ bedeutet,
worin
R¹⁵ und R¹⁶ gleich oder verschieden sind und Wasserstoff, Phenyl oder geradkettiges oder verzweigtes Alkyl mit bis zu 4 Kohlenstoffatomen bedeuten, oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 4 Kohlenstoffatomen bedeutet,
L eine geradkettige oder verzweigte Alkylen- oder Alkenylen-Kette mit jeweils bis zu 6 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 6 Kohlenstoffatomen bedeuten, oder
T oder X eine Bindung bedeutet,
V für ein Sauerstoff- oder Schwefelatom oder für eine Gruppe der Formel -NR¹⁸- steht,
worin
R¹⁸ Wasserstoff oder geradkettiges oder verzweigtes Alkyl mit bis zu 4 Kohlenstoffatomen oder Phenyl bedeutet,
E für Cyclopropyl, -butyl, -pentyl, -hexyl oder -heptyl steht, oder für geradkettiges oder verzweigtes Alkyl mit bis zu 6 Kohlenstoffatomen steht, das gegebenenfalls durch Cyclopropyl, -butyl, -hexyl, -pentyl, -heptyl oder durch Hydroxy substituiert ist, oder für Phenyl steht, das gegebenenfalls durch Fluor, Chlor oder Trifluormethyl substituiert ist,
- R¹ und R²: gemeinsam eine geradkettige oder verzweigte Alkylenkette mit bis zu 6 Kohlenstoffatomen bilden, die durch eine Oxygruppe und/oder durch einen Rest der Formel 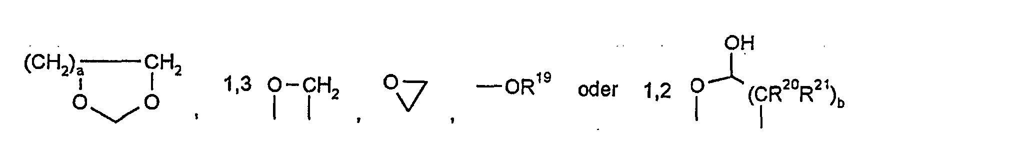 substituiert sein muß,
worin
a und b gleich oder verschieden sind und eine Zahl 1, 2 oder 3 bedeuten,
R¹⁹ Wasserstoff, Cyclopropyl, Cyclopentyl, Cyclohexyl, geradkettiges oder verzweigtes Silylalkyl mit bis zu 7 Kohlenstoffatomen oder geradkettiges oder verzweigtes Alkyl mit bis zu 6 Kohlenstoffatomen bedeutet, das gegebenenfalls durch Hydroxy, geradkettiges oder verzweigtes Alkoxy mit bis zu 4 Kohlenstoffatomen oder durch Phenyl substituiert ist, das seinerseits durch Fluor, Chlor, Brom, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 3 Kohlenstoffatomen oder Benzyl bedeutet, oder oder
R¹⁹ geradkettiges oder verzweigtes Acyl mit bis zu 18 Kohlenstoffatomen oder Benzoyl bedeutet, das gegebenenfalls durch Fluor, Chlor, Brom, Trifluormethyl, Nitro oder Trifluormethoxy substituiert ist, oder
geradkettiges oder verzweigtes Fluoracyl mit bis zu 6 Kohlenstoffatomen bedeutet,
R²⁰ und R²¹ gleich oder verschieden sind, Wasserstoff, Phenyl oder geradkettiges oder verzweigtes Alkyl mit bis zu 4 Kohlenstoffatomen bedeuten, oder
R²⁰ und R²¹ gemeinsam einen Cyclpropyl-, Cyclobutyl-, Cyclopentyl-, Cyclohexyl- oder Cycloheptylring bilden, und die gebildeten Carbocyclen gegebenenfalls bis zu gleich oder verschieden, gegebenenfalls auch geminal, durch Trifluormethyl, Hydroxy, Carboxyl, Azido, Fluor, Chlor, Brom, Nitro, Cyano, Cyclopropyl, Cyclobutyl, Cyclopentyl, Cyclohexyl, Cyclopropyloxy, Cyclopentyloxy, Cyclohexyloxy, durch geradkettiges oder verweigtes Alkoxycarbonyl, Alkoxy oder Alkylthio mit jeweils bis ca. 5 Kohlenstoffatomen oder geradkettiges oder verzweigtes Alkyl mit bis zu 5 Kohlenstoffatomen substituiert sind, das seinerseits bis zu 2-fach gleich oder verschieden durch Hydroxyl, Benzyloxy, Benzoyl, geradkettiges oder verzweitges Alkoxy oder Oxyacyl mit jeweils bis zu 3 Kohlenstoffatomen, Trifluormethyl, und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden durch Phenyl, Benzoyl, Thiophenyl oder Sulfonylbenzyl substituiert sind, die ihrerseits gegebenenfalls durch Fluor, Chlor, Brom, Trifluormethyl, Trifluormethoxy oder Nitro substituiert sind,
und/oder gegebenenfalls durch einen Rest der Formel 1,2

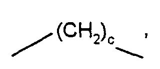

-SO₂-C₆H₅,

-(CO)_{d}-NR₂₃R₂₄

oder

=O

substituiert sind, worin
c eine Zahl 1, 2, 3 oder 4 bedeutet,
d eine Zahl 0 oder 1 bedeutet,
R²³ und R²⁴ gleich oder verschieden sind und Wasserstoff, Cyclopropyl, Cyclobutyl, Cyclopentyl, Cyclohexyl, geradkettiges oder verzweigtes Alkyl mit bis zu 5 Kohlenstoffatomen, Benzyl oder Phenyl bedeuten, das gegebenenfalls durch Fluor, Chlor, Brom, Phenyl oder Trifluormethyl substituiert ist, und/oder die gebildeten Carbocyclen gegebenenfalls durch einen spiroverknüpften Rest der Formel 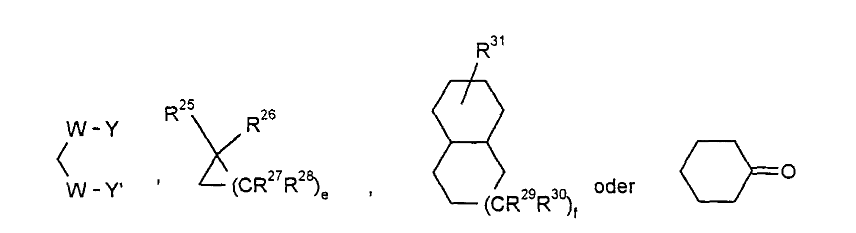 substituiert sind,
worin
W entweder ein Sauerstoff oder ein Schwefelatom bedeutet,
Y und Y' gemeinsam eine 2- bis 5-gliedrige geradkettige oder verzweigte Alkylkette bilden,
e eine Zahl 1, 2, 3, 4, 5 oder 6 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Fluor, Chlor, Brom oder geradkettiges oder verzweigtes Alkyl oder Alkoxy mit jeweils bis zu 5 Kohlenstoffatomen bedeuten, oder
R²⁵ und R²⁶ oder R²⁷ und R²⁸ jeweils gemeinsam eine geradkettige oder verzweigte Alkylkette mit bis zu 5 Kohlenstoffatomen bilden oder
R²⁵ und R²⁶ oder R²⁷ und R²⁸ jeweils gemeinsam einen Rest der Formel 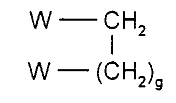 bilden,
worin
W die oben angegebene Bedeutung hat,
g eine Zahl 1, 2, 3, 4, 5 oder 6 bedeutet, und deren Salze und N-Oxide mit Ausnahme von 5(6H)-Quinolone, 3-benzoyl-7,8-dihydro-2,7,7-trimethyl-4-phenyl.

Besonders bevorzugt sind erfindungsgemäße Verbindungen der allgemeinen Formel (I),
in welcher
- A: für Phenyl steht, das gegebenenfalls bis zu 2-fach gleich oder verschieden durch Fluor, Chlor, Brom, Hydroxy, Trifluormethyl, Trifluormethoxy oder durch geradkettiges oder verzweigtes Alkyl oder Alkoxy mit jeweils bis zu 5 Kohlenstoffatomen substituiert ist,
- D: für Phenyl steht, das gegebenenfalls durch Nitro, Trifluormethyl, Phenyl, Fluor, Chlor oder Brom substituiert ist, oder
für einen Rest der Formel

R⁵―L―

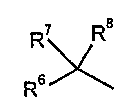

oder

R⁹―T―V―X―

steht,
worin
R⁵, R⁶ und R⁹ unabhängig voneinander Cyclopropyl, Cyclopentyl oder Cyclohexyl bedeuten, oder
Phenyl, Naphthyl, Pyridyl, Tetrazolyl, Pyrimidyl, Pyrazinyl, Phenoxathiin-2-yl, Indolyl, Imidazolyl, Pyrrolidinyl, Morpholinyl, Benzothiazolyl, Benzoxazolyl, Furyl, Chinolyl oder Purin-8-yl bedeuten, wobei die Cyclen, gegebenenfalls bis zu 3-fach, im Fall der stickstoffhaltigen Ringe auch über die N-Funktion, gleich oder verschieden durch Fluor, Chlor, Trifluormethyl, Hydroxy, Cyano, Carboxyl, Trifluormethoxy, geradkettiges oder verzweigtes Alkyl, Alkylthio, Alkylalkoxy, Alkoxy oder Alkoxycarbonyl mit jeweils bis zu 4 Kohlenstoffatomen, Triazolyl, Tetrazolyl, Benzothiazolyl, Trifluormethyl substituiertes Phenyl oder Phenyl substituiert sind und/oder durch eine Gruppe der Formel -OR¹⁰, -SR¹¹ oder -SO₂R¹² substituiert sind,
worin
R¹⁰, R¹¹ und R¹² gleich oder verschieden sind und Phenyl bedeuten, das seinerseits gegebenfalls bis zu 2-fach gleich oder verschieden durch Phenyl, Fluor, Chlor oder durch geradkettiges oder verzweigtes Alkyl mit bis zu 3 Kohlenstoffatomen substituiert ist, oder
R⁵ und/oder R⁶ einen Rest der Formel 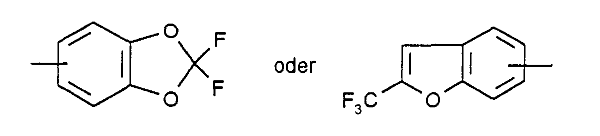 bedeuten,
R⁷ Wasserstoff oder Fluor bedeutet, und
R⁸ Wasserstoff, Fluor, Chlor, Brom, Azido, Trifluormethyl, Hydroxy, Trifluormethoxy, oder geradkettiges oder verzweigtes Alkoxy oder Alkyl mit jeweils bis zu 4 Kohlenstoffatomen oder einen Rest der Formel -NR¹⁵R¹⁶ bedeutet,
worin
R¹⁵ und R¹⁶ gleich oder verschieden sind und Wasserstoff oder geradkettiges oder verzweigtes Alkyl mit bis zu 3 Kohlenstoffatomen bedeuten, oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 4 Kohlenstoffatomen bedeutet,
L eine geradkettige oder verzweigte Alkylen- oder Alkenylen-Kette mit jeweils bis zu 5 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 3 Kohlenstoffatomen bedeuten, oder
T oder X eine Bindung bedeutet,
V für ein Sauerstoff- oder Schwefelatom oder für eine Gruppe der Formel -NR¹⁸ steht,
worin
R¹⁸ Wasserstoff oder geradkettiges oder verzweigtes Alkyl mit bis zu 3 Kohlenstoffatomen bedeutet,
E für Cyclopropyl, Cyclopentyl oder Cyclohexyl oder Phenyl steht, das gegebenenfalls durch Fluor oder Trifluormethyl substituiert ist, oder
für geradkettiges oder verzweigtes Alkyl mit bis zu 4 Kohlenstoffatomen steht, das gegebenenfalls durch Hydroxy substituiert ist,
- R¹ und R²: gemeinsam eine geradkettige oder verzweigte Alkylenkette mit bis zu 5 Kohlenstoffatomen bilden, die durch eine Oxogruppe und/oder einen Rest der Formel 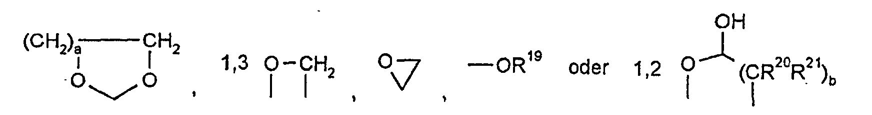 substituiert sein muß,
worin
a und b gleich oder verschieden sind und eine Zahl 1, 2 oder 3 bedeuten,
R¹⁹ Wasserstoff, Cyclopropyl, Cyclopentyl, Cyclohexyl, geradkettiges oder verzweigtes Silylalkyl mit bis zu 6 Kohlenstoffatomen oder geradkettiges oder verzweigtes Alkyl mit bis zu 4 Kohlenstoffatomen bedeutet, das gegebenenfalls durch Hydroxy, geradkettiges oder verzweigtes Alkoxy mit bis zu 3 Kohlenstoffatomen oder durch Phenyl substituiert ist, das seinerseits durch Fluor, Chlor, Brom, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 3 Kohlenstoffatomen oder Benzyl bedeutet, oder
R¹⁹ geradkettiges oder verzweigtes Acyl mit bis zu 15 Kohlenstoffatomen oder Benzoyl bedeutet, das gegebenenfalls durch Fluor, Chlor, Brom, Trifluormethyl, Nitro oder Trifluormethoxy substituiert ist, oder
geradkettiges oder verzweigtes Fluoracyl mit bis zu 4 Kohlenstoffatomen bedeutet,
R²⁰ und R²¹ gleich oder verschieden sind, Wasserstoff, Phenyl oder geradkettiges oder verzweigtes Alkyl mit bis zu 3 Kohlenstoffatomen bedeuten, oder
R²⁰ und R²¹ gemeinsam einen Cyclopropyl-, Cyclopentyl- oder Cyclohexylring bilden, und die gebildeten Carbocyclen gegebenenfalls bis zu 4-fach gleich oder verschieden, gegebenenfalls auch geminal, durch Fluor, Hydroxyl, Trifluormethyl, Carboxyl, Azido, Chlor, Brom, Nitro, Cyano, Cyclopropyl, Cyclobutyl, Cyclopentyl, Cyclohexyl, Cyclopropyloxy, Cyclopentyloxy, Cyclohexyloxy, durch geradkettiges oder verzweigtes Alkoxycarbonyl, Alkoxy oder Alkylthio mit jeweils bis zu 4 Kohlenstoffatomen oder geradkettiges oder verzweigtes Alkyl mit bis zu 4 Kohlenstoffatomen substituiert sind, das seinerseits bis zu 2-fach gleich oder verschieden durch Hydroxyl, Benzyloxy, Trifluormethyl, Benzoyl, Methoxy, Oxyacetyl und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden, durch Phenyl, Benzoyl, Thiophenyl oder Sulfonylbenzyl substituiert sind, die ihrerseits gegebenenfalls durch Fluor, Trifluormethyl, Trifluormethoxy oder Nitro substituiert sind,
und/oder gegebenenfalls durch einen Rest der Formel

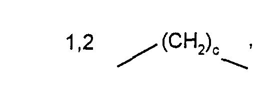

-SO₂-C₆H₅,

-(CO)_{d}-NR²³R²⁴

oder =O
substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cyclopropyl, Cyclopentyl, Benzyl, geradkettiges oder verzweigtes Alkyl mit bis zu 4 Kohlenstoffatomen oder Phenyl bedeuten, das gegebenenfalls durch Fluor, Chlor oder Brom substituiert ist, und/oder die gebildeten Carbocyclen gegebenenfalls durch einen spiroverknüpften Rest der Formel 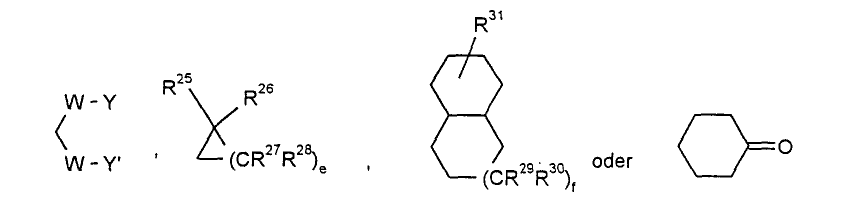 substituiert sind,
worin
W entweder ein Sauerstoff oder ein Schwefelatom bedeutet,
Y und Y' gemeinsam eine 2- bis 6-gliedrige geradkettige oder verzweigte Alkylenkette bilden,
e eine Zahl 1, 2, 3, 4 oder 5 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Fluor, Chlor, Brom oder geradkettiges oder verzweigtes Alkyl oder Alkoxy mit jeweils bis zu 4 Kohlenstoffatomen bedeuten, oder
R²⁵ und R²⁶ oder R²⁷ und R²⁸ jeweils gemeinsam eine geradkettige oder verzweigte Alkylkette mit bis zu 4 Kohlenstoffatomen bilden, oder
- R²⁵ und R²⁶ oder R²⁷ und R²⁸: jeweils gemeinsam einen Rest der Formel 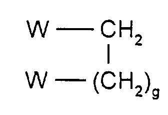 bilden,
worin
W die oben angegebene Bedeutung hat,
g eine Zahl 1, 2, 3, 4, 5, 6 oder 7 bedeutet, und deren Salze und N-Oxide, mit Ausnahme von 5(6H)-Quinolone, 3-benzoyl-7,8-dihydro-2,7,7-trimethyl-4-phenyl.

Ganz besonders bevorzugt sind die Verbindungen der Formel (I), in welchen
- A: für 4-Fluor-Phenyl steht.

Ebenso solche Verbindungen, in denen
- E: für Isopropyl oder Cyclopentyl steht.

Außerdem wurden Verfahren zur Herstellung der erfindungsgemäßen Verbindungen der allgemeinen Formel (I) gefunden, dadurch gekennzeichnet, daß man
[A] im Fall D ≠ Aryl, in Verbindungen der allgemeinen Formel (II) 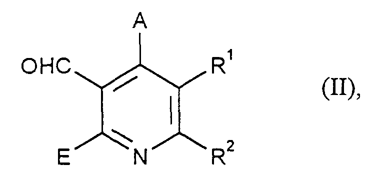 in welcher
   - A, E, R¹ und R²: die oben angegebene Bedeutung haben,
   mit metallorganischen Reagenzien im Sinne einer Grignard-, Wittig- oder Li-organische-Reaktion den Substituenten D in inerten Lösemitteln synthetisiert,
   oder im Fall, daß D für den Rest der Formel R⁹-T-V-X steht, in welcher V ein Sauerstoffatom bedeutet,
[B] entweder Verbindungen der allgemeinen Formel (III) 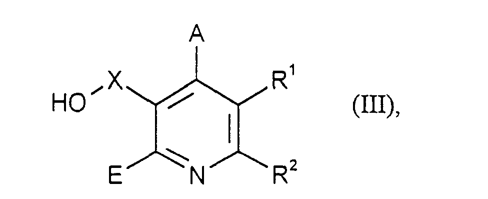 in welcher
   - A, E, X, R¹ und R²: die oben angegebene Bedeutung haben,
   mit Verbindungen der allgemeinen Formel (IV)

   R⁹-T-Z (IV),

   in welcher
   - R⁹ und T: die oben angegebene Bedeutung haben
   und
   - Z: für Halogen, vorzugsweise Chlor oder Brom, steht,
   in inerten Lösemitteln, gegebenenfalls in Anwesenheit einer Base und/oder Hilfsstoffs umsetzt,
   oder
[C] Verbindungen der allgemeinen Formel (III) zunächst durch Umsetzung mit Verbindungen der allgemeinen Formel (V) 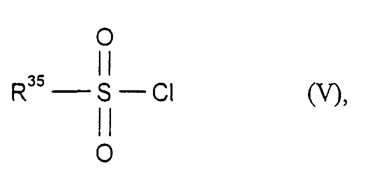 in welcher
   - R³⁵: für geradkettiges Alkyl mit bis zu 4 Kohlenstoffatomen steht,
   in die Verbindungen der allgemeinen Formel (VI) 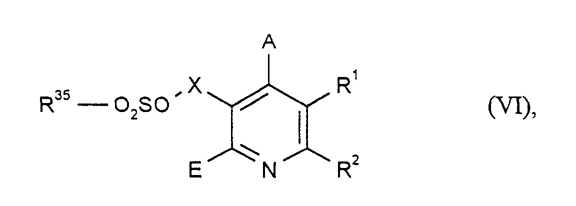 in welcher
   - A, E, X, R¹, R² und R³⁵: die oben angegebene Bedeutung haben,
   überführt und anschließend mit Verbindungen der allgemeinen Formel (VII)

   R⁹-T-V-H (VII),

   in welcher
   - R⁹, T und V: die oben angegebene Bedeutung haben,
   umsetzt und gegebenenfalls Schutzgruppen abspaltet,
   oder
[D] im Fall der Verbindungen der allgemeinen Formel (Ia) 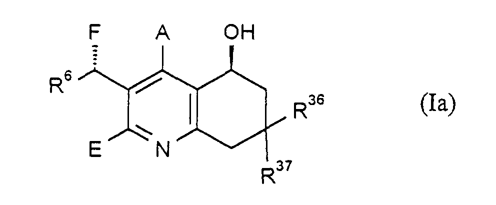 in welcher
   - A und R⁶: die oben angegebene Bedeutung haben,
   - R³⁶ und R³⁷: gleich oder verschieden sind und
   für Trifluormethyl, Halogen, Nitro, Azido, Cyano, Cycloalkyl oder Cycloalkyloxy mit jeweils 3 bis 7 Kohlenstoffatomen stehen, oder
   für geradkettiges oder verzweigtes Alkoxycarbonyl, Alkoxy oder Alkylthio mit jeweils bis zu 6 Kohlenstoffatomen oder für geradkettiges oder verzweigtes Alkyl mit bis zu 6 Kohlenstoffatomen stehen, oder
   für Phenyl, Benzoyl, Thiophenyl oder Sulfonylbenzyl stehen, die ihrerseits gegebenenfalls durch Halogen, Trifluormethyl, Trifluormethoxy oder Nitro substituiert sind, oder
   - R³⁶ und R³⁷: für einen der oben aufgeführten spiro-verknüpften Reste der Formel 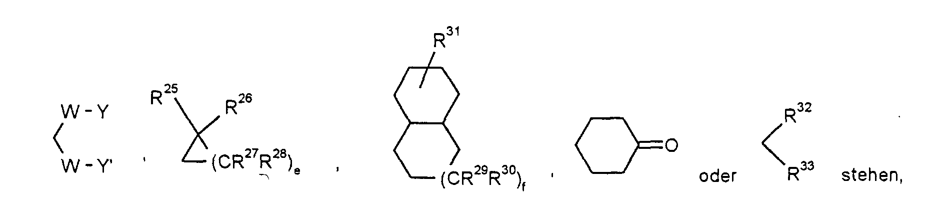
   worin
   - W, Y, Y', R²⁵, R²⁶, R²⁷, R²⁸, e, R²⁹, R³⁰, R³¹, R³² und R³³: die oben angegebene Bedeutung haben,
   Verbindungen der allgemeinen Formel (VIII) 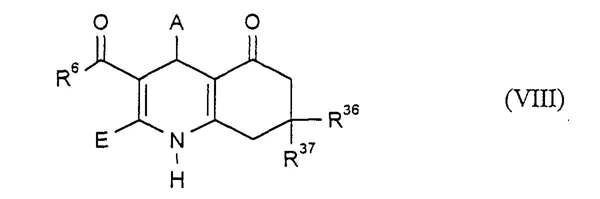 in welcher
   - R⁶, R³⁶, R³⁷, A und E: die oben angegebene Bedeutung haben,
   zunächst zu den Verbindungen der allgemeinen Formel (IX) 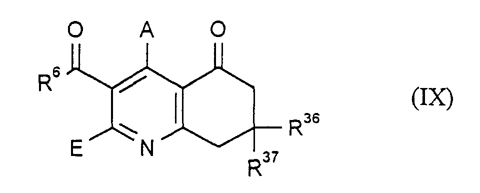 in welcher
   - R⁶, R³⁶, R³⁷, A und E: die oben angegebene Bedeutung haben,
   oxidiert,
   diese in einem nächsten Schritt durch eine asymmetrische Reduktion zu den Verbindungen der allgemeinen Formel (X) 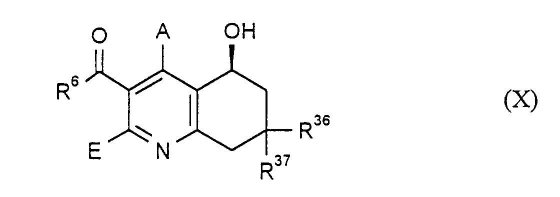 in welcher
   R⁶, R³⁶, R³⁷, A und E die oben angegebene Bedeutung haben,
   umsetzt,
   diese dann durch die Einführung einer Hydroxyschutzgruppe in die Verbindungen der allgemeinen Formel (XI) 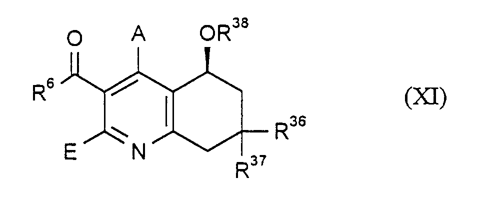 in welcher
   - R⁶, R³⁶, R³⁷, A und E: die oben angegebene Bedeutung haben
   und
   - R³⁸: für eine Hydroxyschutzgruppe, vorzugsweise für einen Rest der Formel -SiR³⁹R⁴⁰R⁴¹ steht,
   worin
   R³⁹, R⁴⁰ und R⁴¹ gleich oder verschieden sind und C₁-C₄-Alkyl bedeuten,
   überführt,
   aus diesem in einem Folgeschritt durch diastereoselektive Reduktion die Verbindungen der allgemeinen Formel (XII) 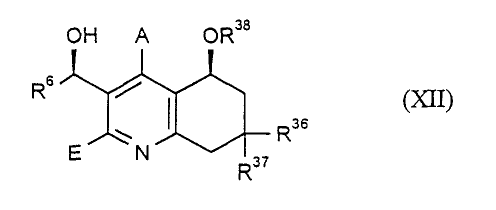 in welcher
   - R⁶, R³⁶, R³⁷, R³⁸, A und E: die oben angegebene Bedeutung haben,
   herstellt,
   und anschließend durch Einführung des Fluorsubstituenten mit Fluorierungsreagentien, wie z.B. DAST und SF₄-Derivaten die Verbindungen der allgemeinen Formel (XIII) 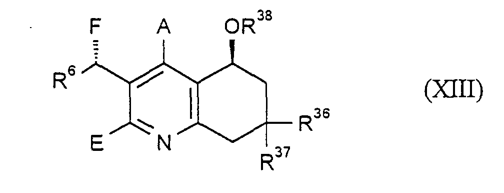 in welcher
   - R⁶, R³⁶, R³⁷, R³⁸, A und E: die oben angegebene Bedeutung haben,
   herstellt,
   und anschließend die Hydroxyschutzgruppe nach üblichen Methoden abspaltet,
   und gegebenenfalls die unter D, E und/oder R¹ und R² aufgeführten Substituenten nach üblichen Methoden variiert oder einführt.

Die erfindungsgemäßen Verfahren können durch folgende Formelschemata beispielhaft erläutert werden:

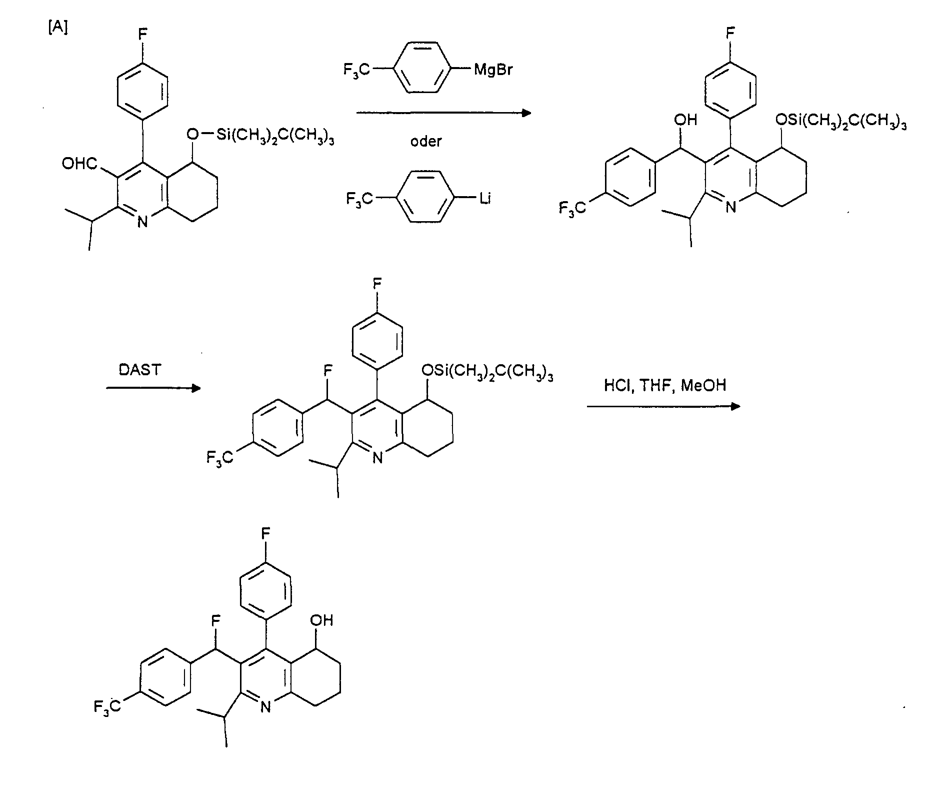

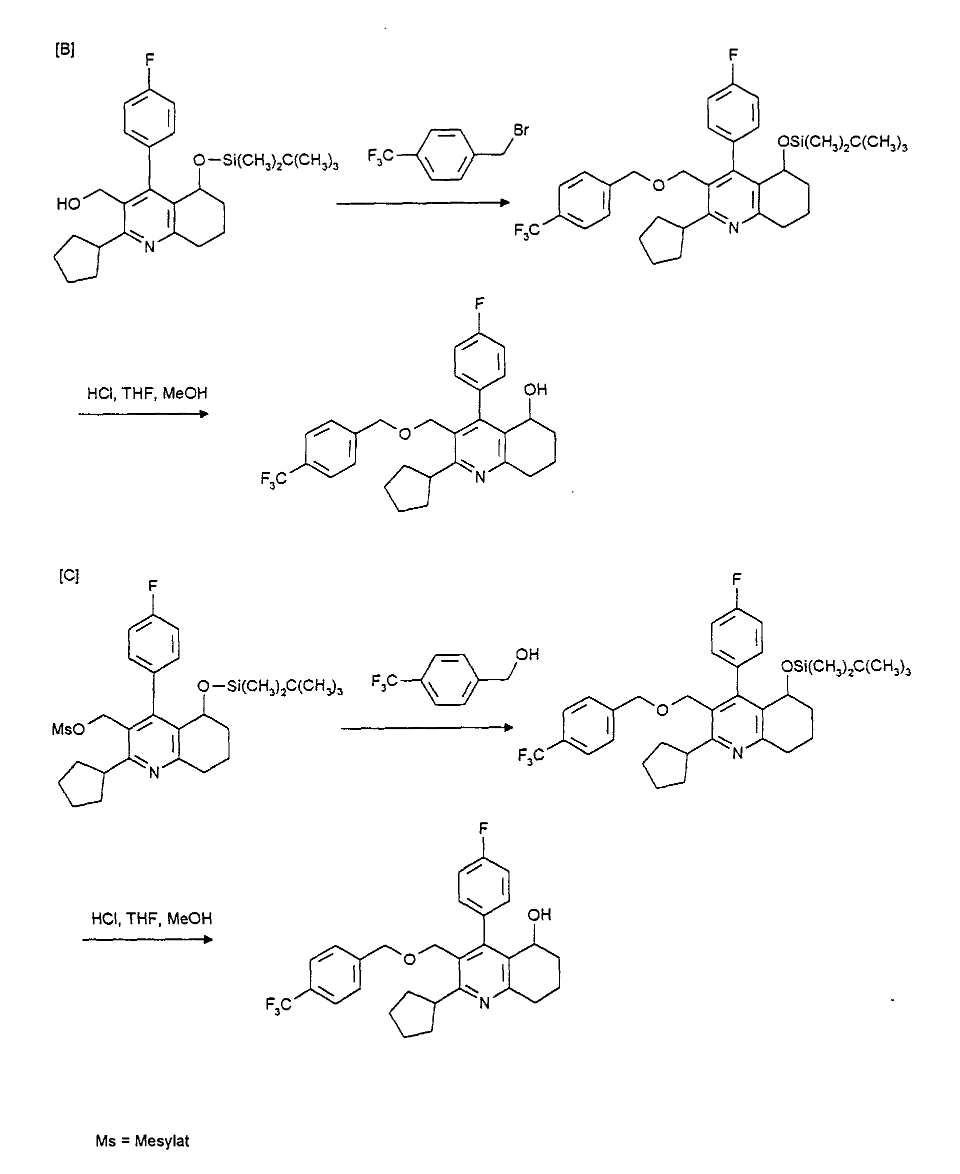

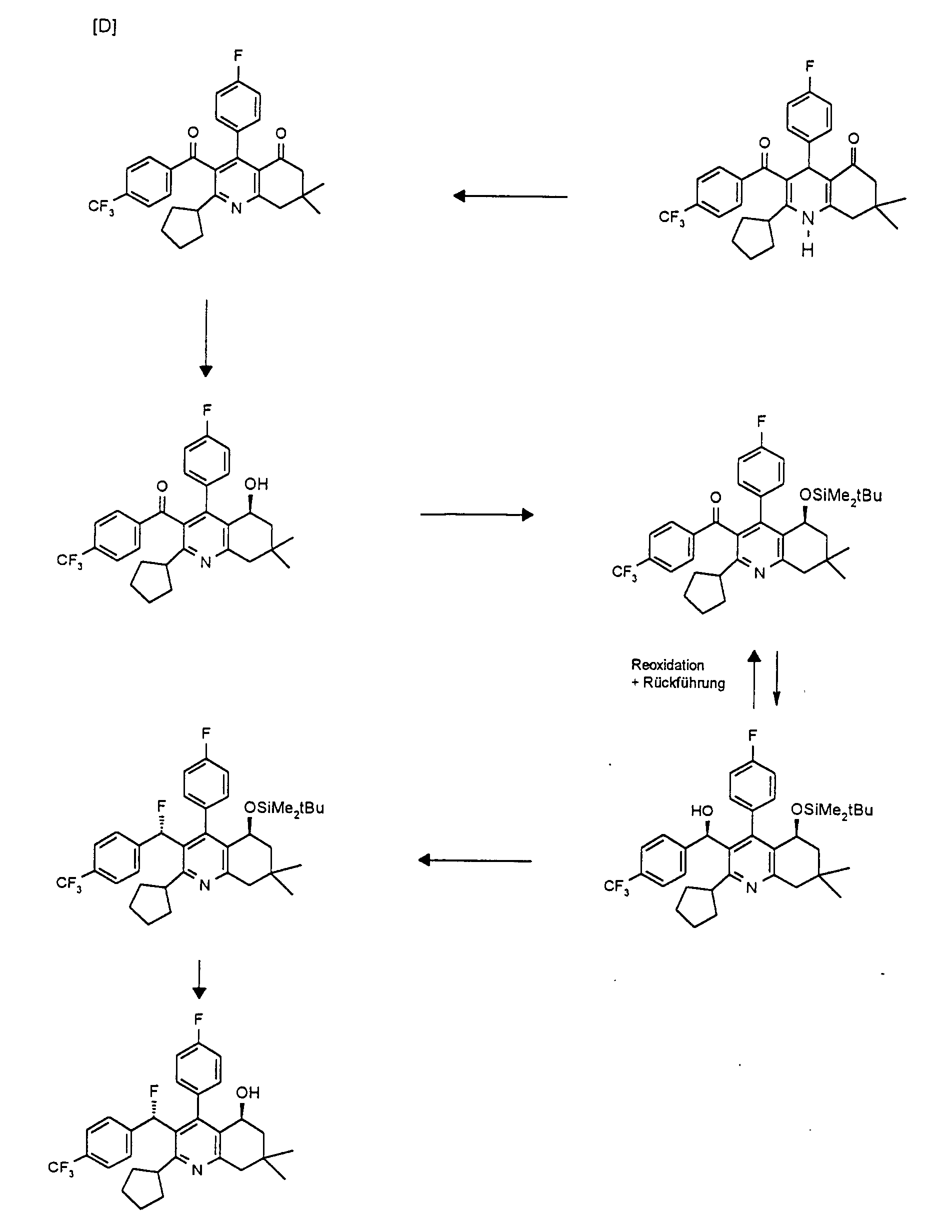

Als Lösemittel für alle Verfahren eignen sich Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen kommen für die einzelnen Schritte die üblichen stark basischen Verbindungen in Frage. Hierzu gehören bevorzugt lithiumorganische Verbindungen wie beispielsweise N-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird N-Butyllithium, Natriumhydrid oder Lithiumdiisopropylamid eingesetzt.

Für die Verfahren [B] und [C] eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydrid oder Kaliumhydroxid eingesetzt.

Als metallorganische Reagenzien eignen sich beispielsweise Systeme wie Mg/Brombenzotrifluorid und p-Trifluormethylphenyllithium.

Die Reduktionen werden im allgemeinen mit Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen in einem Temperaturbereich von -78°C bis +50°C, bevorzugt von -78°C bis 0°C im Falle des DIBAH, 0°C bis Raumtemperatur im Falle des NaBH₄, besonders bevorzugt bei -78°C, jeweils in Abhängigkeit von der Wahl des Reduktionsmittels sowie Lösemittel.

Die Reduktion verläuft im allgemeinen bei Normaldruck, es ist aber auch möglch bei erhöhtem oder erniedrigtem Druck zu arbeiten.

Die Hydrierung erfolgt nach üblichen Methoden mit Wasserstoff in Anwesenheit von Edelmetalkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Bevorzugt wird das Verfahren im Fall [A] zunächst mit Verbindungen der allgemeinen Formel (II), in welcher der Carbocyclus R¹/R² zunächst nur durch eine Gruppe -OSiR^{I}R^{II}R^{III} substituiert ist, worin R^{I}, R^{II} und R^{III} gleich oder verschieden sind und Phenyl oder geradkettiges oder verzweigtes Alkyl mit bis zu 5 Kohlenstoffatomen bedeuten, durchgeführt und nach Abspaltung der Schutzgruppe der oben unter R¹⁹/R²⁰ angegebene Substituent nach üblichen Methoden einführt.

Die Abspaltung der Schutzgruppe erfolgt im allgemeinen in einem der oben aufgeführten Alkohole und THF, vorzugsweise Methanol / THF in Anwesenheit von Salzsäure in einem Temperaturbereich von 0°C bis 50°C, vorzugsweise bei Raumtemperatur, und Normaldruck. In besonderen Fällen wird die Abspaltung der Schutzgruppe mit Tetrabutylammoniumfluorid (TBAF) in THF bevorzugt.

Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Tetrahydropyranyl, tert.Butyldimethylsilyl und Triisopropylsilyl. Besonders bevorzugt ist tert.Butyldimethylsilyl.

Als Lösemittel für die einzelnen Schritte eignen sich Ether wie Diethylether, Dioxan, Tetrahydrofuran, Glykoldimethylether, Diisopropylether oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol. Ebenso ist es möglich, Gemische der genannten Lösemittel zu verwenden.

Als Oxidationsmittel zur Herstellung der Verbindungen der allgemeinen Formel (IX) eignen sich beispielsweise Salpetersäure, Cer(IV)Cer(IV)-ammoniumnitrat, 2,3-Dichlor-5,6-dicyan-benzochinon, Pyridiniumchlorochromat (PCC), Pyridiniumchlorochromat auf basischem Aluminiumoxid, Osmiumtetroxid und Mangandioxid. Bevorzugt sind Mangandioxid und Salpetersäure.

Die Oxidation erfolgt in einem der oben aufgeführten chlorierten Kohlenwasserstoffe und Wasser. Bevorzugt sind Dichlormethan und Wasser.

Das Oxidationsmittel wird in einer Menge von 1 mol bis 10 mol, bevorzugt von 2 mol bis 5 mol, bezogen auf 1 mol der Verbindungen der allgemeinen Formel (VIII), eingesetzt.

Die Oxidation verläuft im allgemeinen bei einer Temperatur von -50°C bis +100°C, bevorzugt von 0°C bis Raumtemperatur.

Die Oxidation verläuft im allgemeinen bei Normaldruck. Es ist aber auch möglich, die Oxidation bei erhöhtem oder erniedrigtem Druck durchzuführen.

Die asymmetrische Reduktion zu den Verbindungen der allgemeinen Formel (X) erfolgt im allgemeinen in einem der oben aufgeführten Ether oder Toluol, vorzugsweise Tetrahydrofuran und Toluol.

Die Reduktion erfolgt im allgemeinen mit enantiomerenreinen 1R,2S-Aminoindanol und Borankomplexen wie BH₃ x THF, BH₃ x DMS und BH₃ x (C₂H₅)₂NC₆H₅. Bevorzugt ist das System Borandiethylanilin / 1R,2S-Aminoindanol.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen bei einer Temperatur von -78°C bis +50°C, bevorzugt von 0°C bis 30°C.

Die Reduktion verläuft im allgemeinen bei Normaldruck, es ist aber auch möglich bei erhöhtem oder erniedrigtem Druck zu arbeiten.

Die Einführung der Hydroxyschutzgruppe erfolgt in einem der oben aufgeführten Kohlenwasserstoffe, Dimethylformamid oder THF, vorzugsweise in Toluol in Anwesenheit von Lutidin in einem Temperaturbereich von -20°C bis +50°C, vorzugsweise von -5°C bis Raumtemperatur und Normaldruck.

Reagenzien zur Einführung der Silylschutzgruppe sind im allgemeinen tert-Butyldimethylsilylchlorid oder tert.-Butyldimethylsilyltrifluormethansulfonat. Bevorzugt ist tert.-Butyldimethylsilyltrifluormethansulfonat.

Die Reduktion zu den Verbindungen der allgemeinen Formel (XI) verläuft in einem der oben aufgeführten Kohlenwasserstoffe, vorzugsweise Toluol.

Die Reduktion zur Herstellung der Verbindungen der allgemeinen Formel (XII) wird im allgemeinen mit üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid, Natrium-bis-(2-methoxyethoxy)-dihydroaluminat oder Lithiumaluminiumhydrid durchgeführt. Ganz besonders bevorzugt wird die Reduktion mit Natrium-bis-(2-methoxyethoxy)-dihydroaluminat durchgeführt.

Das Reduktionsmittel wird im allgemeinen in einer Menge von 1 mol bis 6 mol, bevorzugt von 1 mol bis 3 mol bezogen auf 1 mol der zu reduzierenden Verbindungen, eingesetzt.

Die Reduktion verläuft im allgemeinen bei einer Temperatur von -20°C bis +110°C, bevorzugt von 0°C bis Raumtemperatur.

Die Reduktion verläuft im allgemeinen bei Normaldruck, es ist aber auch möglch bei erhöhtem oder erniedrigtem Druck zu arbeiten.

Bei der Reduktion zu den Verbindungen der allgemeinen Formel (XII) bleiben in der Mutterlauge geringe Reste des falschen Diastereomeren. Diese Reste können mit gängigen Oxidationsmitteln wie z.B. Pyridiniumchlorochromat (PCC) oder aktiviertem Braunstein, insbesondere mit aktiviertem Braunstein zu geschütztem (XI) reoxidiert werden und somit dem Synthesezyklus ohne Ausbeuteverlust zugeführt werden.

Die Einführung des Fluorsubstituenten erfolgt im allgemeinen in einem der oben aufgeführten Kohlenwasserstoffe oder Methylenchlorid, vorzugsweise in Toluol und unter Schutzgasatmosphäre.

Unter SF₄-Derivaten werden im allgemeinen Diethylaminoschwefeltrifluorid oder 2,2'-Bisfluorsubstituierte Amine wie beispielsweise Diethyl-1,2,3,3,3-hexafluorpropylamin hergestellt.

Die Reaktion verläuft im allgemeinen bei einer Temperatur von -78°C bis 100°C, bevorzugt im Falle des Dimethylaminoschwefeltrifluorid bei -78°C bis RT und im Falle des Diethyl-1,1,2,3,3,3-hexafluorpropylamins bei Raumtemperatur bis 80°C.

Die Abspaltung der Schutzgruppe erfolgt im allgemeinen in einem der oben aufgeführten Alkohole und THF, vorzugsweise Methanol / THF in Anwesenheit von Salzsäure in einem Temperaturbereich von 0°C bis 50°C, vorzugsweise bei Raumtemperatur, und Normaldruck. In besonderen Fällen wird die Abspaltung der Schutzgruppe mit Tetrabutylammoniumfluorid (TBAF) in THF bei Raumtemperatur bevorzugt.

Als Derivatisierungen seien beispielhaft folgende Reaktionstypen genannt:

Oxidationen, Reduktionen, Hydrierungen, Halogenierung, Wittig/Grignard-Reaktionen und Amidierungen /Sulfoamidierungen.

Als Basen kommen für die einzelnen Schritte die üblichen stark basischen Verbindungen in Frage. Hierzu gehören bevorzugt lithiumorganische Verbindungen wie beispielsweise n-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird N-Butyllithium, Natriumhydrid oder Lithiumdiisopropylamid eingesetzt.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydroxid oder Kaliumhydroxid eingesetzt.

Als Lösemittel eignen sich für die einzelnen Reaktionsschritte auch Alkohole wie Methanol, Ethanol, Propanol, Butanol oder tert.Butanol. Bevorzugt ist tert.Butanol.

Gegebenenfalls ist es nötig, einige Reaktionsschritte unter Schutzgasatmosphäre durchzuführen.

Die Halogenierungen erfolgen im allgemeinen in einem der oben aufgeführten chlorierten Kohlenwasserstoffen, wobei Methylenchlorid bevorzugt ist.

Als Halogenierungsmittel eignen sich beispielsweise Diethylamino-Schwefeltrifluorid (DAST), Morpholino-Schwefeltrifluorid oder SOCl₂.

Die Halogenierung verläuft im allgemeinen in einem Temperaturbereich von -78°C bis +50°C, bevorzugt von -78°C bis 0°C, jeweils in Abhängigkeit von der Wahl des Halogenierungsmittels sowie Lösemittel.

Die Halogenierung verläuft im allgemeinen bei Normaldurck, es ist aber auch möglich, bei erhöhtem oder erniedrigtem Druck zu arbeiten.

Die Verbindungen der allgemeinen Formeln (II) und (III) sind neu und können hergestellt werden, indem man
durch Umsetzung der Verbindungen der allgemeinen Formel (XIV) 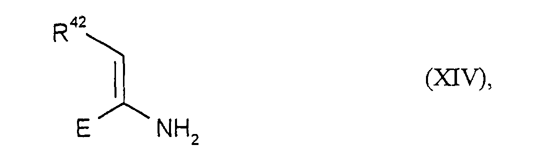 in welcher
- E: die oben angegebene Bedeutung hat
und
- R⁴²: für C₁-C₄-Alkoxycarbonyl oder Aryl (D = Aryl) steht,
mit Aldehyden der allgemeinen Formel (XV)

A-CHO (XV),

in welcher
- A: die oben angegebene Bedeutung hat,
und Verbindungen der allgemeinen Formel (XVI) 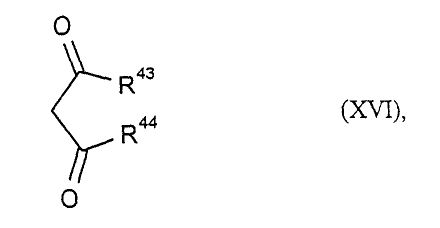 in welcher
- R⁴³ und R⁴⁴: unter Einbezug einer Carbonylgruppe den oben angegebenen Bedeutungsumfang von R¹ und R² umfassen,
die Verbindungen der allgemeinen Formel (XVII) 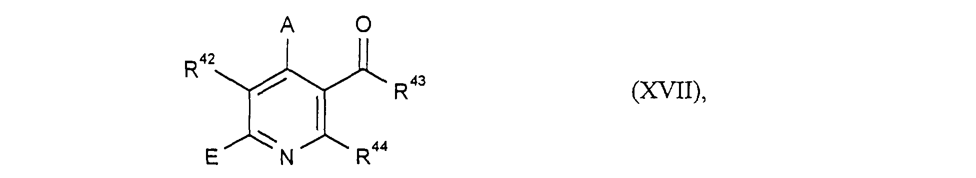 in welcher
- A, E, R⁴², R⁴³ und R⁴⁴: die oben angegebene Bedeutung haben,
herstellt,
und im Fall der Verbindungen der allgemeinen Formel (III) eine Reduktion, wie oben beschrieben, zur Hydroxymethylfunktion anschließt,
und in einem letzten Schritt die Alkoxycarbonylgruppe (R⁴²) durch eine Reduktions-Oxidations-Sequenz in eine Aldehydgruppe überführt.

Als Lösemittel eignen sich für die Oxidation Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Methylenchlorid.

Als Oxidationsmittel eignen sich beispielsweise Cer(IV)-ammoniumnitrat, 2,3-Dichlor-5,6-dicyan-benzochinon, Pyridiniumchlorochromat (PCC), Pyridiniumchlorochromat auf basischem Aluminiumoxid, Osmiumtetroxid und Mangandioxid. Bevorzugt sind Schwefeltrioxid-Pyridinkomplex in DMSO/Methylenchlorid und Pyridiniumchlorochromat auf basischem Aluminiumoxid.

Das Oxidationsmittel wird in einer Menge von 1 mol bis 10 mol, bevorzugt von 2 mol bis 5 mol, bezogen auf 1 mol der Verbindungen der allgemeinen Formel (XVII), eingesetzt.

Die Oxidation verläuft im allgemeinen in einem Temperaturbereich von -50°C bis +100°C, bevorzugt von 0°C bis Raumtemperatur.

Die Oxidation verläuft im allgemeinen bei Normaldruck. Es ist aber auch möglich, die Oxidation bei erhöhtem oder erniedrigtem Druck durchzuführen.

Die Verbindungen der allgemeinen Formeln (IV), (V), (VII), (XIV), (XV) und (XVI) sind an sich bekannt oder nach üblichen Methoden herstellbar.

Die Verbindungen der allgemeinen Formeln (VI) und (XV) sind teilweise bekannt oder neu und können dann wie oben beschrieben hergestellt werden.

Die Verbindungen der allgemeinen Formeln (IX) und (X) sind als Species neu und können wie oben beschrieben hergestellt werden.

Die Verbindungen der allgemeinen Formel (VIII) sind neu und können hergestellt werden, indem man

Verbindungen der allgemeinen Formeln (XVa), (XVIII) und (XIX)

A-CHO (XVa),

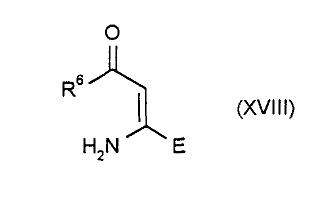

und 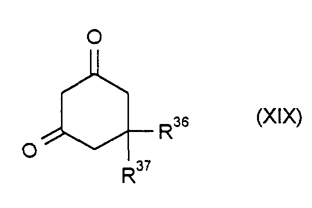 in welcher
- A, E, R⁶, R³⁶ und R³⁷: die oben angegebene Bedeutung haben,
mit einer Säure umsetzt.

Als Lösemittel zur Herstellung der Verbindungen der allgemeinen Formel (VIII) eignen sich die oben aufgeführten Ether oder Alkohole. Bevorzugt ist Diisopropylether.

Als Säuren für die Herstellung der Verbindungen der allgemeinen Formel (VIII) eignen sich im allgemeinen organische Carbonsäuren und anorganische Säuren, wie beispielsweise Oxalsäure, Maleinsäure, Phosphorsäure, Fumarsäure und Trifluoressigsäure. Bevorzugt ist Trifluoressigsäure.

Die Säure wird im allgemeinen in einer Menge von 0,1 mol bis 5 mol, bevorzugt 1 mol, bezogen auf 1 mol der Verbindungen der allgemeinen Formel (XIX) eingesetzt.

Die Reaktion wird im allgemeinen bei Normaldruck durchgeführt. Es ist aber auch möglich die Reaktion bei erhöhtem oder erniedrigtem Druck durchzuführen.

Die Reaktion erfolgt im allgemeinen bei der Rückflußtemperatur des jeweiligen Lösemittels.

Die Verbindungen der allgemeinen Formeln (XV) und (XIX) sind an sich bekannt oder nach üblichen Methoden herstellbar.

Die Verbindungen der allgemeinen Formel (XVIII) sind neu und können hergestellt werden, indem man zunächst durch Umsetzung der Verbindungen der allgemeinen Formel (XX)

E-CO₂-R⁴⁵ (XX)

in welcher
- E: die oben angegebene Bedeutung hat
und
- R⁴⁵: für C₁-C₄-Alkyl steht,
mit Verbindungen der allgemeinen Formel (XXI) 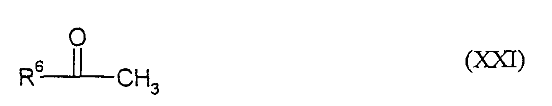 in welcher
- R⁶: die oben angegebene Bedeutung hat,
in einem Lösemittel in Anwesenheit von 18-Krone-6-ether die Verbindungen der allgemeinen Formel (XXII) 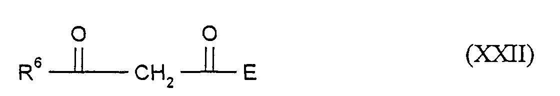 in welcher
- R⁶ und E: die oben angegebene Bedeutung haben,
herstellt
und anschließend mit Ammoniumacetat in inerten Lösemitteln umsetzt.

Als Lösemittel für den ersten Schritt des Verfahrens eignen sich die oben aufgeführten Ether und Kohlenwasserstoffe, wobei Tetrahydrofuran bevorzugt ist.

Als Lösemittel für die Umsetzung mit den Verbindungen der allgemeinen Formel (XXII) eignen sich Alkohole, wie beispielsweise Methanol, Ethanol, Propanol oder Isopropanol. Bevorzugt ist Ethanol.

Alle Schritte des Verfahrens erfolgen bei der jeweiligen Rückflußtemperatur des entsprechenden Lösemittels und bei Normaldruck.

Die Verbindungen der allgemeinen Formeln (XX) und (XXI) sind teilweise bekannt oder können nach bekannten Methoden hergestellt werden.

Die Verbindungen der allgemeinen Formel (XXII) sind als Species partiell neu und können wie oben beschrieben hergestellt werden.

Die erfindungsgemäßen Verbindungen der allgemeinen Formeln (I) und (Ia) haben ein nicht vorhersehbares pharmakologisches Wirkspektrum.

Die erfindungsgemäßen Verbindungen der allgemeinen Formeln (I) und (Ia) besitzen wertvolle, im Vergleich zum Stand der Technik überlegene, pharmakologische Eigenschaften, insbesondere sind sie hochwirksame Inhibitoren des Cholesterin-Ester-Transfer-Proteins (CETP) und stimulieren den Reversen Cholesterintransport. Die erfindungsgemäßen Wirkstoffe bewirken eine Senkung des LDL-Cholesterinspiegels im Blut bei gleichzeitiger Erhöhung des HDL-Cholesterinspiegels. Sie können deshalb zur Behandlung und Prävention von Hypolipoproteinämie, Dyslipidämien, Hypertriglyceridämien, Hyperlipidämien oder Arteriosklerose eingesetzt werden.

Die pharmakologische Wirkung der erfindungsgemäßen Stoffe wurde in folgendem Test bestimmt:

### CETP-Inhibitions-Testung

### Gewinnung von CETP

CETP wird aus humanem Plasma durch Differential-Zentrifugation und Säulenchromatographie in partiell gereinigter Form gewonnen und zum Test verwendet. Dazu wird humanes Plasma mit NaBr auf eine Dichte von 1,21 g pro ml eingestellt und 18 h bei 50.000 Upm bei 4°C zentrifugiert. Die Bodenfraktion (d>1,21 g/ml) wird auf eine Sephadex®Phenyl-Sepharose 4B (Fa. Pharmacia) Säule aufgetragen, mit 0,15 m NaCl/0,001 m TrisHCl pH 7,4 gewaschen und anschließend mit dest. Wasser eluiert. Die CETP-aktiven Fraktionen werden gepoolt, gegen 50mM NaAcetat pH 4,5 dialysiert und auf eine CM-Sepharose® (Fa. Pharmacia)-Säule aufgetragen. Mit einem linearen Gradienten (0-1 M NaCl) wird anschließend eluiert. Die gepoolten CETP-Fraktionen werden gegen 10 mM TrisHCl pH 7,4 dialysiert und anschließend durch Chromatographie über eine Mono Q®-Säule (Fa. Pharmacia) weiter gereinigt.

### Gewinnung von radioaktiv markiertem HDL

50 ml frisches humanes EDTA-Plasma wird mit NaBr auf eine Dichte von 1,12 eingestellt und bei 4°C im Ty 65-Rotor 18 h bei 50.000 Upm zentrifugiert. Die Oberphase wird zur Gewinnung von kaltem LDL verwendet. Die Unterphase wird gegen 3*4 l PDB-Puffer (10 mM Tris/HCl pH 7,4, 0,15 mM NaCl, 1 mM EDTA, 0,02% NaN₃) dialysiert. Pro 10 ml Retentatvolumen wird anschließend 20 µl 3H-Cholesterin (Dupont NET-725; 1 -µC/µl gelöst in Ethanol !) hinzugesetzt und 72 h bei 37°C unter N₂ inkubiert.

Der Ansatz wird dann mit NaBr auf die Dichte 1,21 eingestellt und im Ty 65-Rotor 18 h bei 50.000 Upm bei 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1,26 eingestellt. Je 4 ml dieser Lösung werden in Zentrifugenröhrchen (SW 40-Rotor) mit 4 ml einer Lösung der Dichte 1,21 sowie 4,5 ml einer Lösung von 1,063 überschichtet (Dichtelösungen aus PDB-Puffer und NaBr) und anschließend 24 h bei 38.000 Upm und 20°C im SW 40-Rotor zentrifugiert. Die zwischen der Dichte 1,063 und 1,21 liegende, das markierte HDL enthaltende Zwischenschicht wird gegen 3*100 Volumen PDB-Puffer bei 4°C dialysiert.
Das Retentat enthält radioaktiv markiertes ³H-CE-HDL, das auf ca. 5x10⁶ cmp pro ml eingestellt zum Test verwendet wird.

### CETP-Test

Zur Testung der CETP-Aktivität wird die Übertragung von ³H-Cholesterolester von humanen HD-Lipoproteinen auf biotinylierte LD-Lipoproteine gemessen.
Die Reaktion wird durch Zugabe von Streptavidin-SPA®beads (Fa. Amersham) beendet und die übertragene Radioaktivität direkt im Liquid Scintillation Counter bestimmt.
Im Testansatz werden 10 µl HDL-³H-Cholesterolester (∼ 50.000 cpm) mit 10 µl Biotin-LDL (Fa. Amersham) in 50 mM Hepes / 0,15 m NaCl / 0,1% Rinderserumalbumin / 0,05% NaN₃ pH 7,4 mit 10 µl CETP (1 mg/ml) und 3 µl Lösung der zu prüfenden Substanz (in 10% DMSO / 1% RSA gelöst), für 18 h bei 37°C inkubiert. Anschließend werden 200 µl der SPA-Streptavidin-Bead-Lösung (TRKQ 7005) zugesetzt, 1 h unter Schütteln weiter inkubiert und anschließend im Scintillationszähler gemessen. Als Kontrollen dienen entsprechende Inkubationen mit 10 µl Puffer, 10 µl CETP bei 4°C sowie 10 µl CETP bei 37°C.
Die in den Kontrollansätzen mit CETP bei 37°C übertragene Aktivität wird als 100% Übertragung gewertet. Die Substanzkonzentration, bei der diese Übertragung auf die Hälfte reduziert ist, wird als IC₅₀-Wert angegeben.

In der folgenden Tabelle A sind die IC₅₀-Werte (mol/l) für CETP-Inhibitoren angegeben:

**Tabelle A**

| **Beispiel-Nr.** | **IC**_{**50**}**-Wert (mol/l)** |
|---|---|
| 76 | 6 x 10⁻⁹ |
| 90 | 6 x 10⁻⁸ |
| 195 | 7,5 x 10⁻⁷ |
| 237 | 8 x 10⁻⁷ |
| 244 | 6 x 10⁻⁸ |

### Ex vivo Aktivität der erfindungsgemäßen Verbindungen

Syrische Goldhamster aus werkseigener Zucht werden nach 24-stündigem Fasten narkotisiert (0,8 mg/kg Atropin, 0,8 mg/kg Ketavet® s.c., 30' später 50 mg/kg Nembutal i.p.). Anschließend wird die V.jugularis freipräpariert und kanüliert. Die Testsubstanz wird in einem geeigneten Lösemittel (in der Regel Adalat-Placebolösung: 60 g Glycerin, 100 ml H₂O, ad 1000 ml PEG-400) gelöst und den Tieren über einen in die V.jugularis eingeführten PE-Katheter verabreicht. Die Kontrolltiere erhalten das gleiche Volumen Lösungsmittel ohne Testsubstanz. Anschließend wird die Vene abgebunden und die Wunde verschlossen.
Die Verabreichung der Testsubstanzen kann auch p.o. erfolgen, indem die Substanzen in DMSO gelöst und 0,5% Tylose suspendiert mittels Schlundsonde peroral verabreicht werden. Die Kontrolltiere erhalten identische Volumen Lösemittel ohne Testsubstanz.
Nach verschiedenen Zeitpunkten - bis zu 24 Stunden nach Applikation - wird den Tieren durch Punktion des retro-orbitalen Venenplexus Blut entnommen (ca. 250 µl). Durch Inkubation bei 4°C über Nacht wird die Gerinnung abgeschlossen, anschließend wird 10 Minuten bei 6000 x g zentrifugiert. Im so erhaltenen Serum wird die CETP-Aktivität durch den modifizierten CETP-Test bestimmt. Es wird wie für den CETP-Test oben beschrieben die Übertragung von ³H-Cholesterolester von HD-Lipoproteinen auf biotinylierte LD-Lipoproteine gemessen.
Die Reaktion wird durch Zugabe von Streptavidin-SPA^{R}beads (Fa. Amersham) beendet und die übertragene Radioaktivität direkt im Liquid Scintlation Counter bestimmt.
Der Testansatz wird wie unter "CETP-Test" beschrieben durchgeführt. Lediglich 10 µl CETP werden für die Testung der Serum durch 10 µl der entsprechenden Serumproben ersetzt. Als Kontrollen dienen entsprechende Inkubationen mit Seren von unbehandelten Tieren.
Die in den Kontrollansätzen mit Kontrollseren übertragene Aktivität wird als 100% Übertragung gewertet. Die Substanzkonzentration, bei der diese Übertragung auf die Hälfte reduziert ist wird als ED₅₀-Wert angegeben.

**Tabelle B:**

| ED₅₀-Werte für ex vivo Aktivität | | |
|---|---|---|
| Bsp.-Nr. | ED₅₀ | % Hemmung bei 30 mg/kg |
| 115 | <30mg/kg | 61,9 |
| 117 | <30mg/kg | 86,0 |
| 170 | <30mg/kg | 60,1 |
| 181 | >30mg/kg | 46,4 |
| 184 | <30mg/kg | 53,5 |

### In vivo Aktivität der erfindungsgemäßen Verbindungen

Bei Versuchen zur Bestimmung der oralen Wirkung auf Lipoproteine und Triglyceride wird syrischen Goldhamstern aus werkseigener Zucht Testsubstanz in DMSO gelöst und 0,5% Tylose suspendiert mittels Schlundsonde peroral verabreicht. Zur Bestimmung der CETP-Aktivität wird vor Versuchsbeginn durch retro-orbitale Punktion Blut entnommen (ca. 250 µl). Anschließend werden die Testsubstanzen peroral mittels einer Schlundsonde verabreicht. Die Kontrolltiere erhalten identische Volumen Lösemittel ohne Testsubstanz. Anschließend wird den Tieren das Futter entzogen und zu verschiedenen Zeitpunkten - bis zu 24 Stunden nach Substanzapplikation - durch Punktion des retroorbitalen Venenplexus Blut entnommen. Durch Inkubation von 4°C über Nacht wird die Gerinnung abgeschlossen, anschließend wird 10 Minunten bei 6000 x g zentrifugiert. Im so erhaltenen Serum wird der Gehalt an Cholesterin und Triglyceriden mit Hilfe modifizierter kommerziell erhältlicher Enzymtests bestimmt (Cholesterin enzymatisch 14366 Merck, Triglyceride 14364 Merck). Serum wird in geeigneter Weise mit physiologischer Kochsalzlösung verdünnt.
100 µl Serum-Verdünnung werden mit 100 µl Testsubstanz in 96-Lochplatten versetzt und 10 Minuten bei Raumtemperatur inkubiert. Anschließend wird die optische Dichte bei einer Wellenlänge von 492 nm mit einem automatischen Platten-Lesegerät bestimmt. Die in den Proben enthaltene Triglycerid- bzw. Cholesterinkonzentration wird mit Hilfe einer parallel gemessenen Standardkurve bestimmt.
Die Bestimmung des Gehaltes von HDL-Cholesterin wird nach Präzipitation der ApoB-haltigen Lipoproteine mittels eines Reagenziengemisch (Sigma 352-4 HDL Cholesterol Reagenz) nach Herstellerangaben durchgeführt

**Tabelle C:**

| HDL-Anstieg bei in vivo-Versuchen | | |
|---|---|---|
| Bsp.-Nr. | Dosis [mg/kg] | % HDL-Anstieg |
| 76 | 2 x 3 | + 15,81 |
| 91 | 2 x 3 | + 12,58 |
| 209 | 2 x 3 | + 25,94 |
| 211 | 2 x 3 | + 7,54 |
| 237 | 2 x 3 | + 21,03 |

### In vivo Wirksamkeit an transgenen hCETP-Mäusen

Transgenen Mäusen aus eigener Zucht (Dinchuck, Hart, Gonzalez, Karmann, Schmidt, Wirak; BBA (1995), 1295, 301) wurden die zu prüfenden Substanzen im Futter verabreicht. Vor Versuchsbeginn wurde den Mäusen retroorbital Blut entnommen, um Cholesterin und Triglyceride im Serum zu bestimmen. Das Serum wurde wie oben für Hamster beschrieben durch Inkubation bei 4°C über Nacht und anschließender Zentrifugation bei 6000 x g gewonnen. Nach einer Woche wurde den Mäusen wieder Blut entnommen, um Lipoproteine und Triglyceride zu bestimmen. Die Veränderung der gemessenen Parameter werden als prozentuale Veränderung gegenüber dem Ausgangswert ausgedrückt.

**Tabelle D**

| Bsp.-Nr. | HDL | LDL | Triglyceride |
|---|---|---|---|
| 76 (400 ppm) | + 31,25 % | - 15,3 % | - 11,7 % |

Die Erfindung betrifft außerdem die Kombination von Cycloalkano-Pyridinen der allgemeinen Formeln (I) und (Ia) mit einem Glucosidase- und/oder Amylasehemmer zur Behandlung von familiärer Hyperlipidaemien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formeln (I) und (Ia).

Weiterhin können die erfindungsgemäßen Verbindungen in Kombination mit Cholesterin senkenden Vastatinen oder ApoB-senkenden Prinzipien kombiniert werden, um Dyslipidemien, kombinierte Hyperlipidemien, Hypercholesterolemien oder Hypertriglyceridemien zu behandeln.

Die genannten Kombinationen sind auch zur primären oder sekundären Prävention koronarer Herzerkrankungen (z.B. Myokardinfarkt) einsetzbar.

Vastatine im Rahmen der Erfindung sind beispielsweise Lovastatin, Simvastatin, Pravastatin, Fluvastatin, Atorvastatin und Cerivastatin. ApoB senkende Mittel sind zum Beispiel MTP-Inhibitoren.

Bevorzugt ist die Kombination von Cerivastatin oder ApoB-Inhibitoren mit einer der oben aufgeführten erfindungsgemäßen Verbindungen der allgemeinen Formeln (I) und (Ia).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise intravenös, oral, parenteral oder perlingual, insbesondere oral.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Verwendete Abkürzungen:

- Cy =: Cyclohexan
- EE =: Essigester
- PE =: Petrolether
- THF =: Tetrahydrofuran
- DAST =: Dimethylaminoschwefeltrifluorid
- PTS =: para-Toluolsulfonsäure
- PDC =: Pyridiniumdichromat
- PE/EE =: Petrolether / Essigsäureethylester
- Tol =: Toluol

### Ausgangsverbindungen

### Beispiel I:

### 4-(4-Fluorphenyl)-2-isopropyl-5-oxo- 1,4,5,6,7,8-hexahydrochinolin-3-carbonsäuremethylester

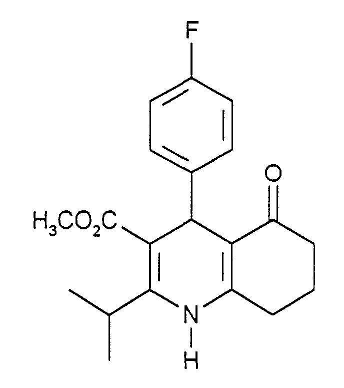

50,14 g (0,404 mol) p-Fluorbenzaldehyd, 45,3 g (0,404 mol) 1,3-Cyclohexandion und 57,89 g (0,404 mol) 3-Amino-4-methyl-pent-2-en-säure-methylester werden in 1000 ml Ethanol 60 Stunden unter Rückfluß gekocht. Die Mischung wird auf Raumtemperatur abgekühlt und bis zur Trockene eingeengt. Der Rückstand wird in 500 ml Toluol heiß gelöst, unter Abkühlen mit 1 l Petrolether versetzt und das auskristallisierende Produkt abgesaugt.
Ausbeute: 100,8 g (72,6 % d. Th.)
R_{f}=0,15 (Toluol/EE 8:1)

### Beispiel II:

### 4-(4-Fluorphenyl)-2-isopropyl-5-oxo-5,6,7,8-tetrahydrochinolin-3-carbonsäuremethylester

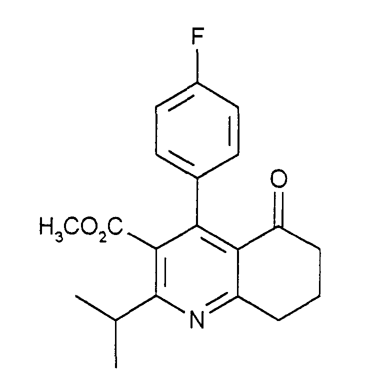

Zu einer Lösung von 46,04 g (0,1341 mol) der Verbindung aus Beispiel I in 645 g Dichlormethan p.a. gibt man 30,44 g (0,1341 mol) 2,3-Dichlor-5,6-dicyan-pbenzochinon (DDQ) und rührt über Nacht bei Raumtemperatur. Danach wird über 500 ml Kieselgel 60 abgesaugt, mit 700 ml Dichlormethan nachgewaschen, die vereinigten Filtrate bis zur Trockene eingeengt.
Ausbeute: 24,2 g (52,87 % d. Th.)
R_{f}=0,54 (Toluol/EE 8:2)

### Beispiel III:

### 4-(4-Fluorphenyl)-2-isopropyl-5-hydroxy-5,6,7,8-tetrahydrochinolin-3-carbonsäuremethylester

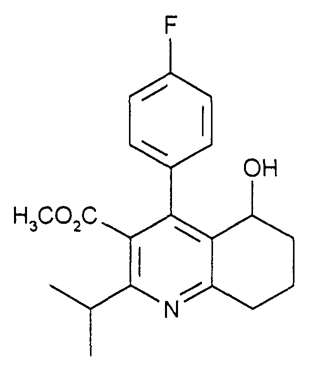

70,64 g (0,207 mol) der Verbindung aus Beispiel II werden unter Argon in 706 g Toluol p.a. gelöst, auf -78°C gekühlt und 228 ml (0,228 mol; 1,1 eq.) Diisobutylaluminiumhydrid (DIBAL-H; 1,0 molar in Toluol) innerhalb von 20 min zugetropft. Nach 5 min Rühren bei -78°C werden nochmals 35 ml (0,15 eq.) DIBAL-H zugegeben und 10 min gerührt. Danach läßt man bei -78°C 500 ml 20 %ige Kaliumnatriumtartratlösung zutropfen, wobei die Temperatur langsam auf 20°C ansteigt. Nach 1 Stunde Nachrührzeit wird die wässrige Phase abgetrennt, zweimal mit Essigsäureethylester extrahiert, die vereinigten organischen Phasen über Natriumsulfat getrocknet, filtriert und eingeengt. Der Rückstand wird durch Chromatographie an 1000 ml Kieselgel 60 mit Toluol, Toluol-EE-Gemischen (9:1, 8:2) gereinigt. Die Fraktionen mit der gewünschten Verbindung werden gesammelt, bis auf 100 ml eingeengt und mit Petrolether versetzt. Das ausgefallene Kristallisat wird abgesaugt und im Hochvakuum über Nacht getrocknet.
Ausbeute: 1. Fraktion: 61,69 g (86,8 % d. Th.)
Ausbeute: 2. Fraktion: 6,34 g (8,9 % d. Th.)
R_{f}= 0,14 (Toluol/EE 9:1)

### Beispiel IV:

### 5-(tert.Butyldimethylsilyloxy)-4-(4-fluorphenyl)-2-isopropyl-5,6,7,8-tetrahydrochinolin-3-carbonsäure-methylester

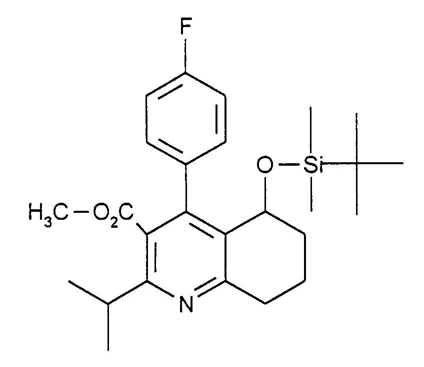

68,0 g (0,198 mol) der Verbindung aus Beispiel III werden in 340 g DMF p.a. gelöst und nacheinander mit 59,69 g (0,396 mol; 2 eq.) tert. Butyldimethylsilylchlorid, 48,54 g (0,713 mol; 3,6 eq.) Imidazol und 0,484 g (0,00396 mol, 0,02 eq.) N-Dimethylaminopyridin versetzt. Es wird über Nacht bei Raumtemperatur gerührt, in 800 ml Ammoniumchloridlösung und 400 ml Essigsäureethylester verteilt und mit 6 molarer Salzsäure ein pH-Wert von 5 bis 6 eingestellt. Die organische Phase wird abgetrennt, die wässrige Phase noch zweimal mit Essigsäureethylester extrahiert, die vereinigten organischen Phasen über Natriumsulfat getrocknet, filtriert und eingeengt. Der Rückstand - gelöst in Toluol - wird auf 1800 ml Kieselgel aufgetragen, anfangs mit Toluol und später mit Toluol/EE (9:1) eluiert. Nach Einengen der Eluate erhält man ein weißes, kristallines Produkt.
Ausbeute: 87,5 g (96,7 % d. Th.)
R_{f}=0,68 (Toluol/EE 9:1)

### Beispiel V:

### 5-(tert.Butyldimethylsilyloxy)-4-(4-fluorphenyl)-3-hydroxymethyl-2-isopropyl-5,6,7,8-tetrahydrochinolin

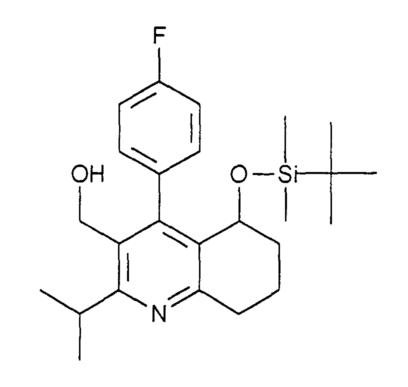

87,4 g (0,191 mol) der Verbindung aus Beispiel IV werden in 500 g Toluol p.a. gelöst und unter Argon auf -78°C gekühlt. 690 ml (0,690 mol; 3,61 eq.) DIBAL-H (1,0 molar in Toluol) werden in 1 Stunde zugetropft und danach noch 1,5 Stunden bei -78°C gerührt. Zu der auf -78°C gekühlten Lösung gibt man vorsichtig 30 ml Kaliumnatriumtartratlösung hinzu und rührt 30 min bei -78°C. Danach läßt man den Ansatz auf Raumtemperatur ansteigen, schüttet 400 ml Kaliumnatriumtartratlösung hinzu und verdünnt mit Essigsäureethylester. Zum Schluß werden noch 1,2 l Kaliumnatriumtartratlösung zugegeben, wobei allmählich zwei nahezu klare Lösungen entstehen. Die organische Phase wird abgetrennt, die wässrige noch zweimal mit Essigsäureethylester extrahiert. Die vereinigten organischen Phasen werden mit Natriumsulfat getrocknet, filtriert und eingeengt. Der halbfeste Rückstand wird in 400 ml Toluol gelöst, auf 1100 ml Kieselgel 60, das zuvor mit Toluol konditioniert wurde, aufgetragen und nacheinander mit Toluol, Toluol/EE (9:1) eluiert. Die Fraktionen, die die gewünschte Verbindung enthalten, werden eingeengt, das zurückgewonnene Öl mit Petrolether versetzt, wobei ein Kristallbrei ausfällt.
Ausbeute: 75,52 g (92,0 % d. Th.)
R_{f}=0,28 (Toluol/EE 9:1)

### Beispiel VI:

### 5-(tert.Butyldimethylsilyloxy)-4-(4-fluorphenyl)-2-isopropyl-5,6,7,8-tetrahydrochinolin-3-carbaldehyd

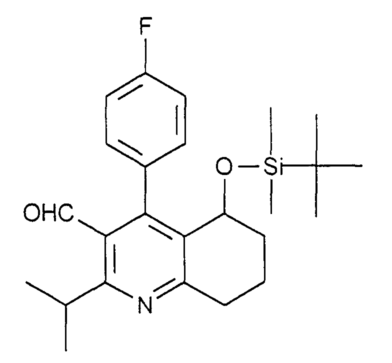

Zu einer Lösung von 67,1 g (0,156 mol) der Verbindung aus Beispiel V in 671 g Dichlormethan gibt man 31,8 g (0,312 mol; 2 eq.) neutrales Aluminiumoxid und 67,3 g (0,312 mol; 2 eq.) Pyridiniumchlorochromat (PCC) und rührt 1,5 Stunden bei Raumtemperatur. Die Reaktionslösung wird auf 1100 ml Kieselgel 60 (trocken) aufgetragen und danach zunächst mit Toluol und später mit Essigsäureethylester/Methanol (9:1) eluiert. Die Fraktionen, die die gewünschte Verbindung enthalten, werden eingeengt, das ausgefallene Material abgesaugt und mit wenig Toluol gewaschen. Das gewonnene Material wird anschließend in 100 ml Toluol gelöst, auf 250 ml Kieselgel 60 aufgetragen und mit Toluol, Toluol/EE (9:1) eluiert. Die Eluate werden eingeengt, das resultierende Öl mit Petrolether kristallisiert.
Ausbeute: 1. Fraktion: 28,8 g (43,1 % d.Th.)
Ausbeute: 2. Fraktion: 10,05 g (15,1 % d. Th.)
R_{f}= 0,72 (Toluol/EE 9:1)

### Beispiel VII

### 5-(tert.Butyldimethylsilyloxy)-4-(4-fluorphenyl)-3-[hydroxy-(4-trifluormethylphenyl)-methyl]-2-isopropyl-5,6,7,8-tetrahydrochinolin (Dia A/Dia B-Gemisch)

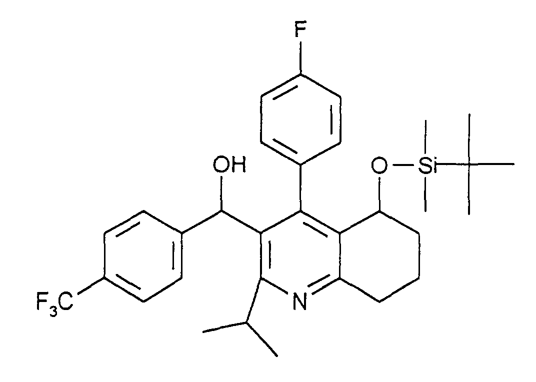

341 mg (14,03 mmol; 6 eq.) Magnesiumspäne werden in 30 ml THF p.a. vorgelegt, unter Argon zum Rückfluß erhitzt und 1,052 g (4,676 mmol; 2 eq.) 4-Brombenzotrifluorid mittels Spritze pur zugetropft. Es wird 45 min unter Rückfluß gekocht, danach läßt man auf Raumtemperatur abkühlen (Grignard-Reagenz). 1,0 g (2,338 mmol) der Verbindung aus Beispiel VI werden in 20 ml THF p.a. gelöst, unter Argon auf ca -78°C gekühlt und dann das Grignard-Reagenz unter Rühren zugegeben. Das Kältebad wird entfernt und der Ansatz 1 Stunde gerührt. Die Reaktionslösung wird in 200 ml konz. Ammoniumchloridlösung und 250 ml Essigsäureethylester unter Rühren verteilt, die organische Phase abgetrennt, die wässrige noch zweimal mit Essigsäureethylester extrahiert, die verenigten organischen Phasen mit Natriumsulfat getrocknet, filtriert, eingeengt und im Hochvakuum über Nacht getrocknet.
Ausbeute: 1,18 g (97,5 % d. Th.; Dia A/Dia B-Gemisch)
Die Trennung der beiden Diastereomerenpaare (Dia A und Dia B) erfolgt durch Chromatographie an 100 ml Kieselgel 60, konditioniert mit Cyclohexan. Das Diastereomerengemisch - gelöst in 4 ml Cyclohexan - wird an Kieselgel 60 zunächst mit Cyclohexan und anschließend mit Cyclohexan/THF (9:1) eluiert. Man erhält nach Einengen der Fraktionen die 2 Diastereomerenpaare.
Ausbeute: Dia A: 789 mg (65,2 % d. Th.)
R_{f}=0,42 (Cy/THF 9:1)
Ausbeute: Dia B: 410 mg (33,9 % d. Th.)
R_{f} = 0,24 (Cy/THF 9:1)

### Beispiel VIII

### 5-(tert.Butyldimethylsilyloxy)-4-(4-fluorphenyl)-3-[fluor-(4-trifluormethyl-phenyl)-methyl]-2-isopropyl-5,6,7,8-tetrahydrochinolin (Dia A/Dia B-Gemisch)

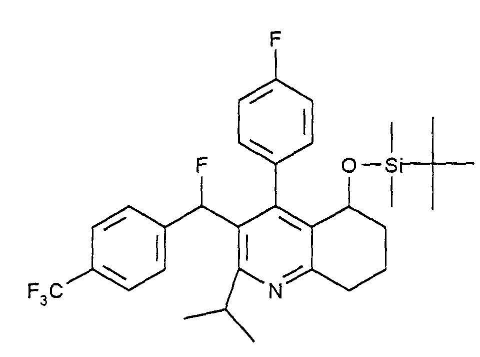

Zu einer Lösung von 876 mg (1,521 mmol) des Diastereomerengemisches A/B aus Beispiel VII in 70 ml Dichlormethan p.a. werden bei -78° C unter Argon 0,302 ml Diethylaminoschwefeltrifluorid (DAST) mittels Spritze zugegeben, danach das Kältebad weggenommen und 30 min nachgerührt. Danach wird die Reaktionslösung in Essigsäureethylester/Ammoniumchlorid-Lösung eingerührt, die organische Phase abgetrennt, die wässrige noch dreimal mit Essigsäureethylester extrahiert, das gesamte organische Extrakt mit Natriumsulfat getrocknet, filtriert, eingeengt und im Hochvakuum getrocknet.
Ausbeute: 690 mg (78,5 % d. Th.)
R_{f}=0,57 (Toluol/EE 9:1)

### Beispiel IX

### 5-(tert.Butyldimethylsilyloxy)-4-(4-fluorphenyl)-3-[fluor-(4-trifluormethyl-phenyl)-methyl]-2-isopropyl-5,6,7,8-tetrahydrochinolin (Dia A)

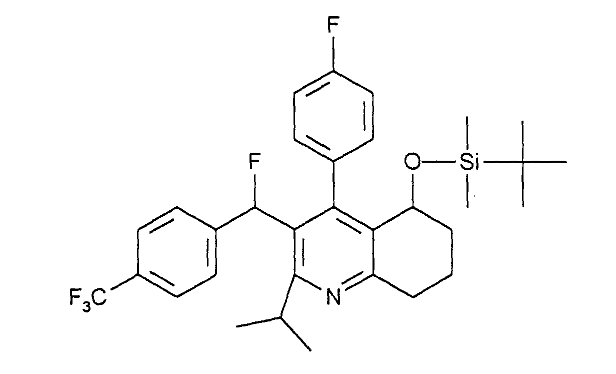

Analog Beispiel VIII werden 250 mg (0,436 mmol) der Verbindung Dia A aus Beispiel VII in 10 ml Dichlormethan p.a. mit 0,086 ml (0,654 mmol; 1,5 eq.)
DAST bei -78°C umgesetzt.
Ausbeute: 233 mg (92,8 % d. Th.)
R_{f}= 0,76 (Cy/THF 9:1)

### Beispiel X

### 5-(tert.Butyldimethylsilyloxy)-4-(4-fluorphenyl)-3-[fluor-(4-trifluormethyl-phenyl)-methyl]-2-isopropyl-5,6,7,8-tetrahydrochinolin (Dia B)

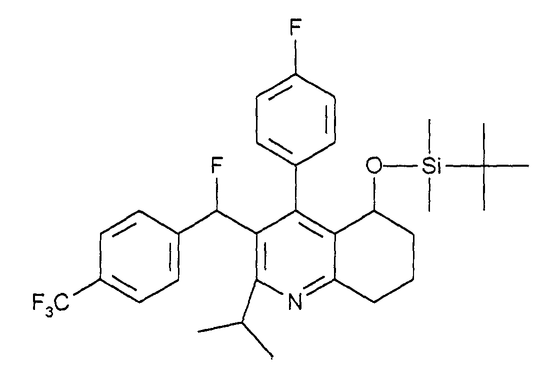

Analog Beispiel VIII werden 250 mg (0,436 mmol) der Verbindung Dia B aus Beispiel VII in 10 ml Dichlormethan p.a. mit 0,086 ml (0,654 mmol; 1,5 eq.)
DAST bei -78°C umgesetzt.
Ausbeute: 246 mg (98,4 % d. Th.)
R_{f} = 0,76 (Cy/THF 9:1)

### Beispiel XI

### 8-Brom-5-(tert.butyldimethylsilyloxy)-4-(4-fluorphenyl)-3-[fluor-(4-trifluormethylphenyl)-methyl]-2-isopropyl-5,6,7,8-tetrahydrochinolin

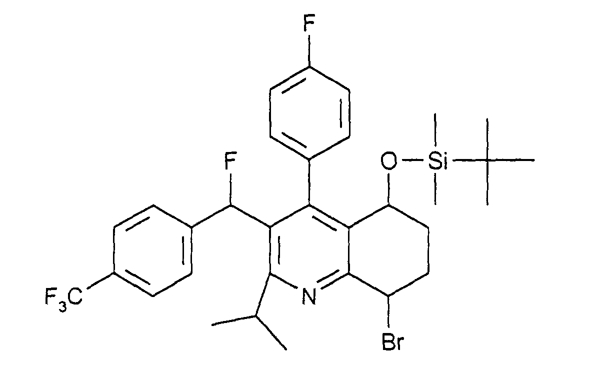

Unter Argon werden zu einer Lösung von 18,7 g (32,5 mmol) der Verbindung aus Beispiel X in 500 ml Tetrachlorkohlenstoff 9,3 g (52,0 mmol) N-Bromsuccimid und 500 mg Azoisobuttersäurenitril gegeben. Es wird auf Rückfluß erhitzt, wobei nach 10 min heftige Reaktion eintritt. Nach 2,5 Stunden wird auf Raumtemperatur abgekühlt, abgesaugt und eingeengt. Das Rohprodukt wird an Kieselgel 60 mit Cy/EE 15:1 eluiert, die Fraktionen eingeengt und im Hochvakuum getrocknet.
Ausbeute: 9,9 g (47 % d. Th.)
R_{f} = 0,58 (Cy/EE 9:1)

### Beispiel XII

### 8-Butyl-5-(tert.butyldimethylsilyloxy)-4-(4-fluorphenyl)-3-[fluor-(4-trifluormethylphenyl)-methyl]-2-isopropyl-5,6,7,8-tetrahydrochinolin

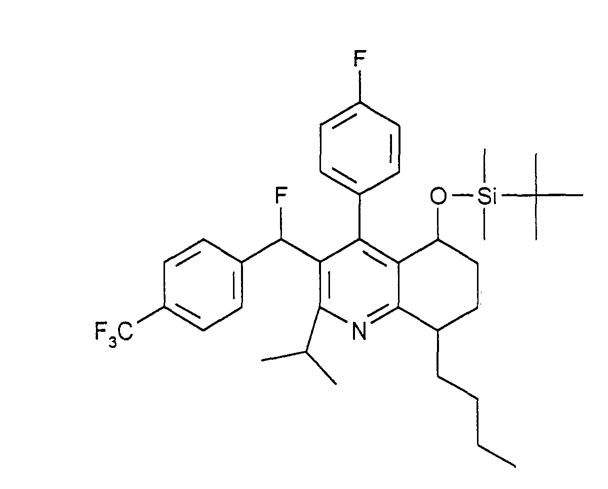

Unter Argon werden 179 mg Kupfer-(I)-cyanid (2 mmol) in 3 ml absolutem Toluol suspendiert und unter Vakuum abgezogen. Danach wird in 2,6 ml absolutem THF suspendiert und auf -65°C gekühlt. Bei dieser Temperatur werden 2,5 ml 1,6 molare n-Butyllithiumlösung (4 mmol) zugetropft und unter zwischenzeitlichem Ansteigen der Temperatur auf -30°C 1 Stunde nachgerührt. Bei -65°C wird zu dieser Lösung eine Lösung von 654 mg (1 mmol) der Verbindung aus Beispiel XI in 2 ml absolutem THF getropft und 1 Stunde gerührt. Zur Aufarbeitung wird mit einer Mischung aus 4,5 ml gesättigter Ammoniumchloridlösung und 0,5 ml konz. Ammoniaklösung versetzt, mit 30 ml Wasser verdünnt und dreimal mit je 15 ml Dieethylether extrahiert. Die vereinigten organischen Phasen werden über Natriumsulfat getrocknet, filtriert und eingeengt. Das Rohprodukt wird an Kieselgel (0,04 - 0,0063 mm) mit Cy:EE 98:2 eluiert.
Ausbeute: 200 mg (32% d.Th.)
R_{f} : 0,33 (Cy:EE 98:2)

### Beispiel XIII

### 4-(4-Fluorphenyl)-3-[fluor-(4-trifluormethyl-phenyl)-methyl]-2-isopropyl-5,6-dihydro-chinolin-5-ol

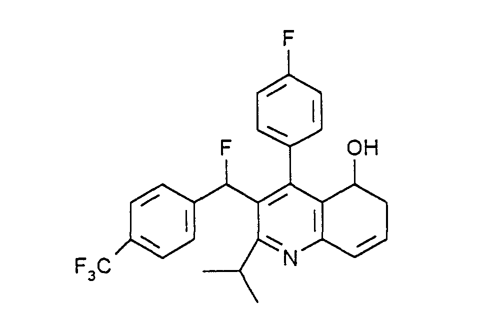

Zu einer Lösung von 1,3 g (2 mmol) 8-Brom-5-(tert.butylsilyloxy)-4-(4-fluorphenyl)-3-[fluor-(4-trifluormethyl-phenyl)-methyl]-2-isopropyl-5,6,7,8-tetrahydrochinolin in 13 ml THF werden bei Raumtemperatur 10,9 ml 1,1 molare Tetrabutylammoniumfluoridlösung getropft. Nach 1 h wird eine Mischung aus 100 ml Wasser und 50 ml Toluol eingerührt. Die Phasen werden getrennt und die wäßrige Phase mit Toluol nachextrahiert. Die vereinigten organischen Phasen werden mit Wasser neutral gewaschen, über Natriumsulfat getrocknet und eingeengt. Das Rohprodukt wird an Kieselgel (0,04-0,063 mm) mit Cy/EE 8:2 eluiert, die Fraktion eingeengt und das Produkt kristallisiert.
Ausbeute: 640 mg (70 % d. Th.)
R_{f}=0,17 (Cy/EE 8:2)

### Beispiel XIV

### 1-Cyclopentyl-3-(4-trifluormethylphenyl)-propan-1,3-dion

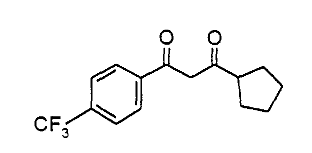

1,97 kg Kalium-tert.-butylat, 2,26 kg Cyclopentancarbonsäuremethylester, 1,66 kg p-Trifluormethylacetophenon und 36 g 18-Krone-6-ether wurden in 18 ltr. Tetrahydrofuran 4 Stunden am Rückfluß gekocht. Quenchen der Reaktion erfolgte mit 16 ltr. 10%iger Salzsäure bei Raumtemperatur. Die wäßrige Phase wurde mit Essigester extrahiert und die vereinigten organischen Phasen mit gesättigter Kochsalzlösung gewaschen. Nach Abdestillieren des Lösungsmittels wurde im Ölpumpenvakuum bei 1,5 mbar destilliert. Es resultierten 1,664 kg 1-Cyclopentyl-3-(4-trifluormethylphenyl)-propan-1,3-dion als Öl, das beim Stehenlassen durchkristallisierte.

Siedepunkt: 138-145 °C/1,5 mbar.

### Beispiel XV

### 3-Amino-3-cyclopentyl-1-(4-trifluormethylphenyl)-propenon

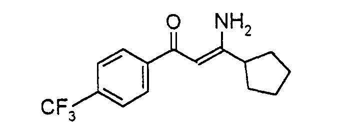

1622,6 g 1-Cyclopentyl-3-(4-trifluormethylphenyl)-propan-1,3-dion und 730 g Ammoniumacetat wurden über Nacht in 4,9 ltr. Ethanol am Rückfluß gekocht. Das Ethanol wurde im Vakuum abgezogen und der Rückstand in 4 ltr. Methylenchlorid aufgenommen. Die Lösung wurde einmal mit Wasser und zweimal mit gesättigter Natriumhydrogencarbonatlösung gewaschen und mit Natriumsulfat getrocknet. Nach dem Abdestillieren des Methylenchlorids wurde der Rückstand aus 6 ltr. heißem Cyclohexan umkristallisiert. Nach dem Trocknen resultierten 1018 g farblose Kristalle mit einer Reinheit von 98.6% nach HPLC.

Schmelzpunkt: 106 °C.
DC: R_{f} = 0,2 (Toluol/Essigester 4:1)

### Beispiel XVI

### 2-Cyclopentyl-4-(4-fluorphenyl)-7,7-dimethyl-3-(4-trifluormethylbenzoyl)-4,6,7,8-tetrahydro-1H-chinolin-5-on

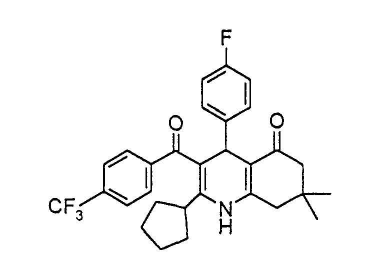

984 g 3-Amino-3-cyclopentyl-1-(4-trifluormethylphenyl)-propenon, 714 g Dimedon, 647,8 g p-Fluorbenzaldehyd und 139,3 ml Trifluoressigsäure wurden in 15 ltr. Diisopropylether 5 Stunden am Rückfluß gekocht. Nach dem Abkühlen wurden die ausgefallenen Kristalle abgesaugt, mit Diisopropylether gewaschen und getrocknet. Es resultierten 843 g mit einer Reinheit nach HPLC von 98,9 %.
Schmelzpunkt: 117 °C.
DC: R_{f} = 0,2 (Toluol/Essigester 4:1)

### Herstellungsbeispiele

### Beispiel 1

### 4-(4-Fluorphenyl)-3-[fluor-(4-trifluormethyl-phenyl)-methyl]-2-isopropyl-5,6,7,8-tetrahydrochinolin-5-ol (Dia A/Dia B-Gemisch)

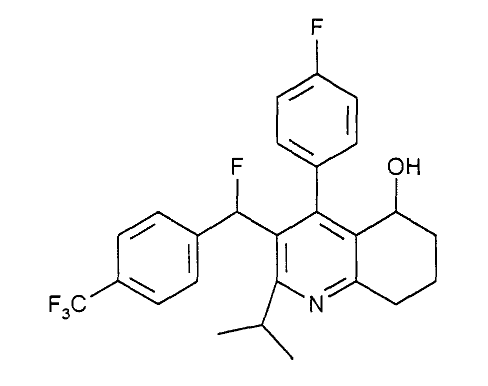

Zu 680 mg (1,177 mmol) der Verbindung aus Beispiel VIII, gelöst in 30 ml Methanol und 15 ml THF, gibt man 10 ml 3 molare Salzsäure und rührt 1 Stunde bei Raumtemperatur. Die Reaktionslösung wird in 100 ml gesättigter Natriumhydrogencarbonatlösung eingerührt, die mit 100 ml Essigsäureethylester überschichtet ist. Die organische Phase wird abgetrennt, die wässrige noch zweimal mit Essigsäureethylester extrahiert. Die vereinigten organischen Phasen werden einmal mit gesättigter Kochsalzlösung gewaschen, über Natriumsulfat getrocknet, filtriert und eingeengt. Der Rückstand wird an 50 g Kieselgel 60 nacheinander mit Toluol und Toluol/EE (8:2) chromatographiert.
Ausbeute: 240 mg (44,2 % d. Th.)
R_{f}= 0,19 (Toluol/EE 9:1)

### Beispiel 2

### 4-(4-Fluorphenyl)-3-[fluor-(4-trifluormethyl-phenyl)-methyl]-2-isopropyl-5,6,7,8-tetrahydrochinolin-5-ol (Dia A)

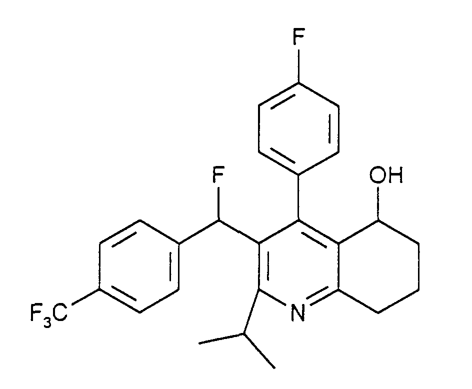

Analog Beispiel 1 werden 223 mg (0,387 mmol) der Verbindung aus Beispiel IX in 9 ml Methanol und 9 ml THF mit 3 ml 3 molarer Salzsäure über Nacht bei Raumtemperatur gerührt. Die Chromatographie des Rohproduktes erfolgt an 40 ml Kieselgel 60, das zuvor mit Cyclohexan equilibriert und danach mit Cyclohexan unter Zusatz von THF mit einem Gradienten von 10 bis 20 % eluiert wurde.
Ausbeute: 167 mg (93,3 % d. Th.)
Rf= 0,43 (Cy/THF 8:2)

### Beispiel 3

### 4-(4-Fluorphenyl)-3-[fluor-(4-trifluormethyl-phenyl)-methyl]-2-isopropyl-5,6,7,8-tetrahydrochinolin-5-ol (Dia B)

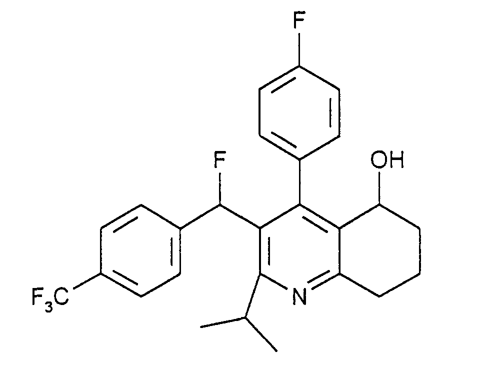

Analog Beispiel 1 werden 236 mg (0,410 mmol) der Verbindung aus Beispiel X in 9 ml Methanol und 9 ml THF mit 3 ml 3 molarer Salzsäure über Nacht bei Raumtemperatur gerührt. Die Chromatographie des Rohproduktes erfolgt an 40 ml Kieselgel 60, das zuvor mit Cyclohexan equilibriert wurde, mit Cyclohexan unter Zusatz von THF mit einem Gradienten von 10 bis 20 %.
Ausbeute: 182 mg (98,9 % d. Th.)
R_{f} = 0,41 (Cy/THF 8:1)

### Beispiel 4 und Beispiel 5

### 4-(4-Fluorphenyl)-3-[fluor-(4-trifluormethyl-phenyl)-methyl]-2-isopropyl-5,6,7,8-tetrahydrochinolin-5-ol (Enantiomer I und II)

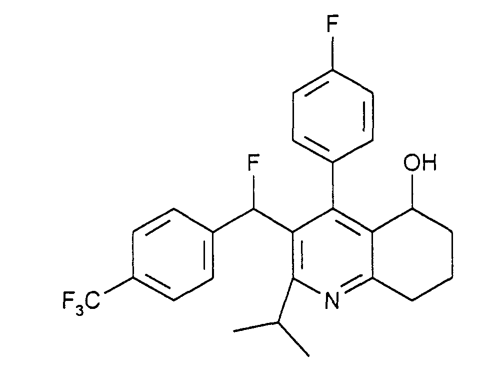

82 mg der Verbindung Dia B aus Beispiel 3 werden in einem Gemisch von 8 ml n-Heptan und 2-Propanol (9:1) gelöst und an einer präparativen HPLC-Säule (250 x 20 mm; gefüllt mit Chiralcel OD; 20 µm) getrennt. Als Elutionsmittel wird ein Gemisch von n-Heptan (LiChrosolv) und 2-Propanol p.a. (98:2) verwendet. Bei einer Temperatur von 40°C, einer Laufzeit von 30 min und einem Fluß von 7,0 ml/min werden insgesamt 20 Injects von jeweils 0,4 ml ausgeführt (Detektion bei 230 nm). Man erhält 9 Fraktionen, die nach analytischer HPLC-Kontrolle in einen vorderen (Enantiomer I; Retention: 6,13 min) und einen hinteren Peak (Enantiomer II; Retention: 8,10 min) differenziert und isoliert werden. Die hintere Fraktion (Retention: 8,10 min) wird an der Chiracel OD-Säule mittels n-Heptan/2-Propanol rechromatographiert.
Ausbeute Enantiomer I: 37 mg (45,1 % d. Th.)
Ausbeute Enantiomer II: 32 mg (39,6 % d. Th.)
Analytisches HPLC:
Säule: 250 x 4,6 mm (Chiralcel OD-H; 5 µm)
Fluß: 1,0 ml/min
Laufmittel: 98 % n-Heptan (LiChrosolv), 2 % 2-Propanol p.a.
Temperatur: 40°C
Auftragsvolumen: 10 µl
Detektion: 220 nm

### Beispiel 6

### 8-Butyl-4-(4-fluorphenyl)-3-[fluor-(4-trifluormethyl-phenyl)-methyl]-2-isopropyl-5,6,7,8-tetrahydrochinolin-5-ol

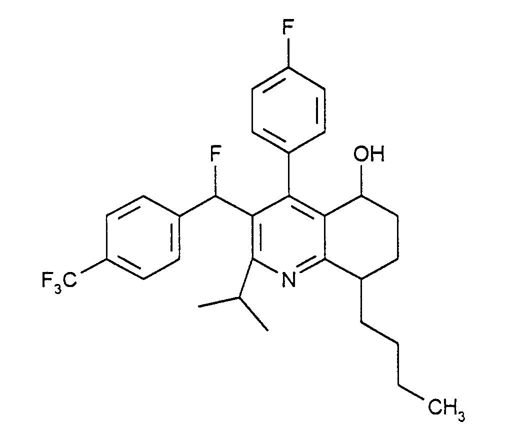

180 mg (0,28 mmol) der Verbindung aus Beispiel XII werden für 16 Stunden in einer Lösung aus 16,8 ml THF und 2,8 ml 3 molarer Salzsäure bei Raumtemperatur gerührt. Danach wird in 70 ml gesättigte Natriumhydrogencarbonatlösung eingerührt und mit 20 ml Toluol verdünnt. Die organische Phase wird abgetrennt, mit Wasser gewaschen, über Natriumsulfat getrocknet, filtriert und eingeengt. Das Rohprodukt wird an 25 g Kieselgel 60 mit Cyclohexan/Essigsäureethylester 9:1 eluiert.
Ausbeute: 44 mg (30 % d. Th.)
R_{f}=0,20 (Cy/EE 9:1)

### Beispiel 7

### 4-(4-Fluorphenyl)-3-[fluor-(4-trifluormethyl-phenyl)-methyl]-2-isopropyl-5,6,7,8-tetrahydrochinolin-5-on

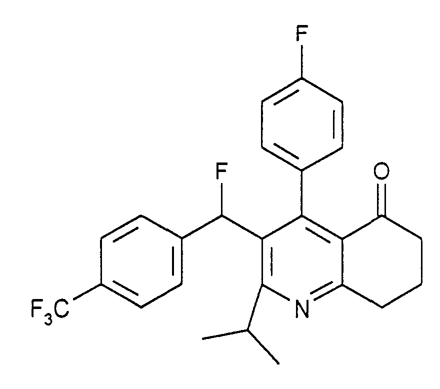

Zu einer Lösung von 16 g (36,4 mmol) der Verbindung aus Beispiel 2 in 655 ml Dichlormethan werden unter kräftigem Rühren 11,16 g (109,2 mmol) neutrales Aluminiumoxid und 23,54 g Pyridiniumchlorochromat portionsweise eingerührt. Nach 1 Stunde werden 140 g Kieselgel 60 zugegeben und abgesaugt. Das Eluat wird eingeengt und getrocknet.
Ausbeute: 10,5 g (70 % d. Th.)
R_{f} = 0,55 (Cy/EE 6:4)

### Beispiel 8

### 4-(4-Fluorphenyl)-3-[fluor-(4-trifluormethyl-phenyl)-methyl]-2-isopropyl-6-(4-trifluormethyl-phenyl)-methyl-5,6,7,8-tetrahydrochinolin-5-on

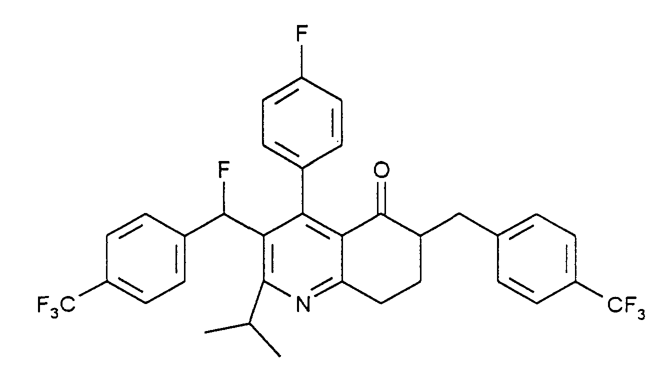

Unter Argon werden bei -70°C in 4 ml absolutem THF 0,88 ml (1,4 mmol) 1,6 molarer n-Butyllithiumlösung in n-Hexan und 0,20 ml Diisopropylamin eingerührt und 1 Stunde bei -50°C gerührt. Zu dieser Lösung wird bei -50°C eine Lösung der Verbindung aus Beispiel 7 (460 mg, 1 mmol) zugetropft und 1 weitere Stunde gerührt. Folgend wird eine Lösung von 335 mg (1,4 mmol) Trifluormethylbenzylbromid in 1 ml absolutem THF zugetropft und nach 1 Stunde bei -50°C mit Wasser hydrolisiert. Zur Aufarbeitung wird eine Mischung aus 5 %iger Kochsalzlösung und Toluol eingerührt, die Phasen getrennt, die wässrige Phase nachextrahiert und die vereinigten organischen Phasen über Natriumsulfat getrocknet, filtriert und eingeengt. Das Rohprodukt wird an 90 g Kieselgel 60 mit Cyclohexan/Essigsäureethylester 9:1 eluiert.
Ausbeute: 443 mg (72 % d. Th.)
R_{f}=0,23 (Cy/EE 9:1)

### Beispiel 9

### 4-(4-Fluorphenyl)-3-[fluor-(4-trifluormethyl-phenyl)-methyl]-2-isopropyl-6-(4-trifluormethyl-phenyl)-methyl-5,6,7,8-tetrahydrochinolin-5-ol

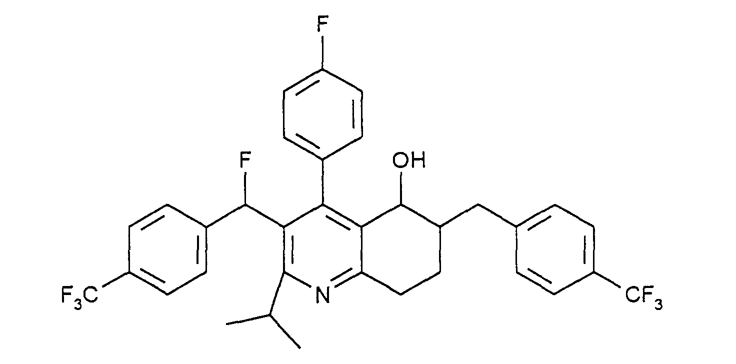

Zu einer Lösung von 100 mg (0,32 mmol) der Verbindung aus Beispiel 8 in 1,6 ml Toluol werden bei -70°C 0,48 ml einer 1 molaren Diisobutylaluminiumhydridlösung (DIBAL-H) in Toluol getropft. Nach 2 Stunden wird mit 5 ml 20 %iger Natriumkaliumtartratlösung versetzt und 0,5 Stunden bei Raumtemperatur gerührt. Die organische Phase wird abgetrennt, über Natriumsulfat getrocknet, filtriert und eingeengt. Das Rohprodukt wird an Kieselgel 60 mit Cyclohexan/Essigsäureethylester 85:15 eluiert.
Ausbeute: 58 mg (59 % d. Th.)
R_{f}=0,23 (Cy/EE 85:15)

In Analogie zu den oben aufgeführten Vorschriften wurden die in die folgenden Tabellen aufgeführten Verbindungen hergestellt:

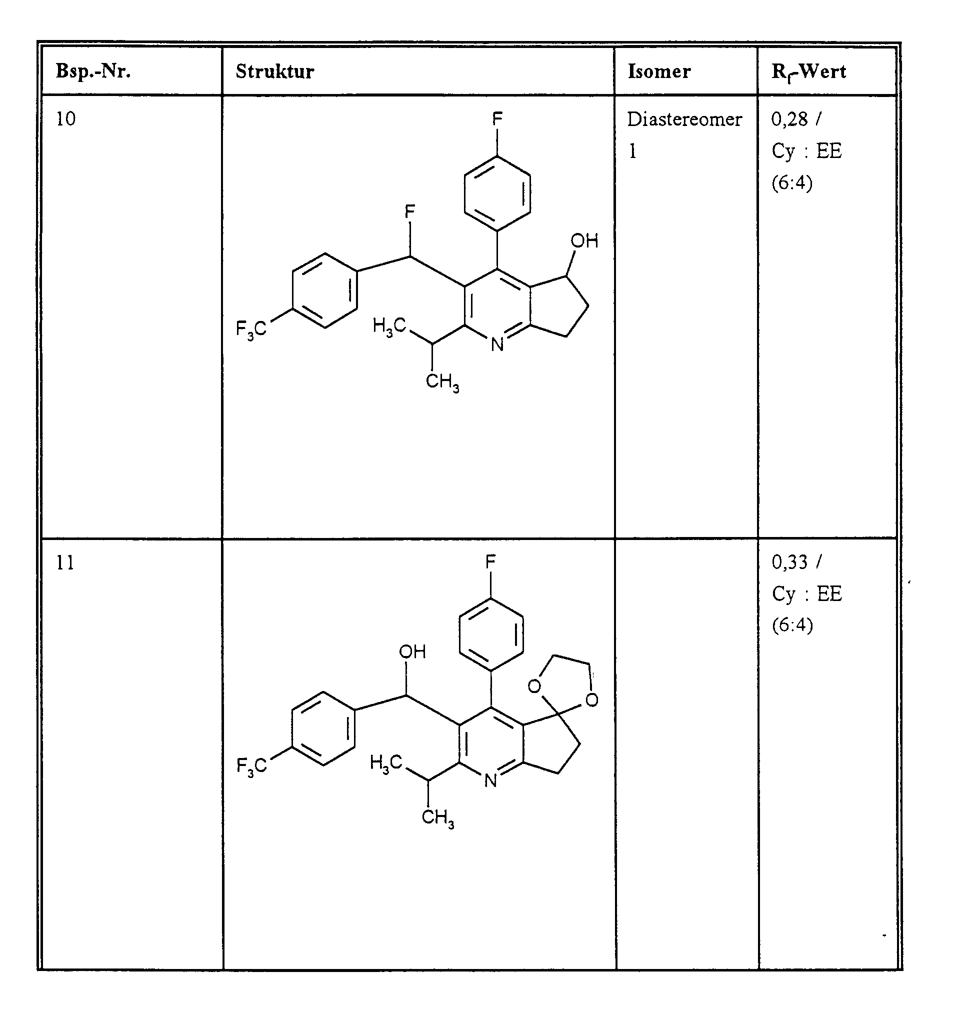

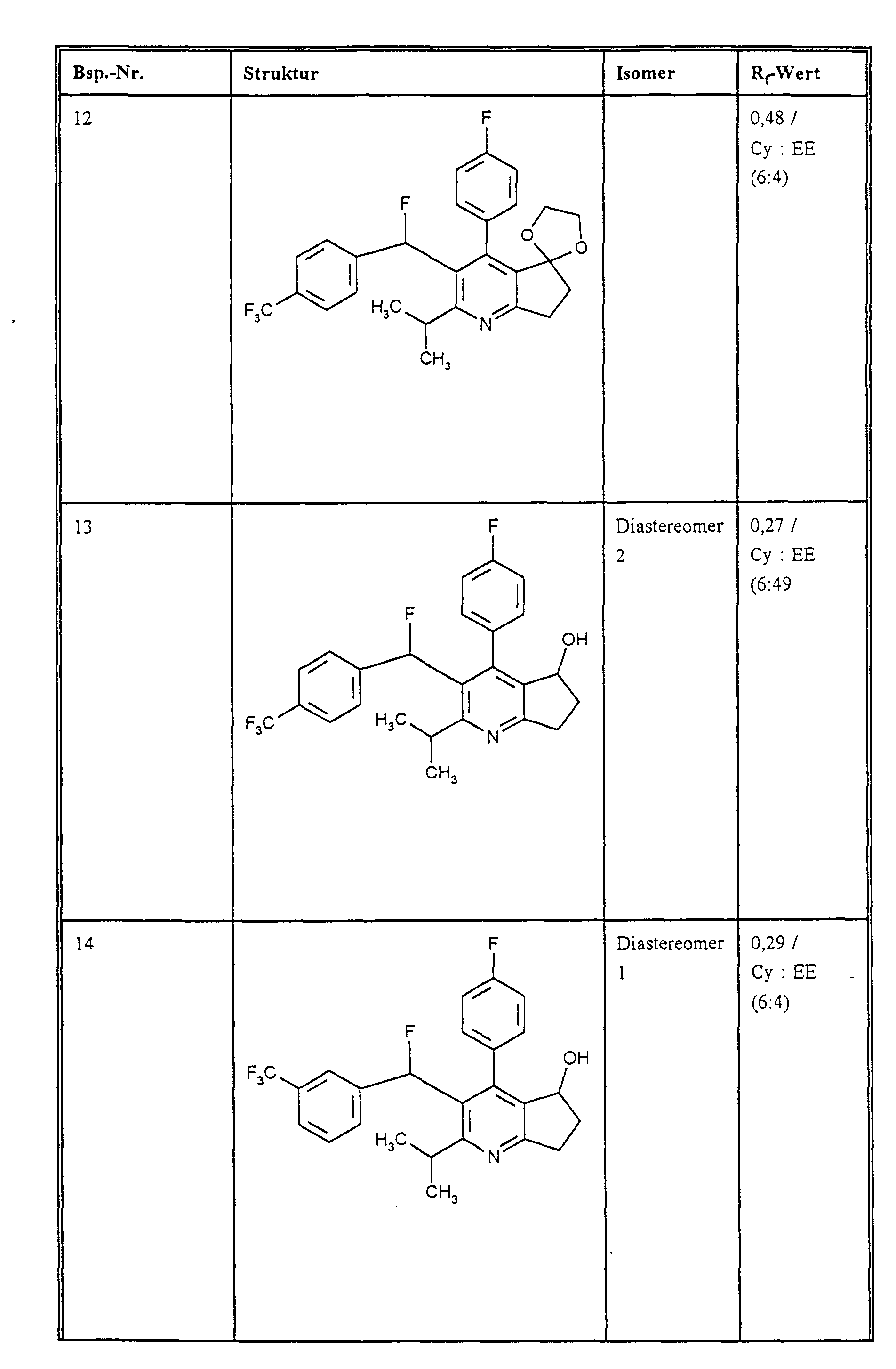

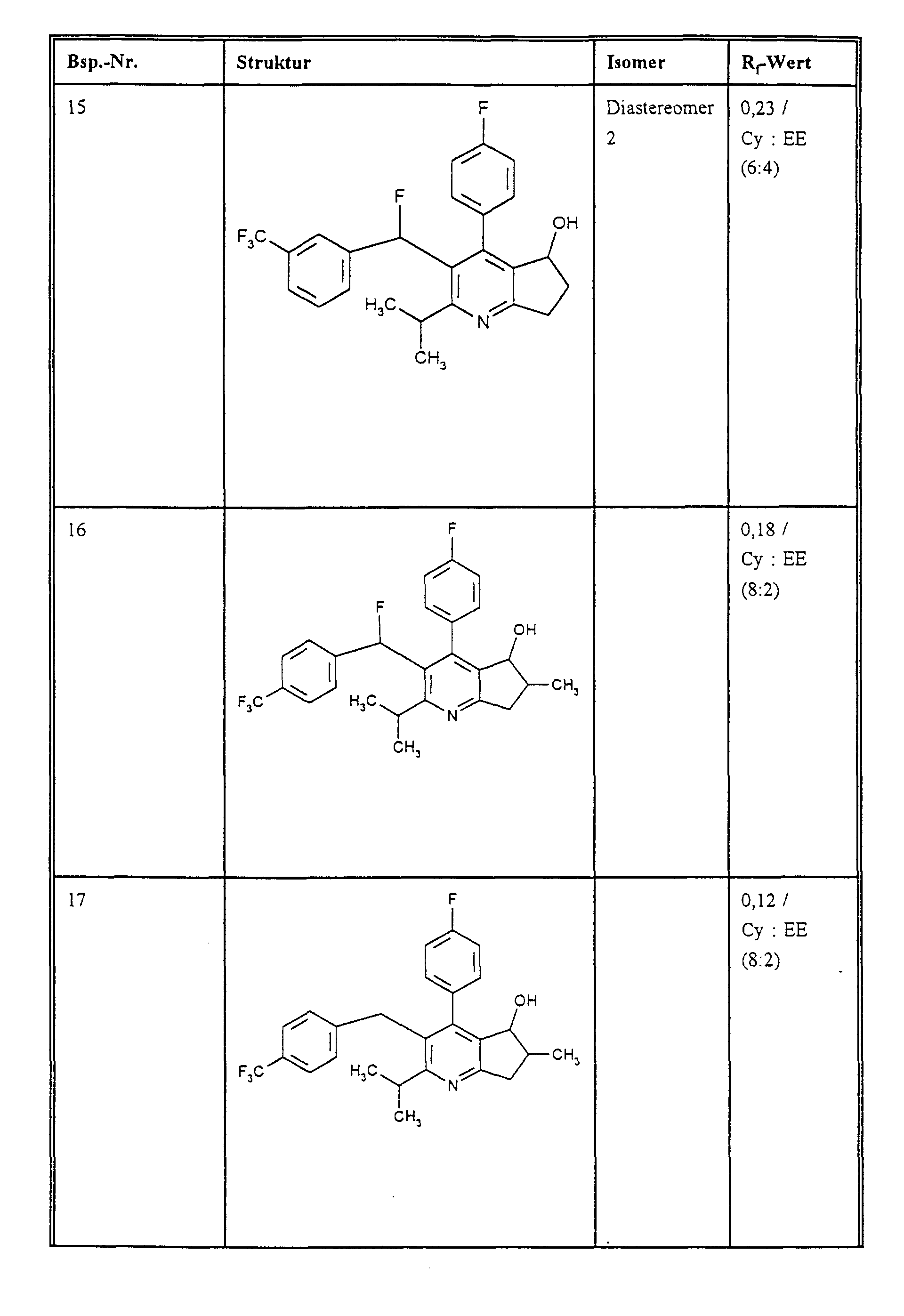

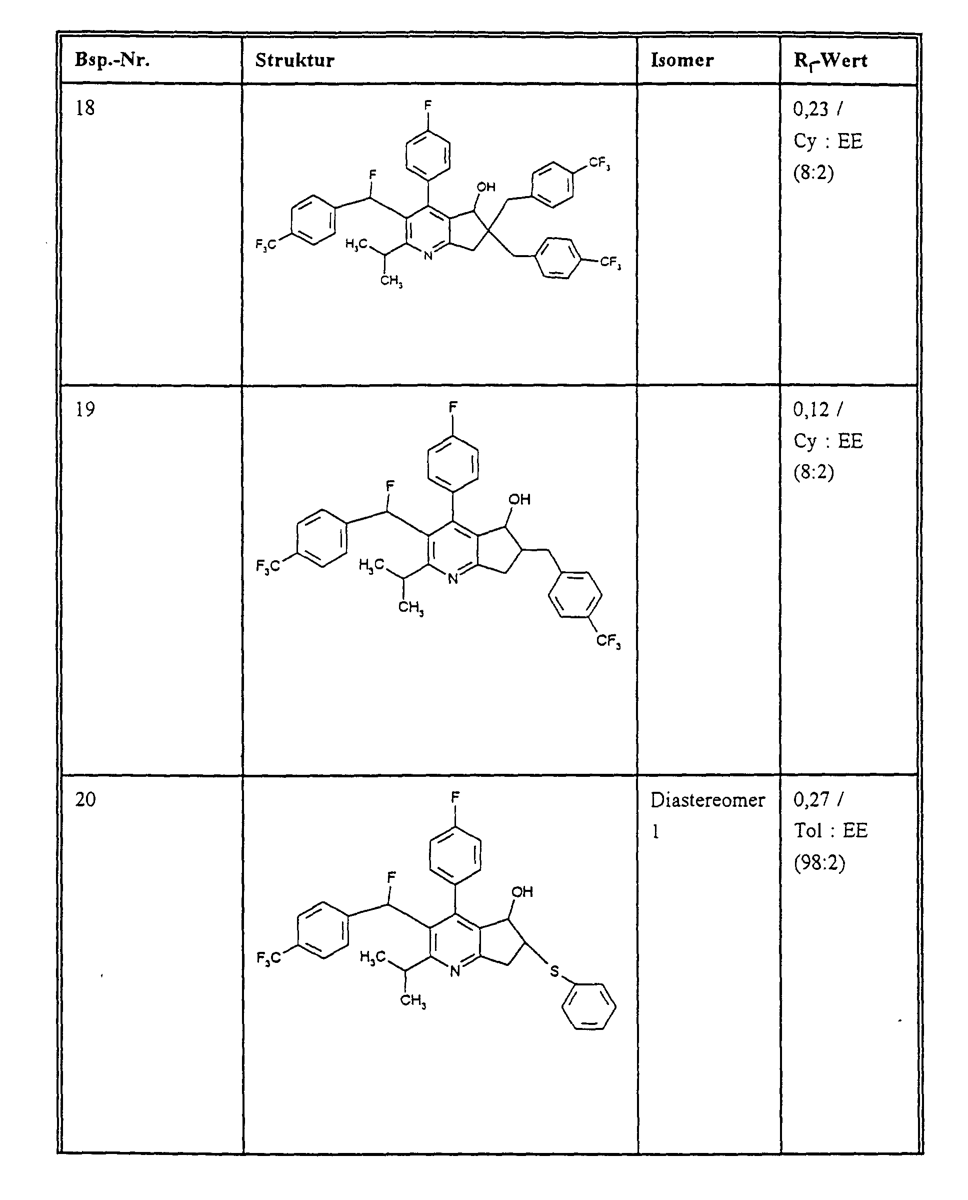

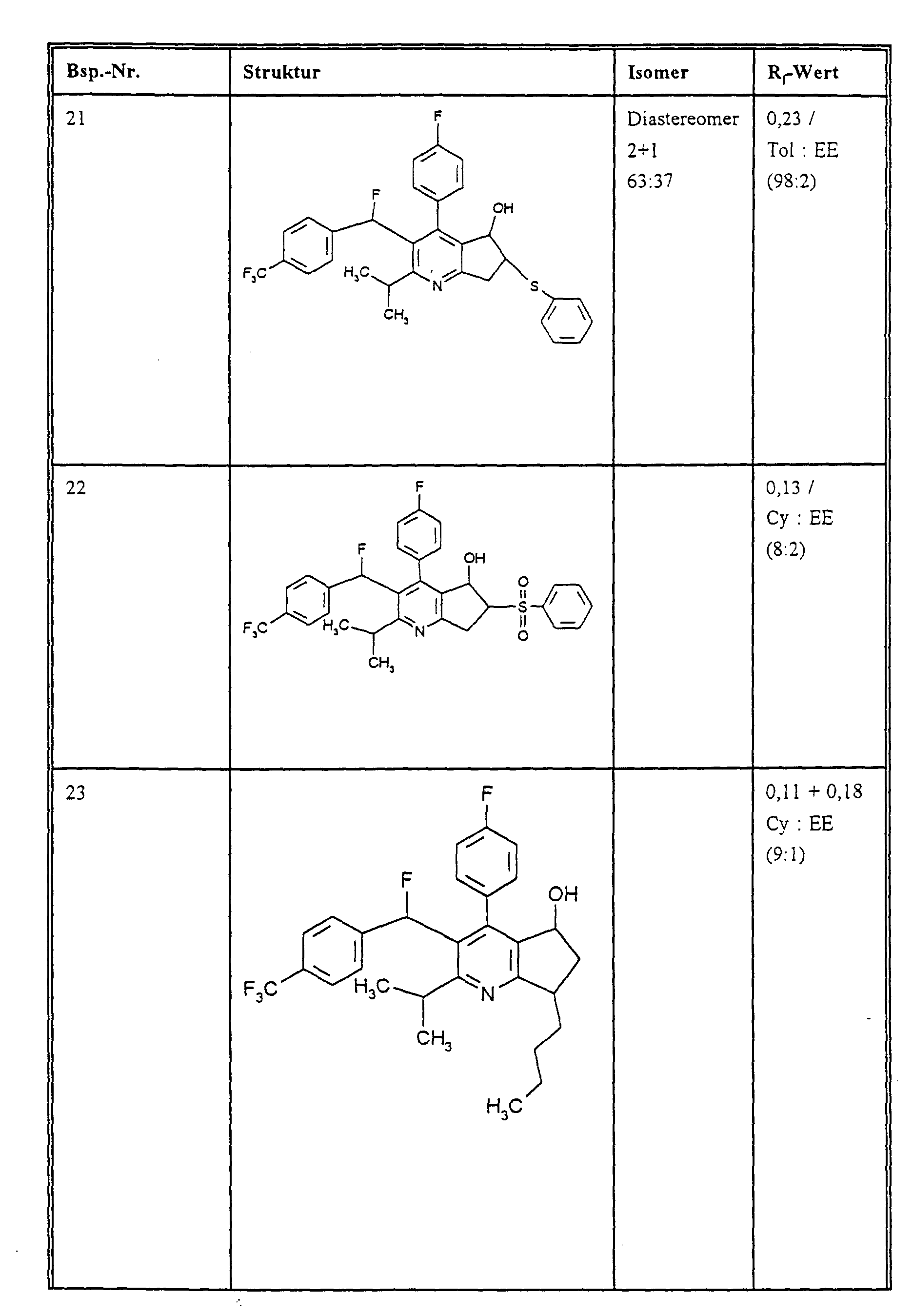

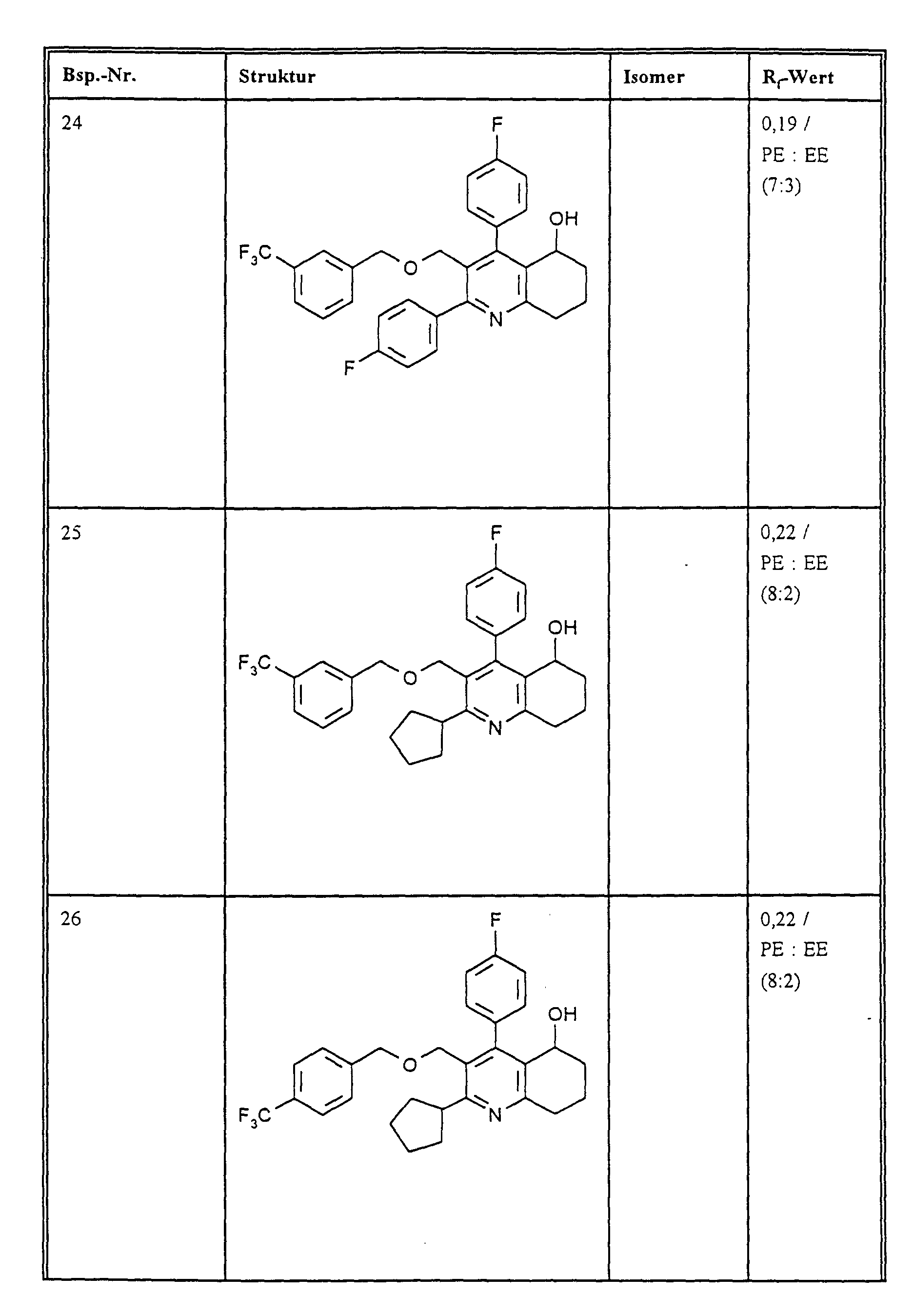

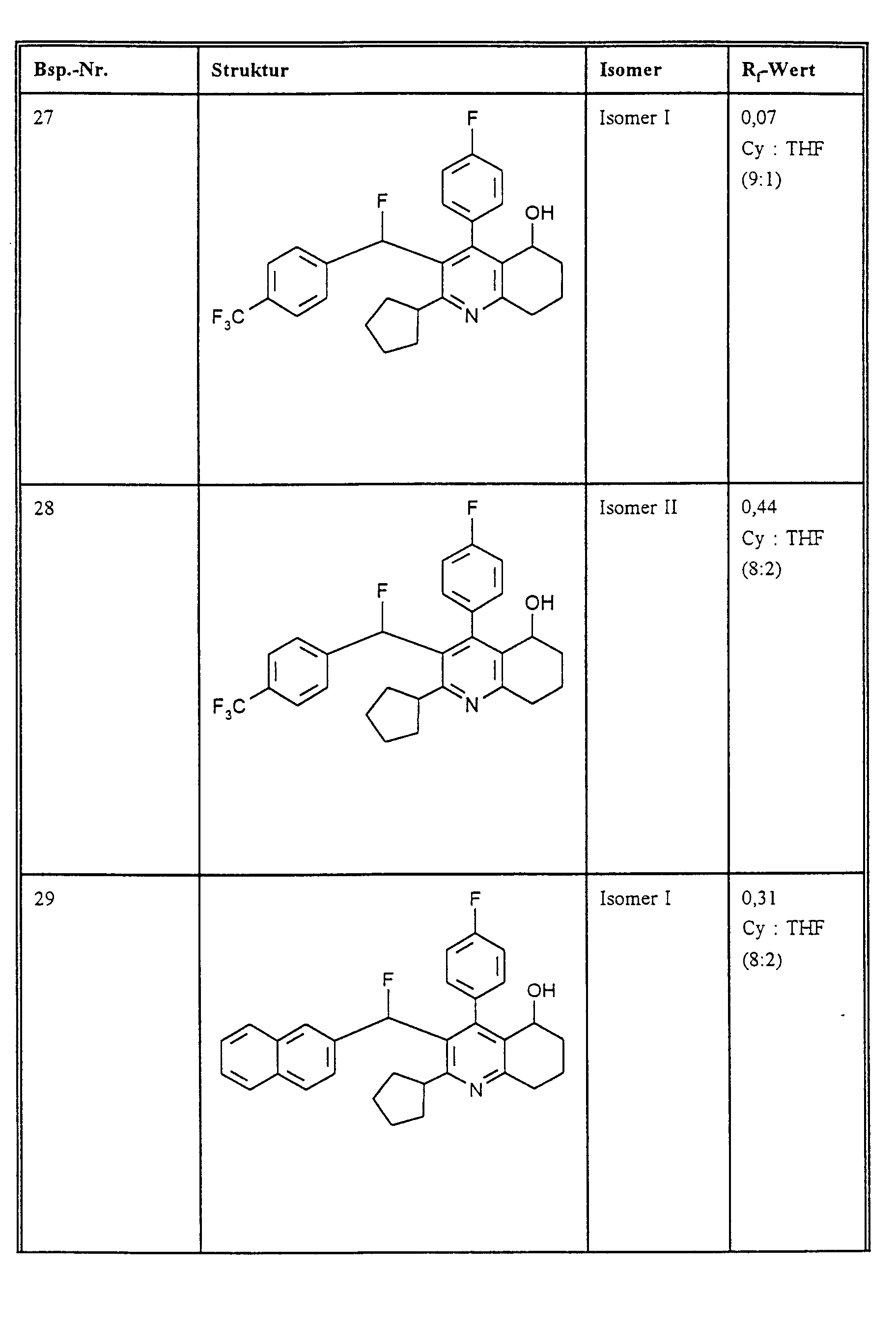

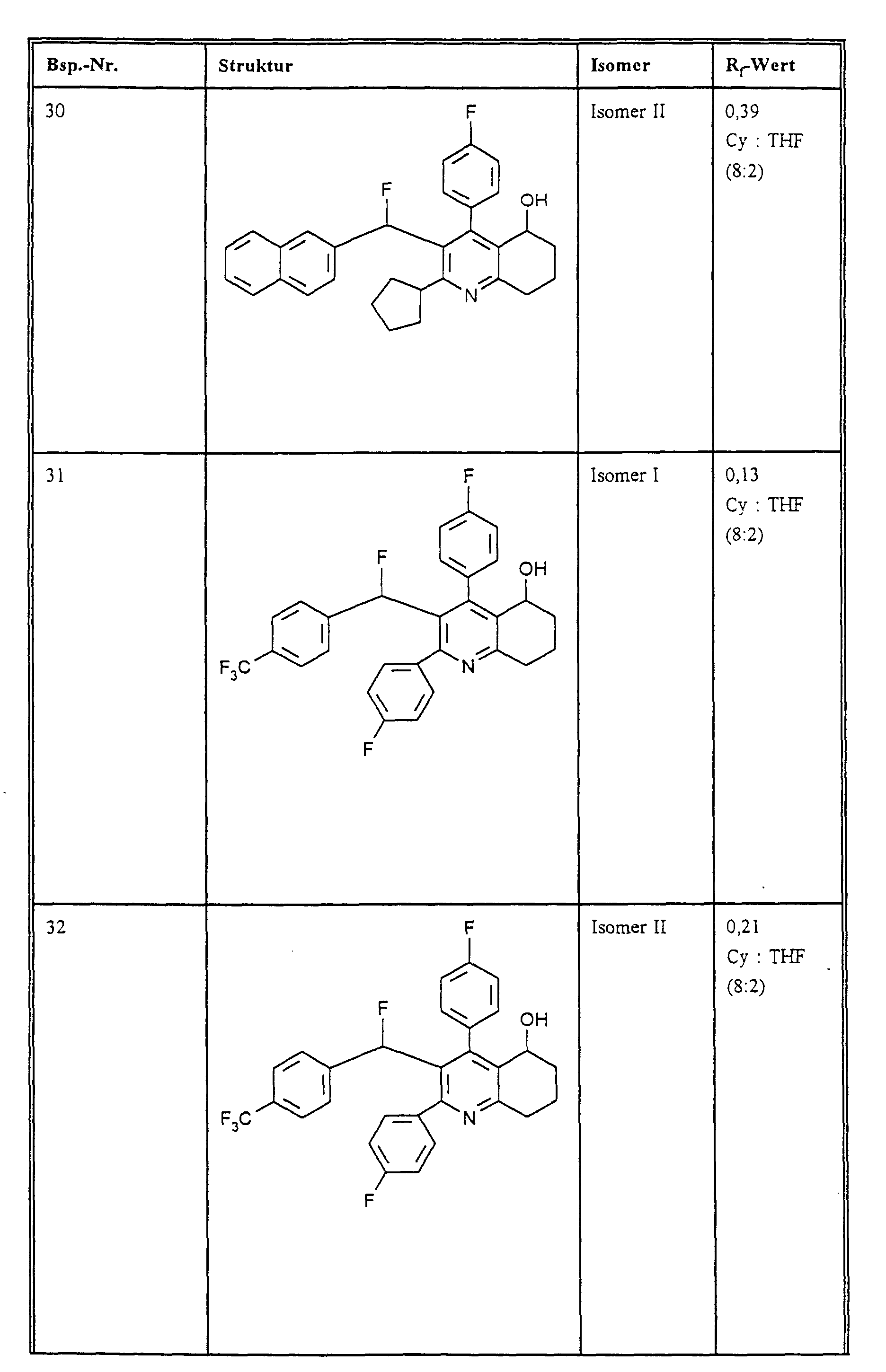

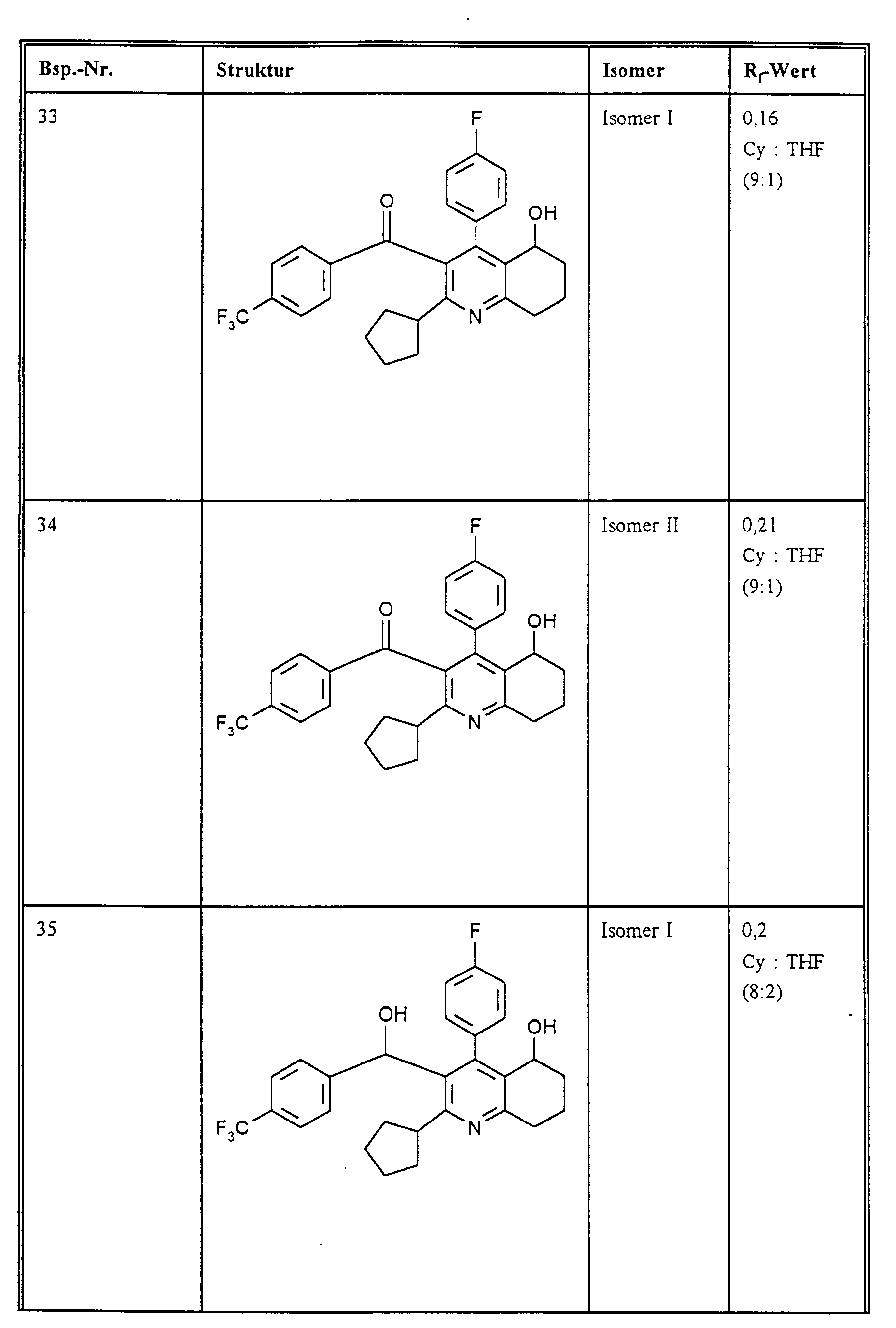

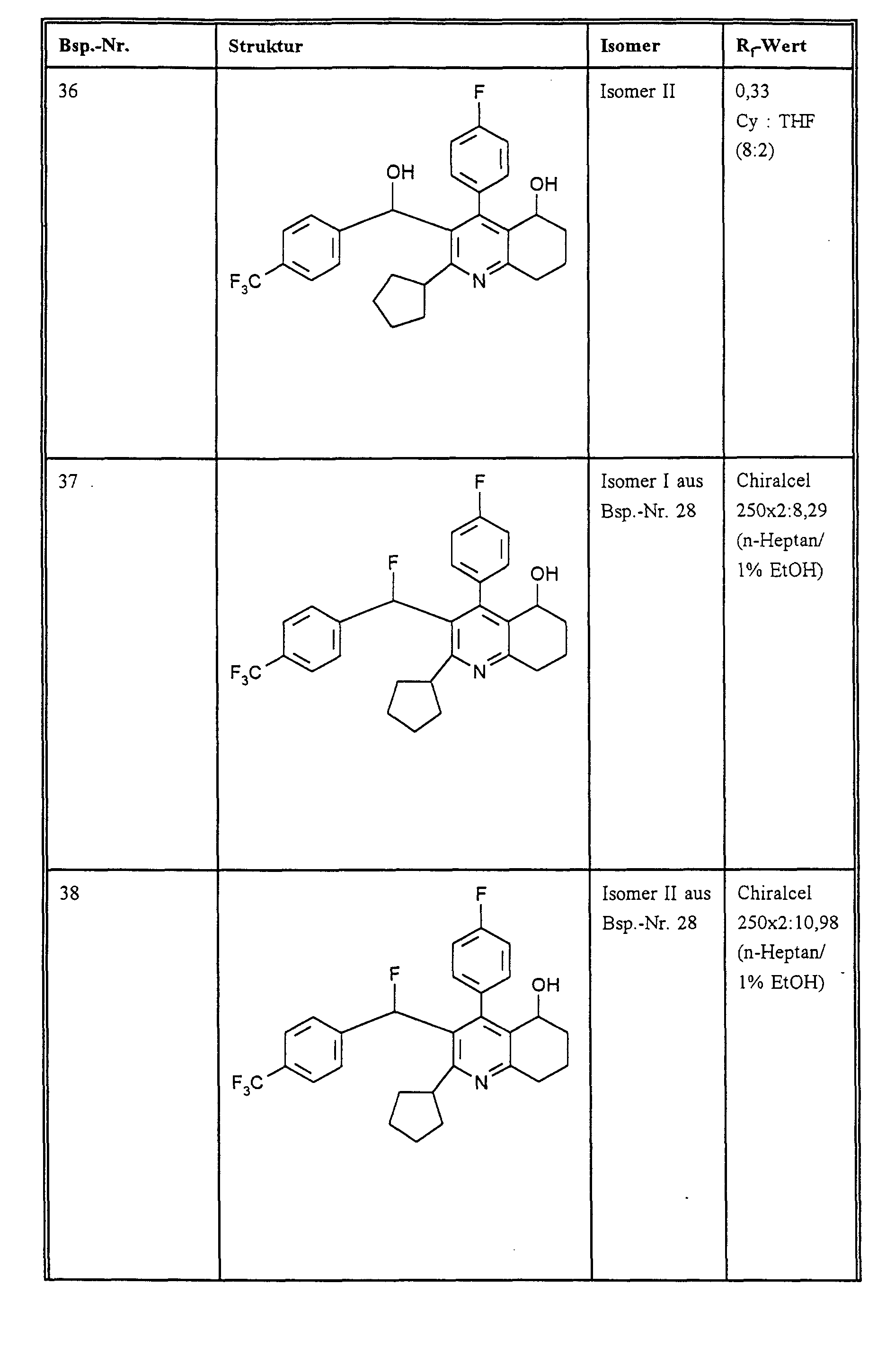

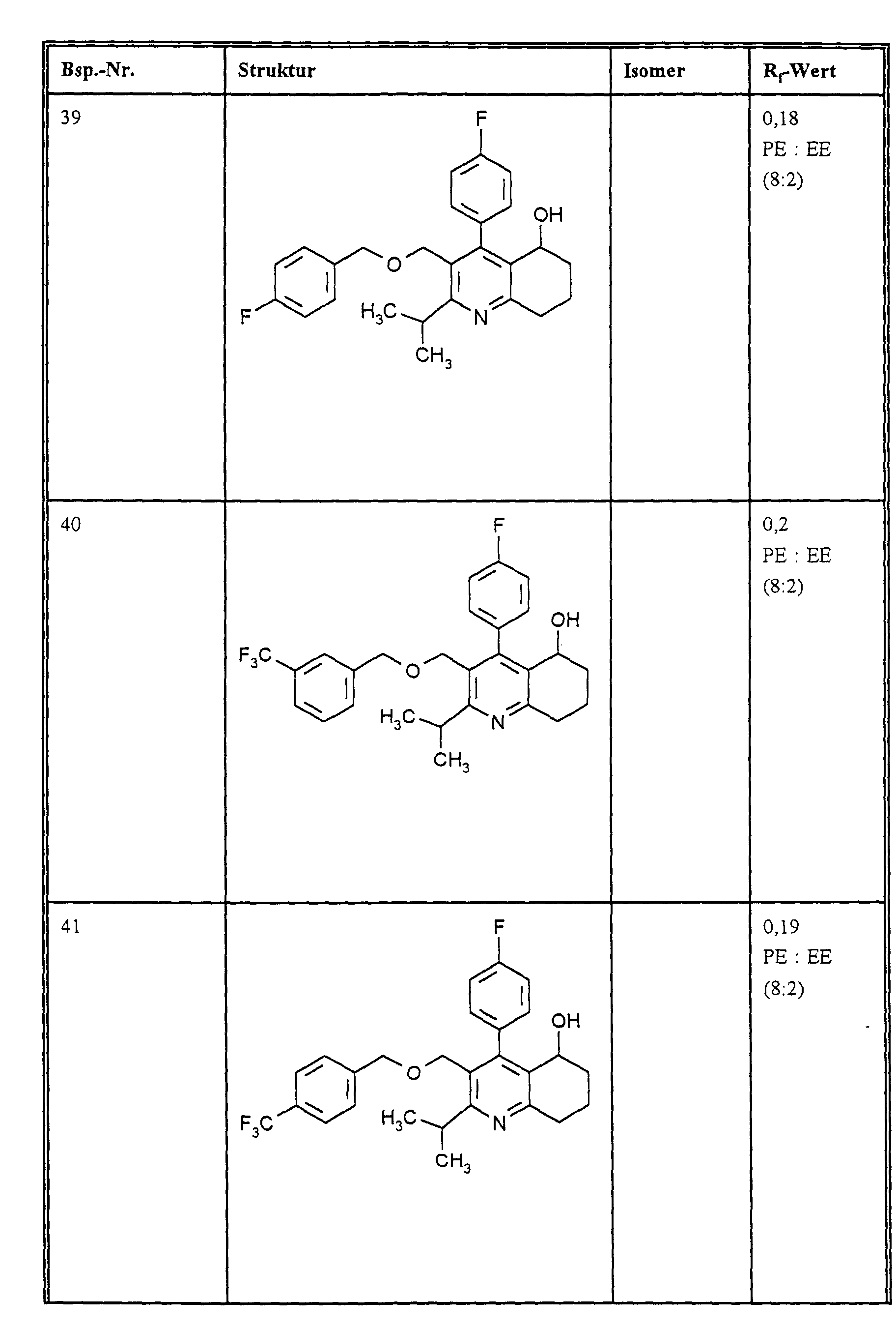

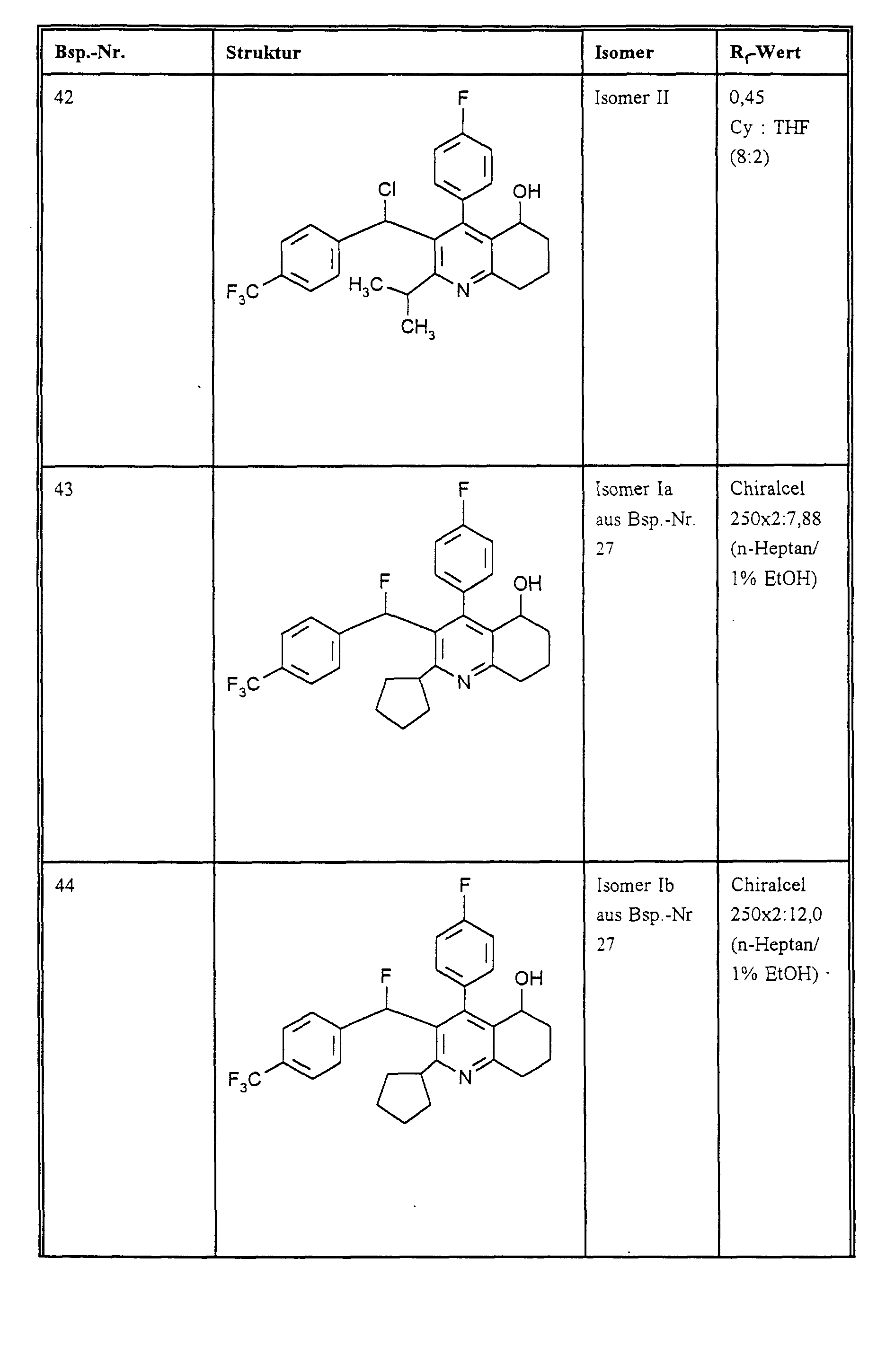

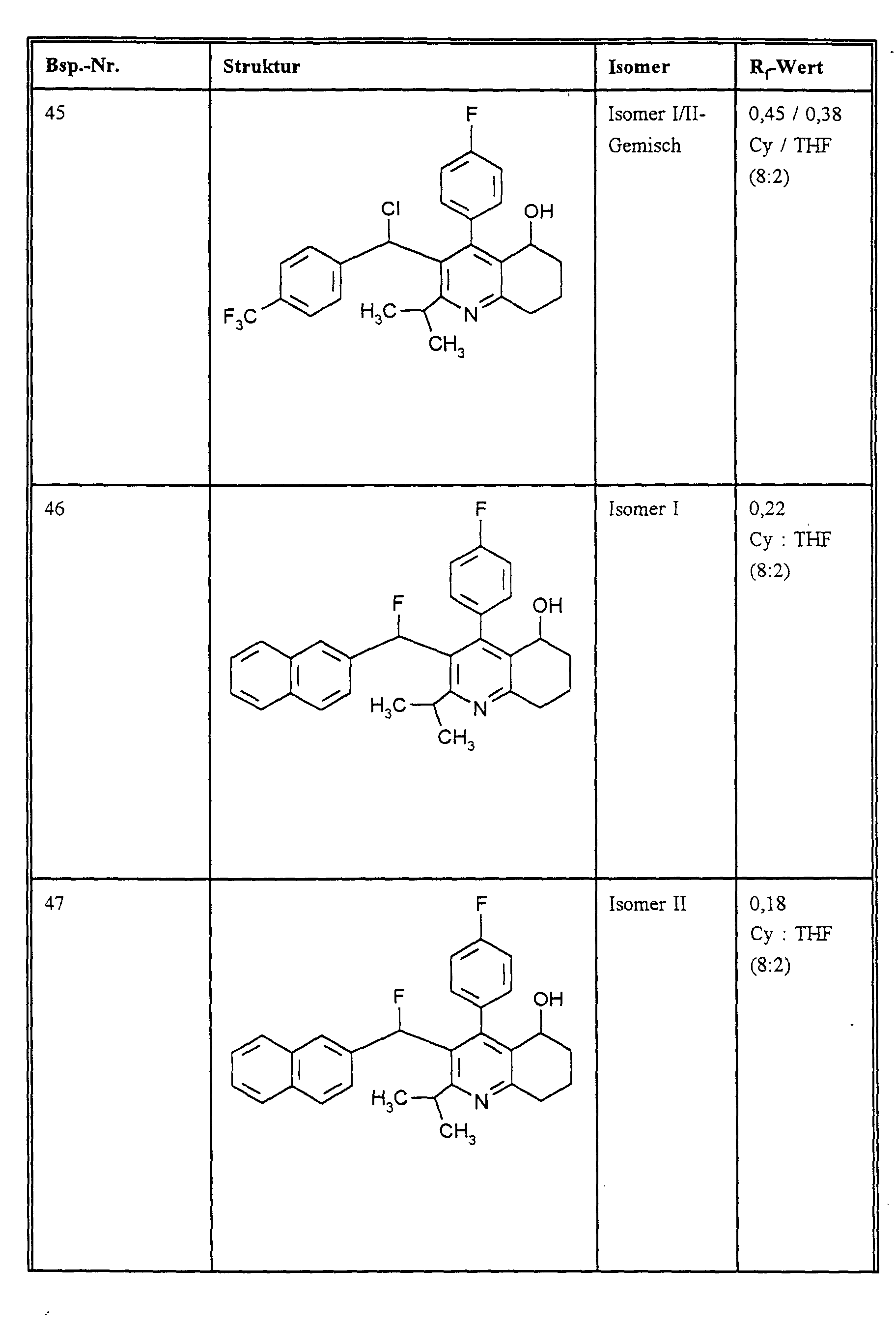

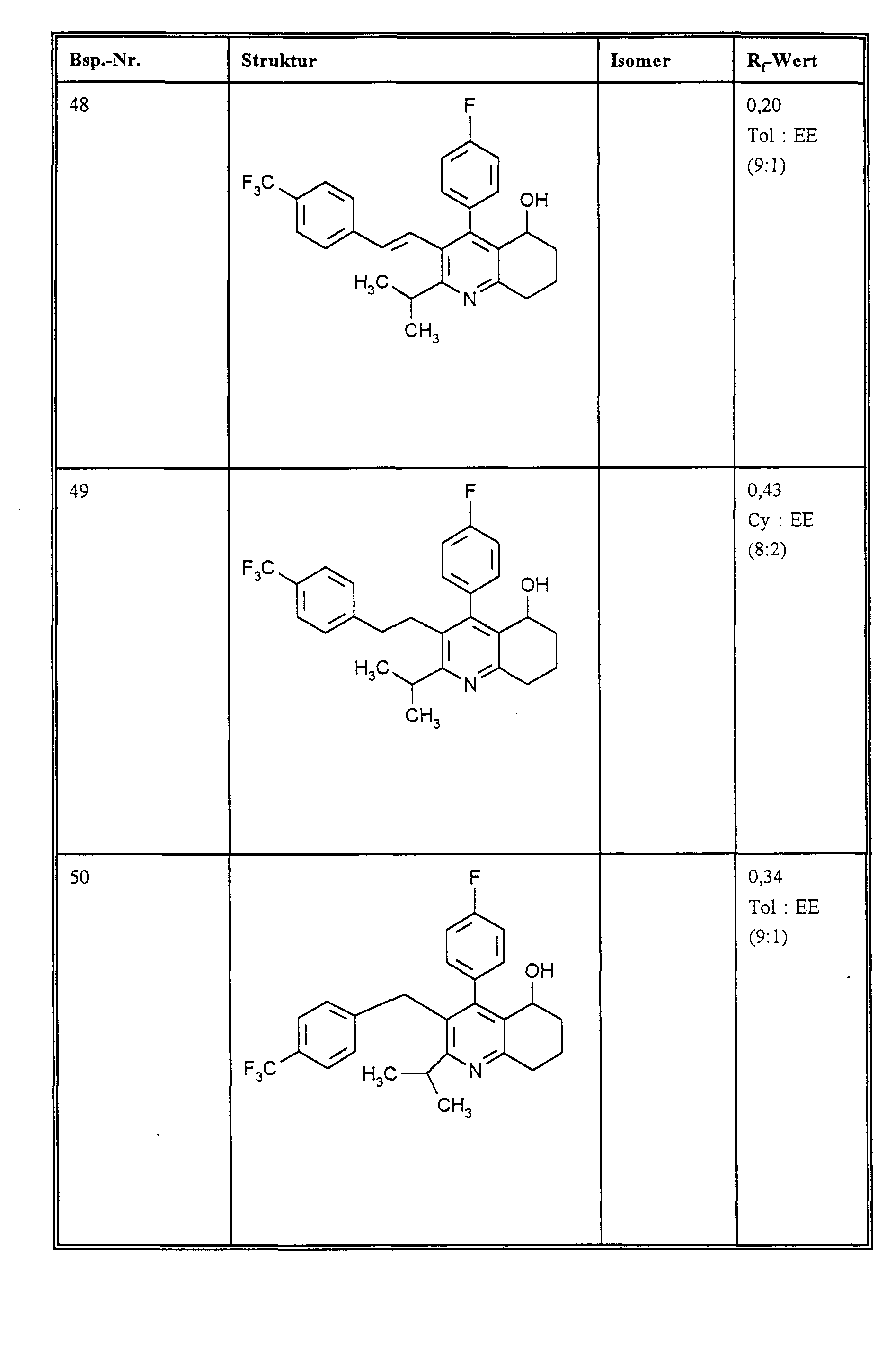

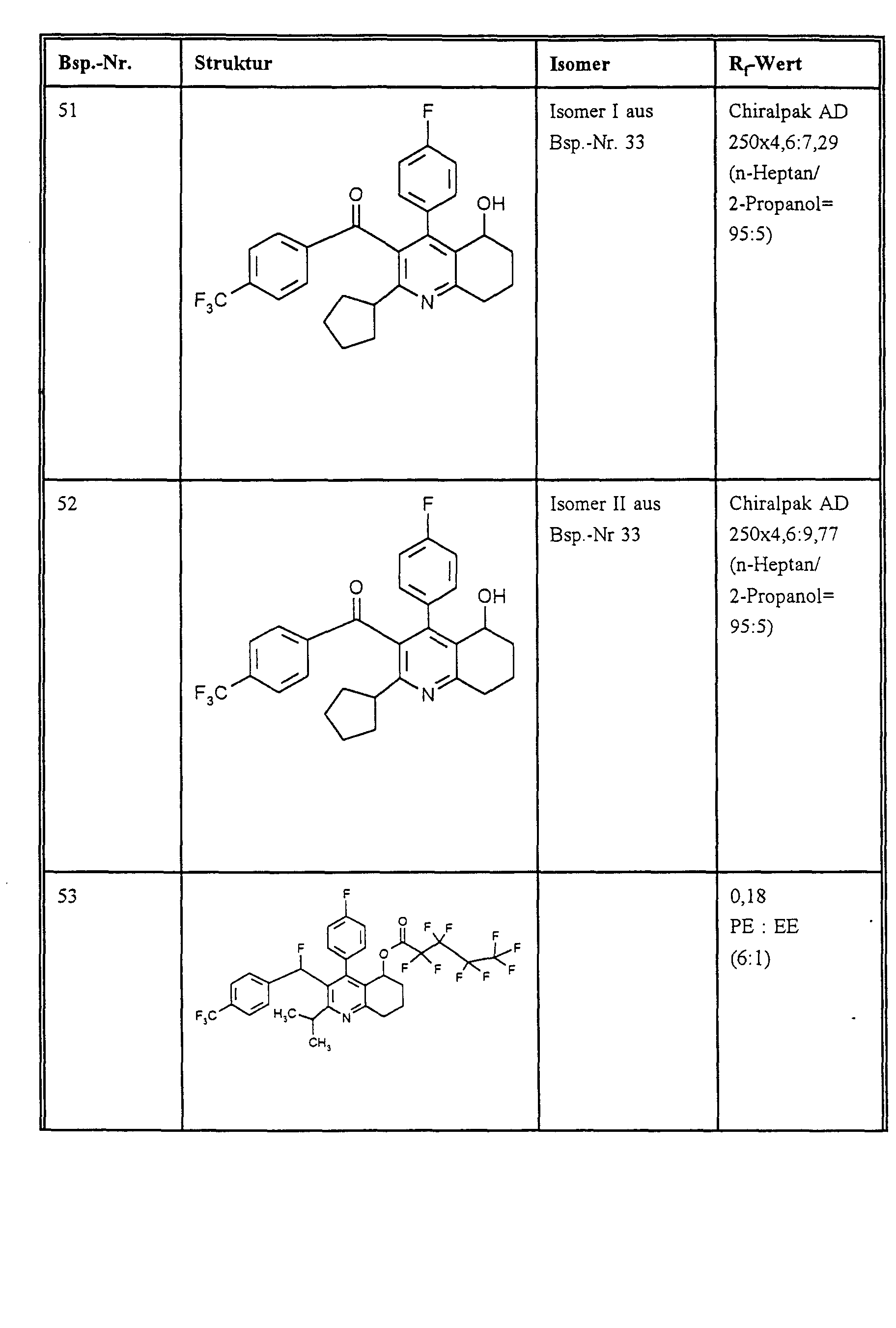

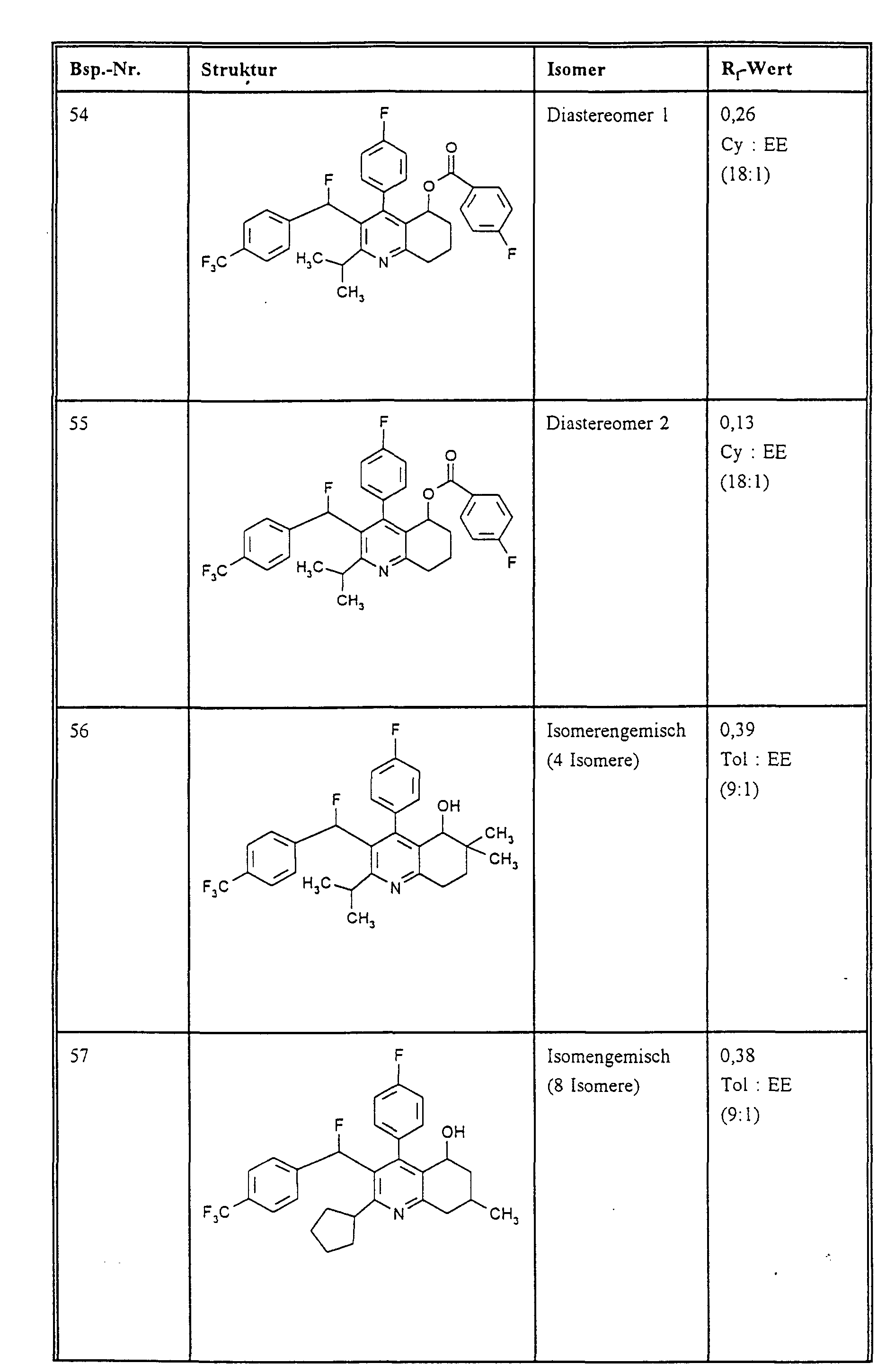

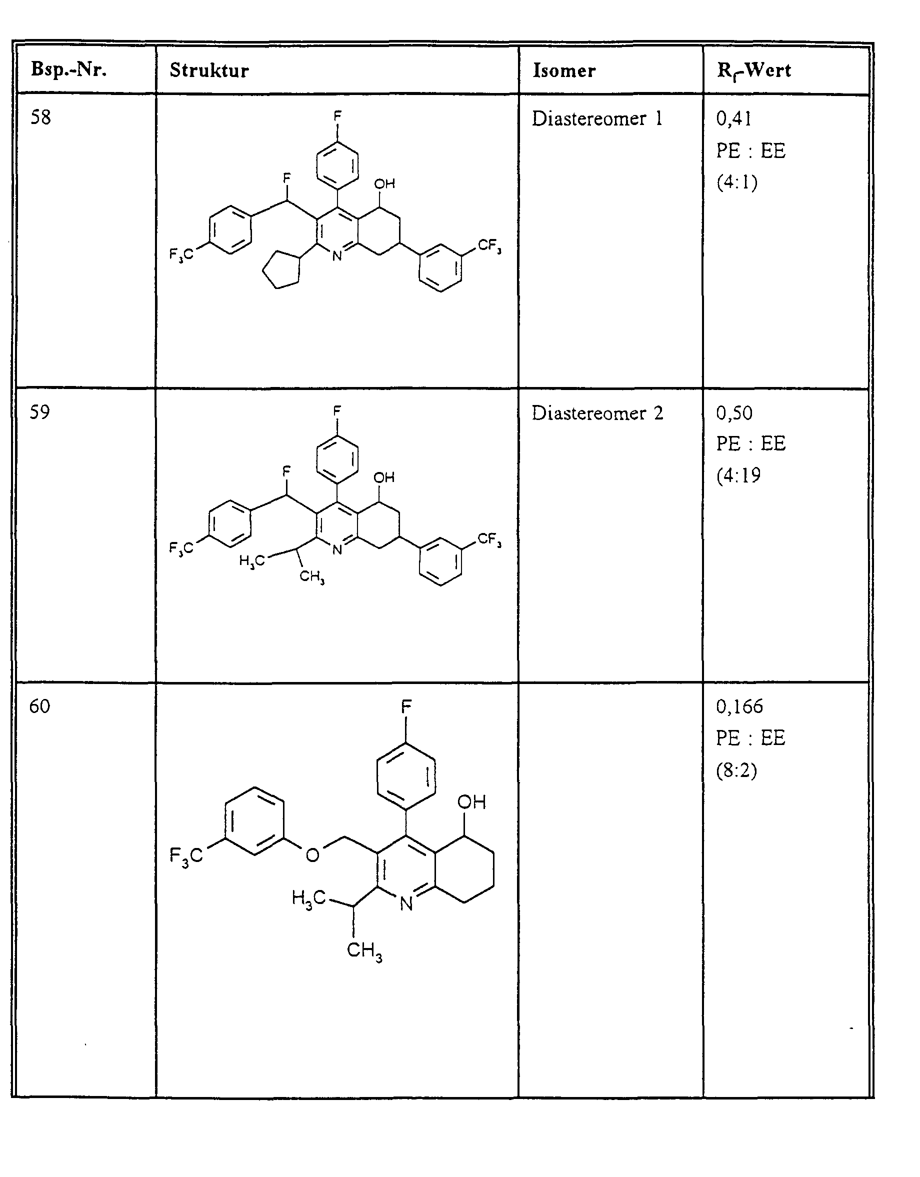

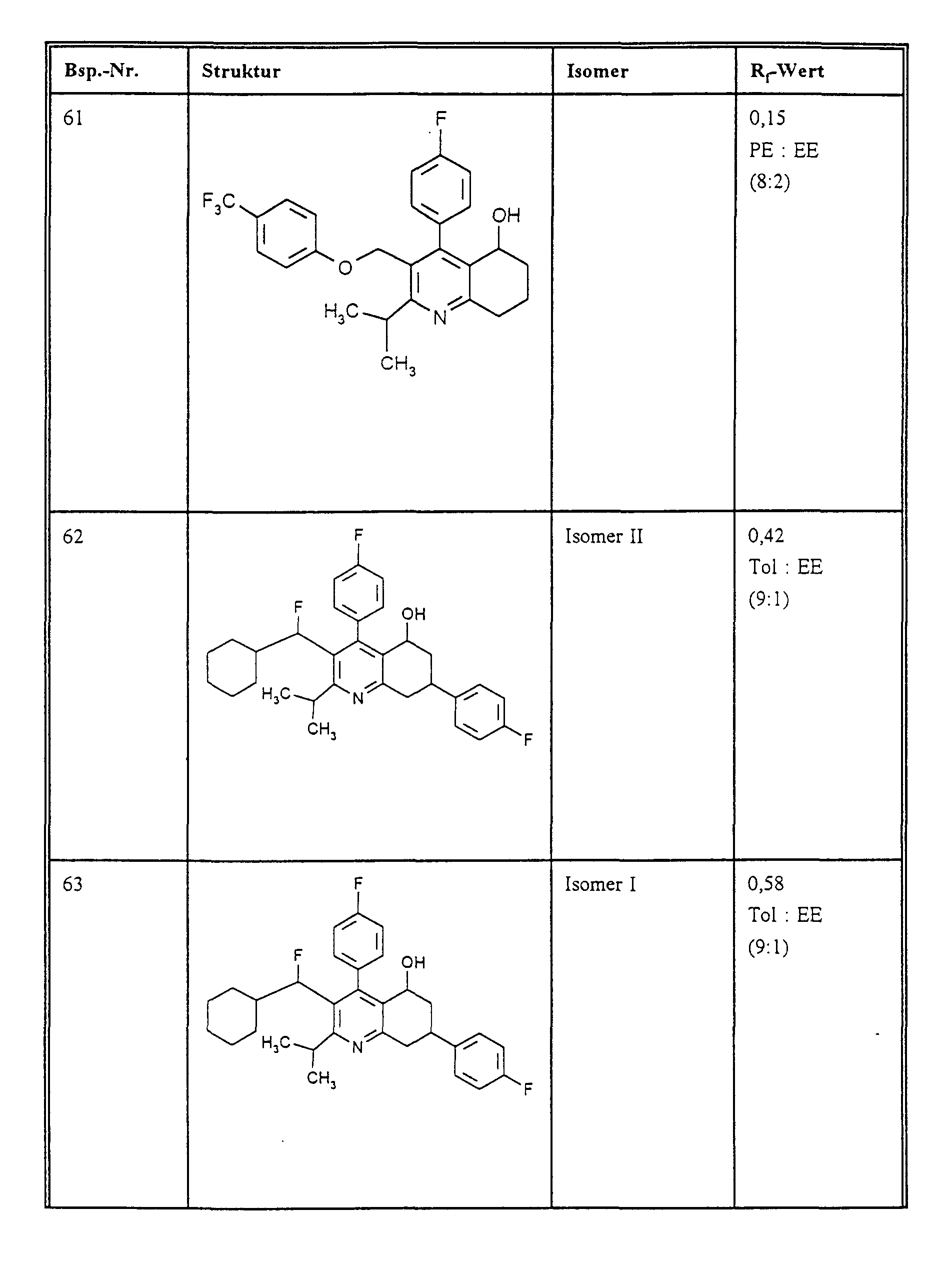

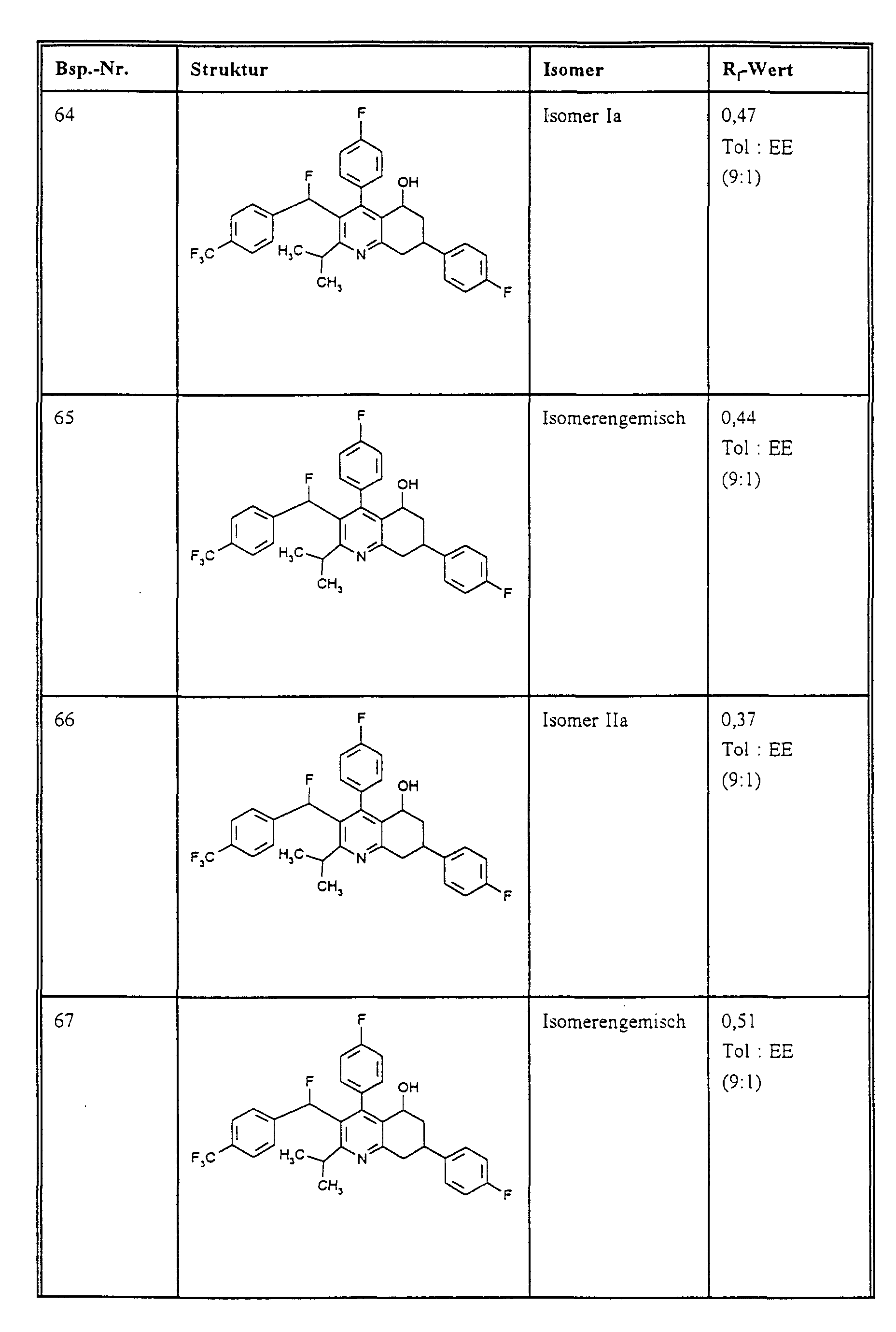

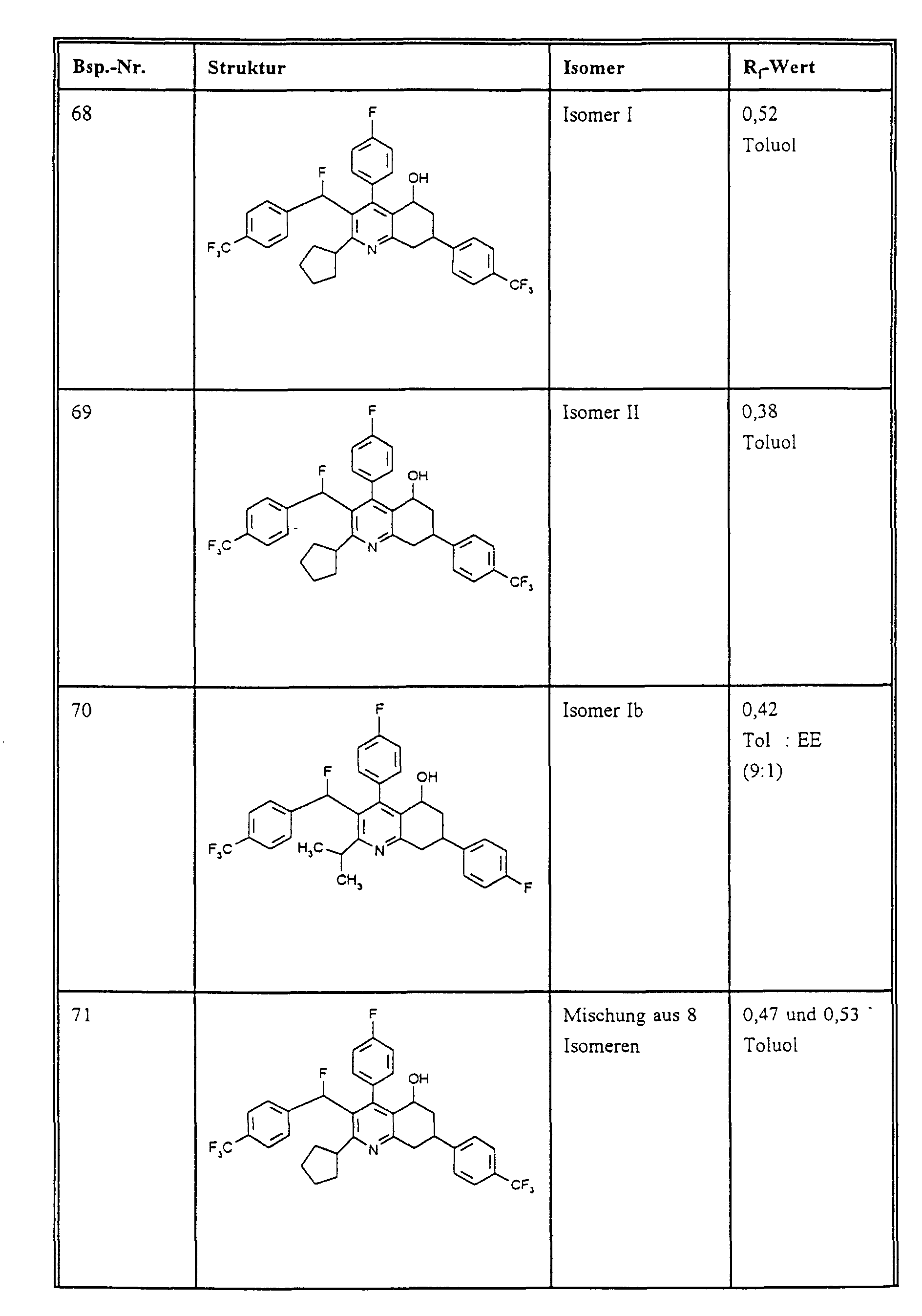

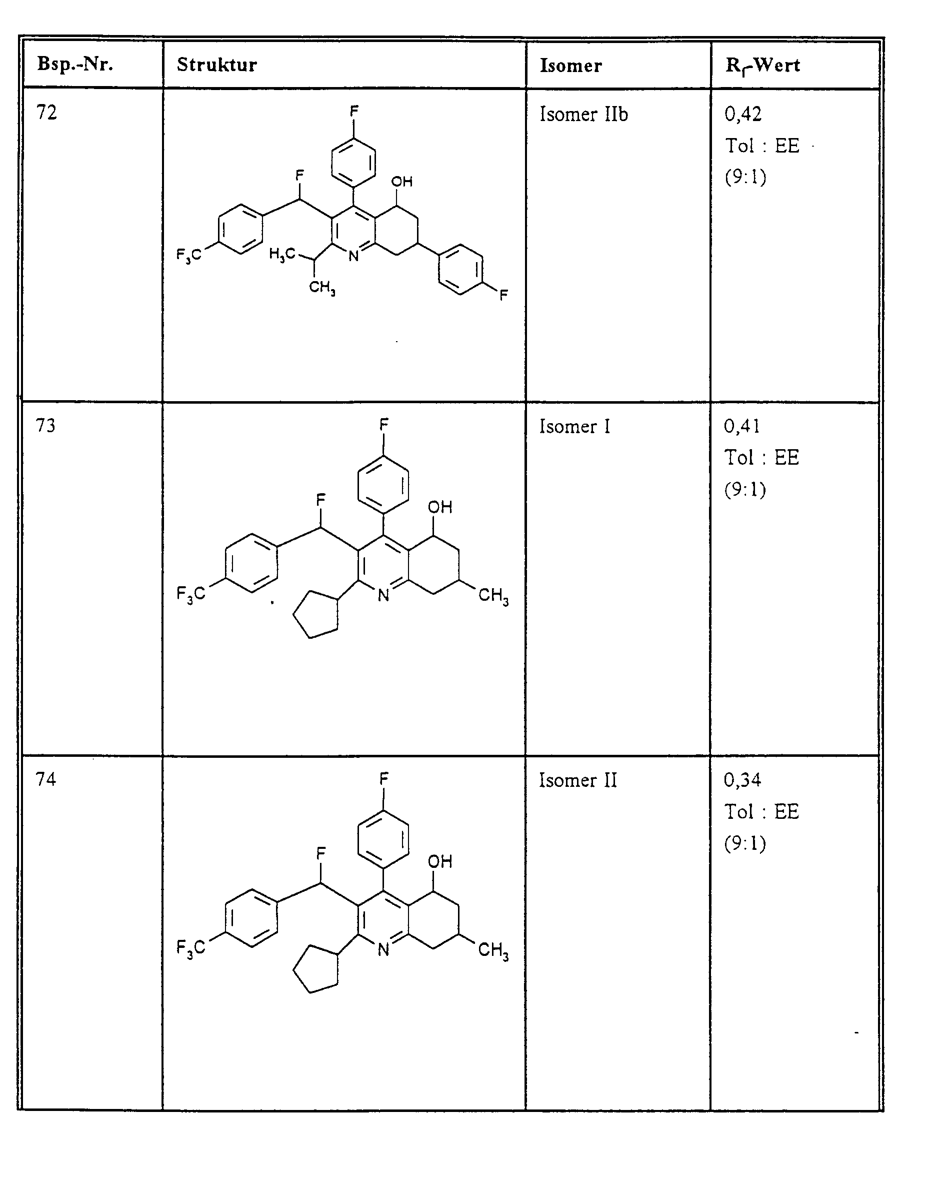

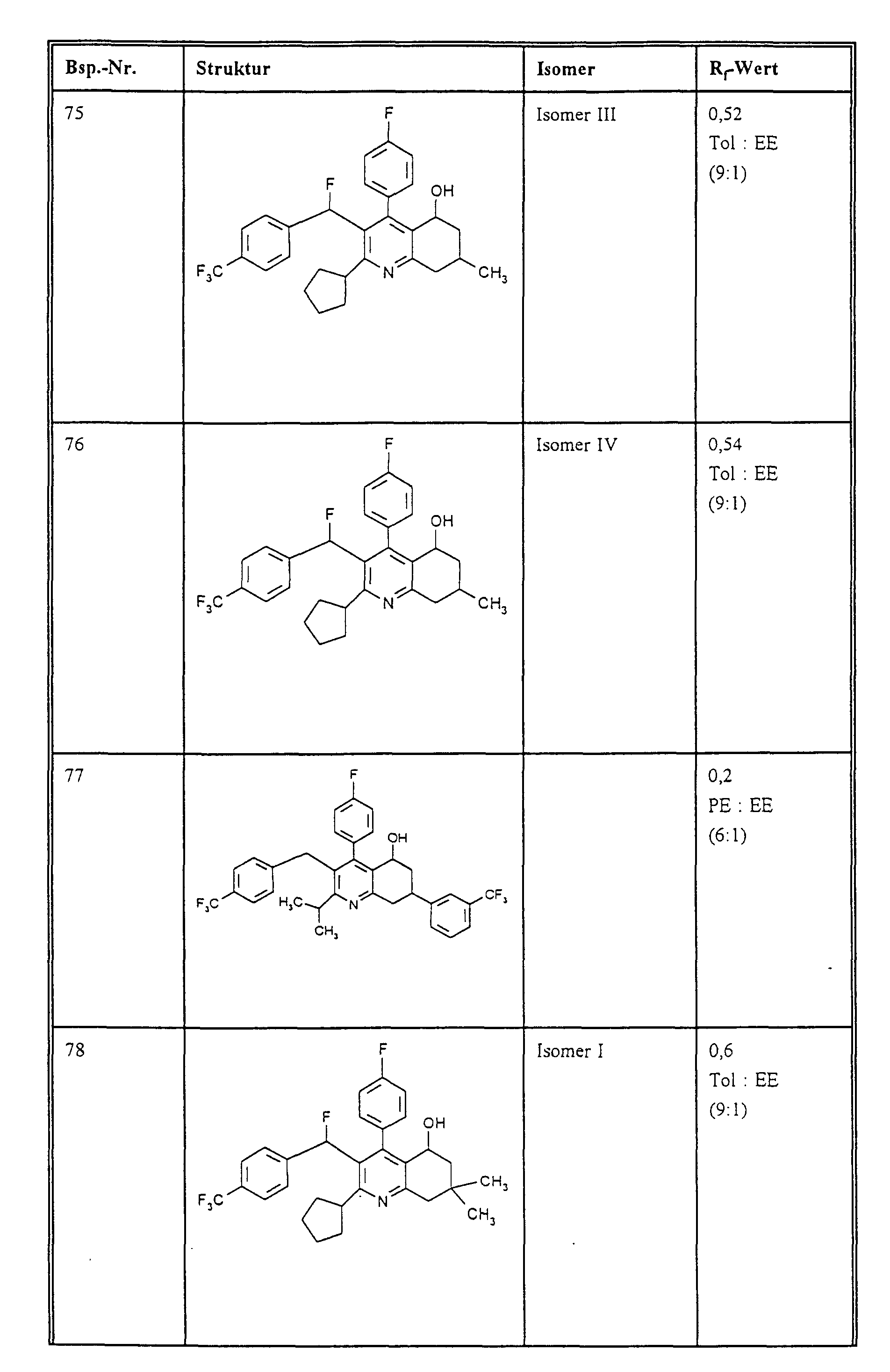

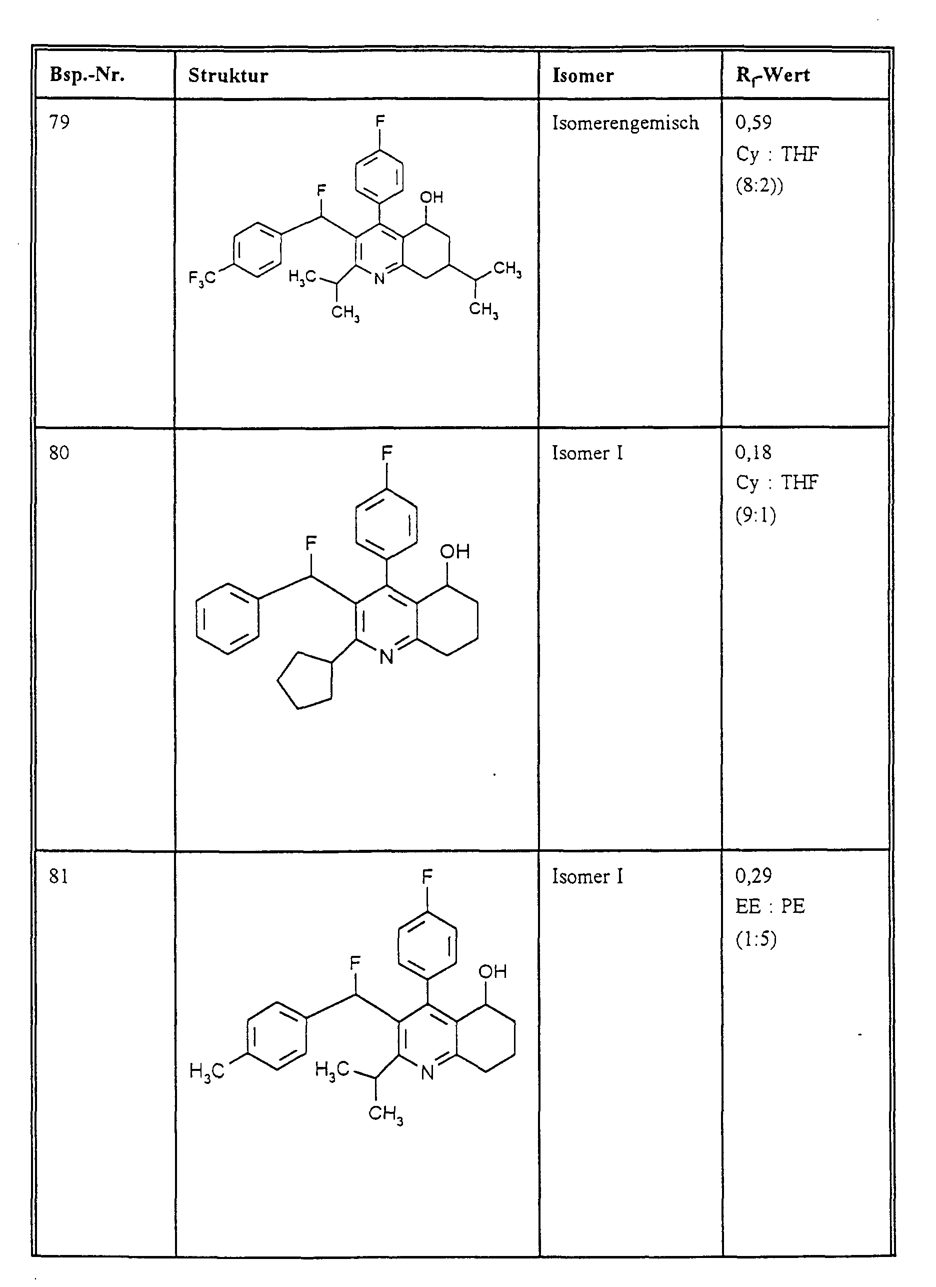

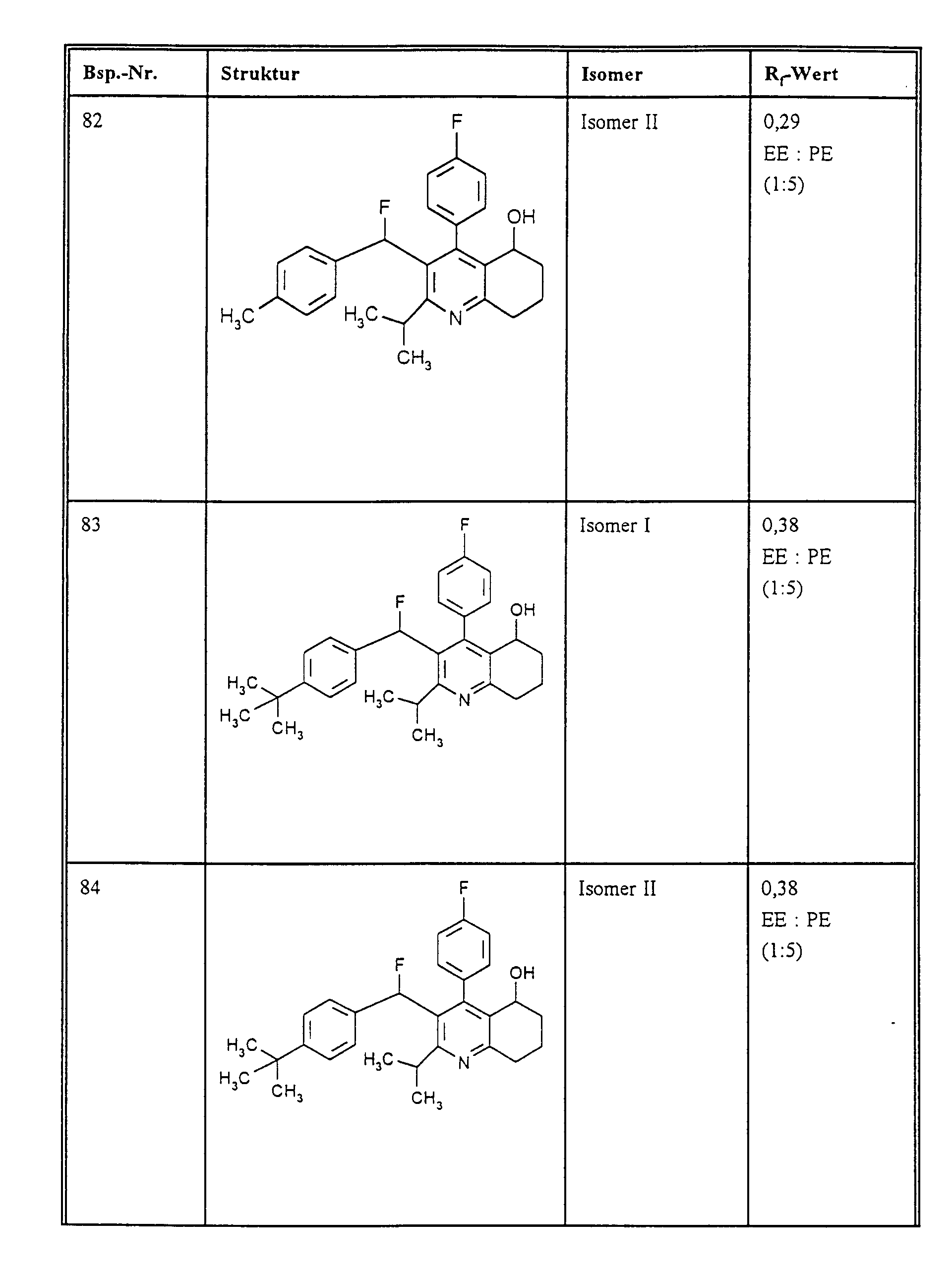

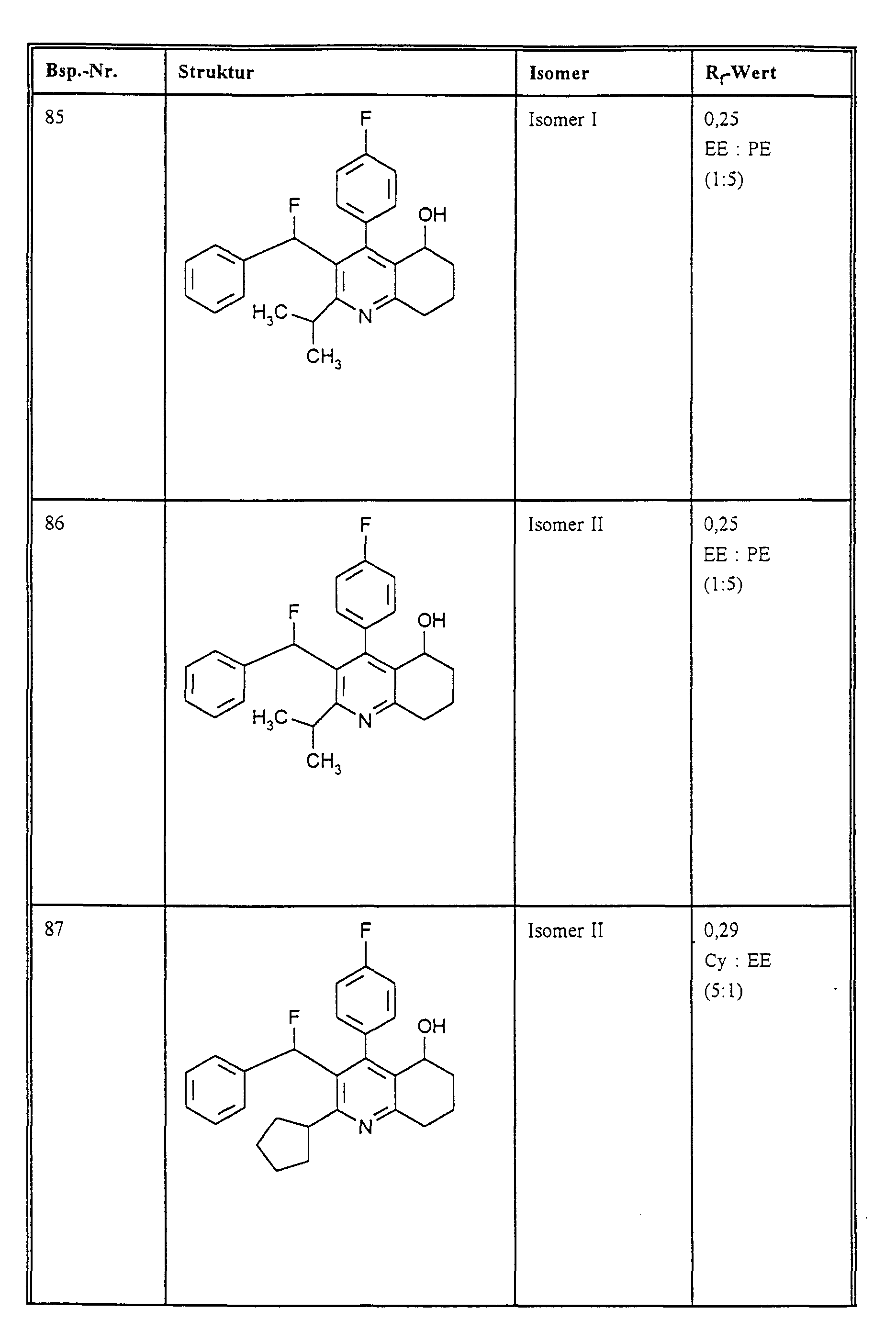

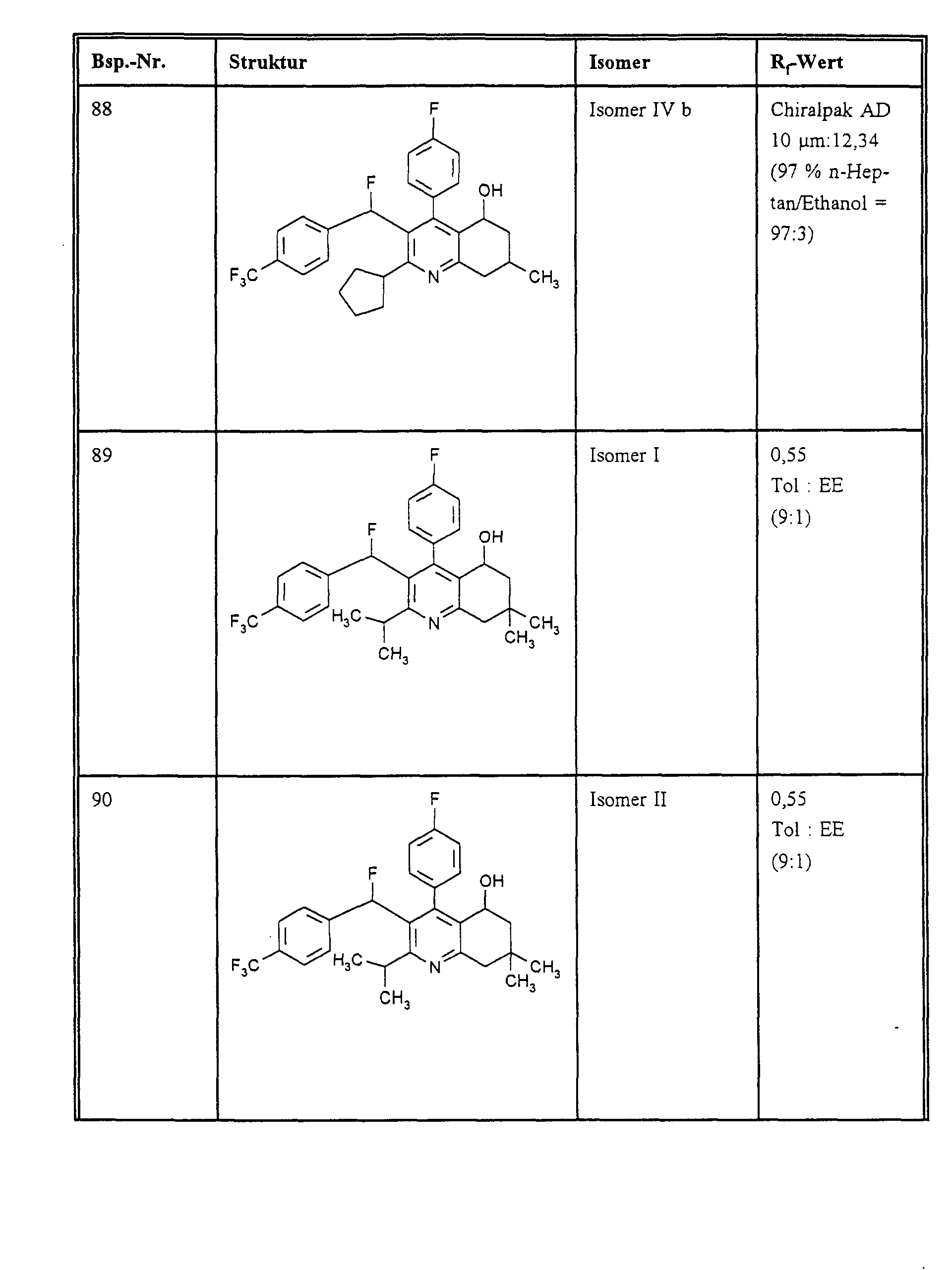

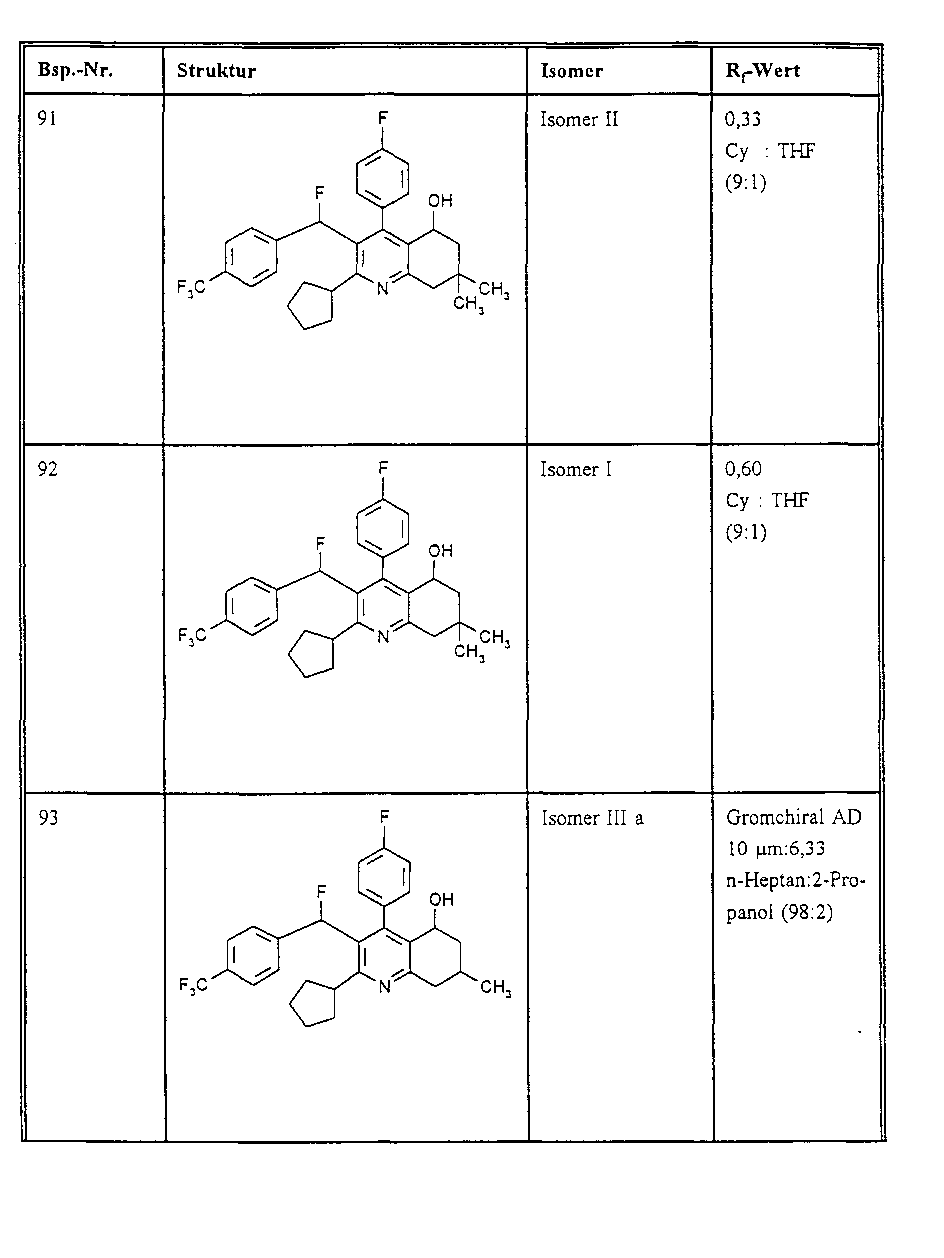

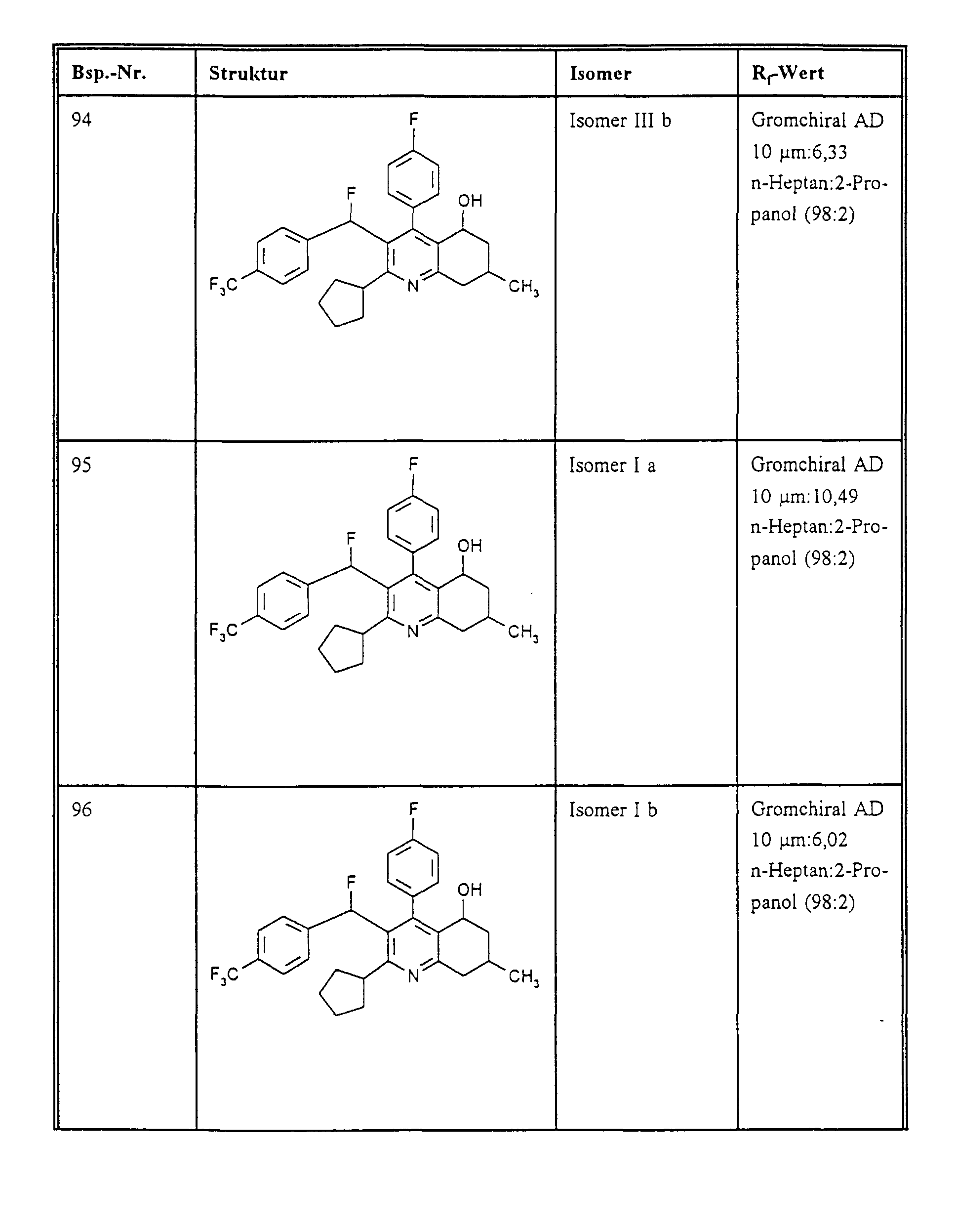

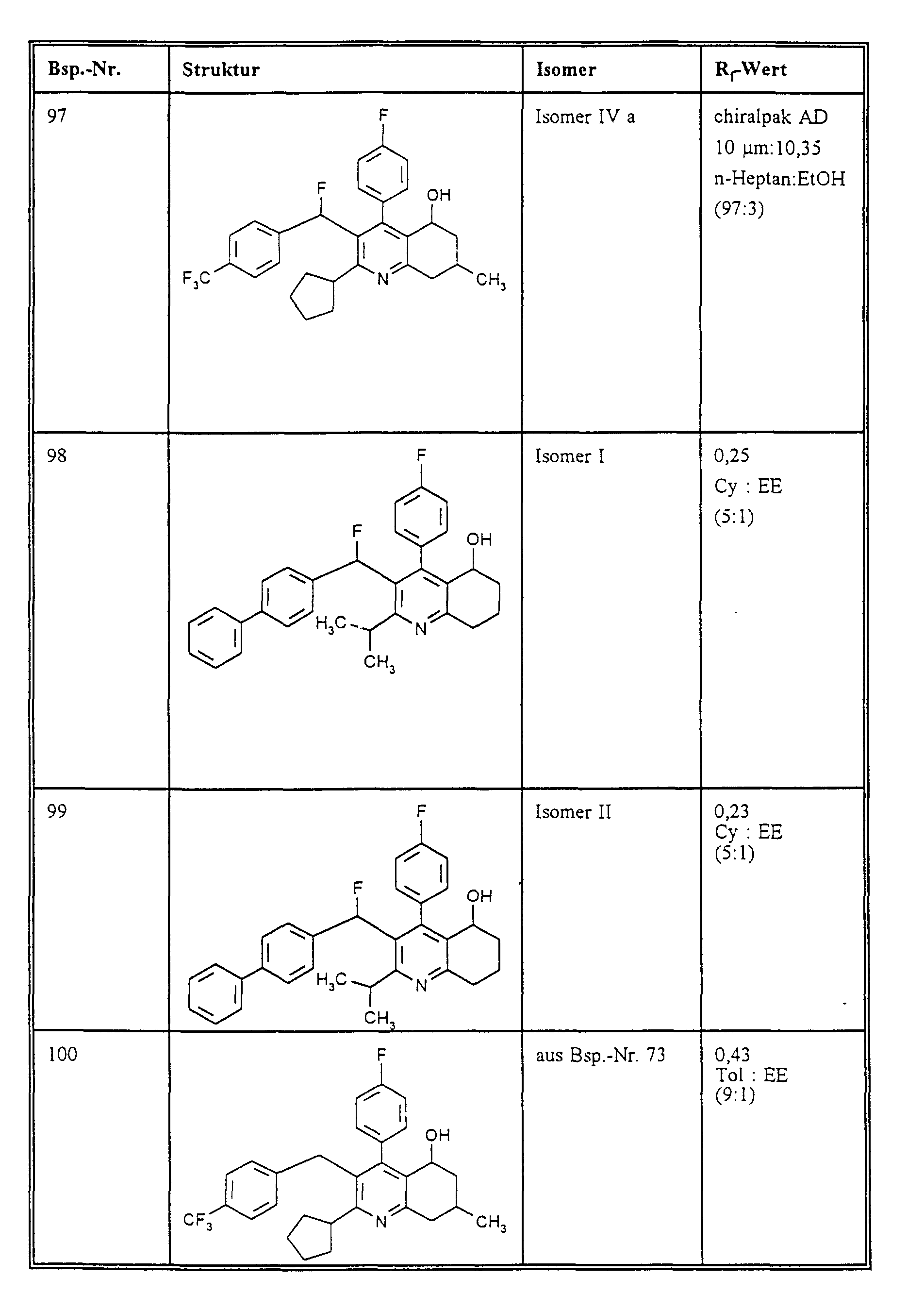

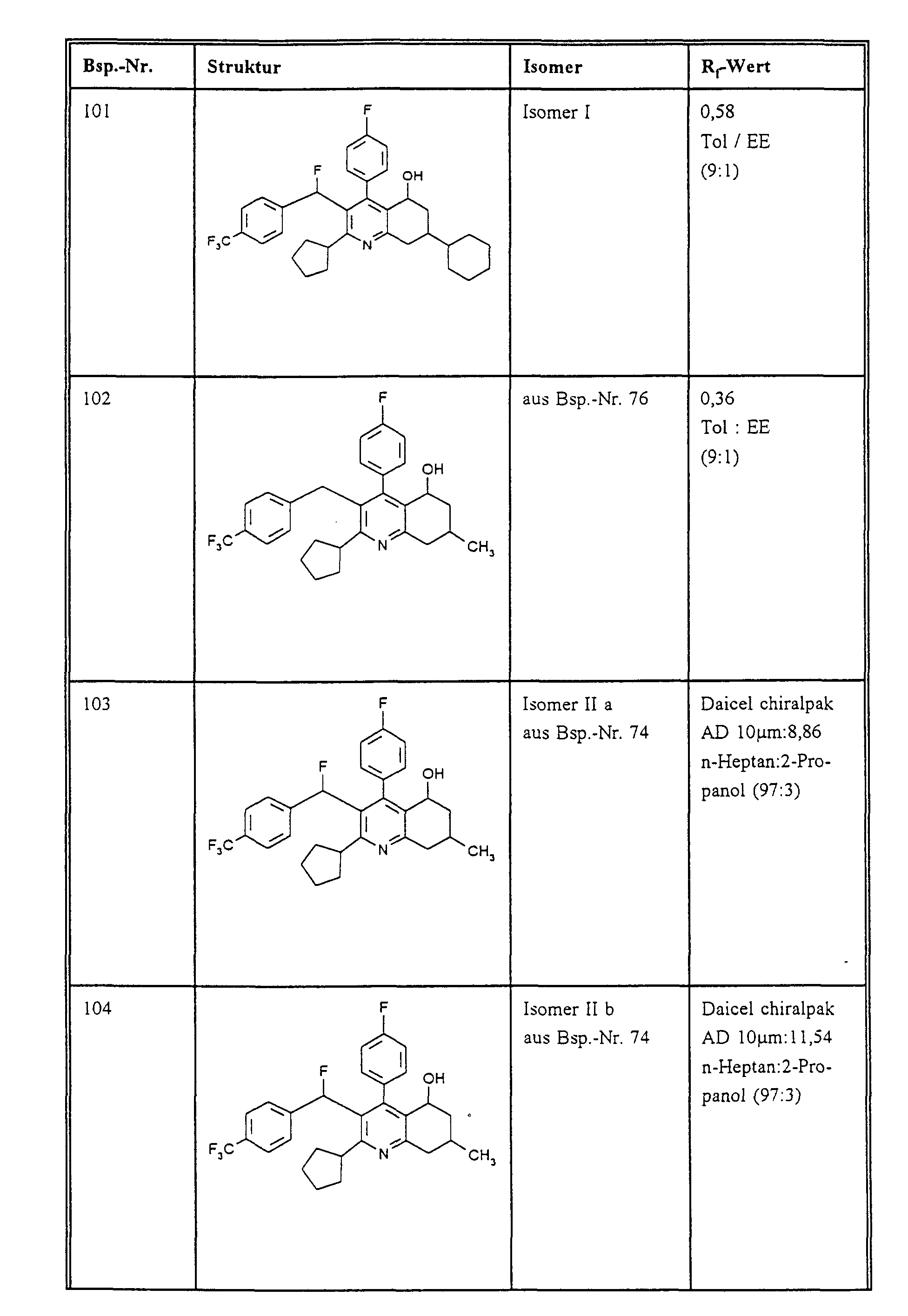

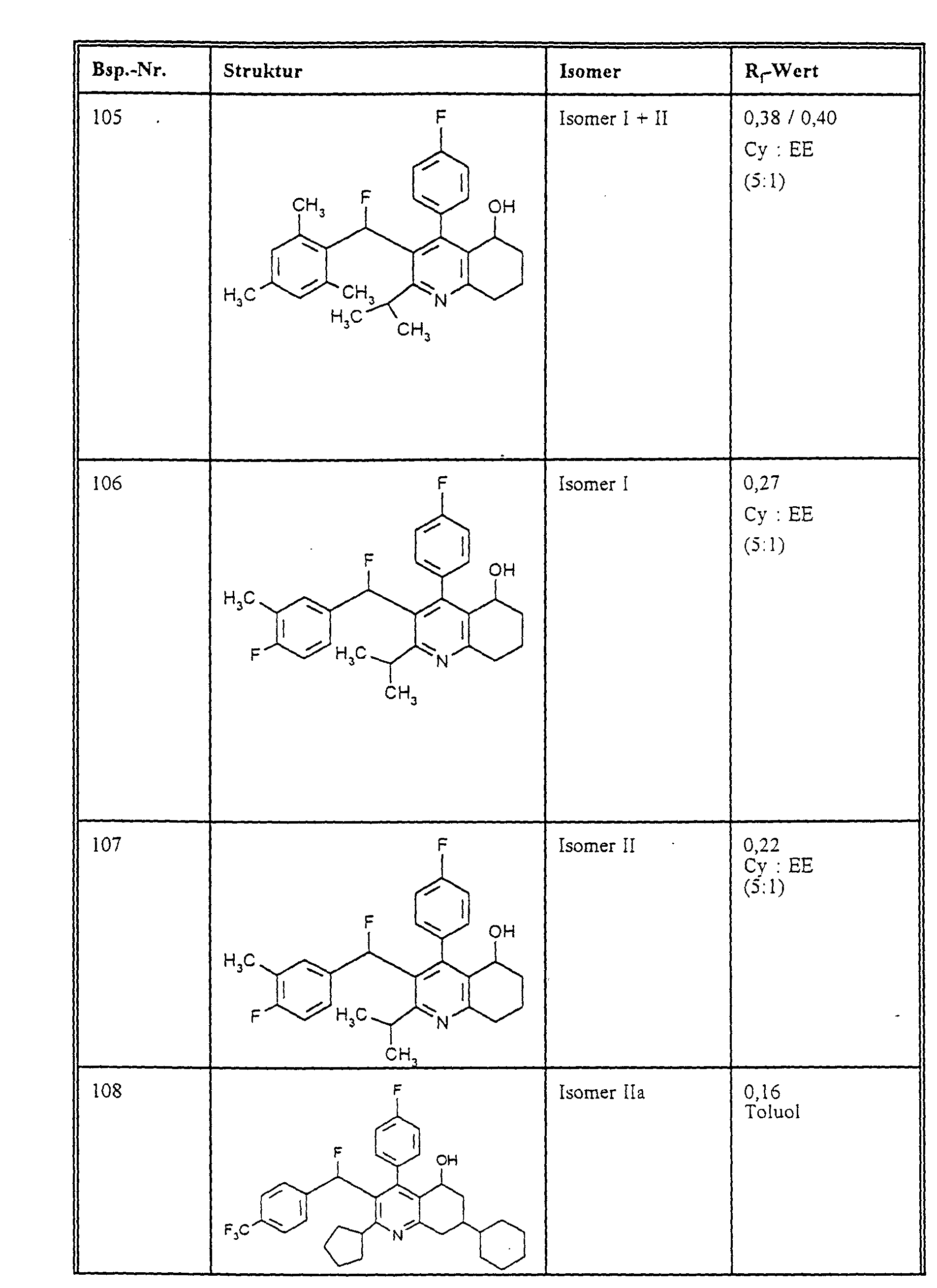

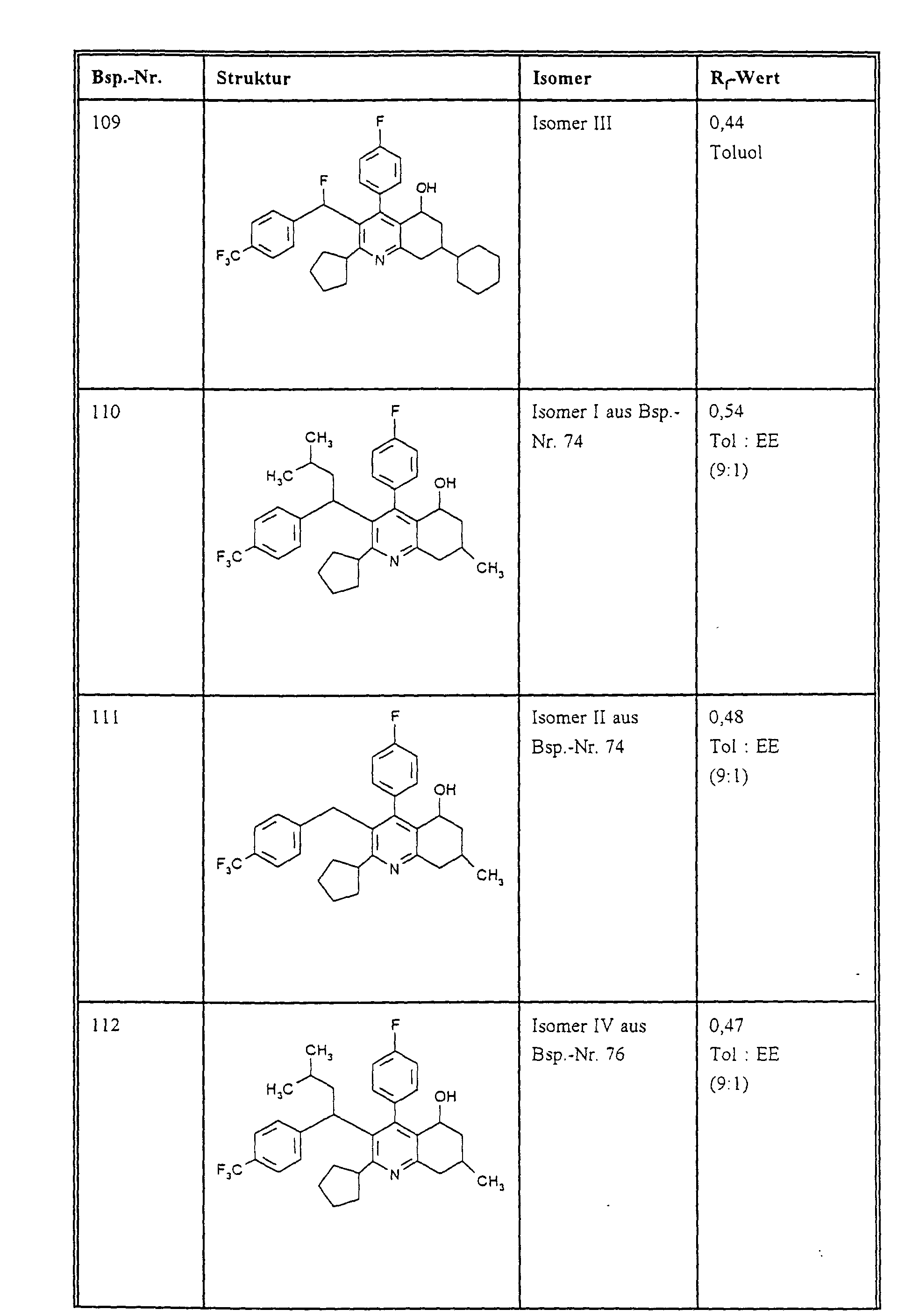

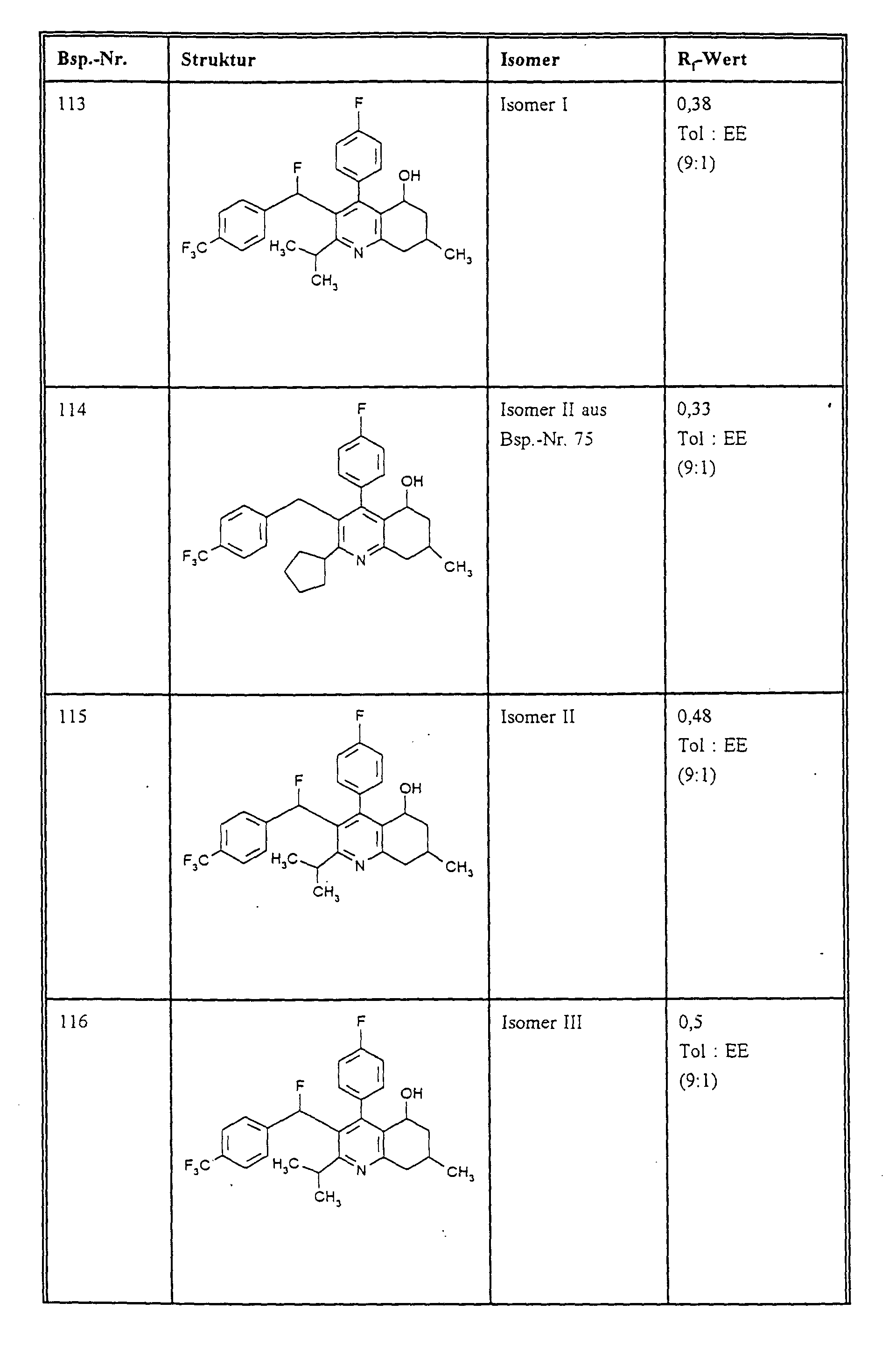

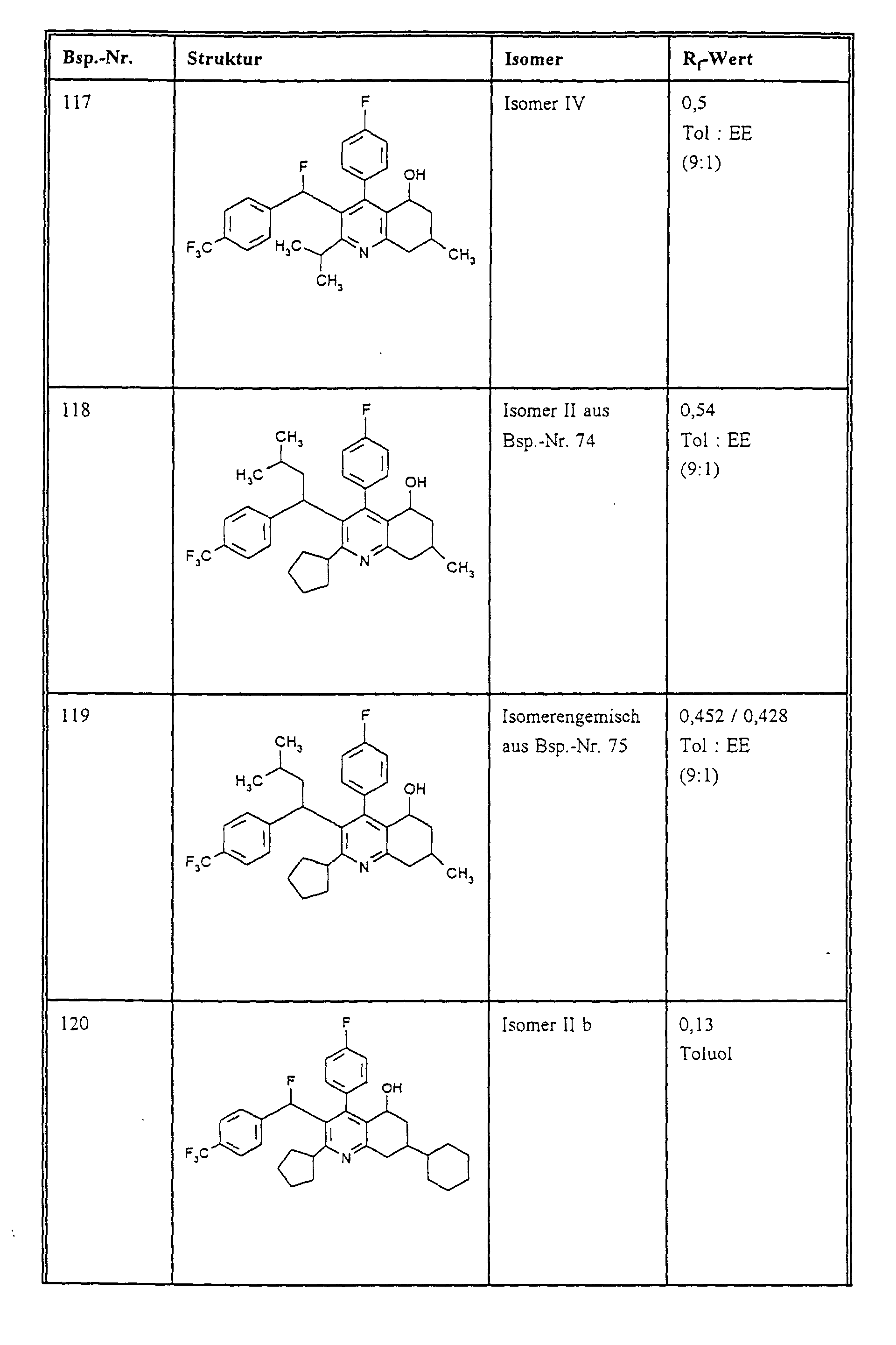

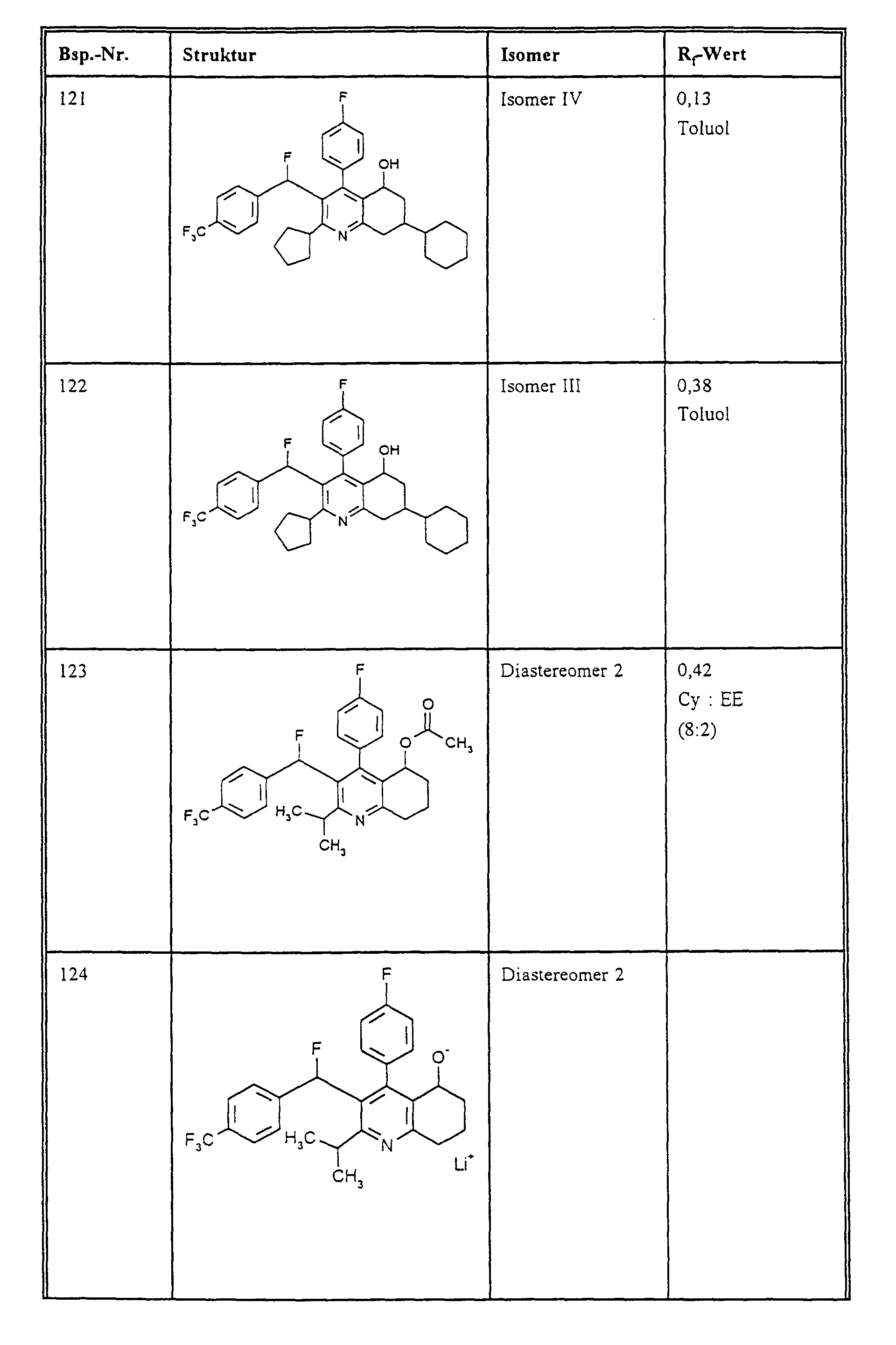

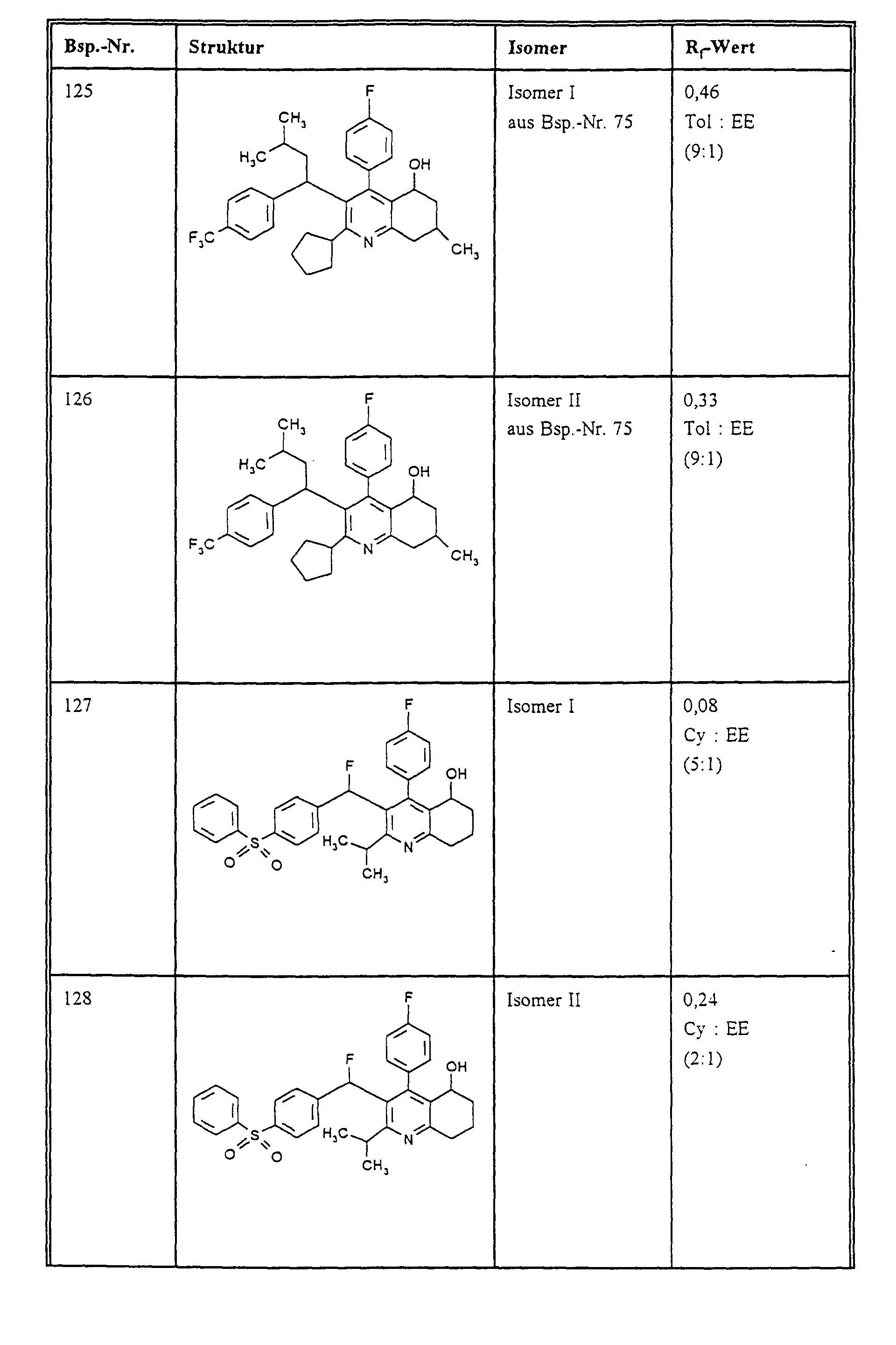

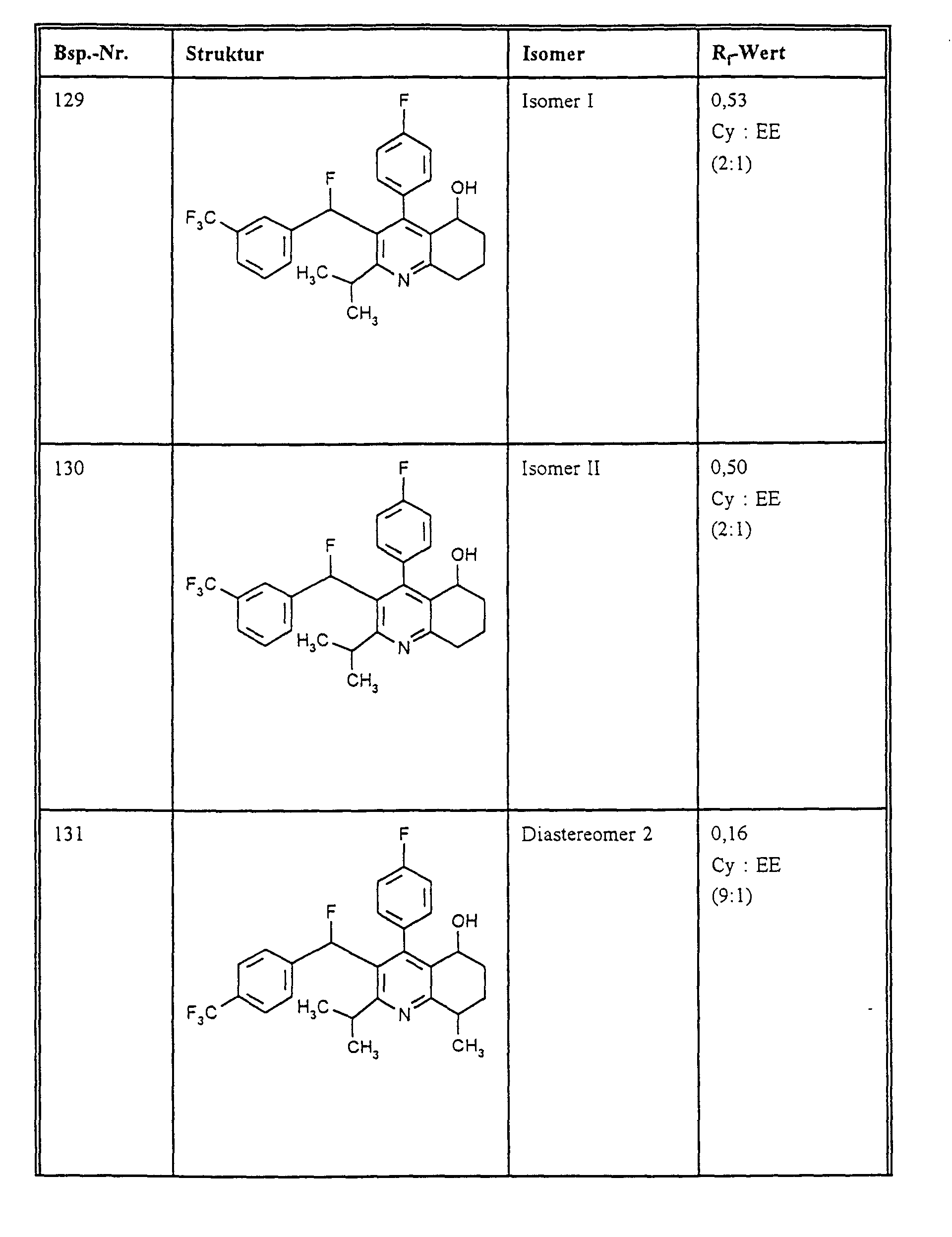

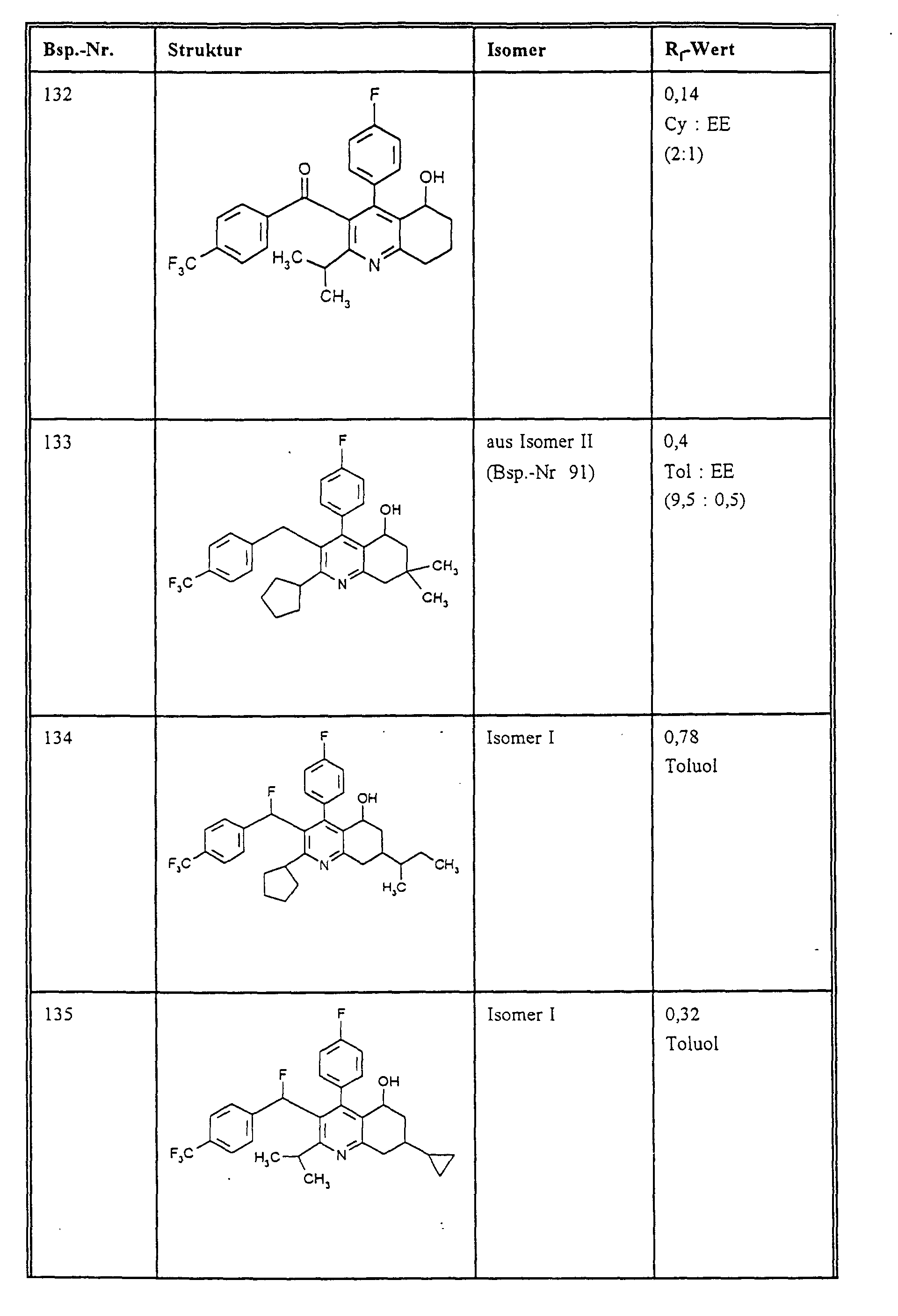

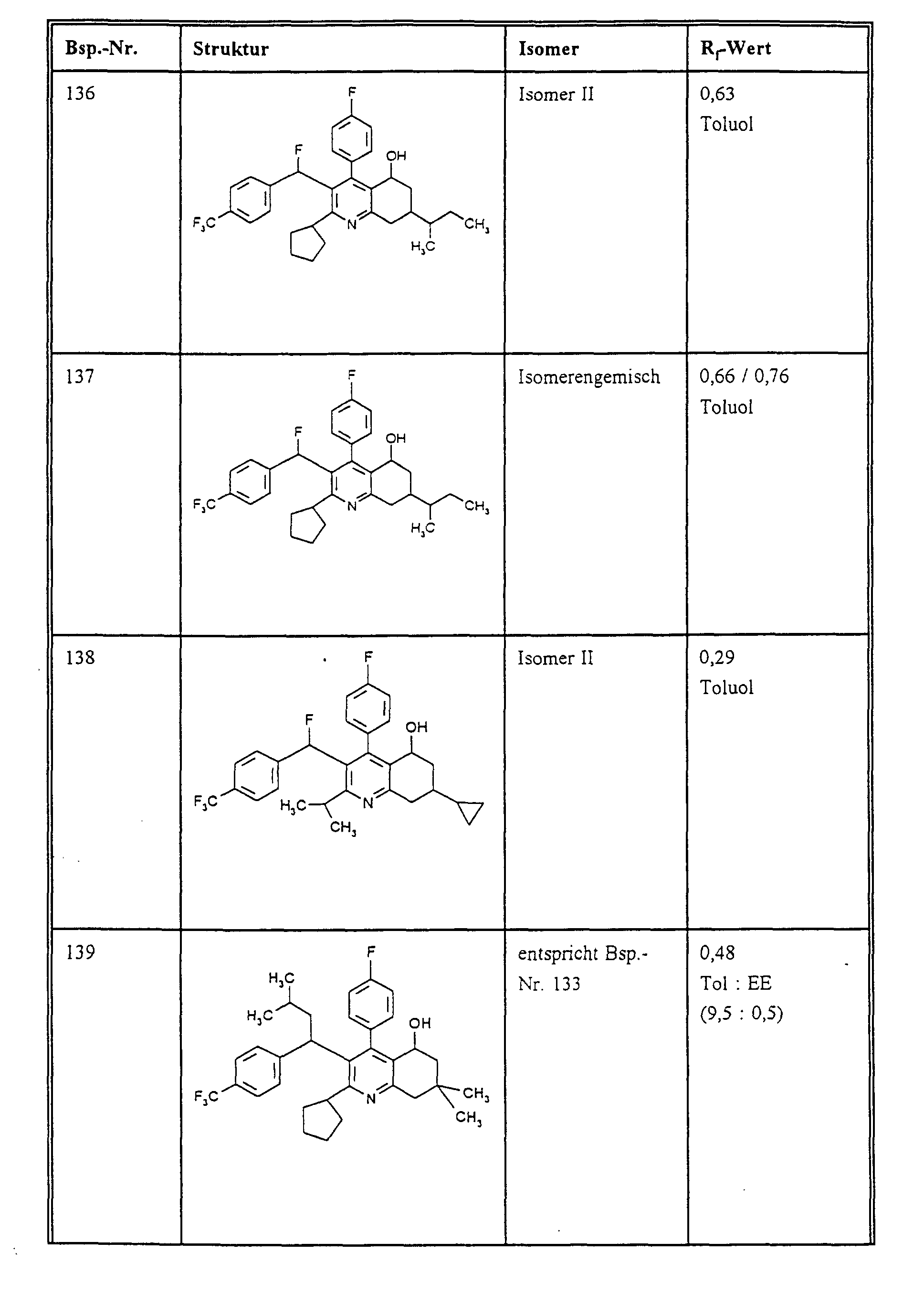

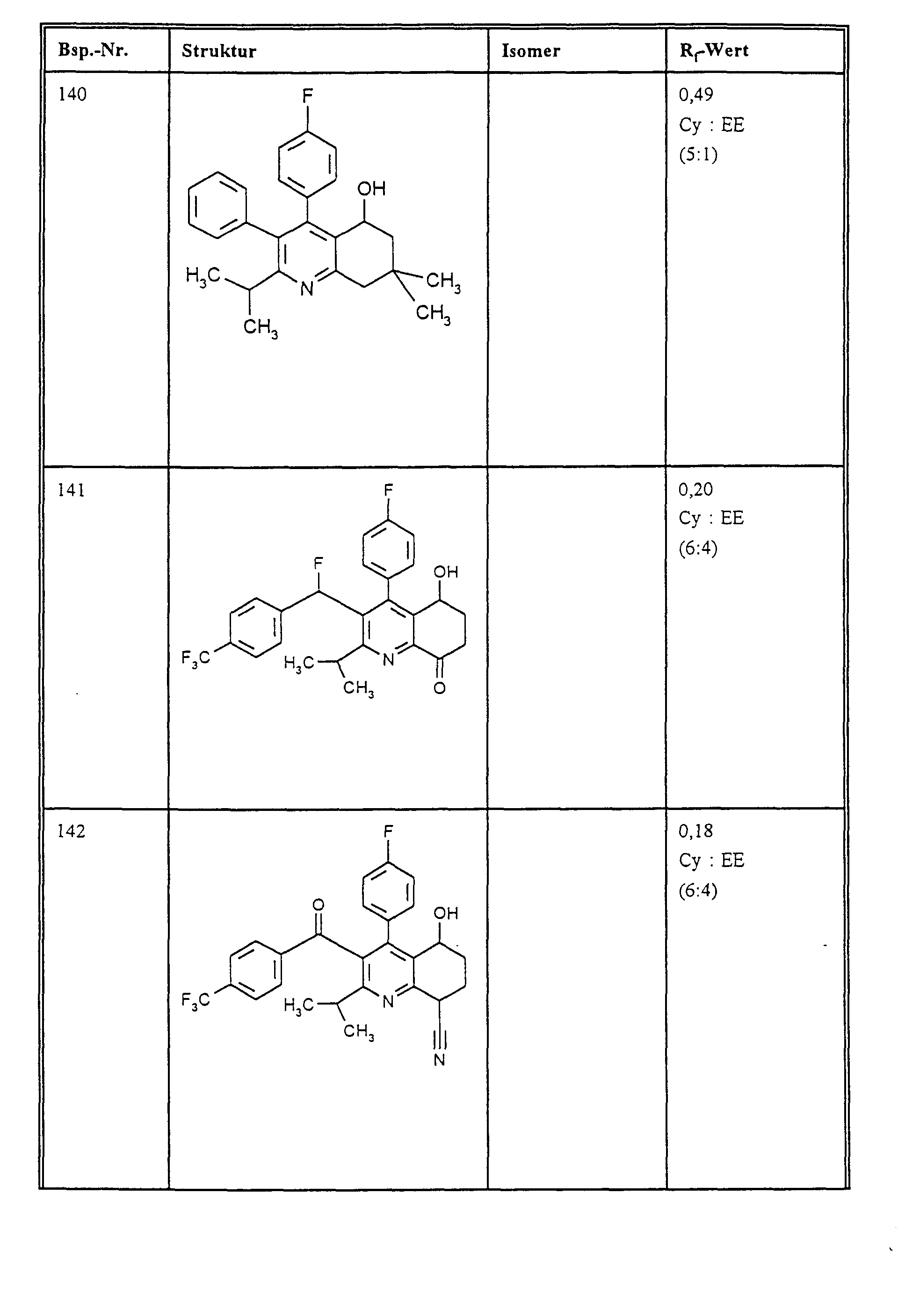

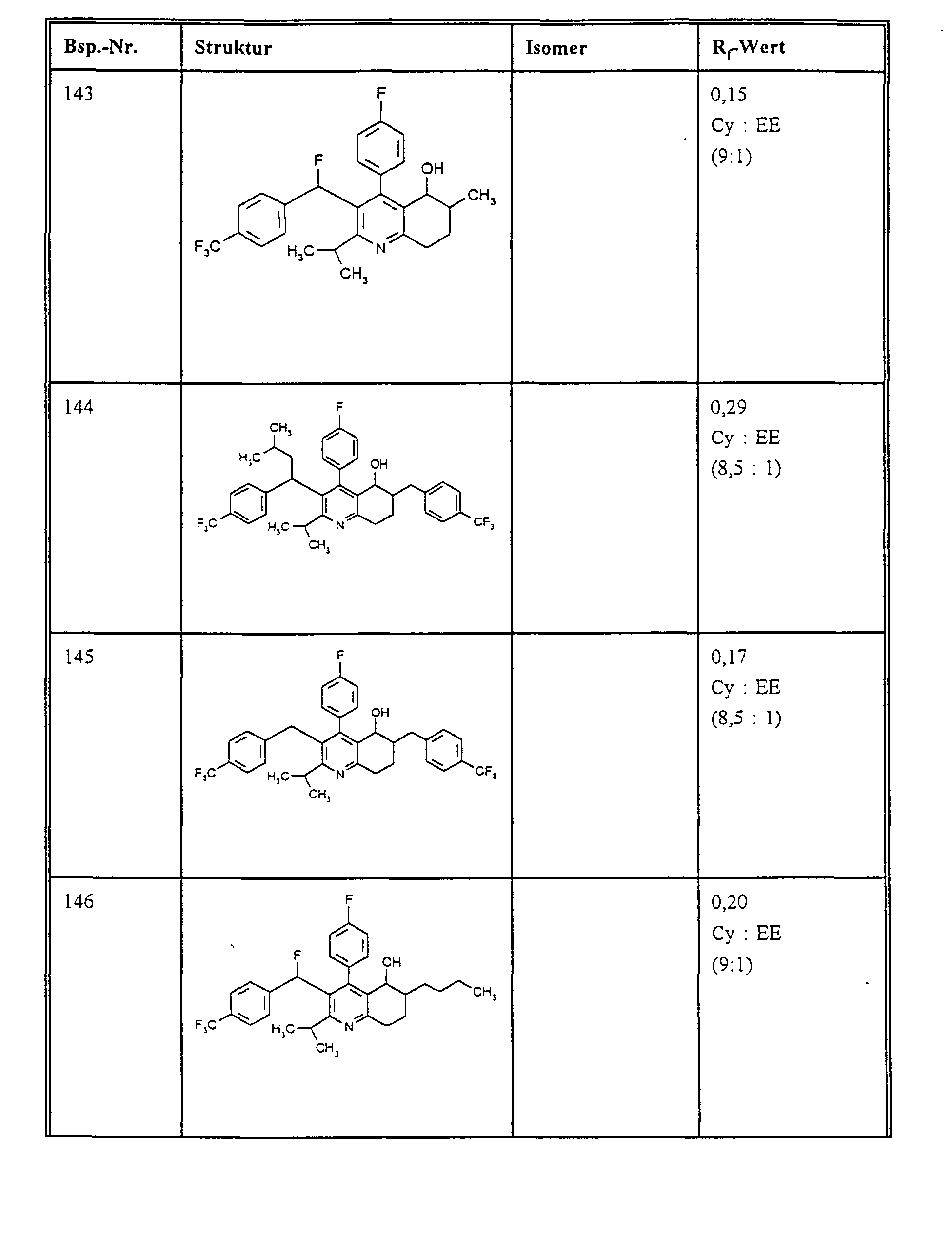

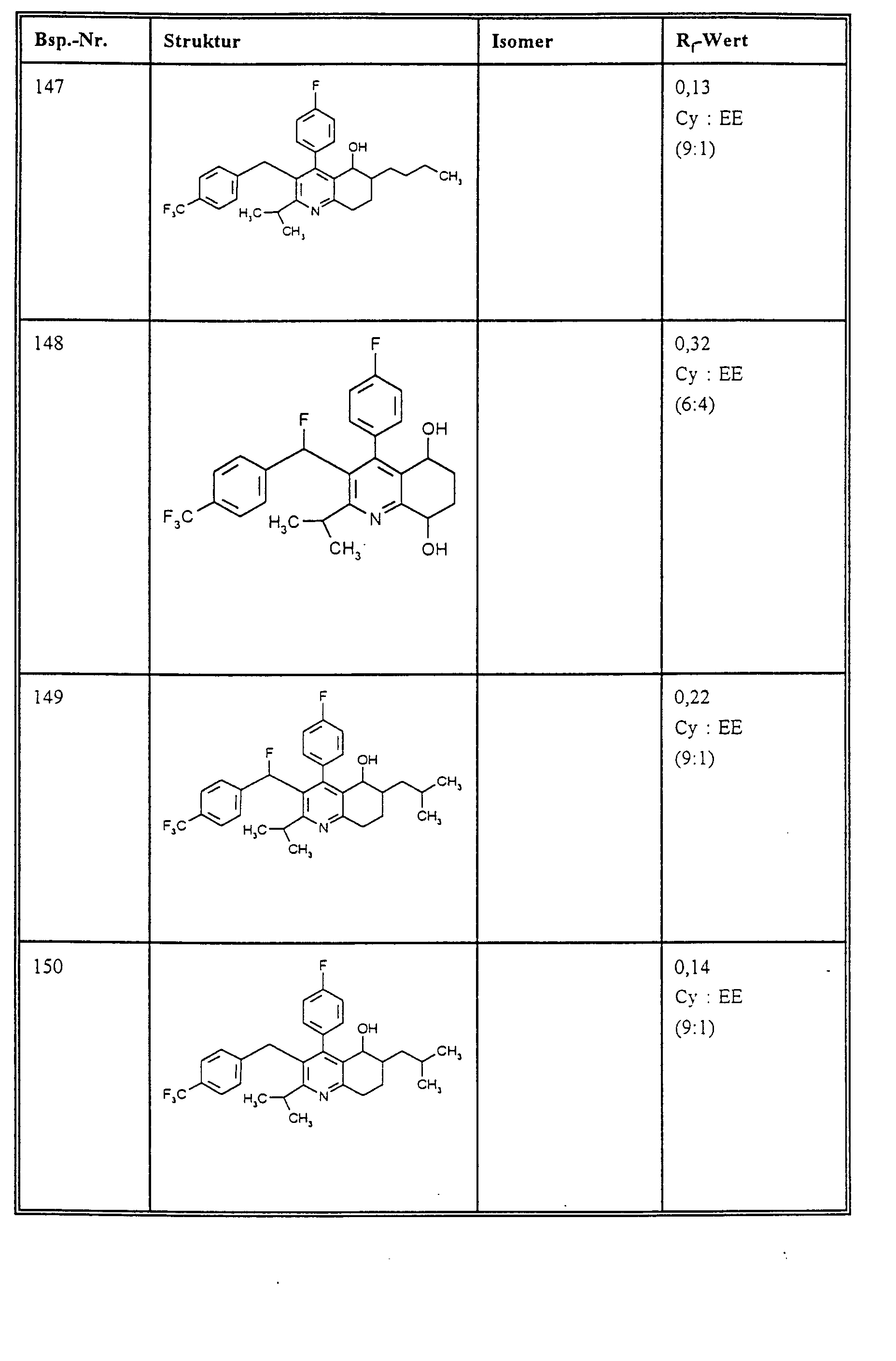

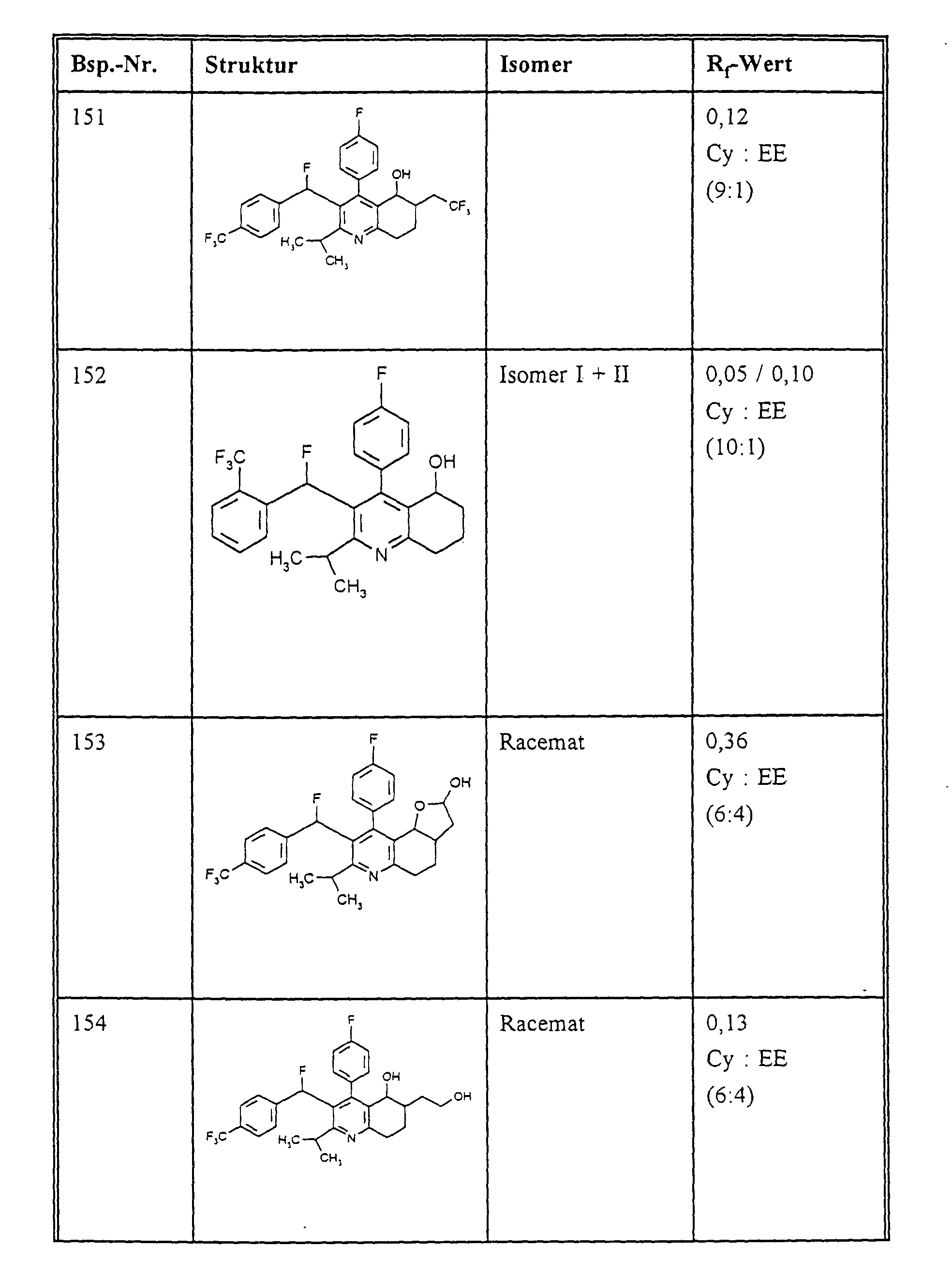

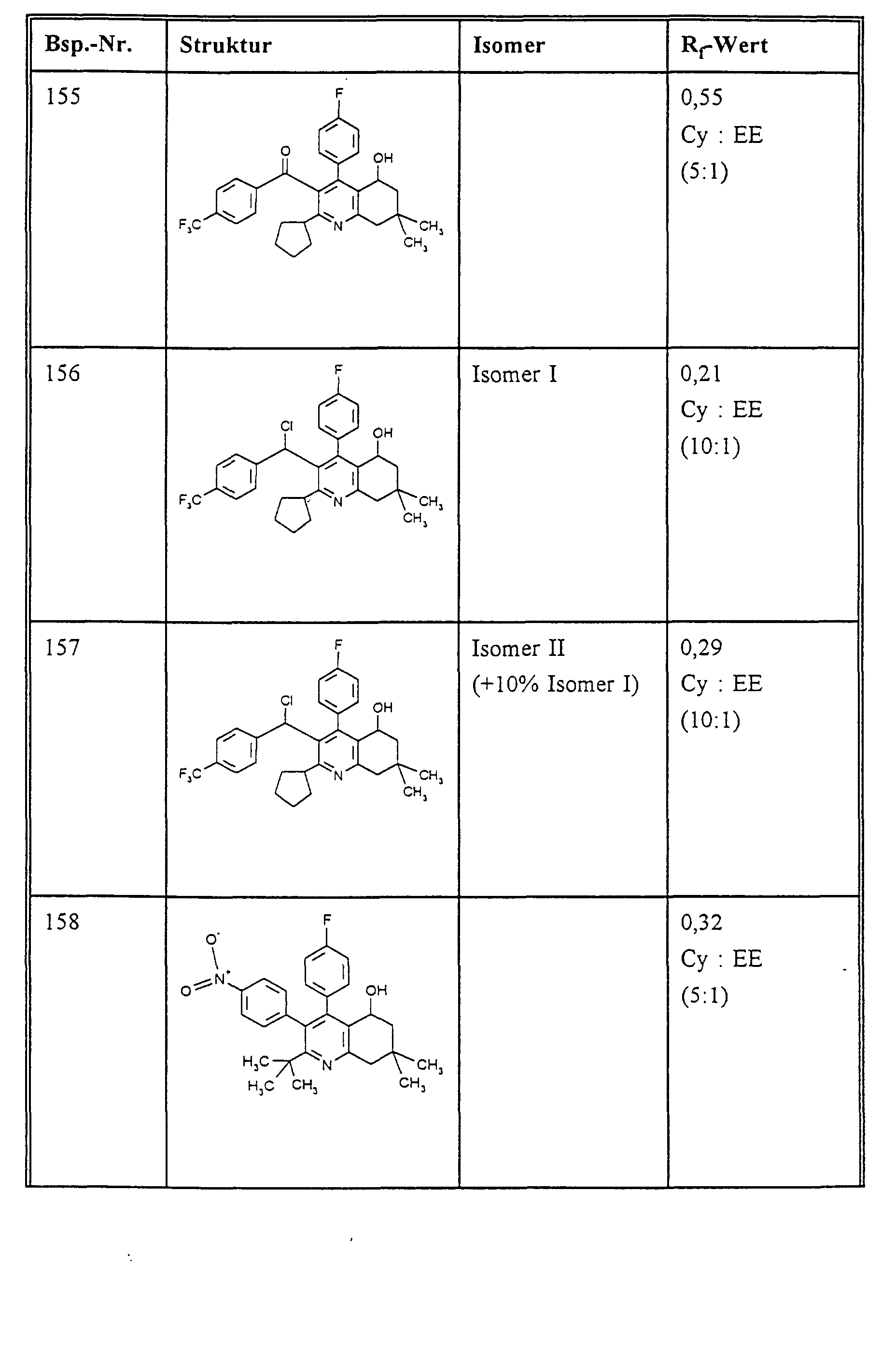

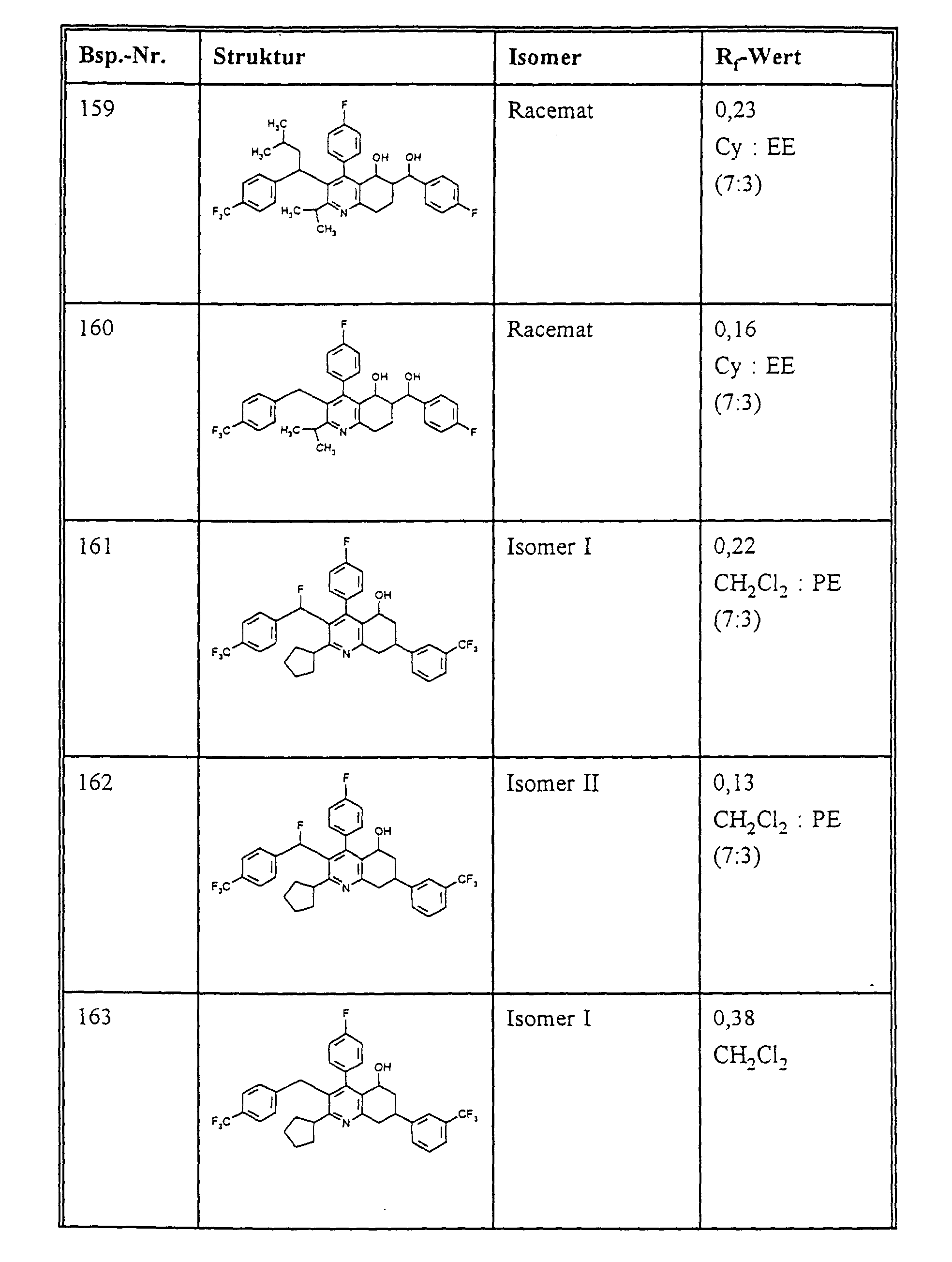

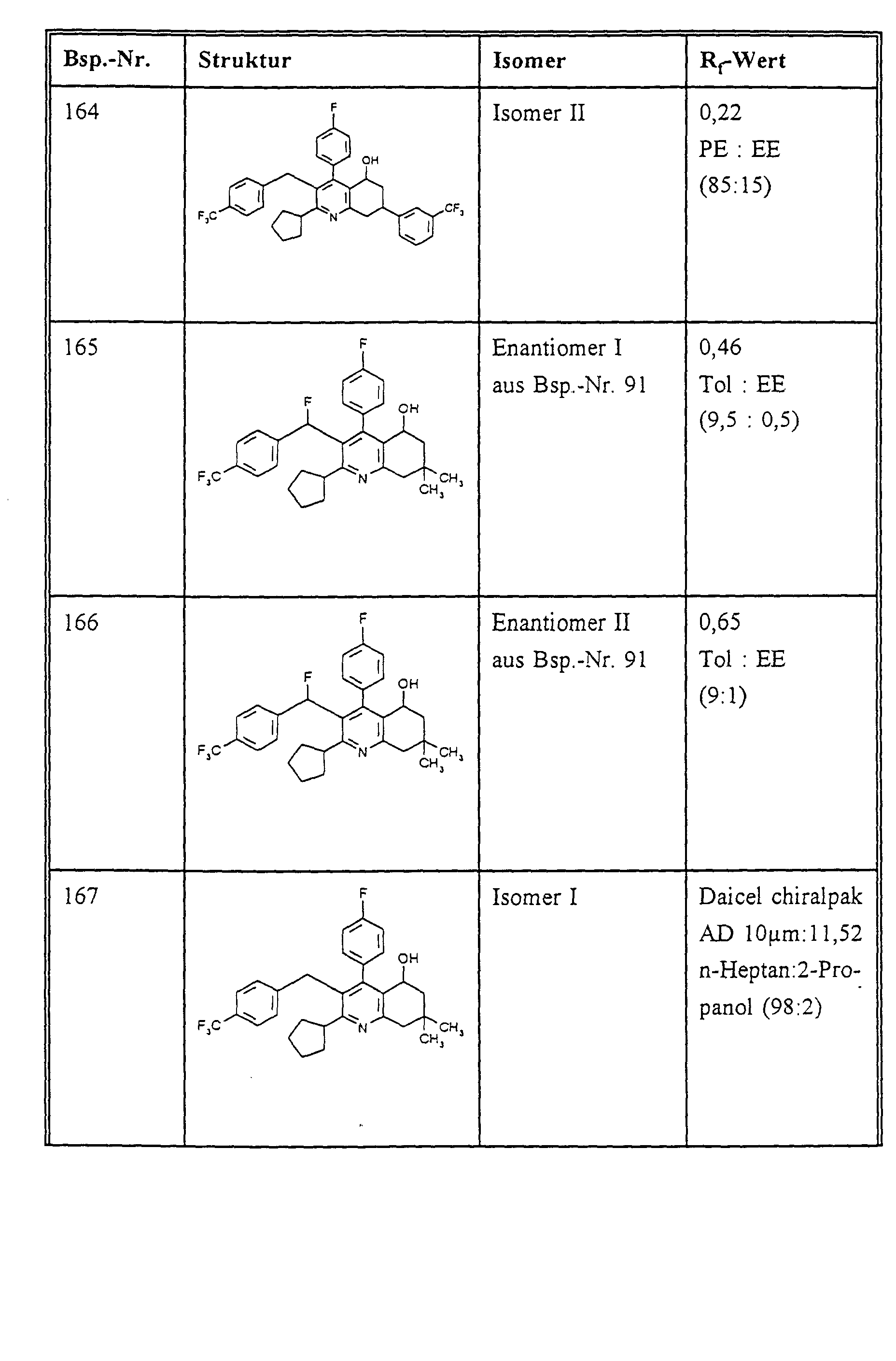

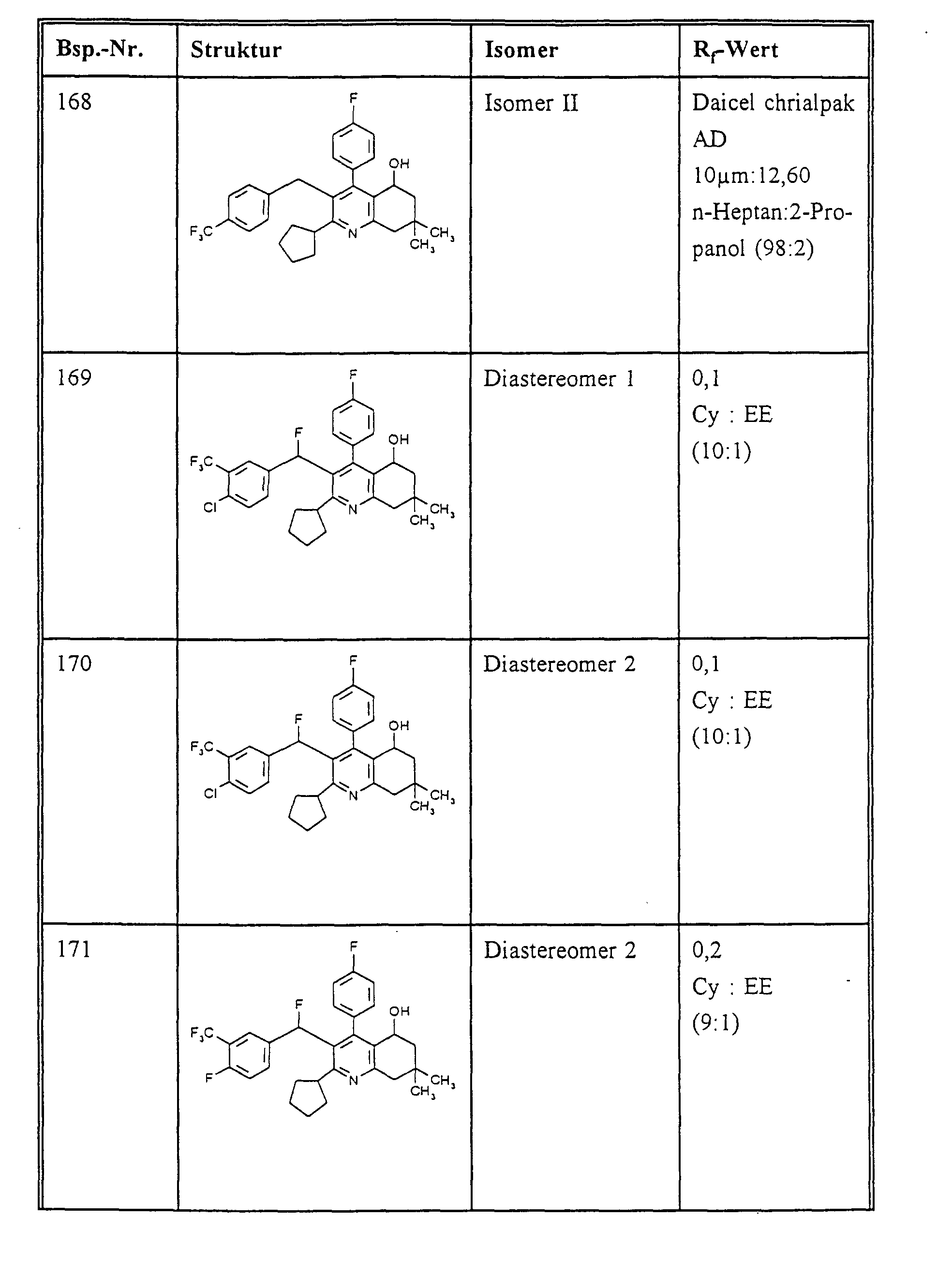

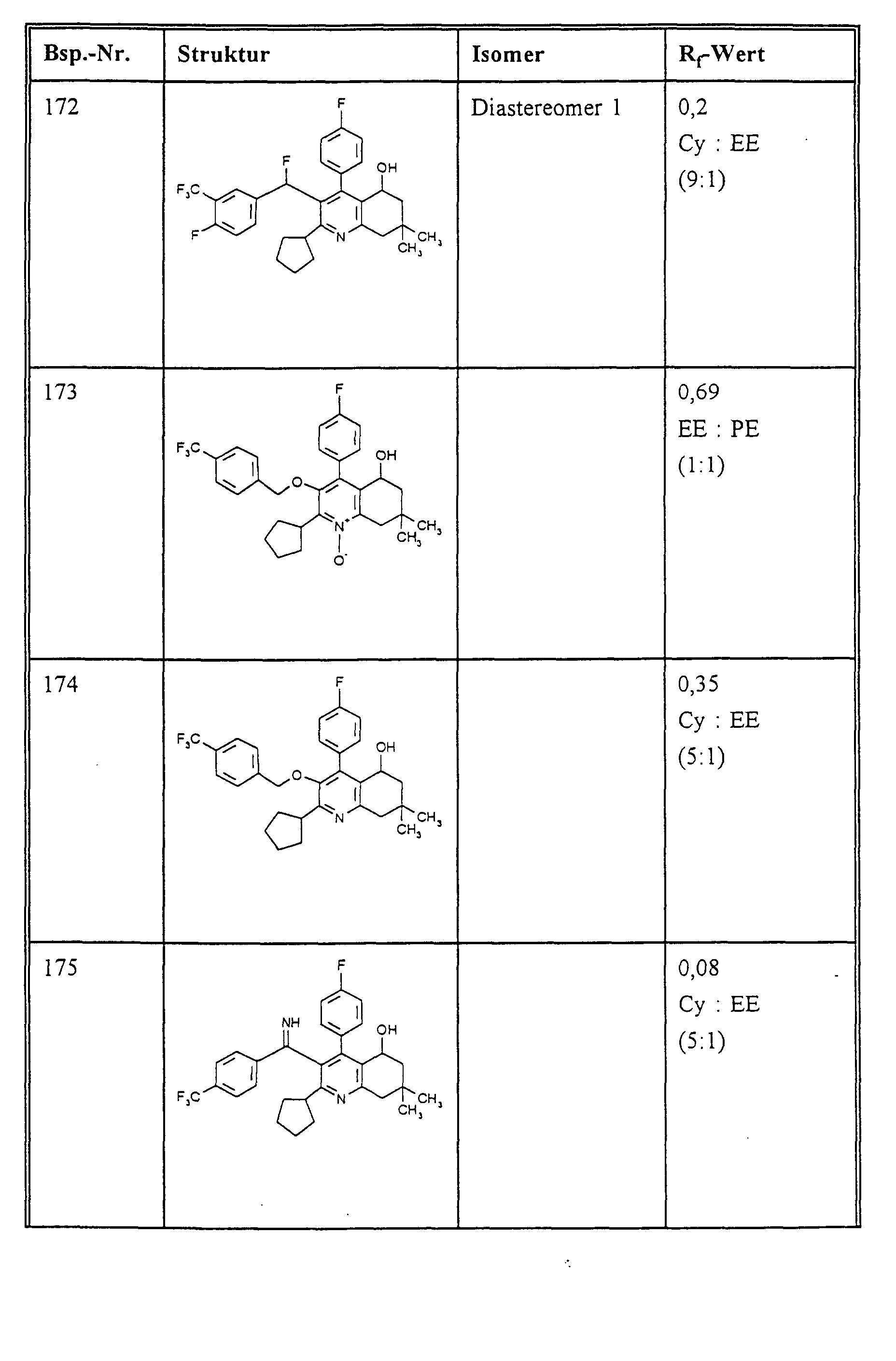

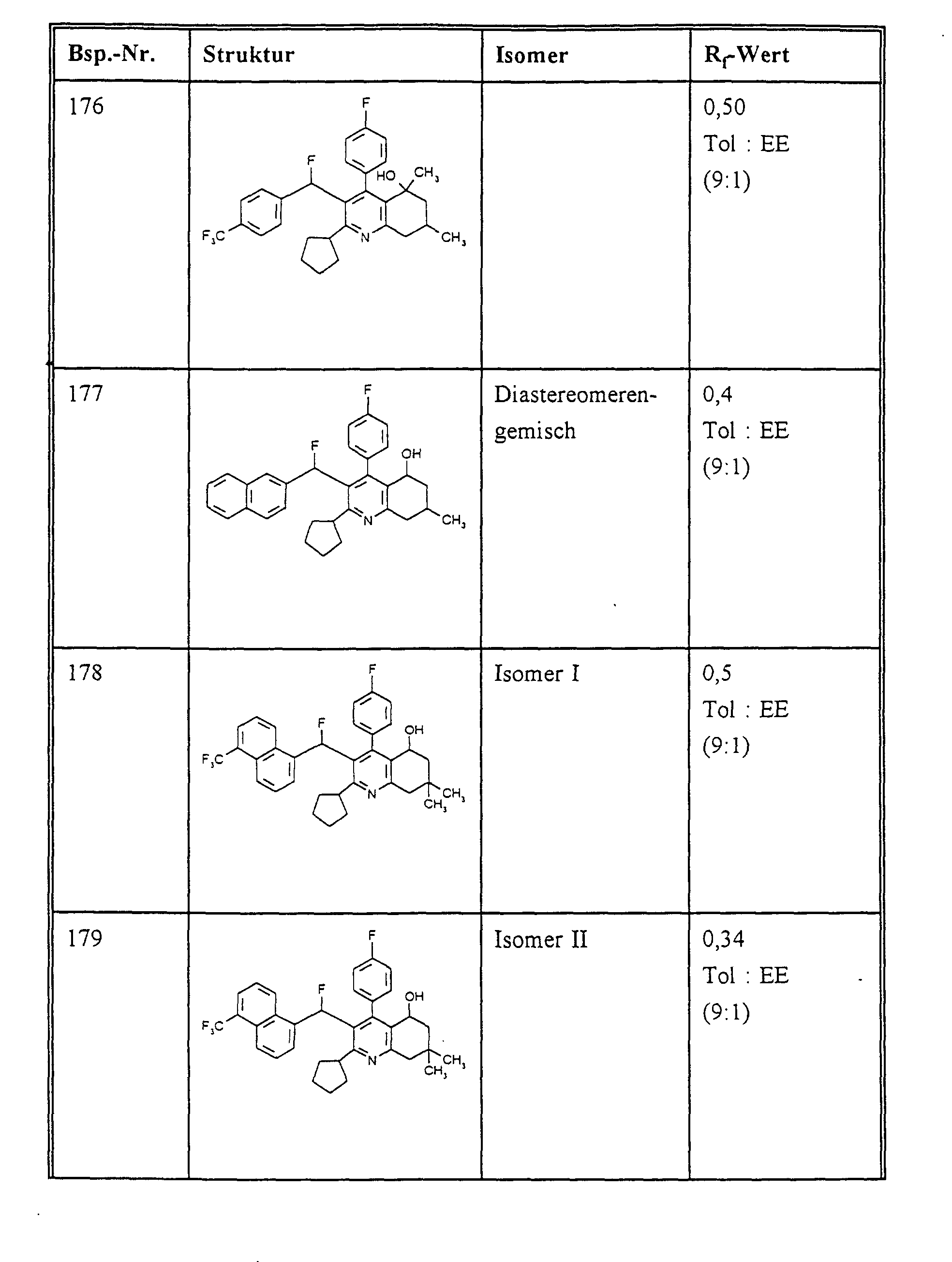

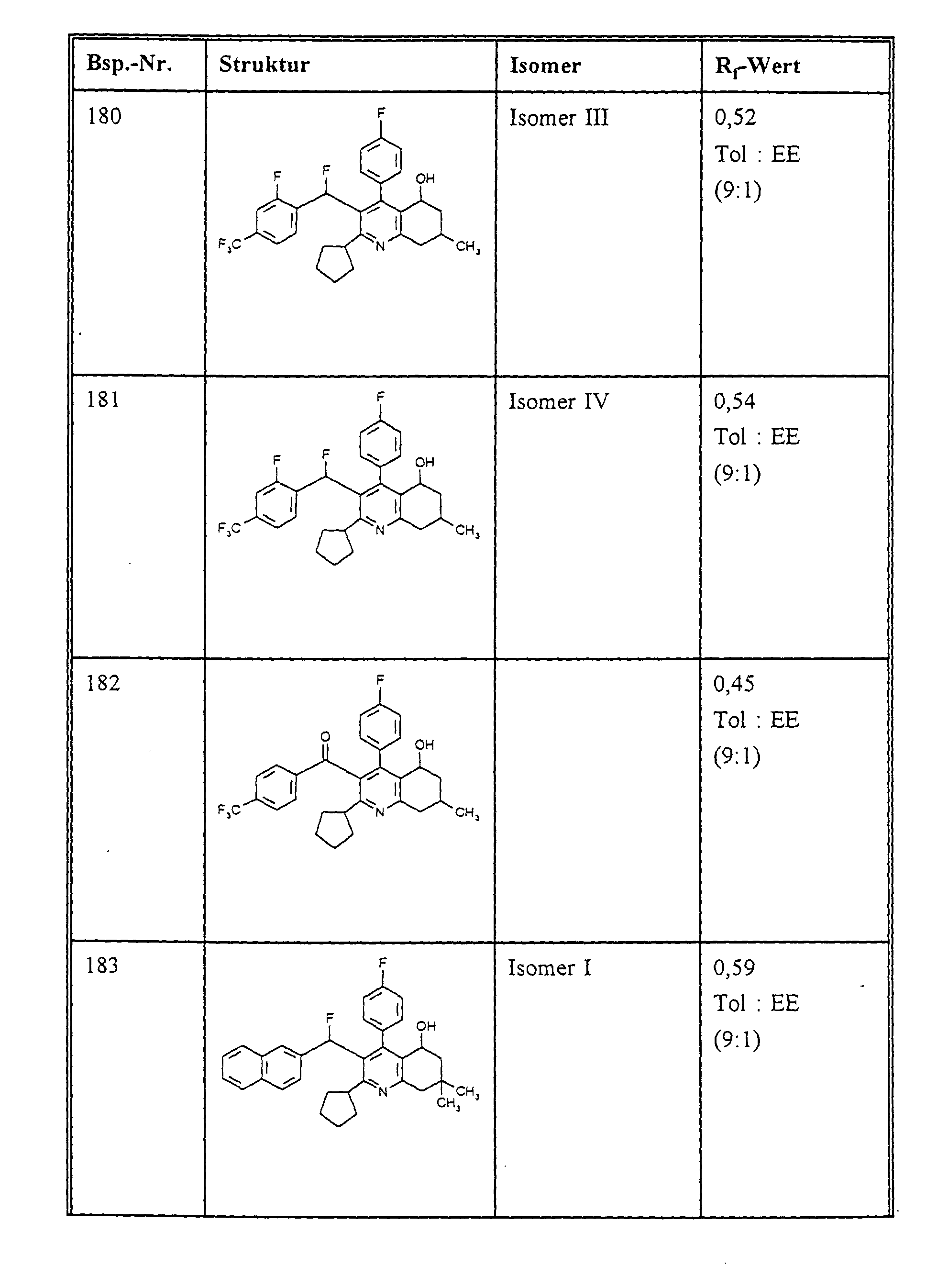

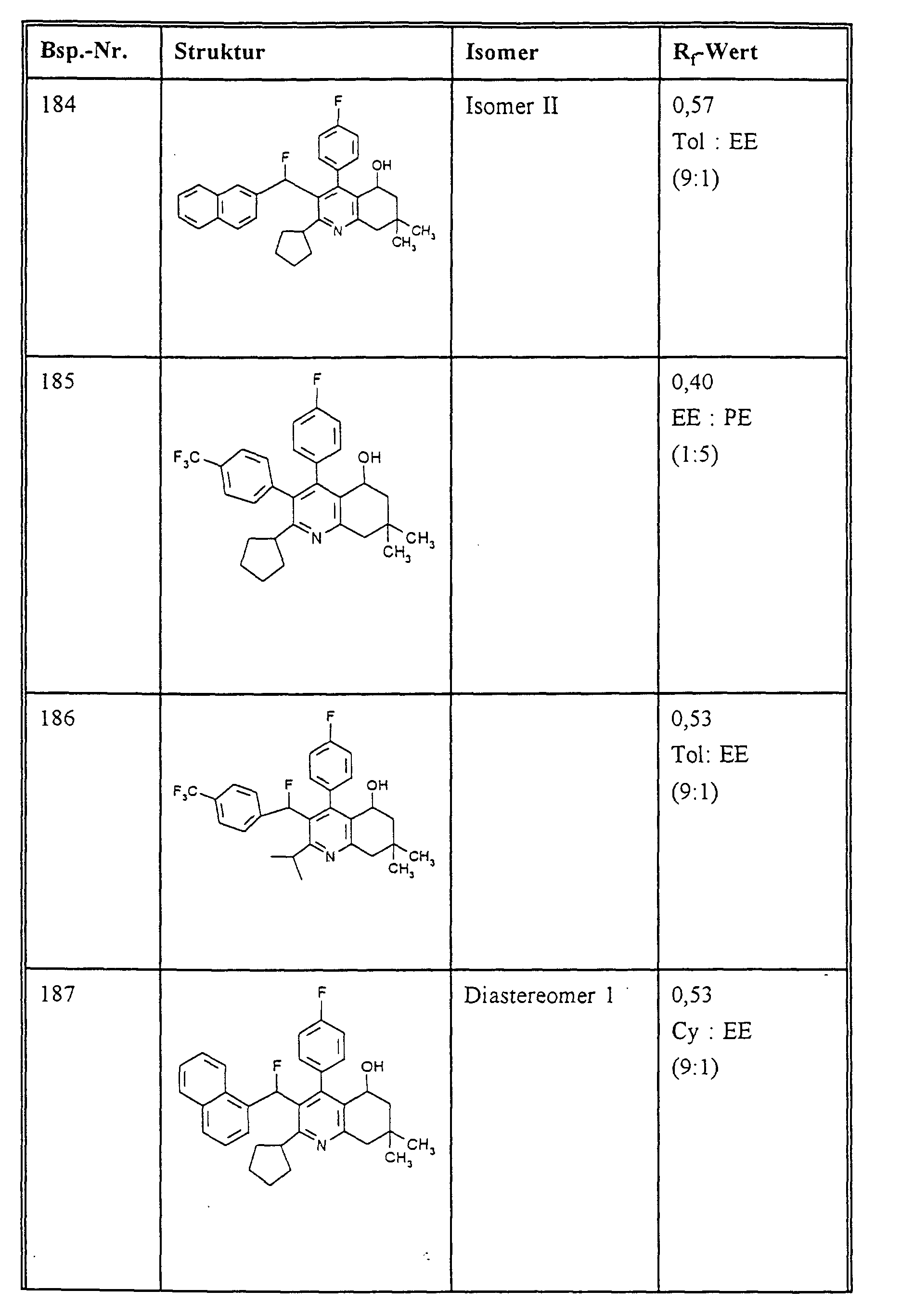

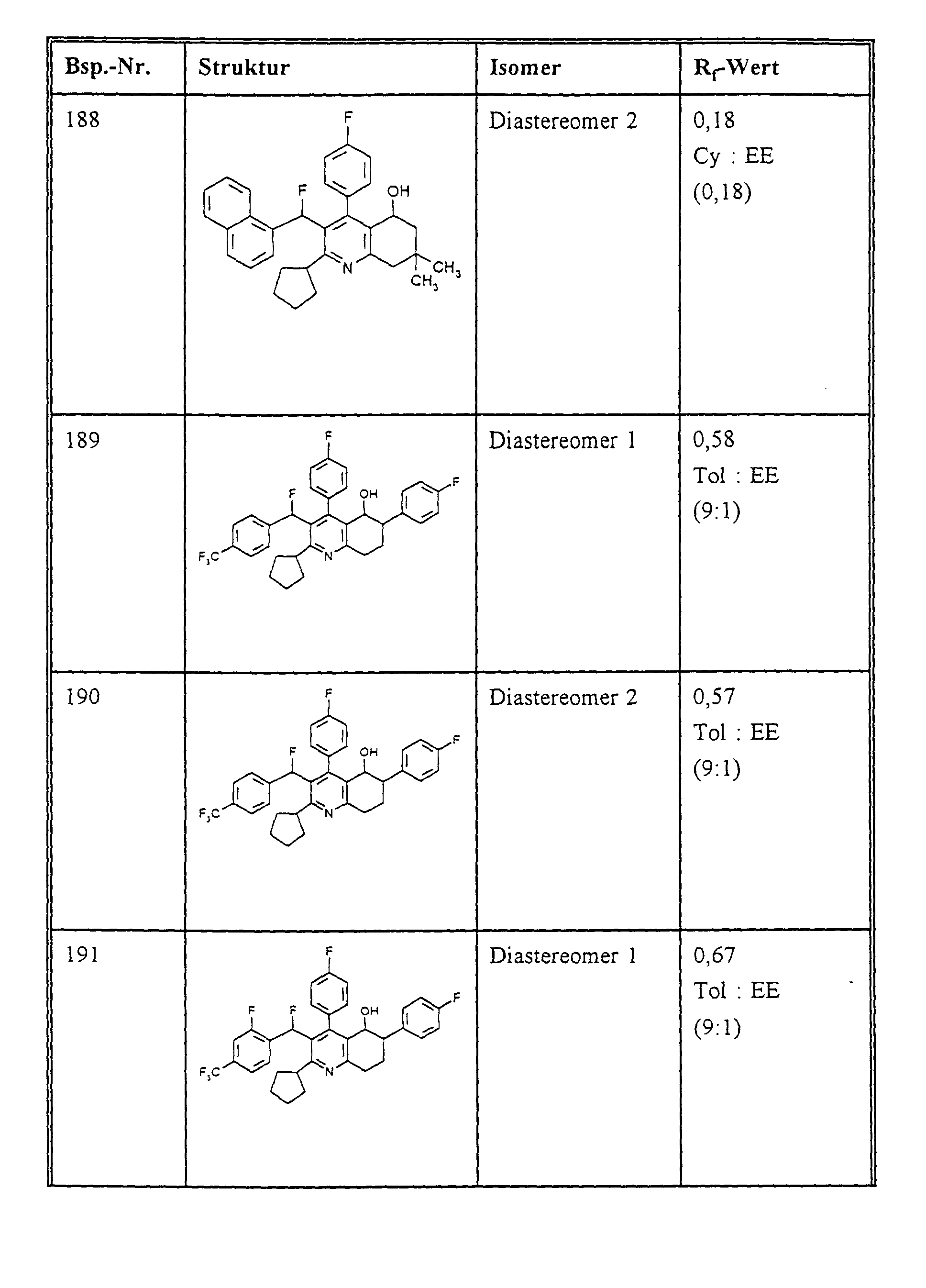

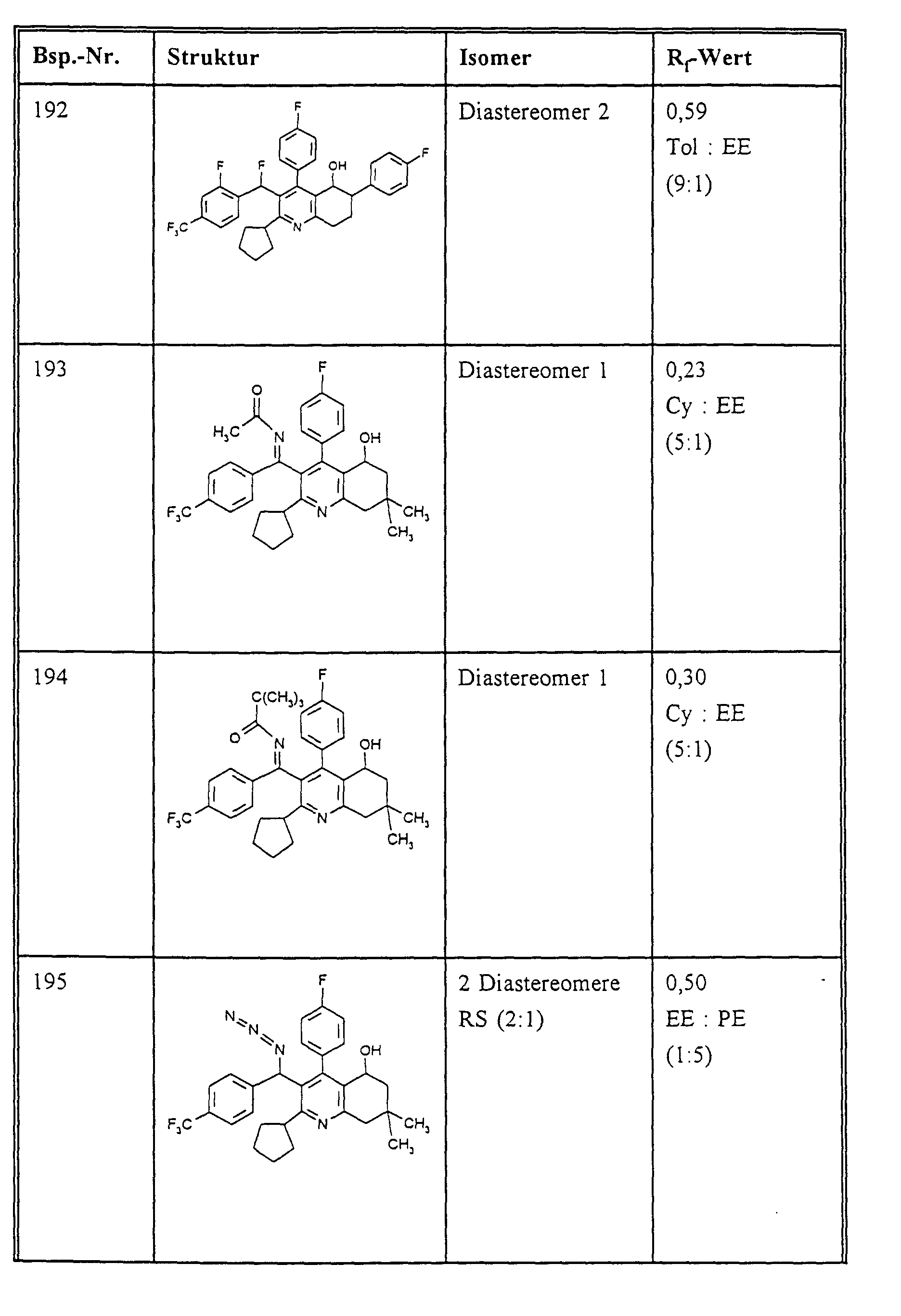

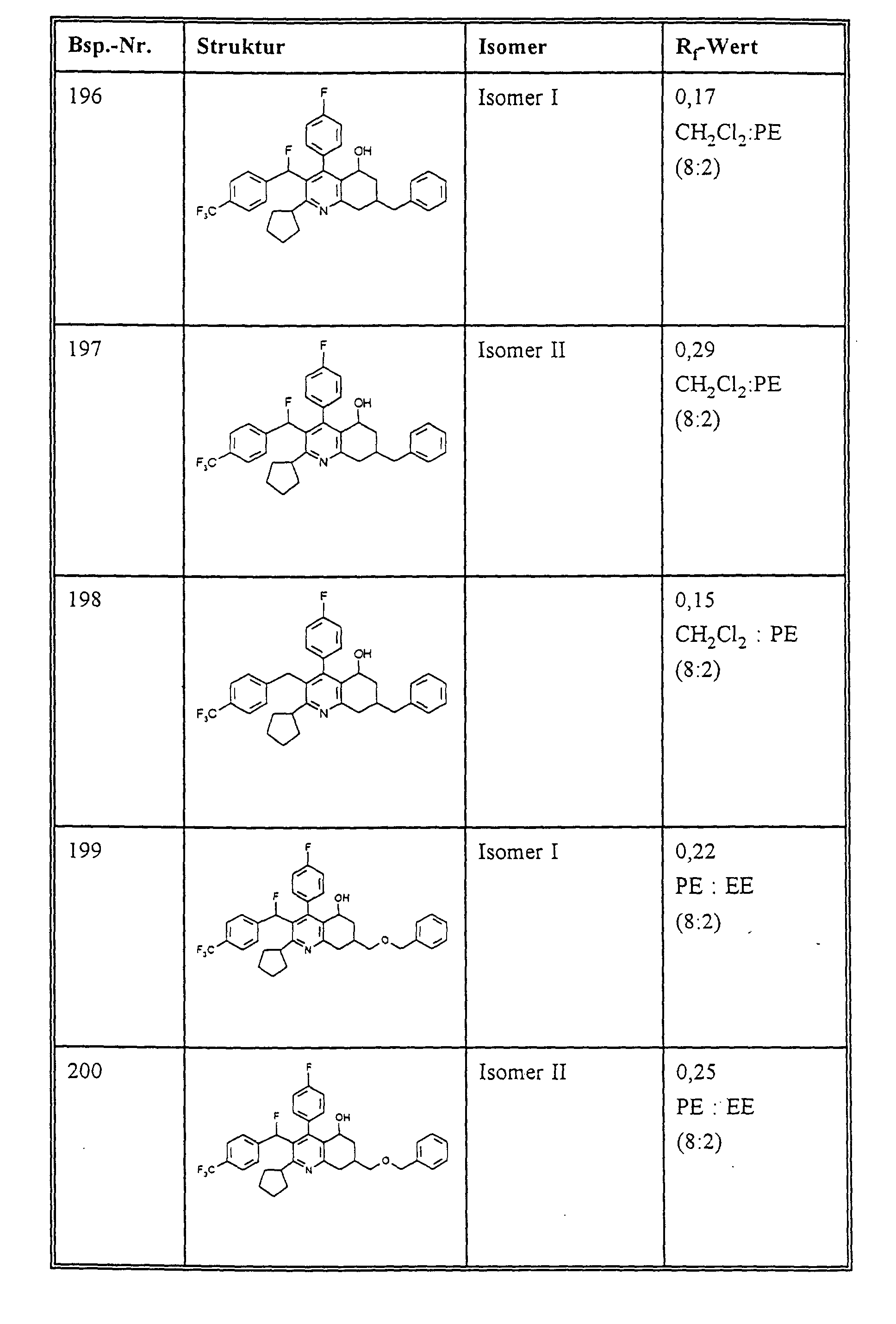

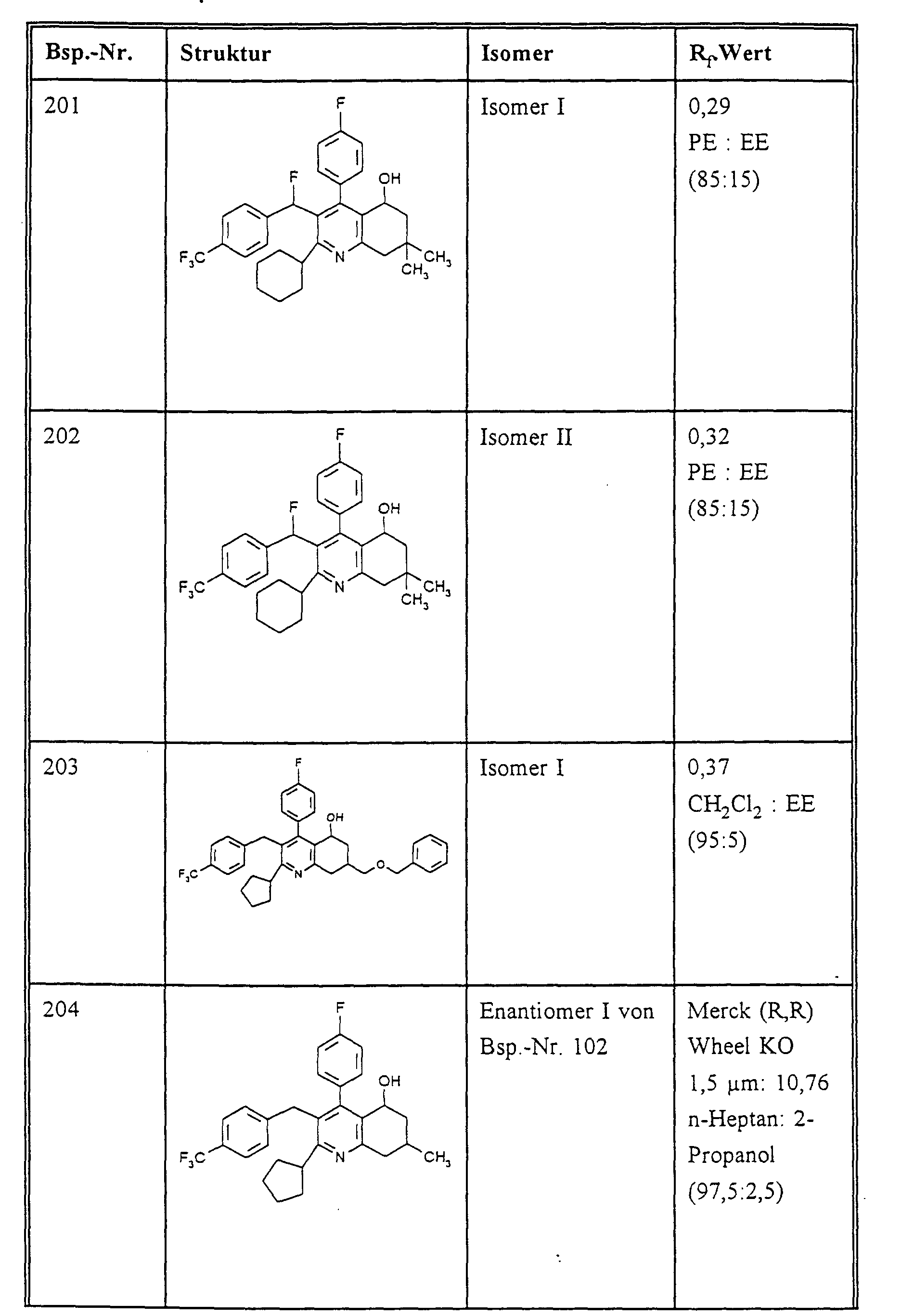

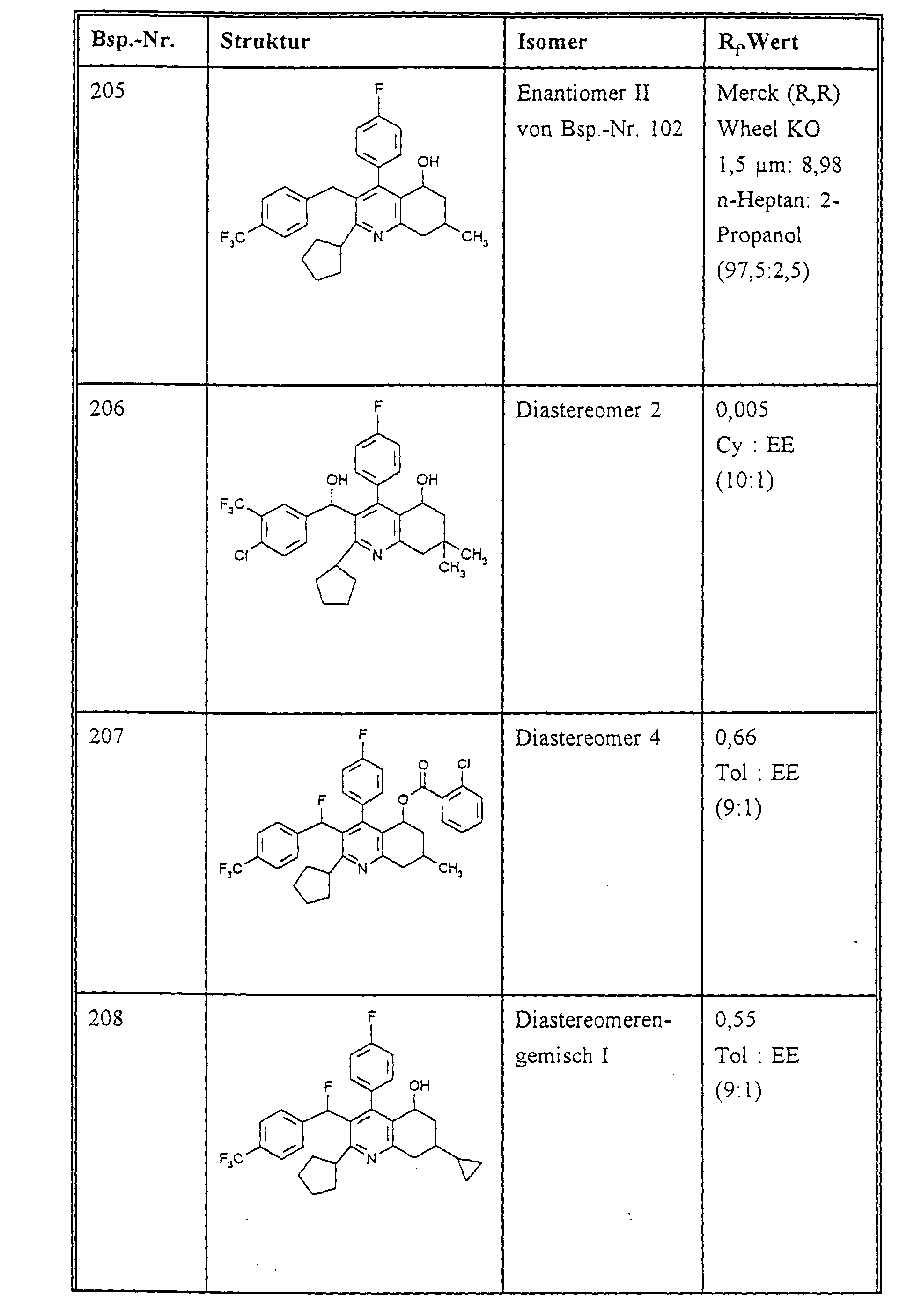

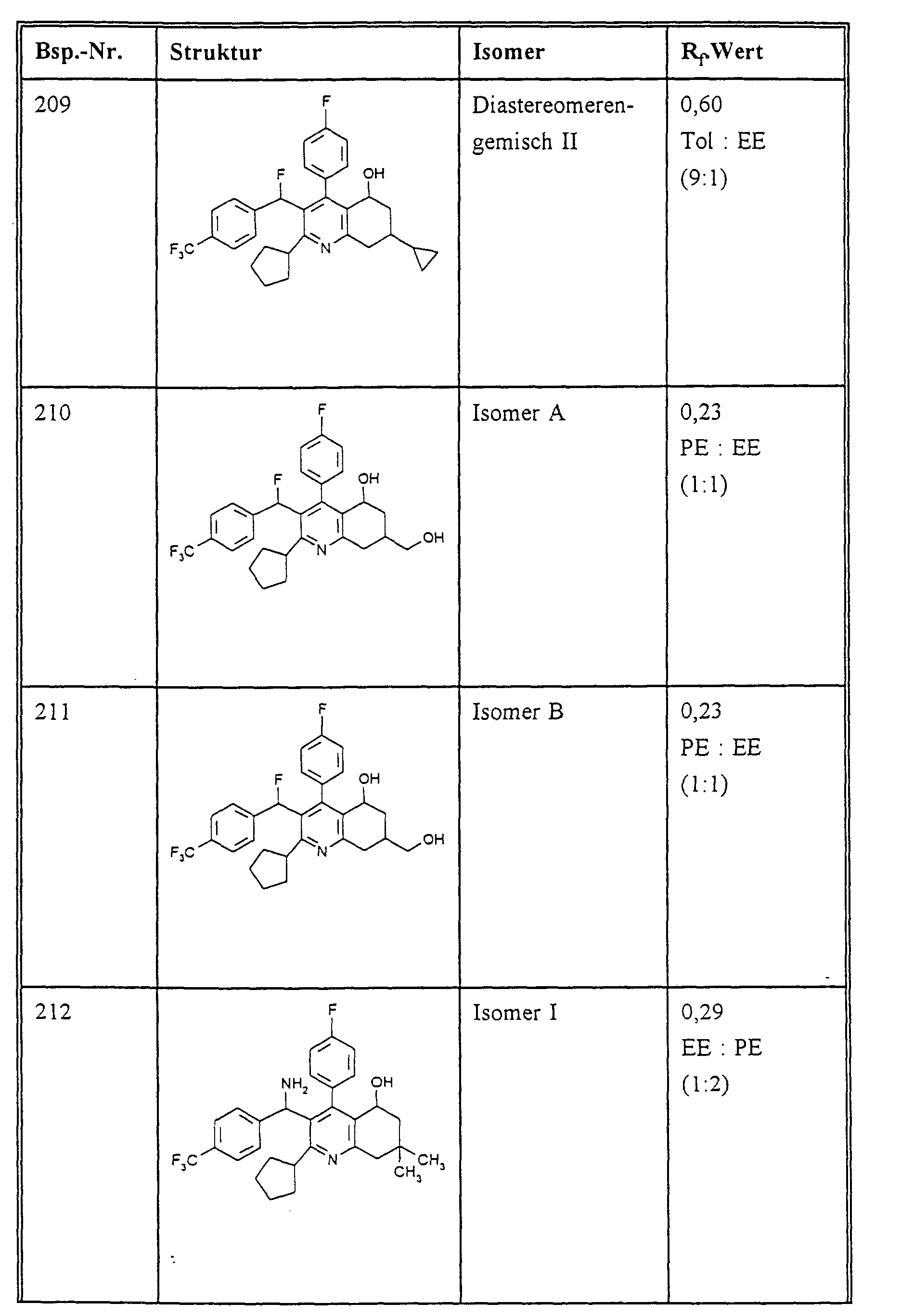

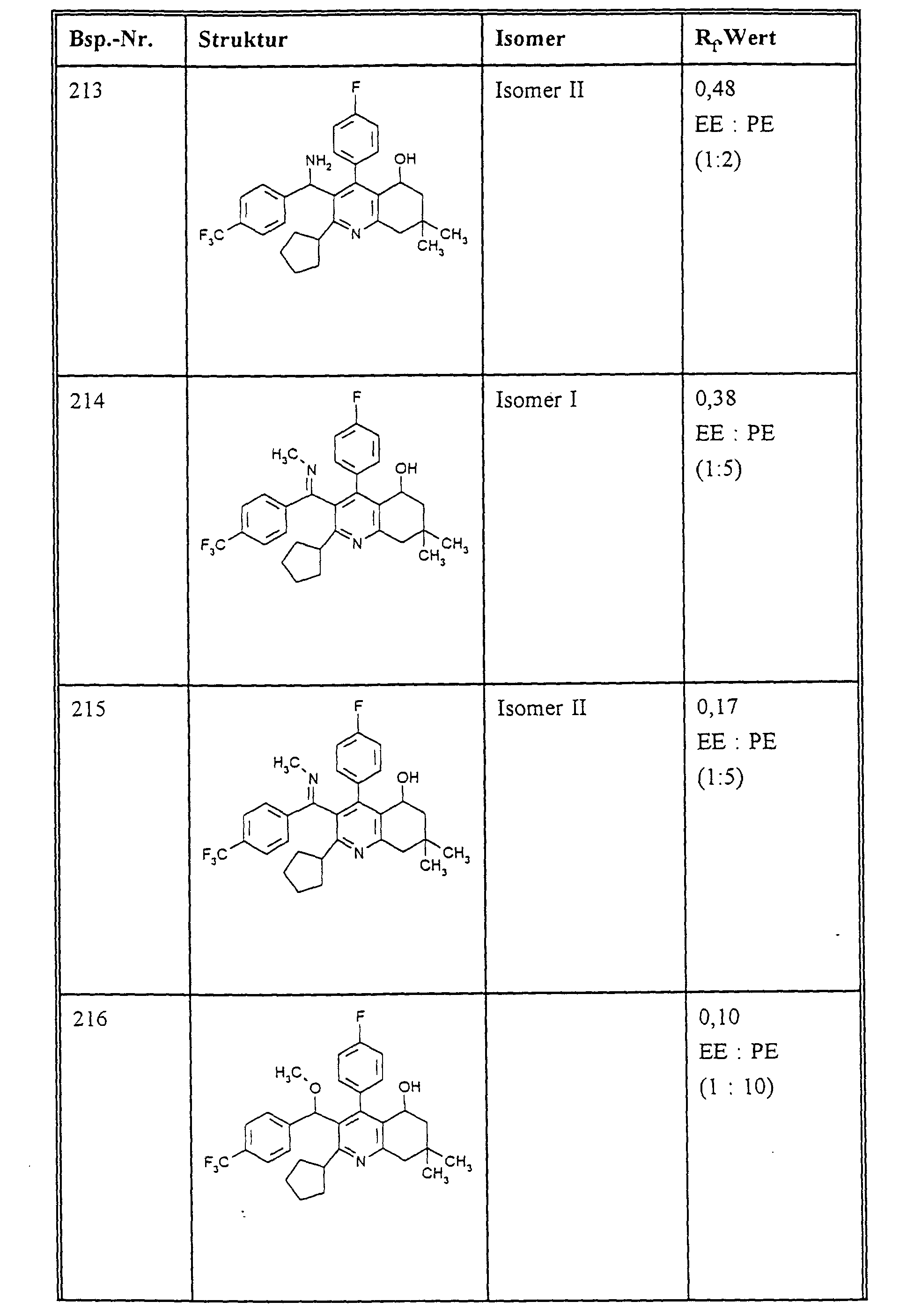

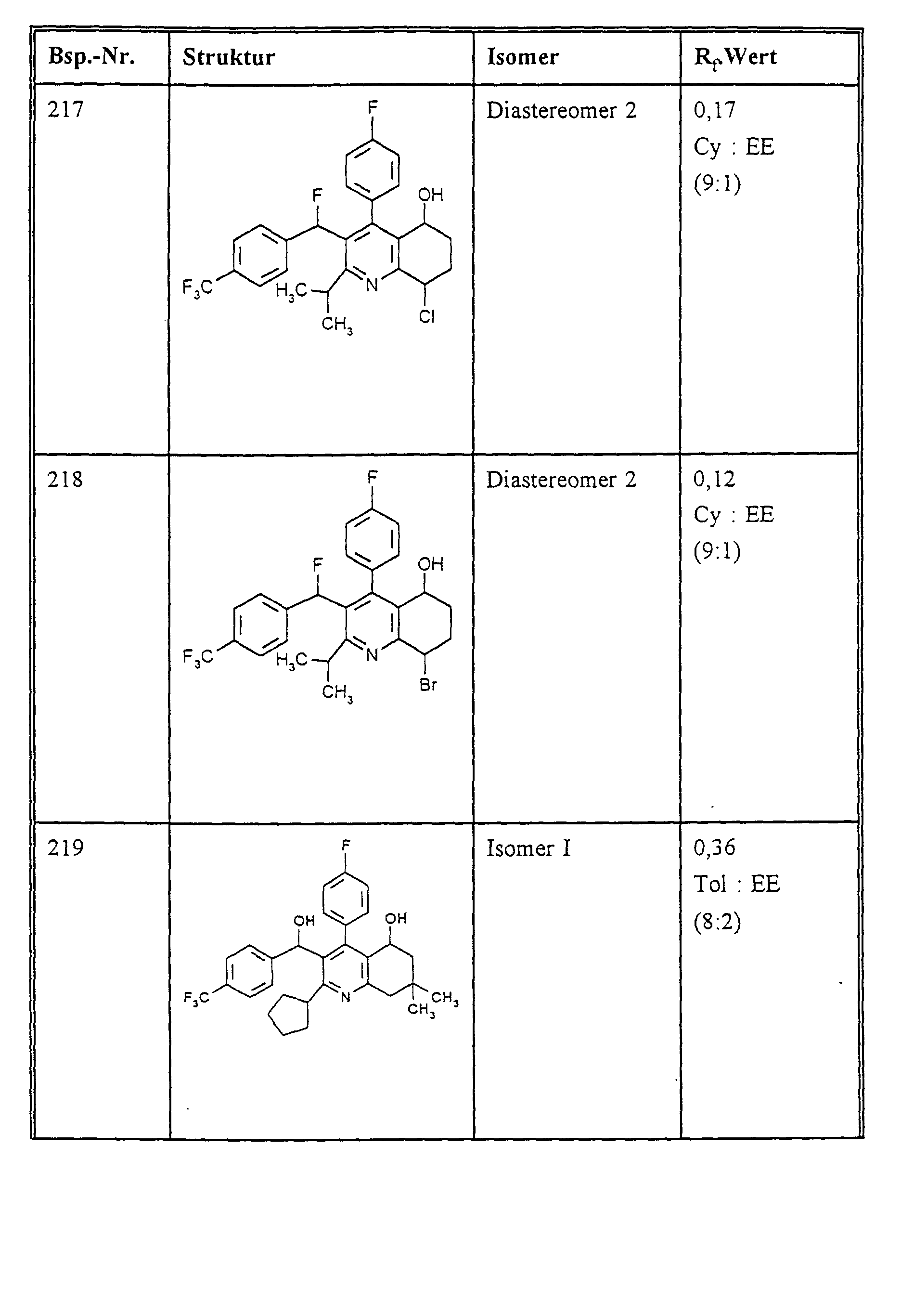

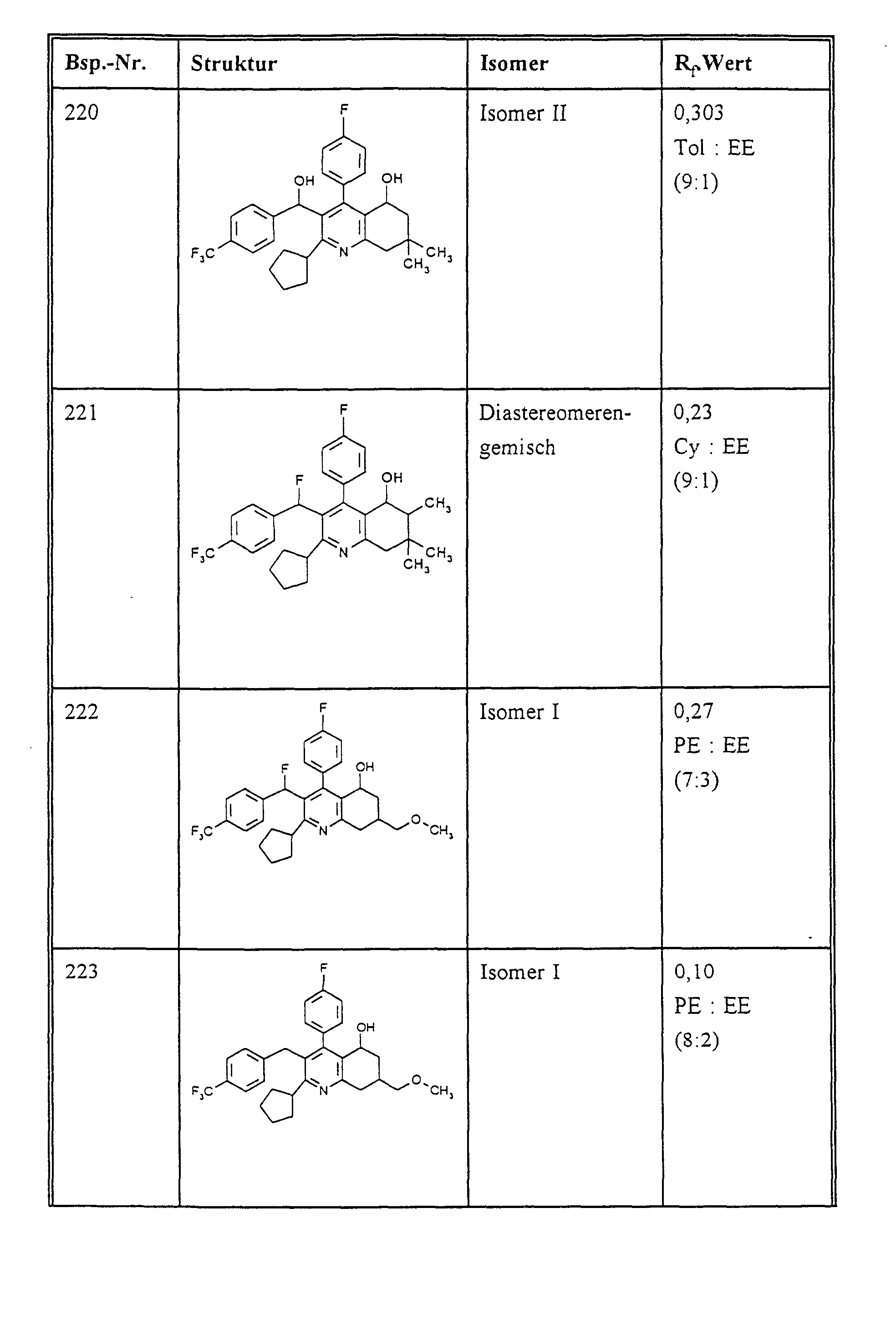

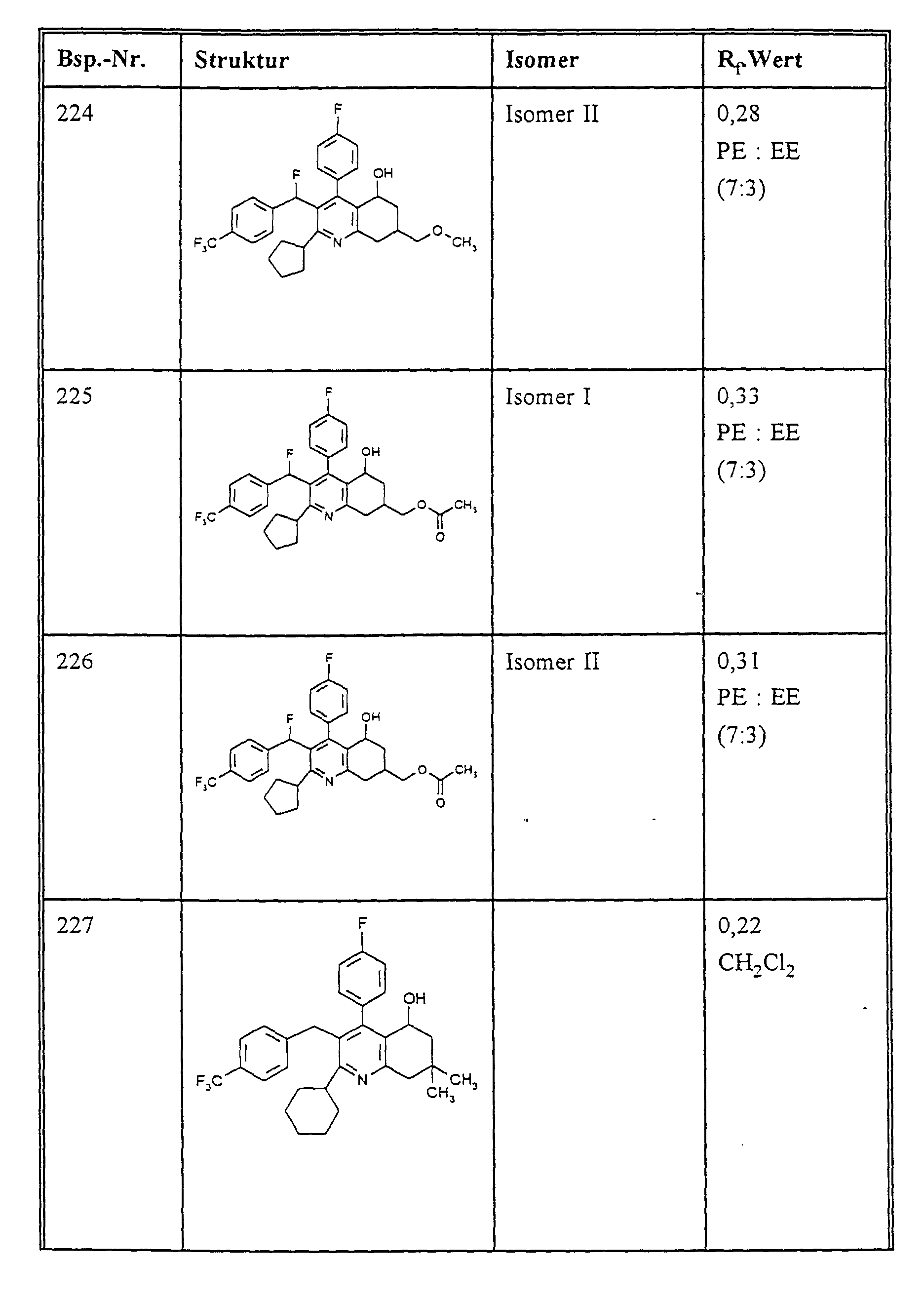

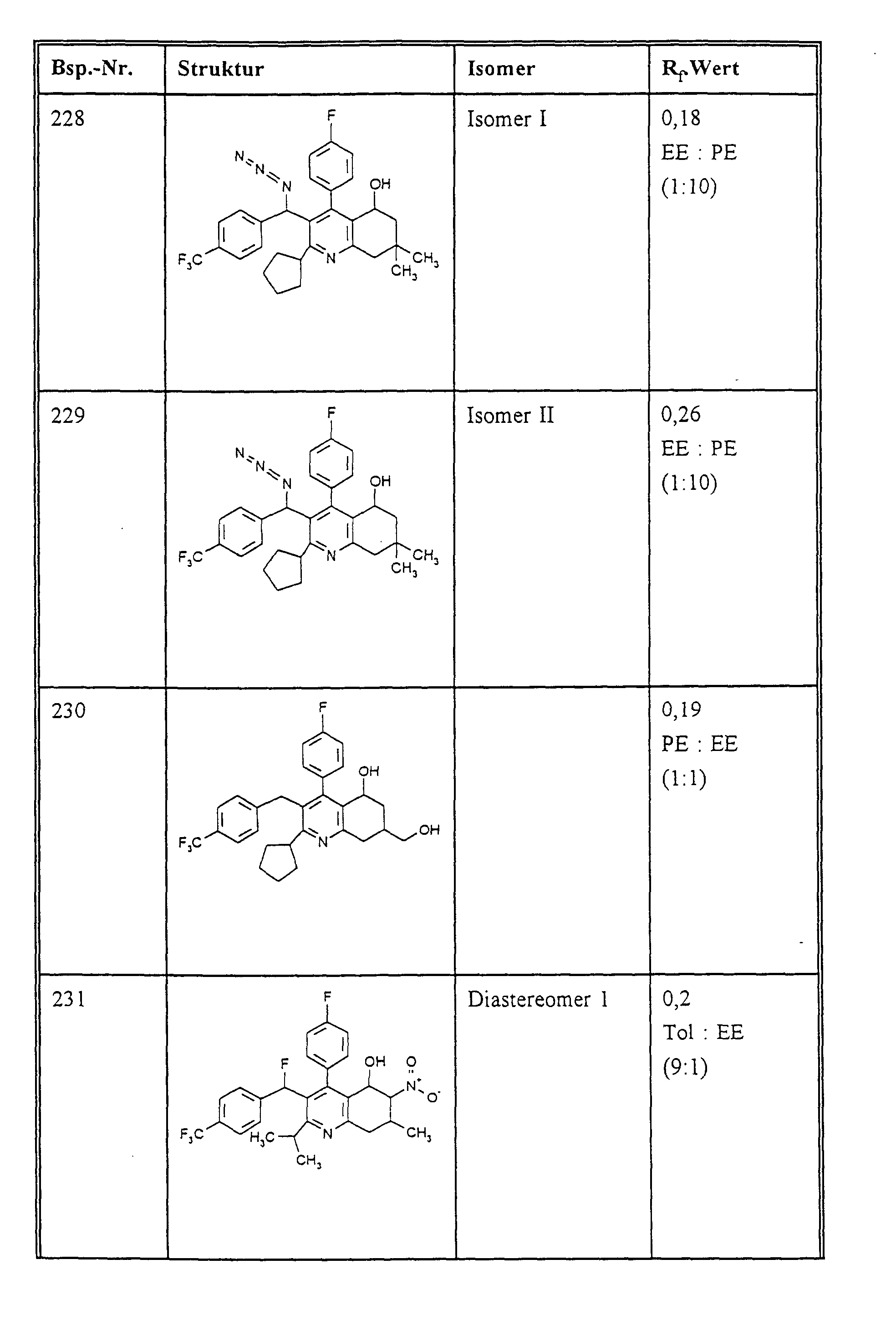

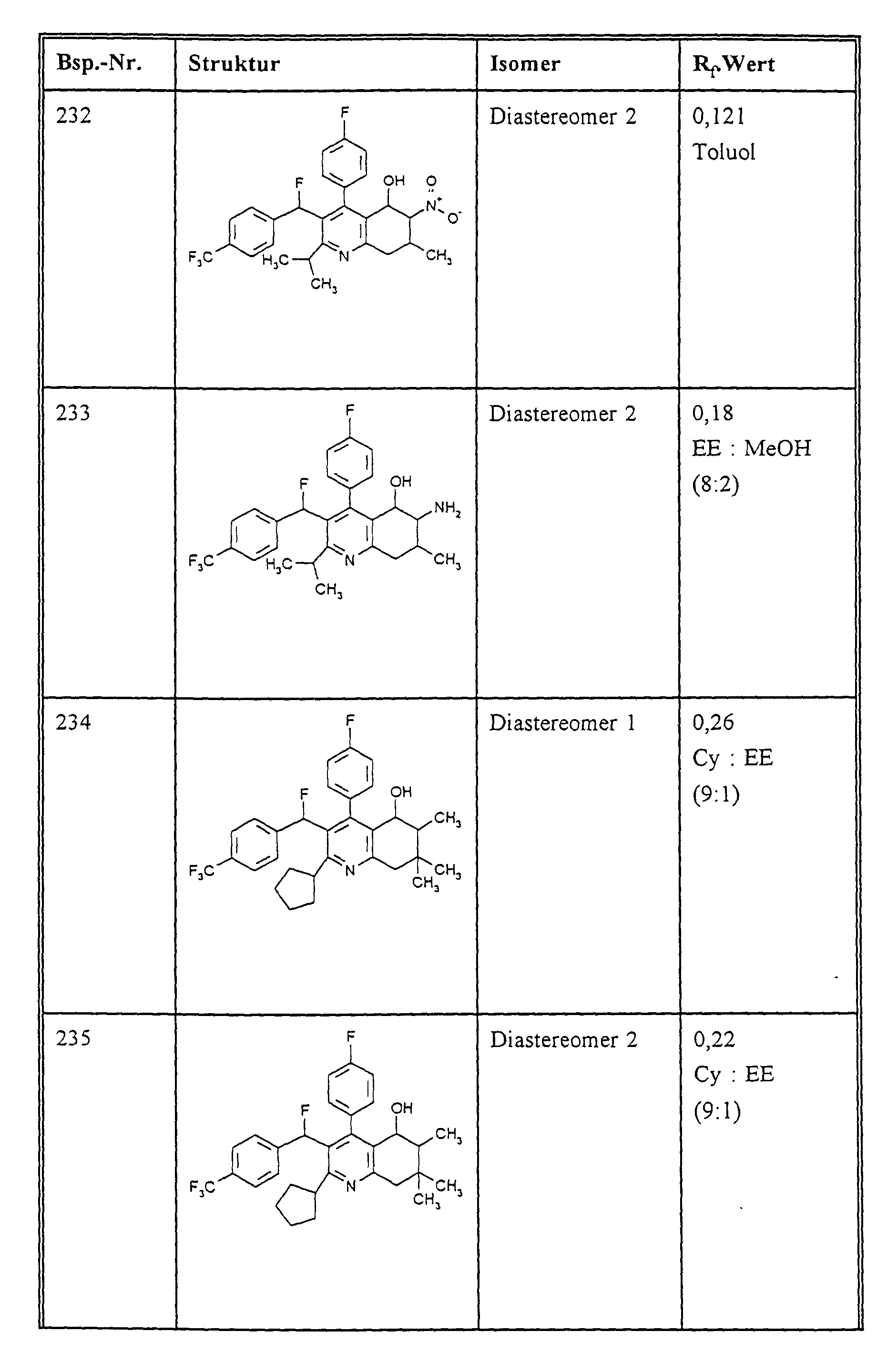

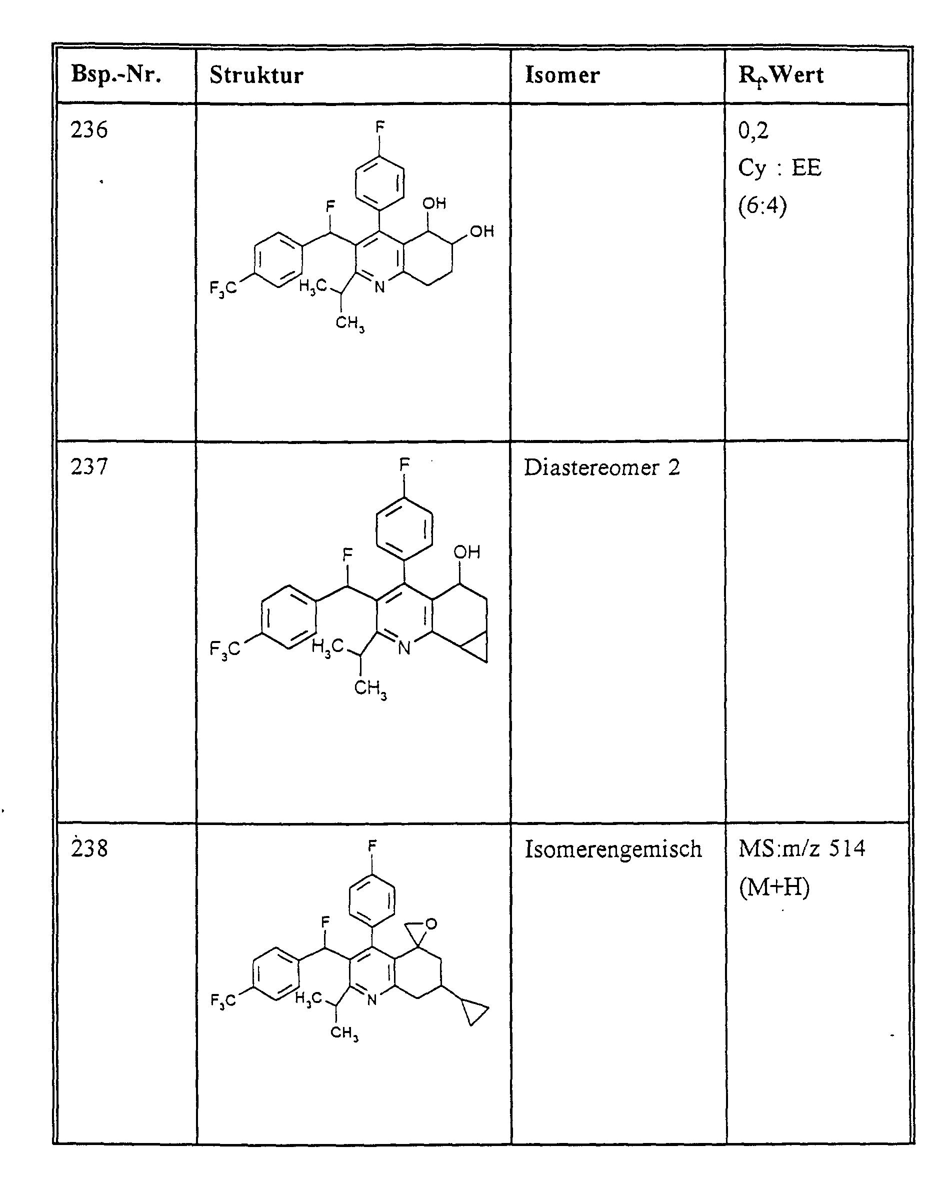

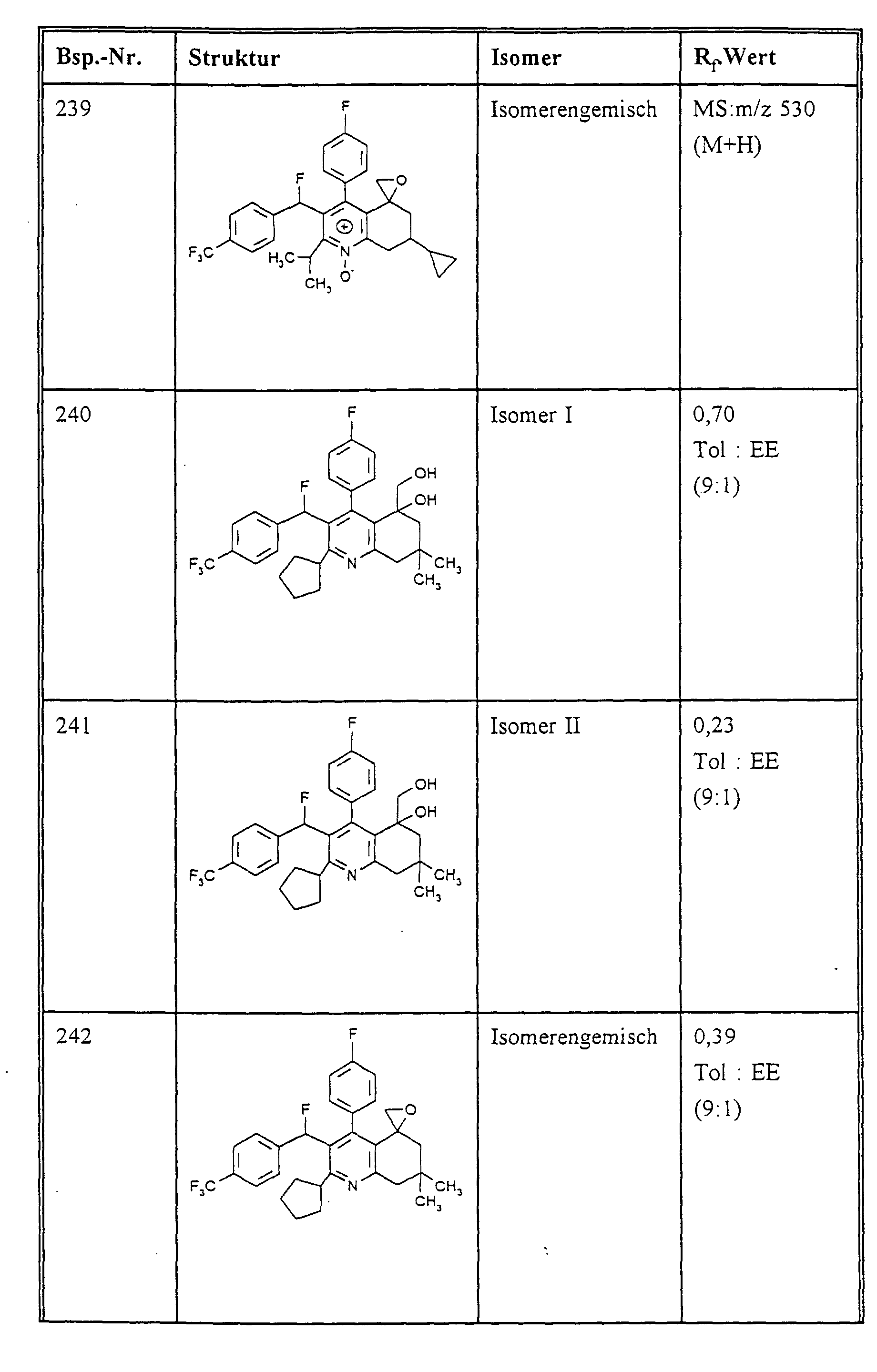

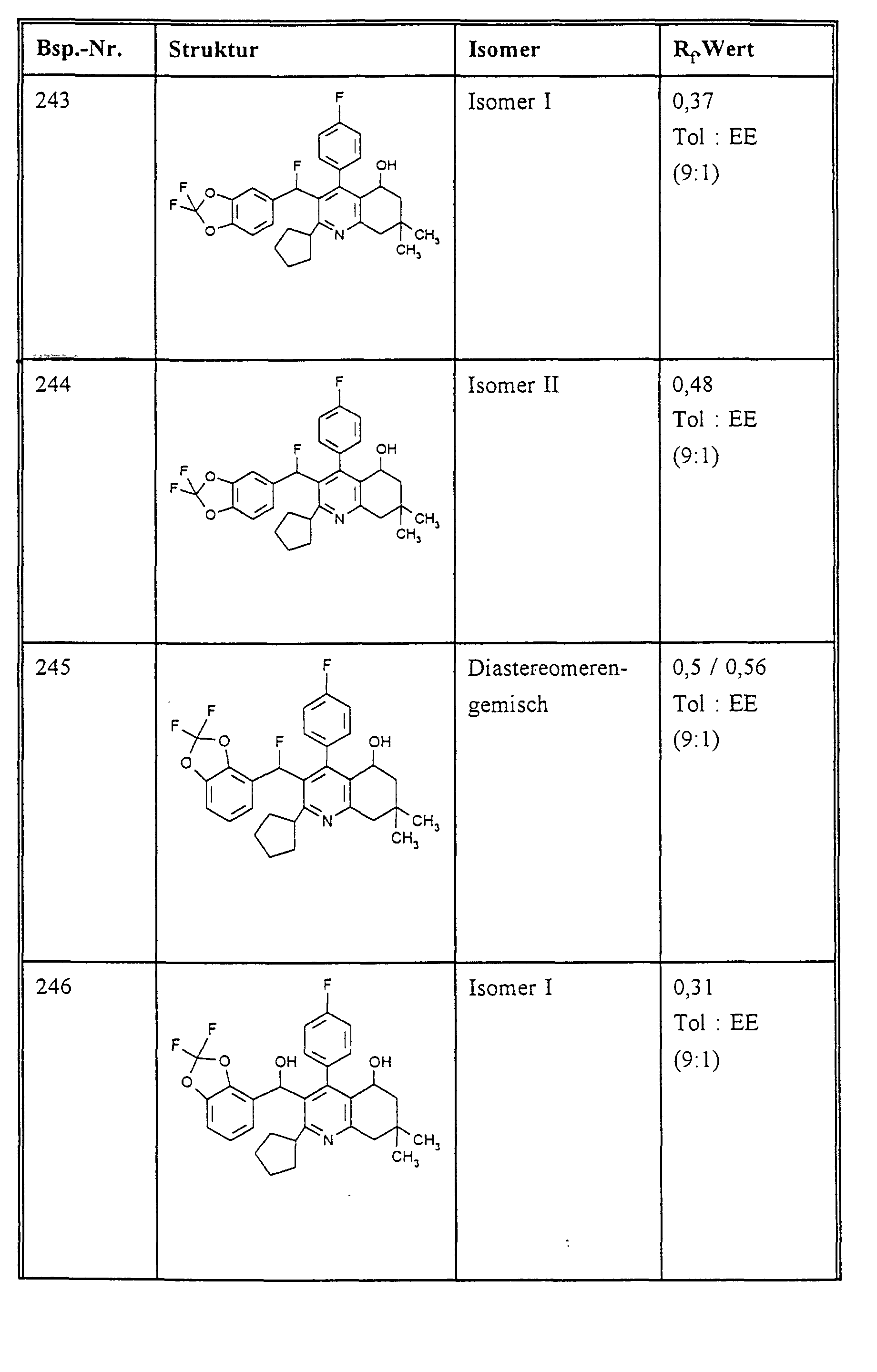

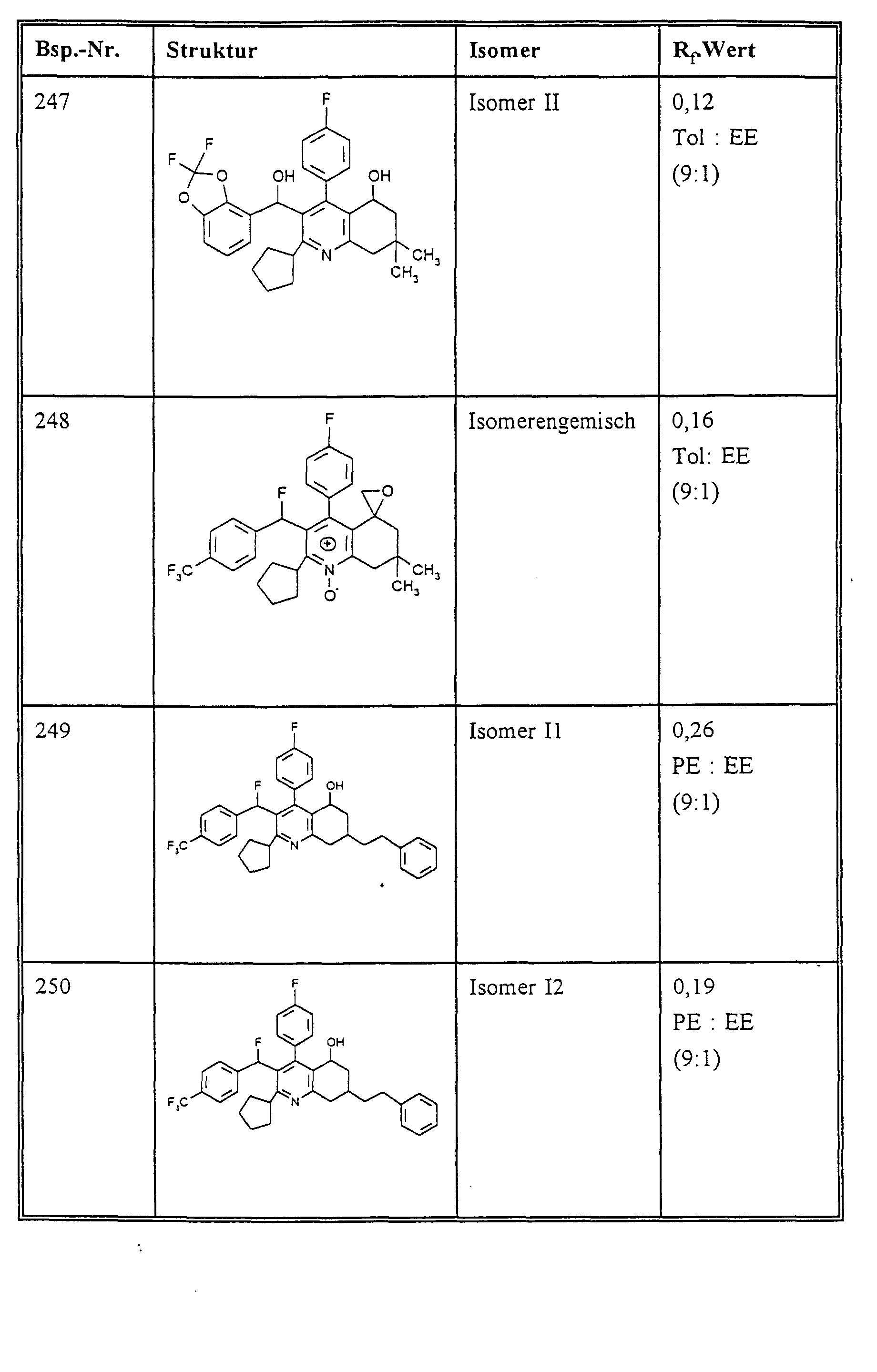

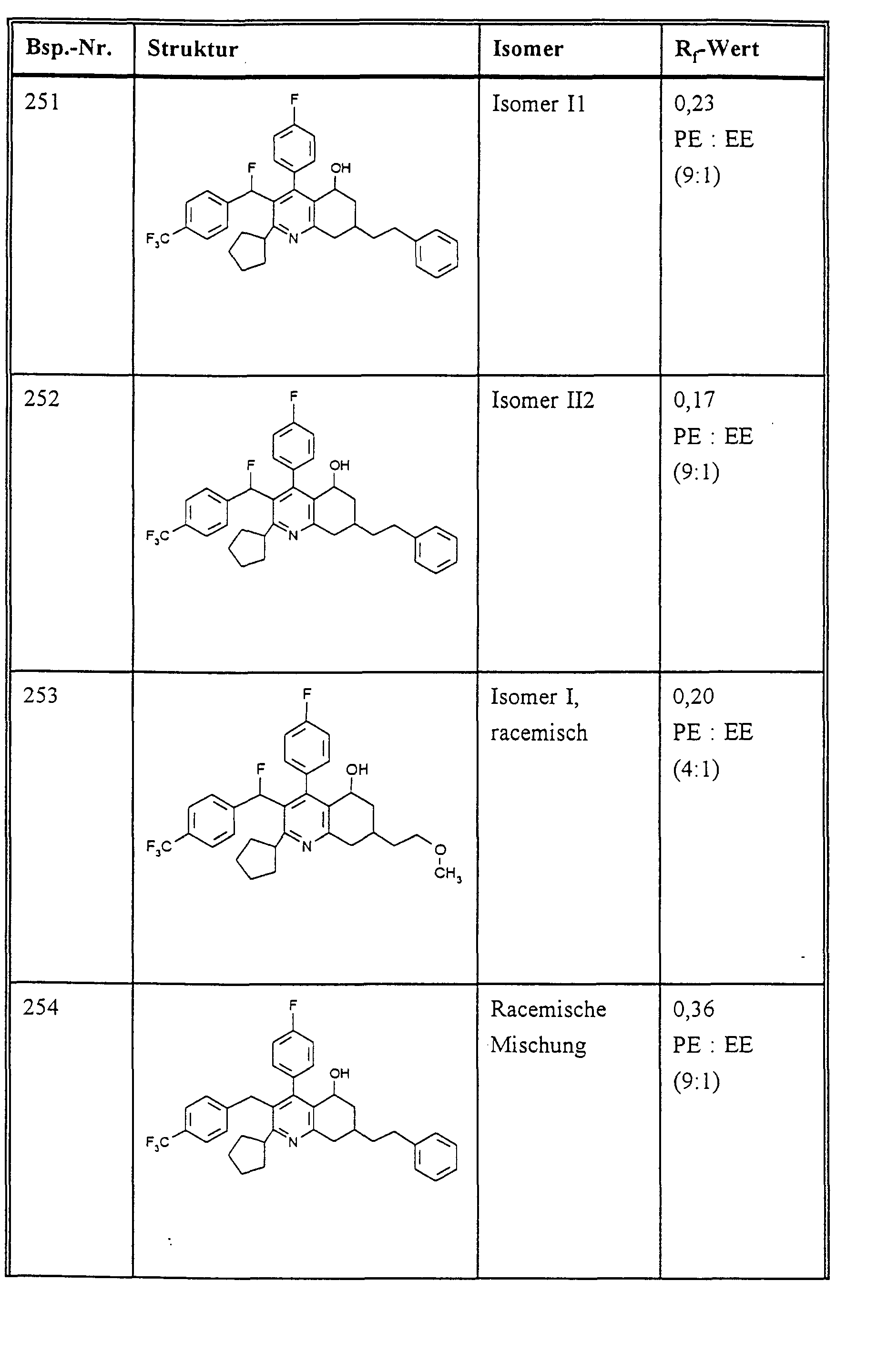

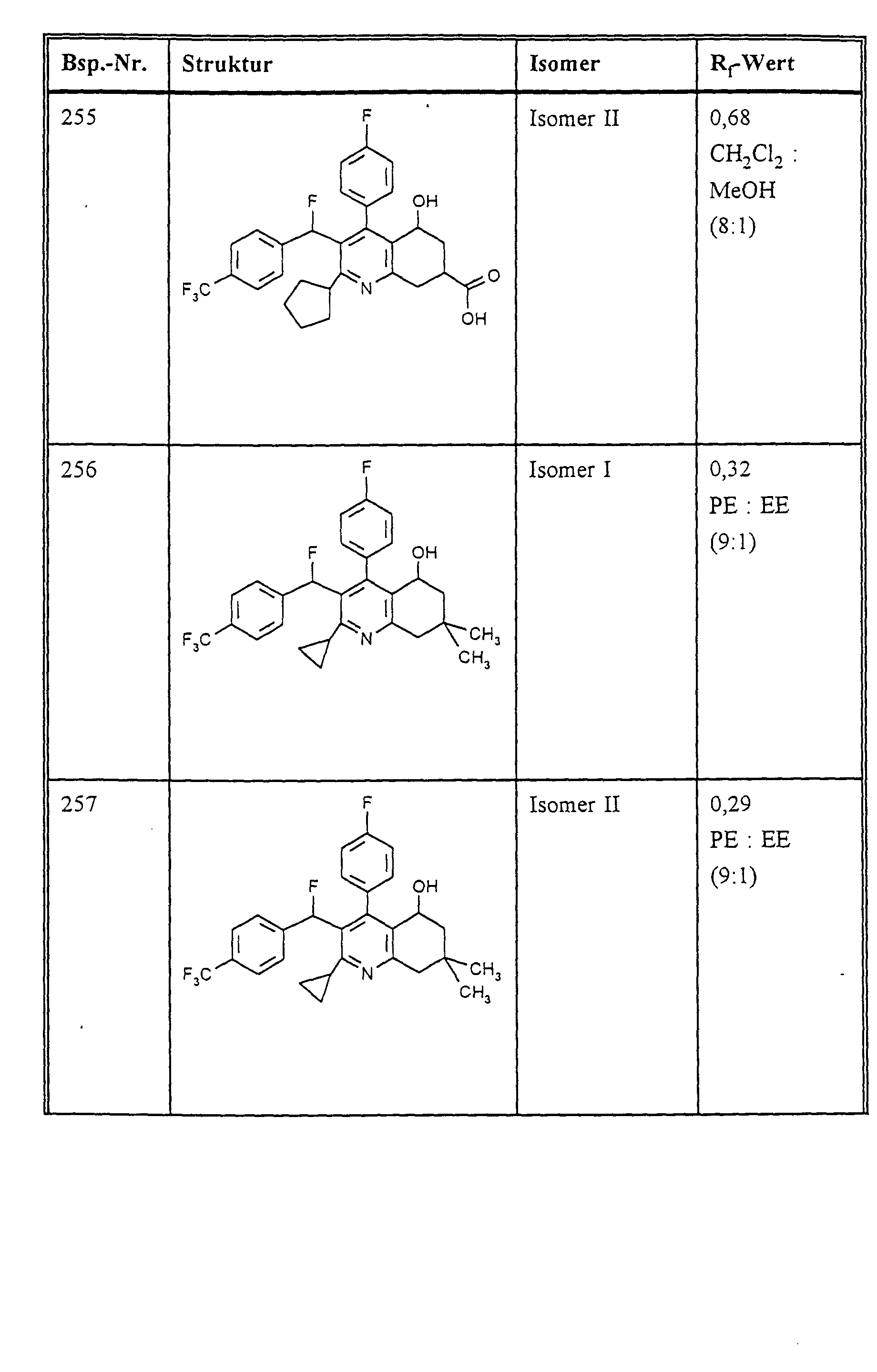

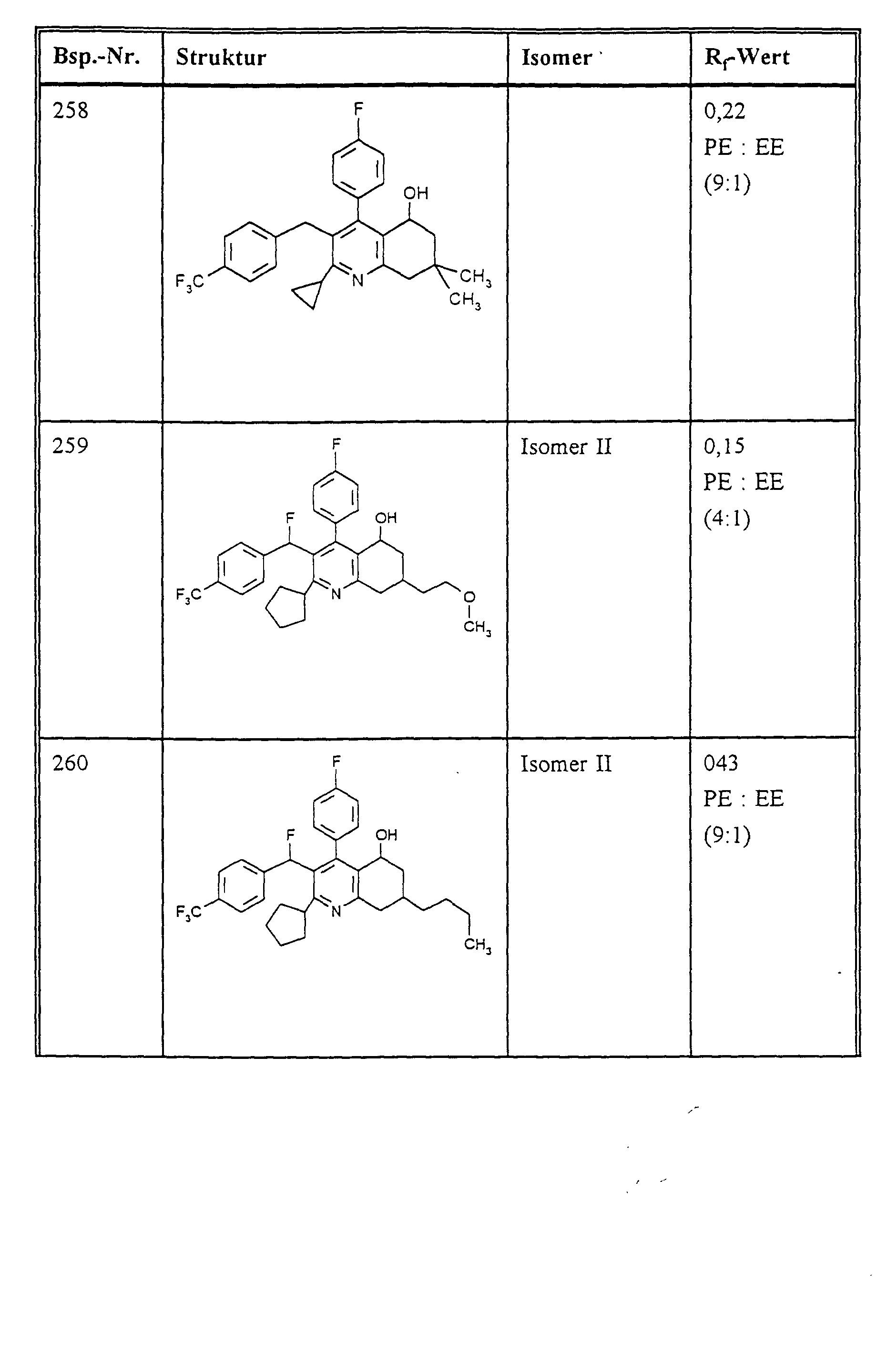

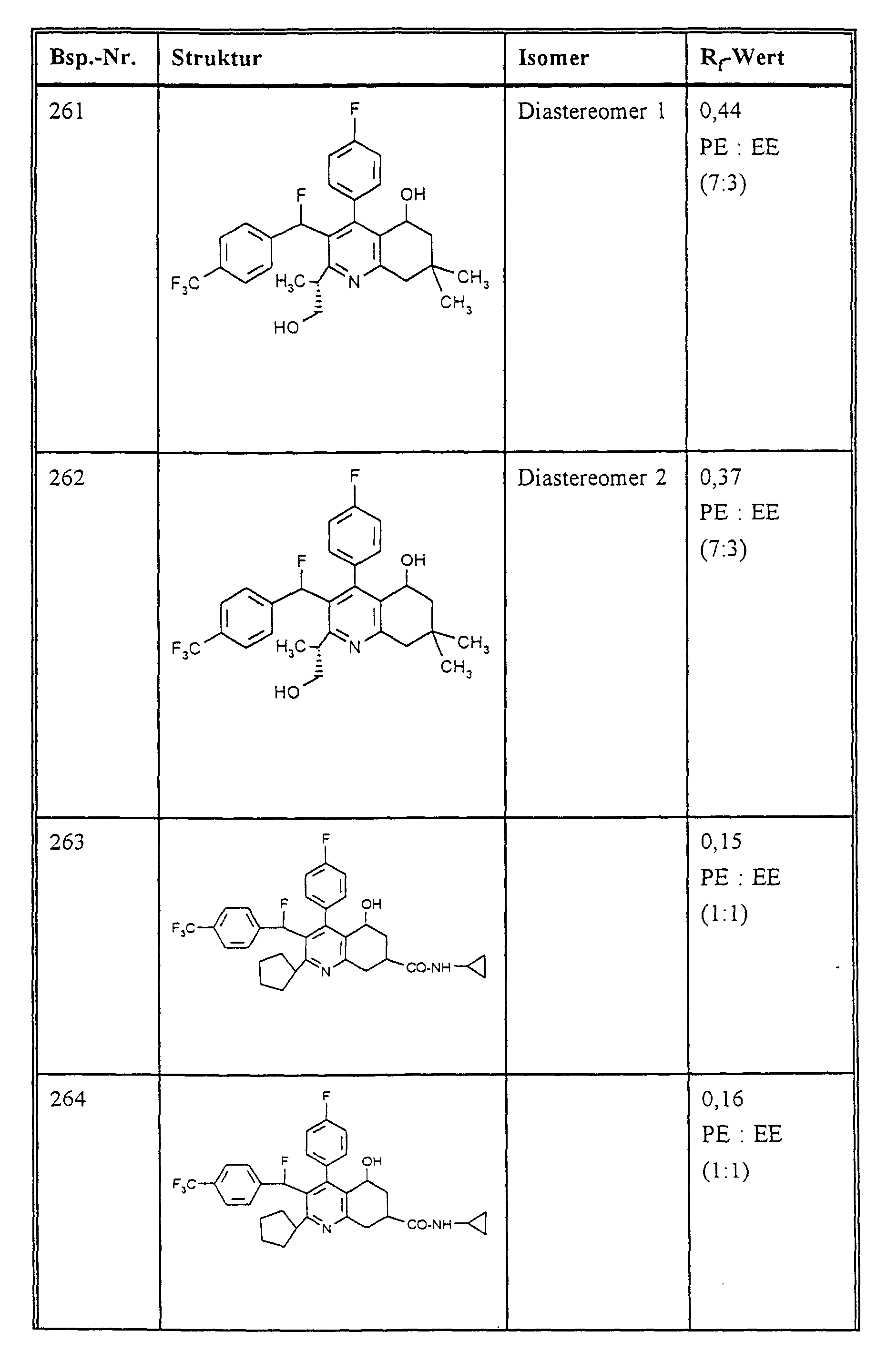

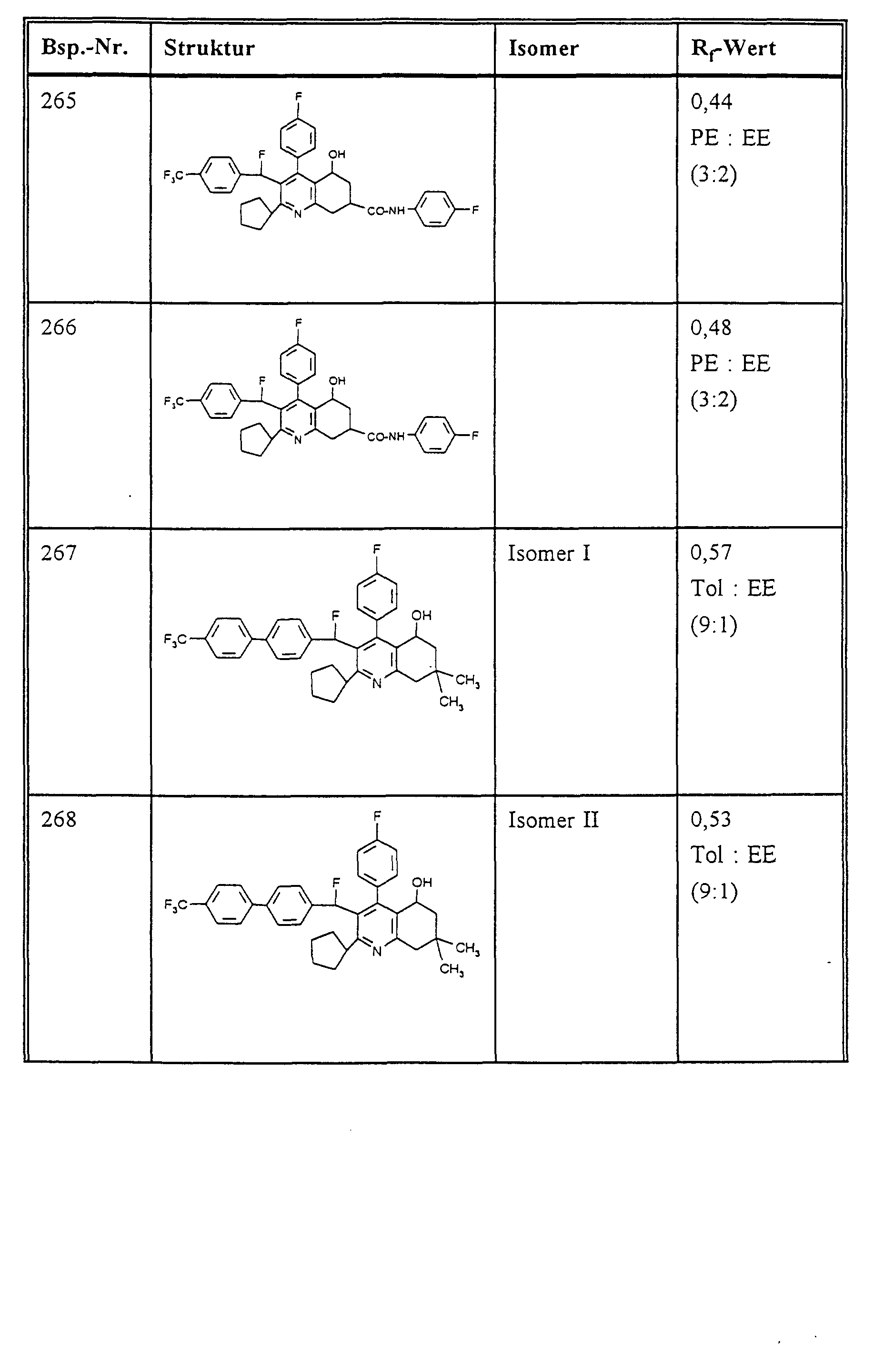

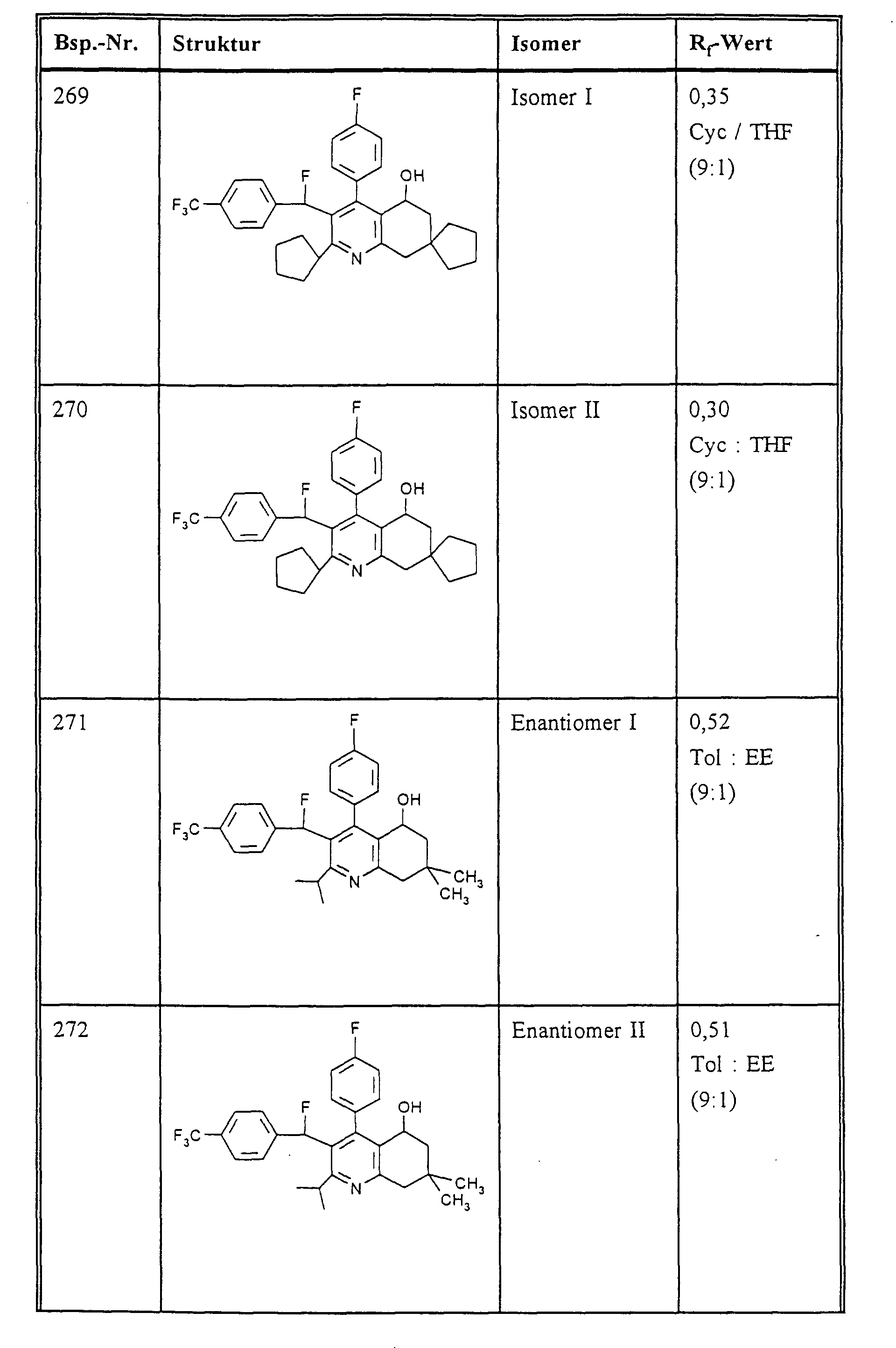

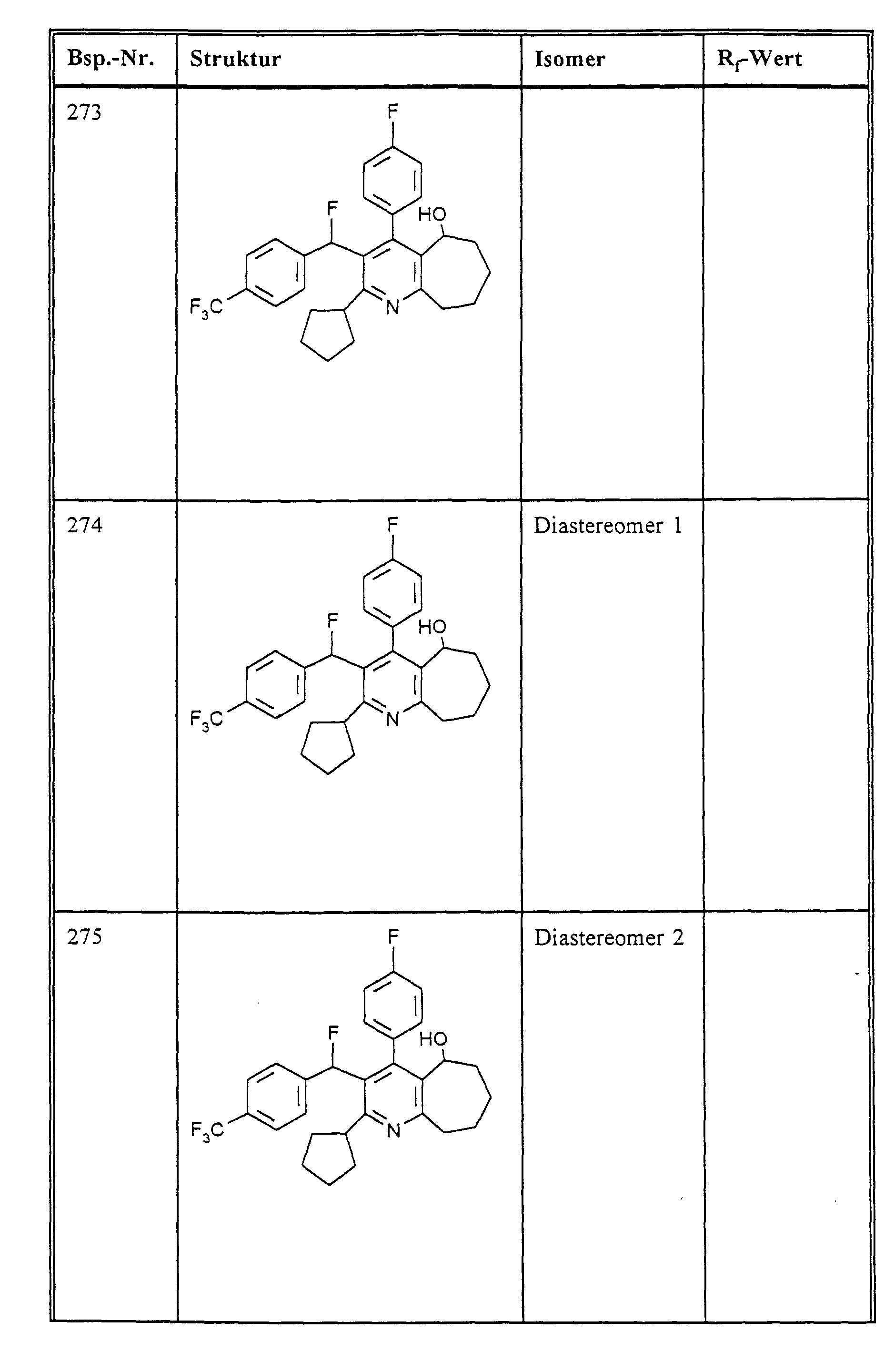

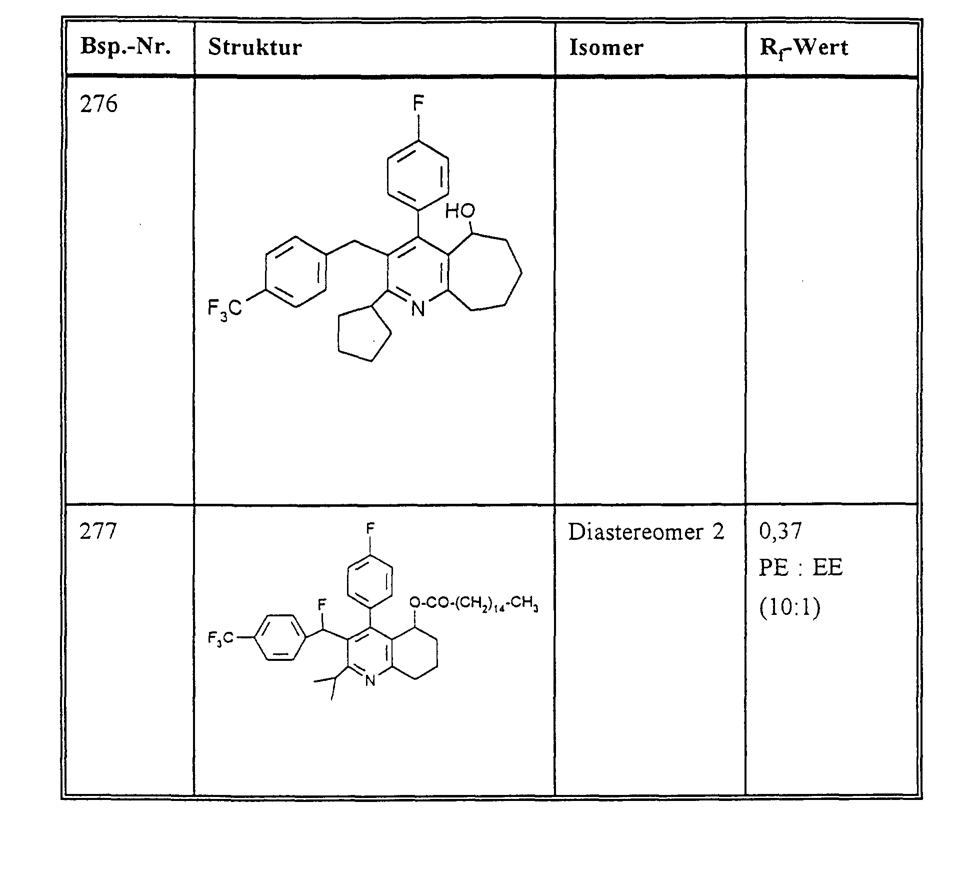

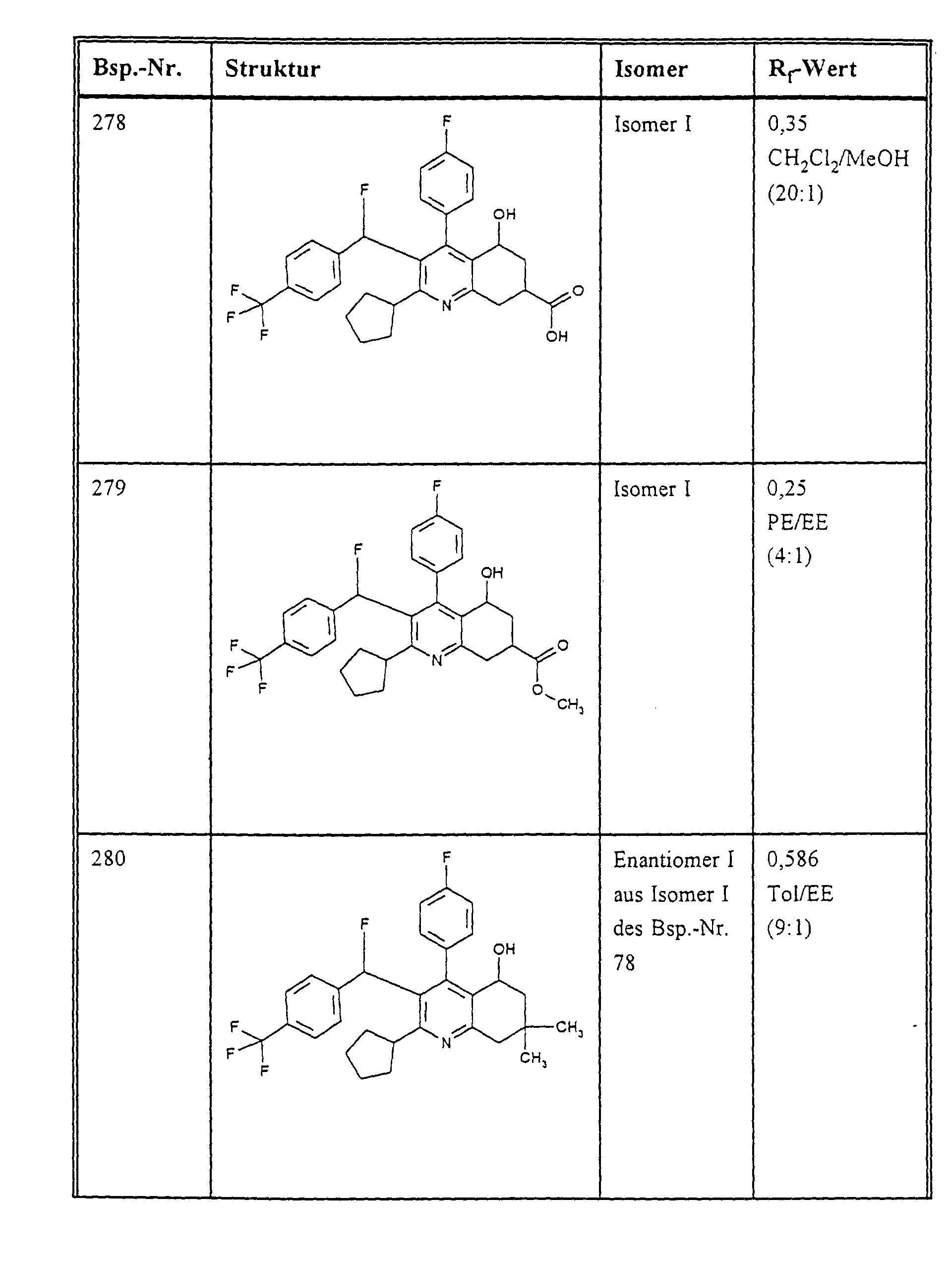

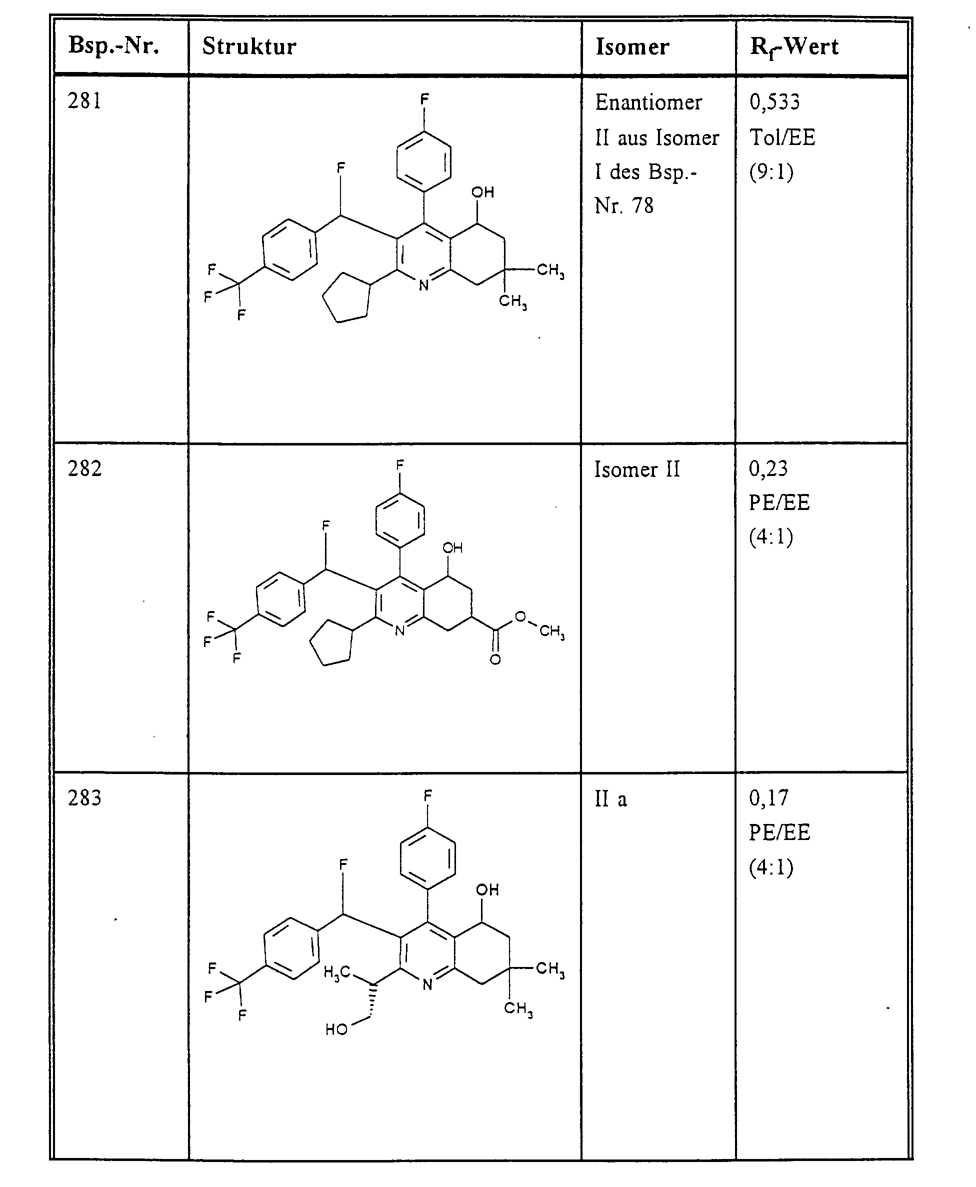

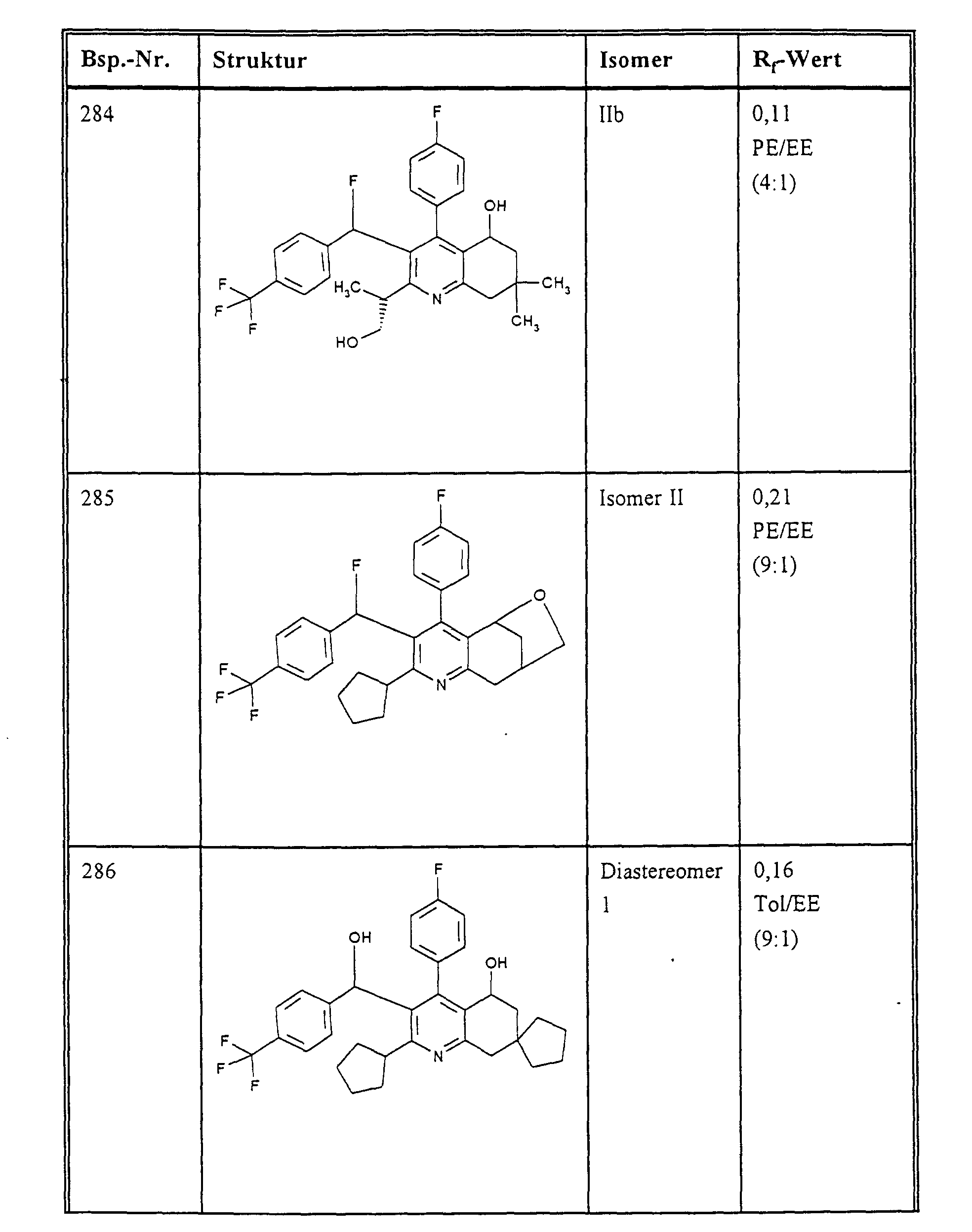

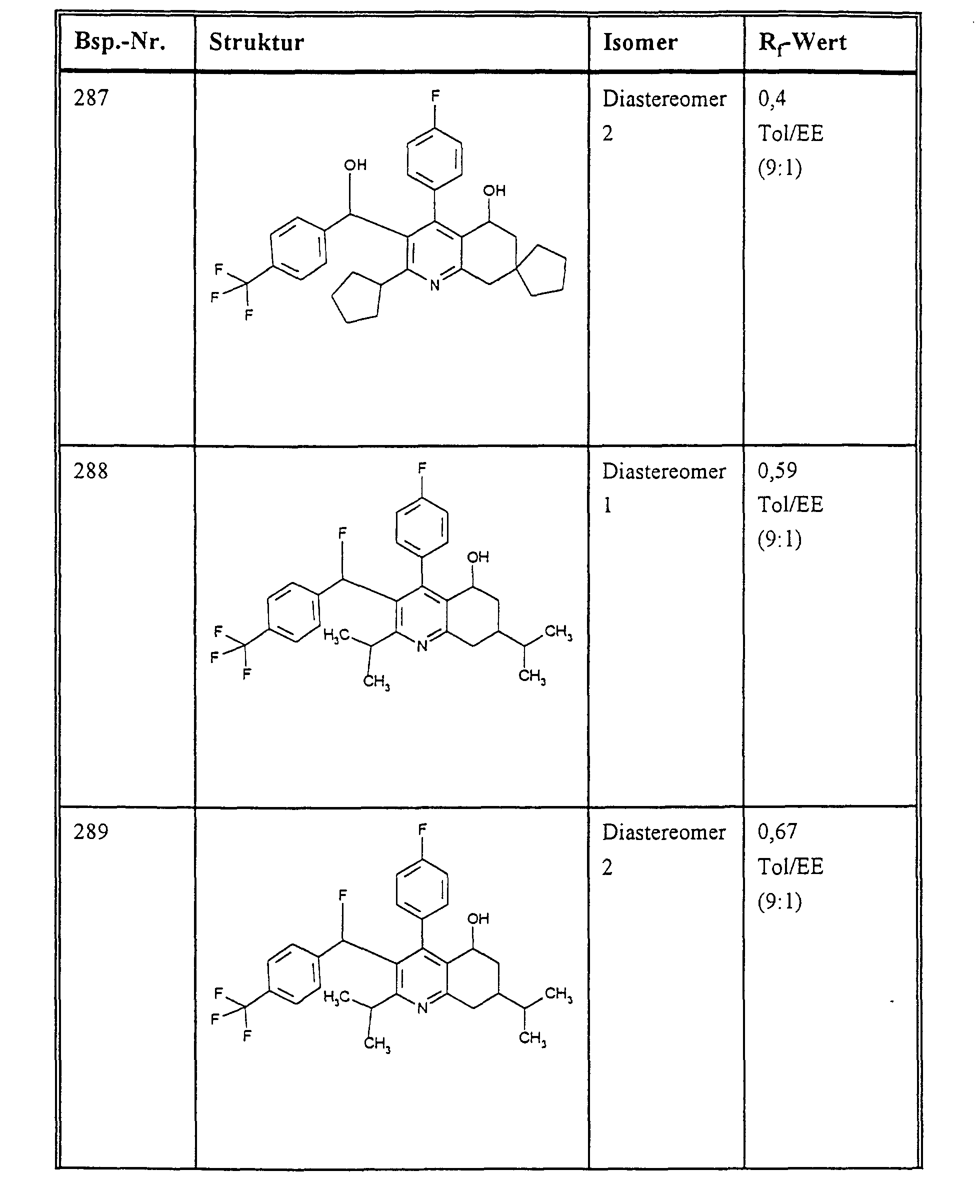

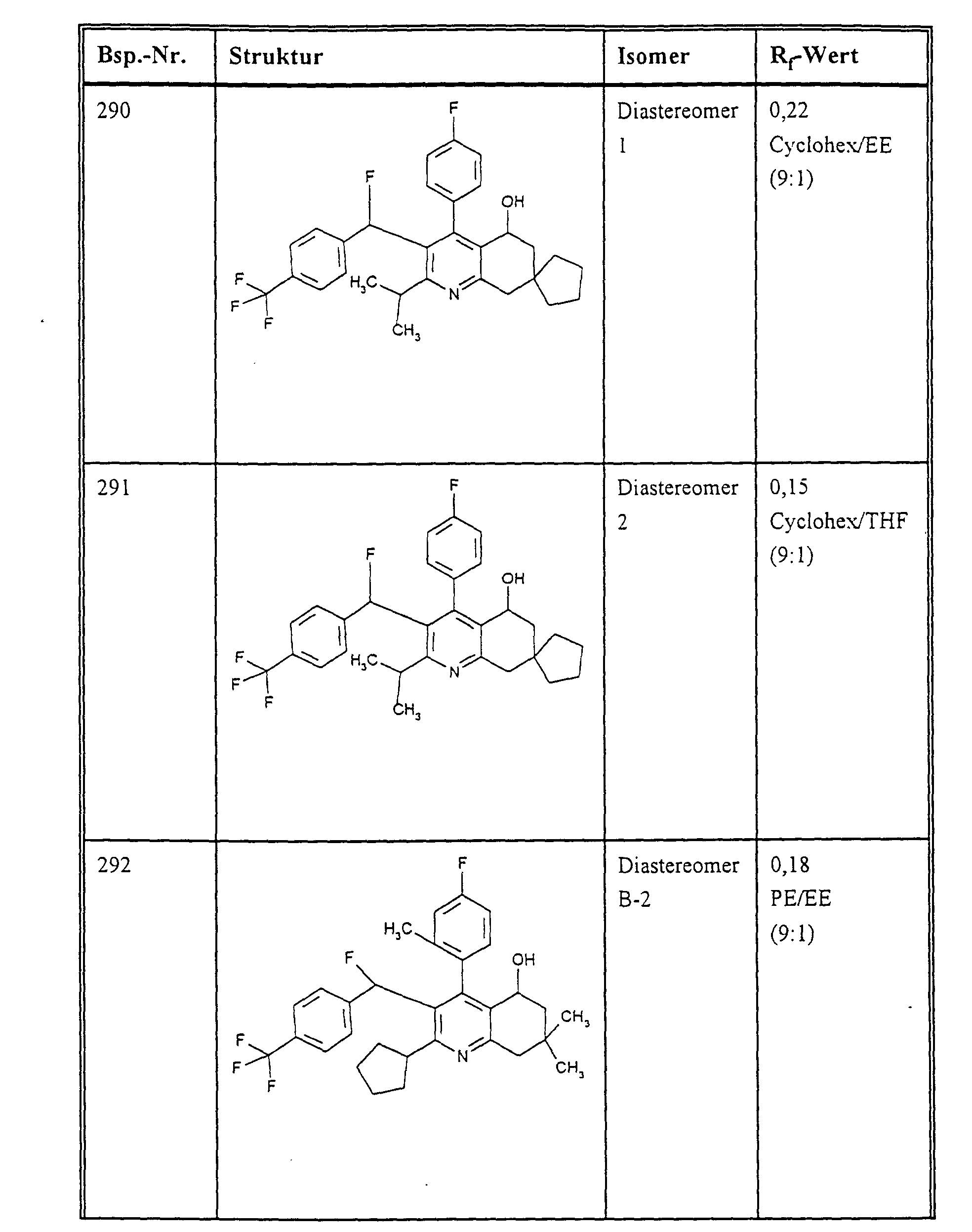

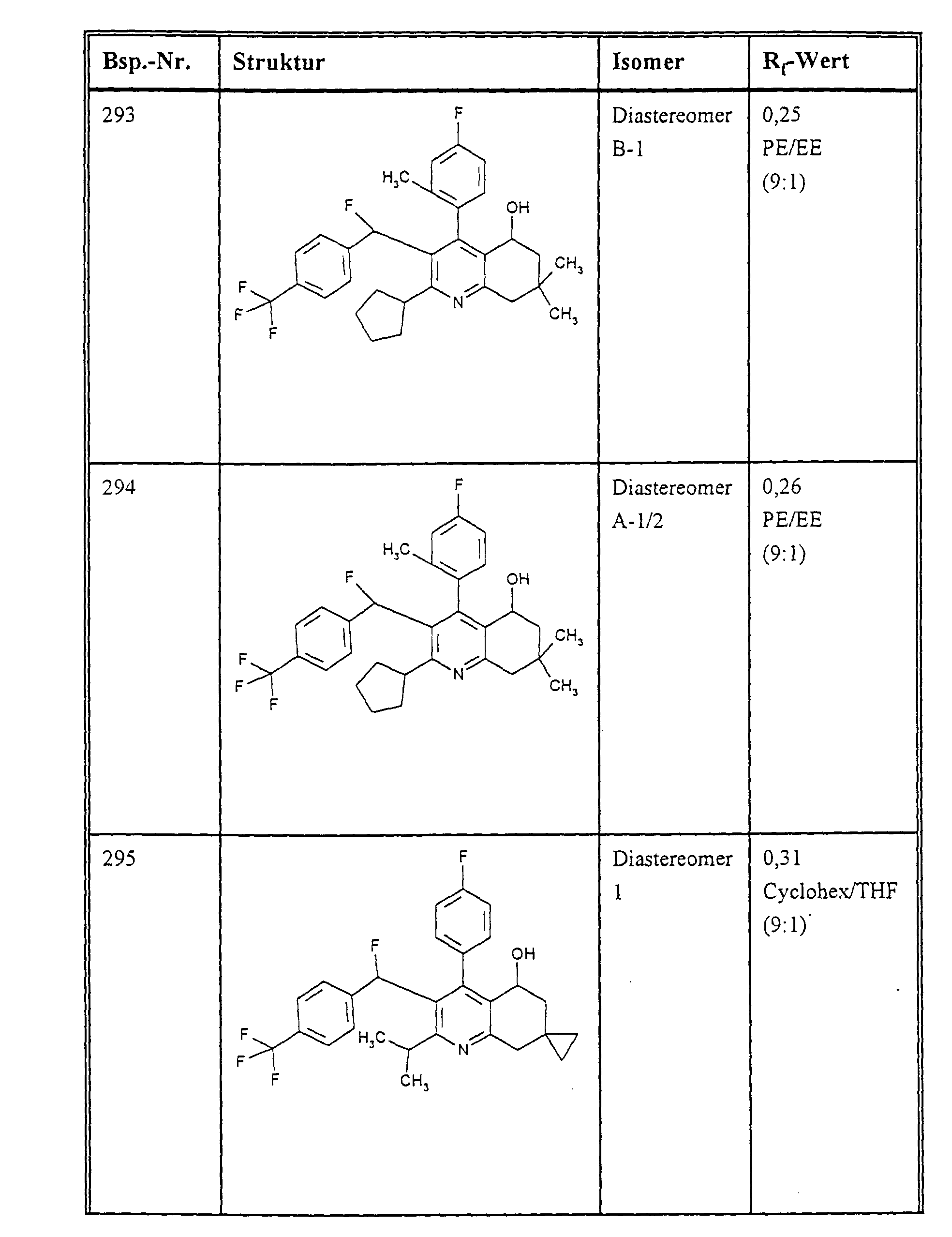

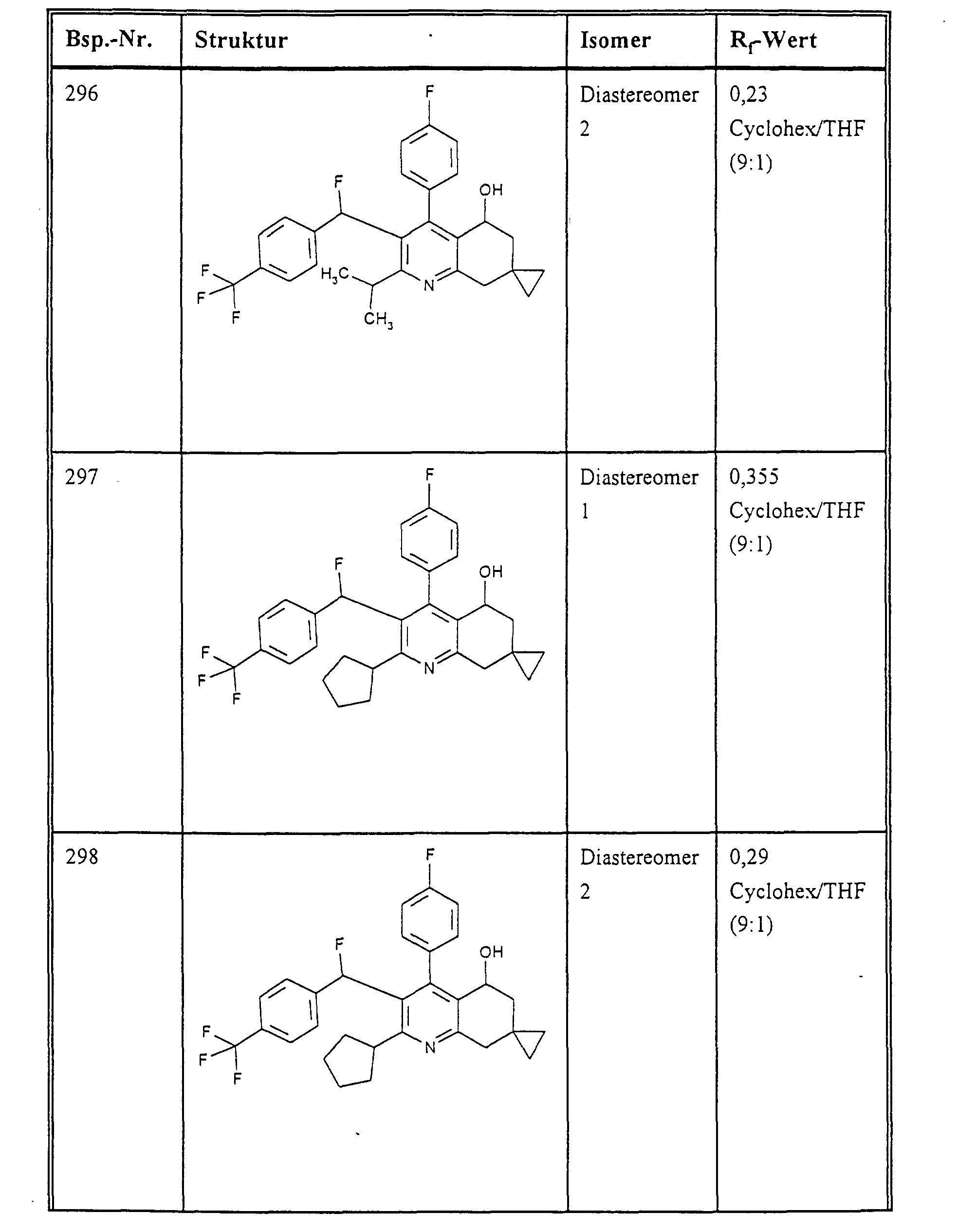

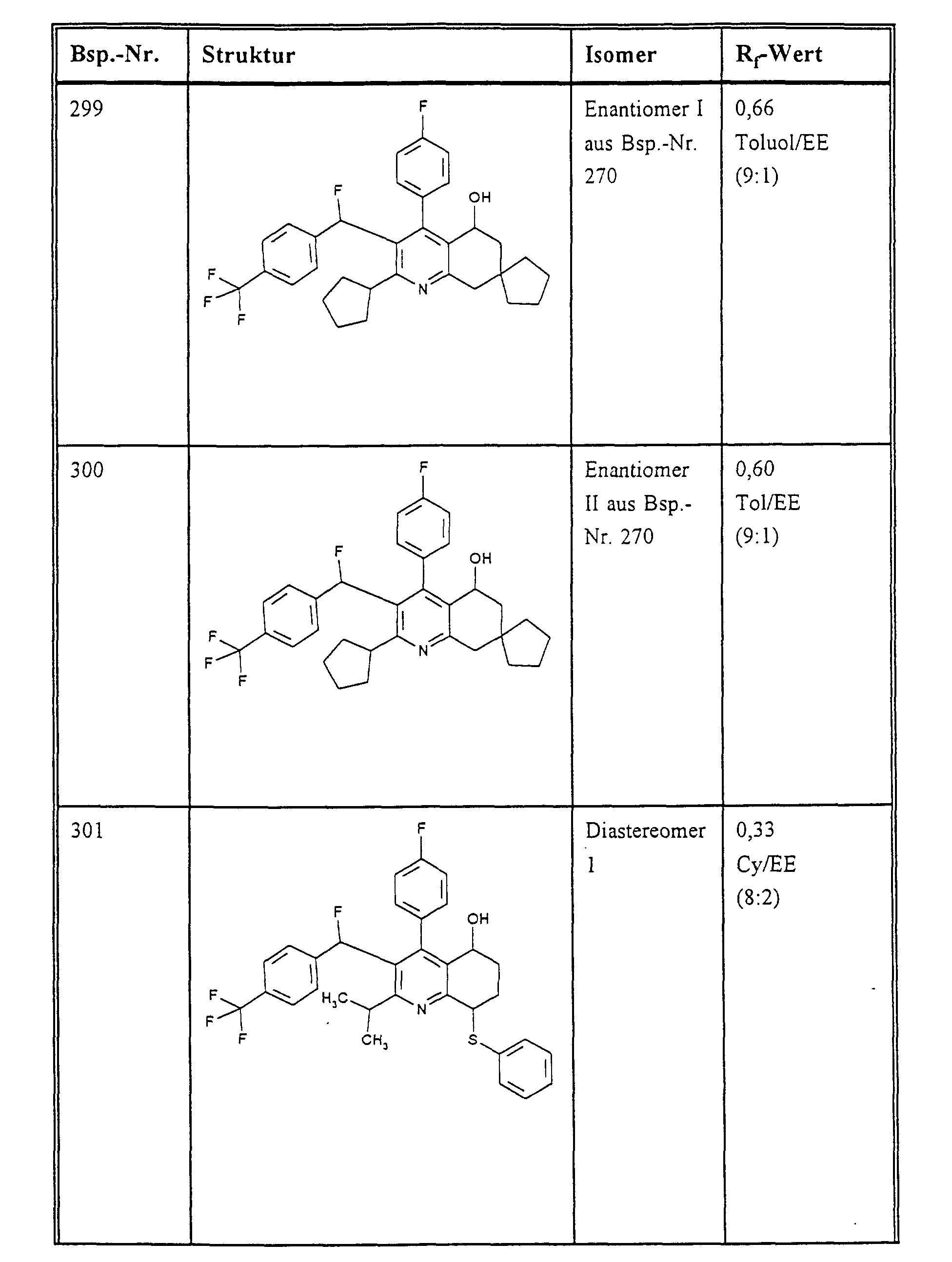

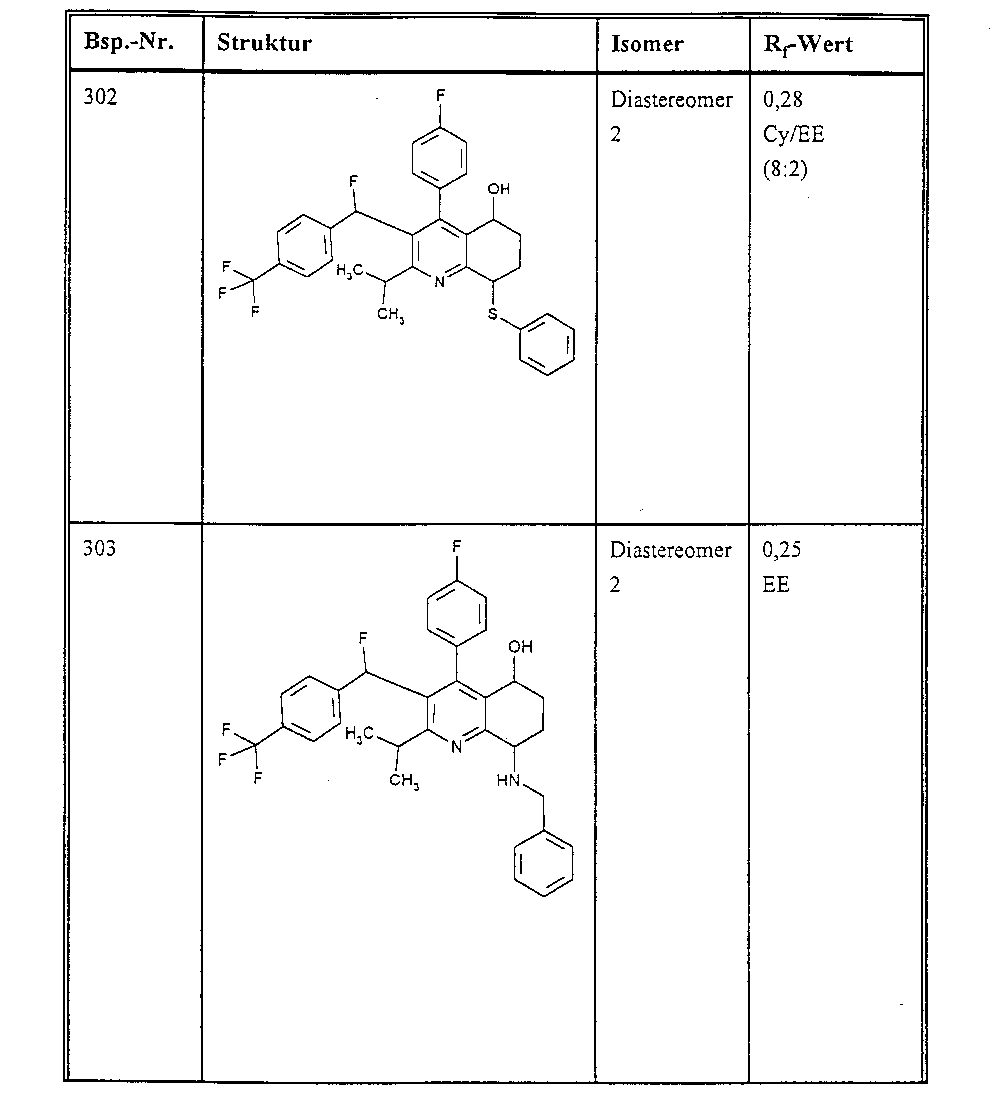

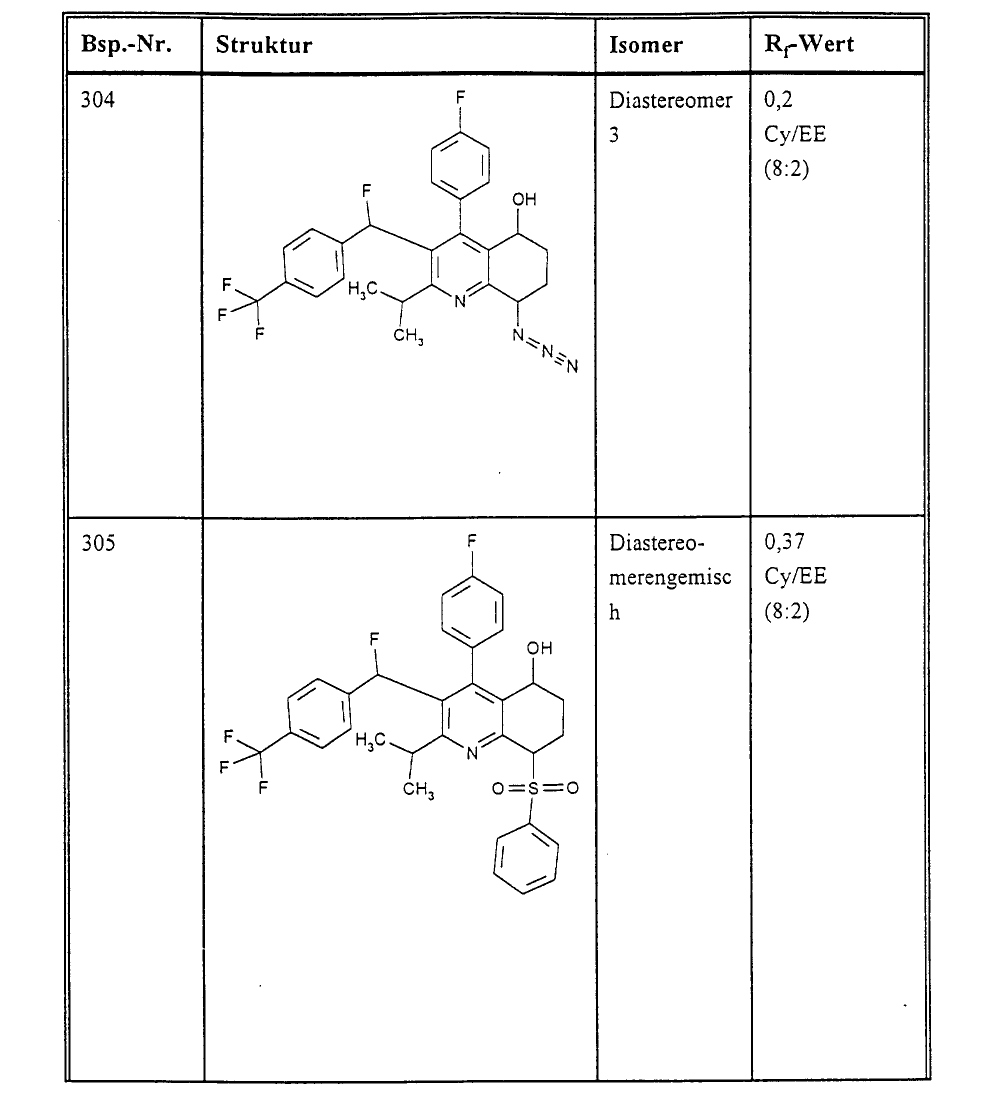

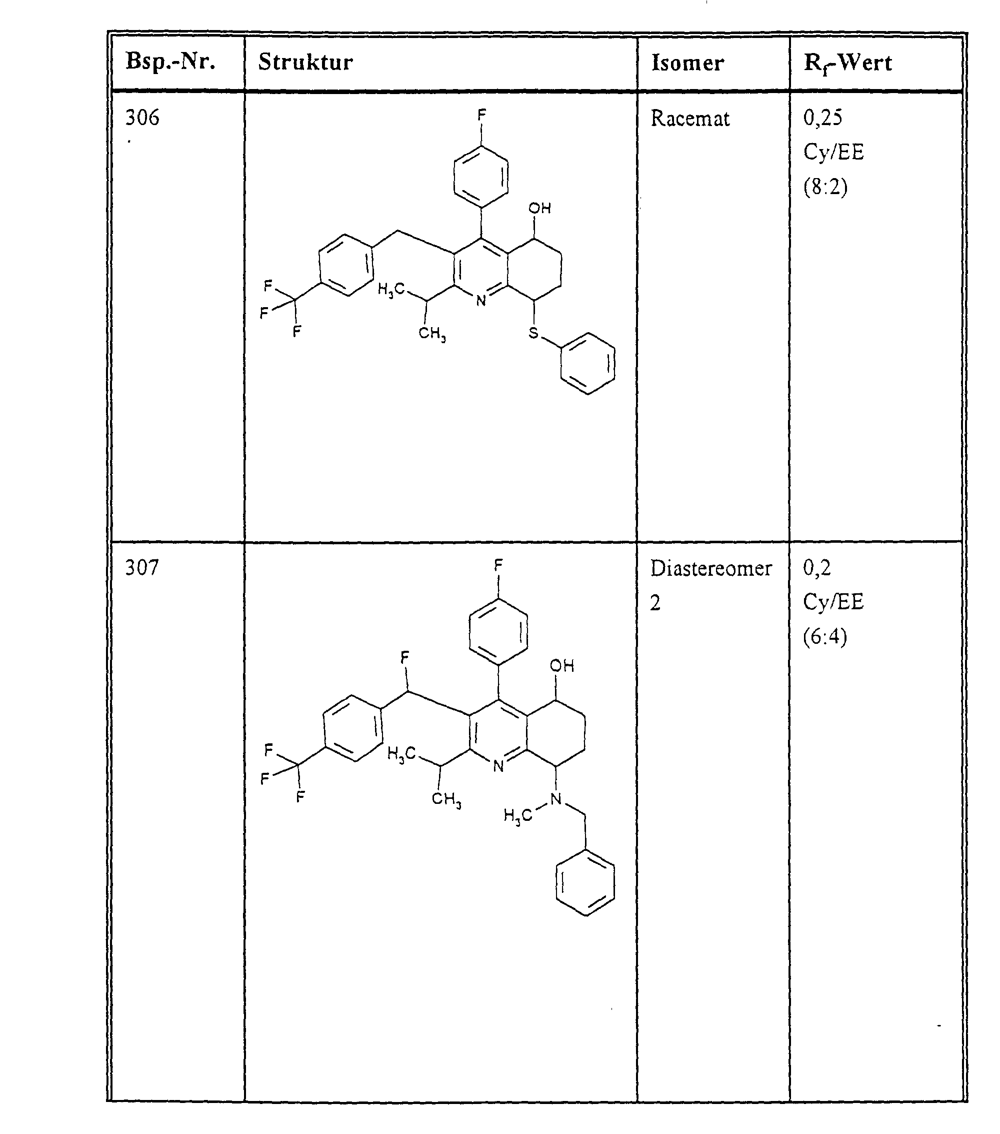

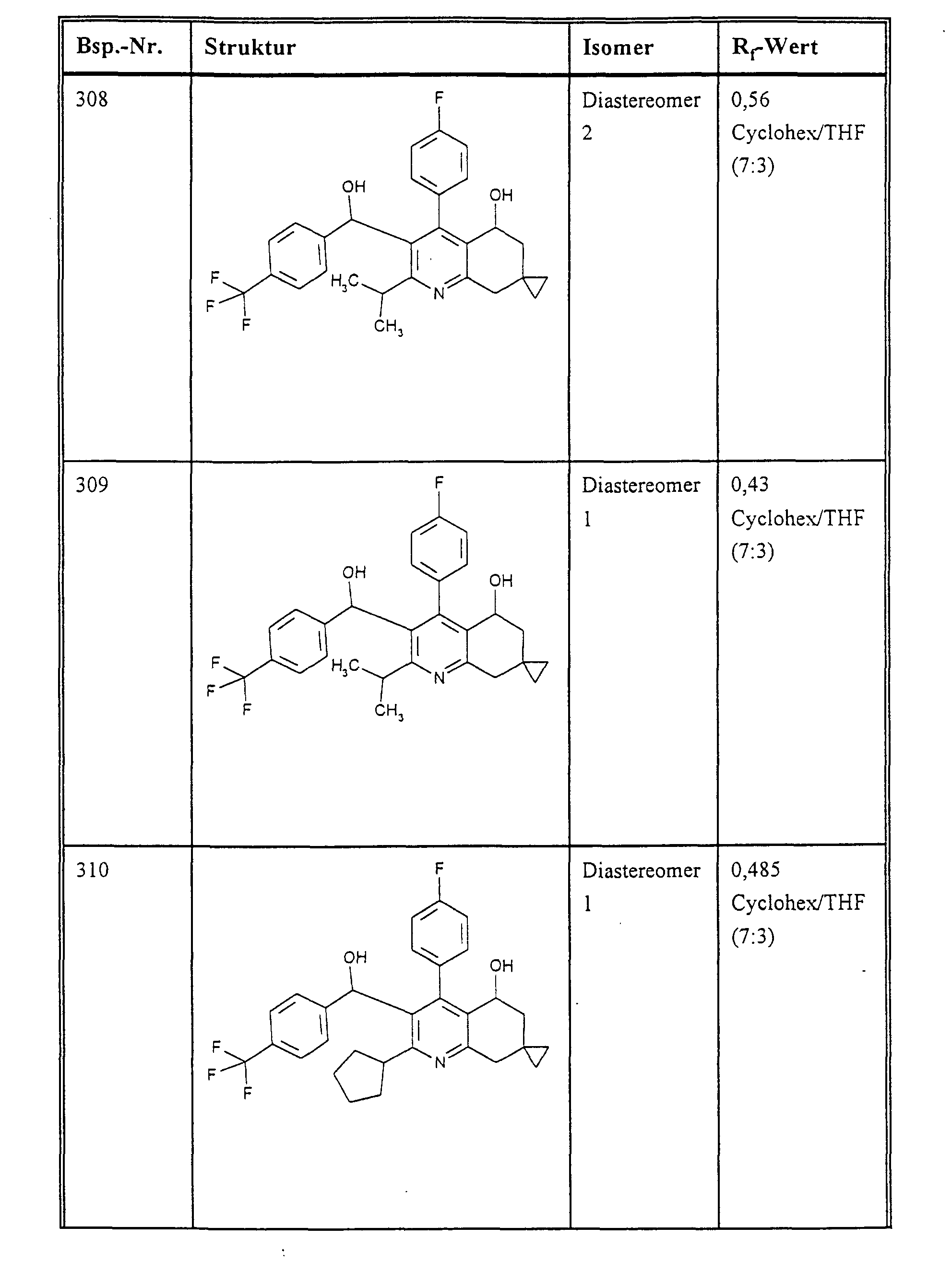

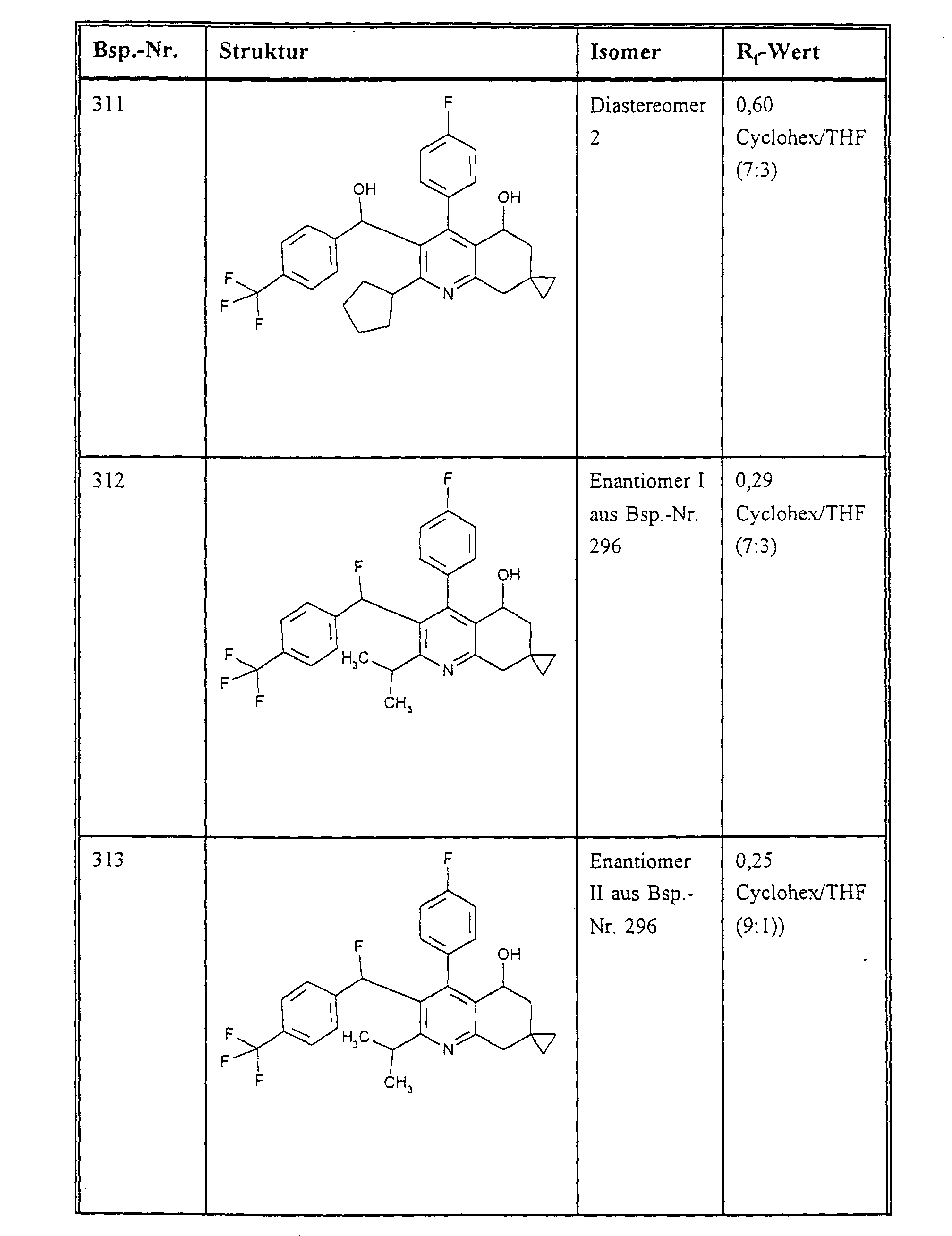

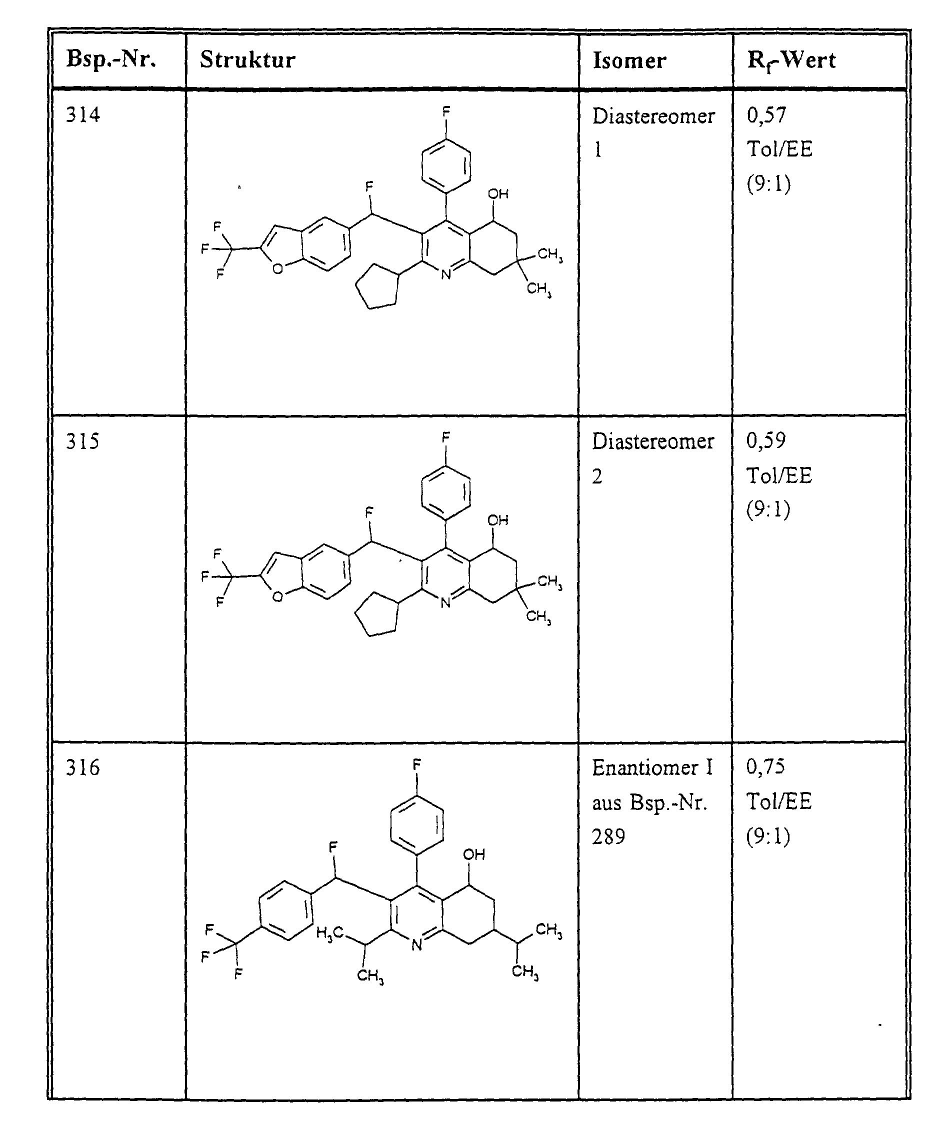

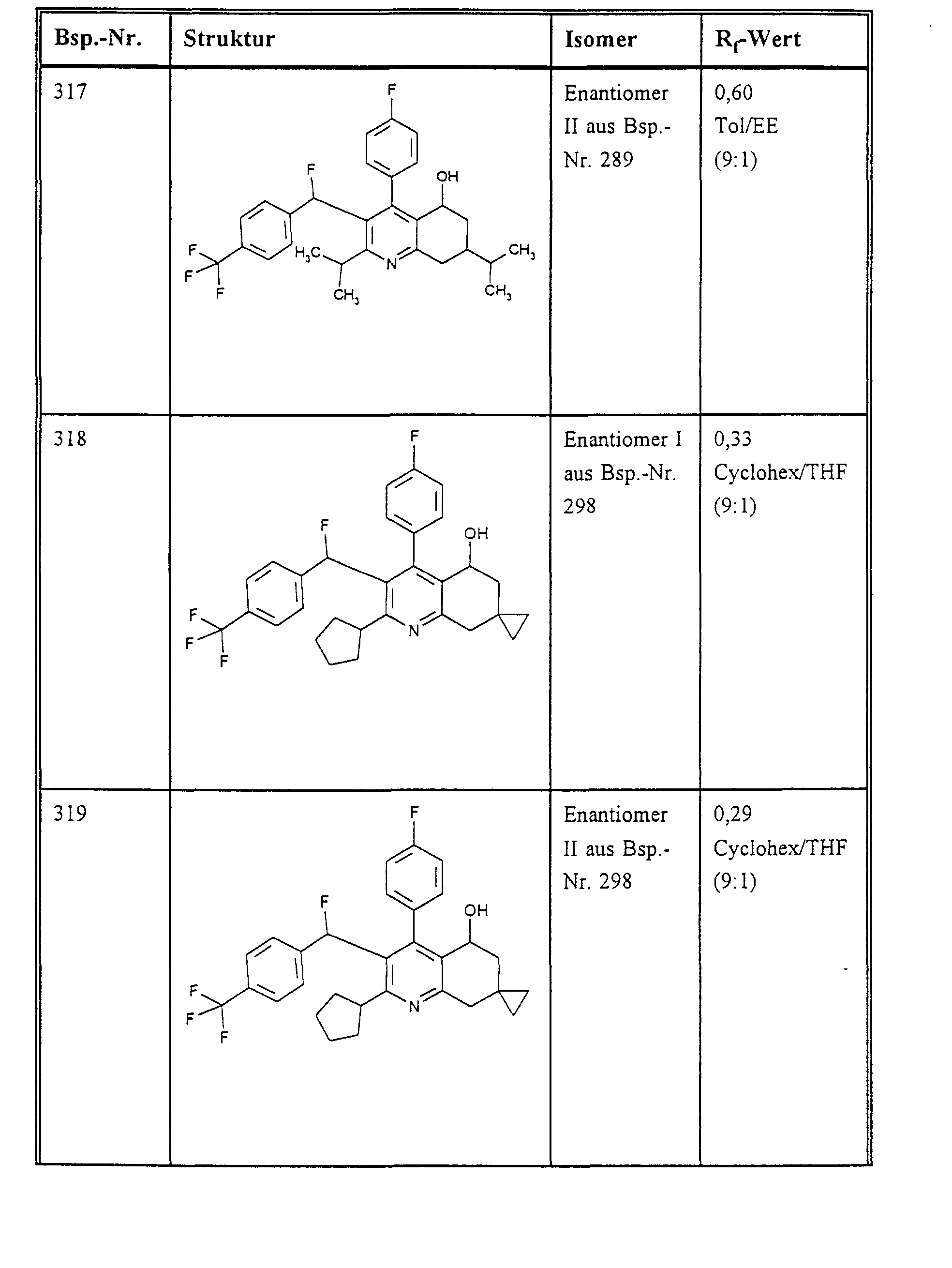

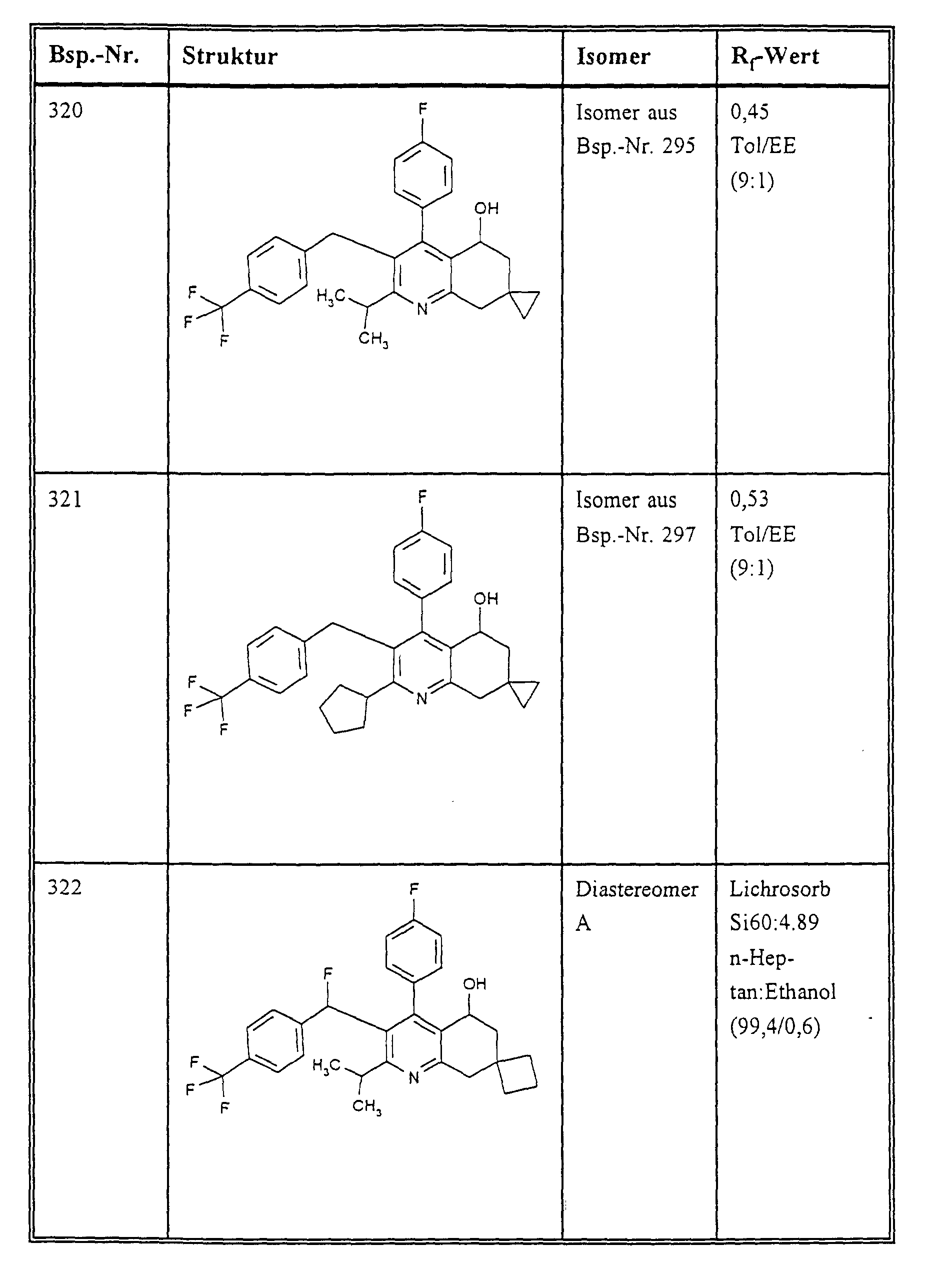

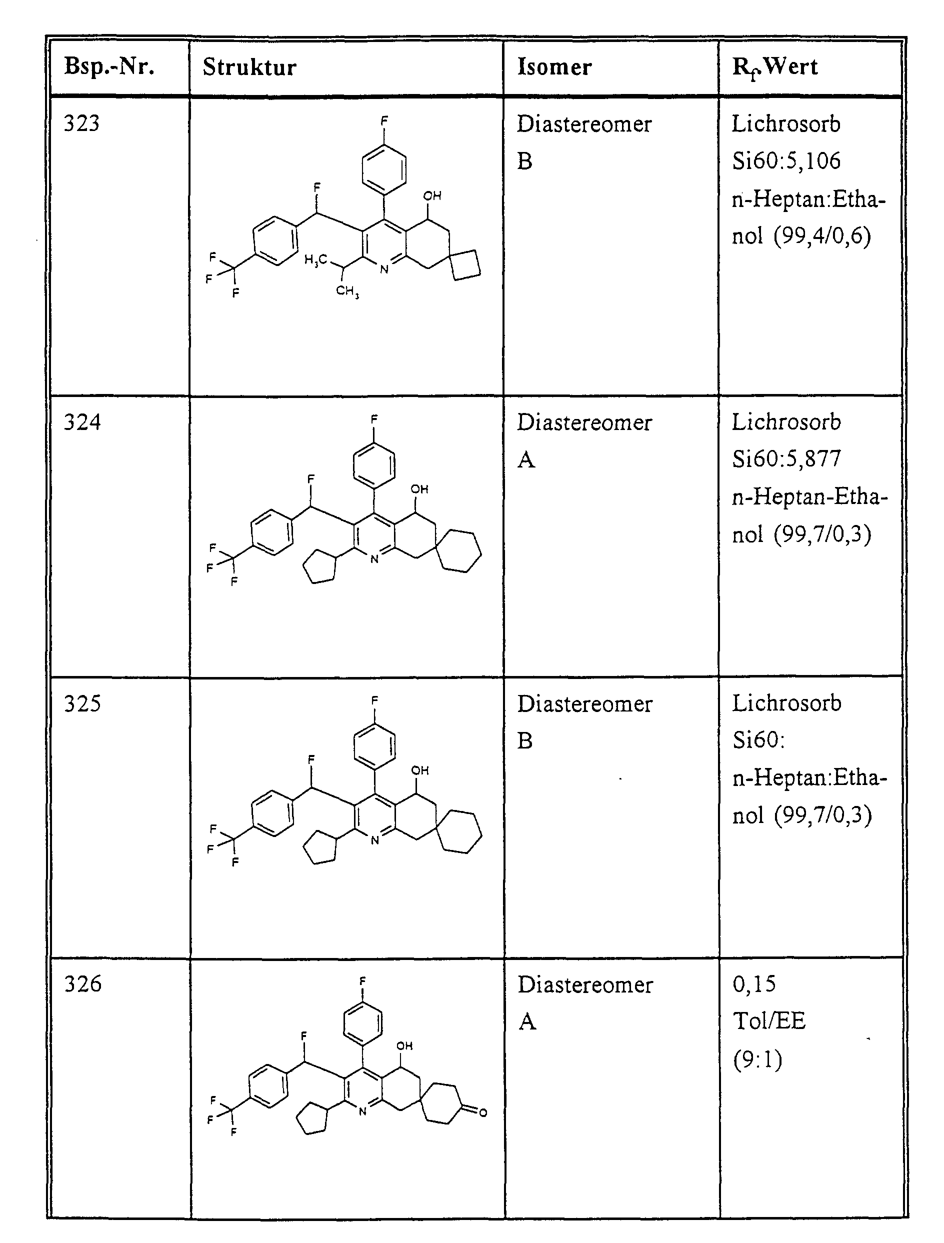

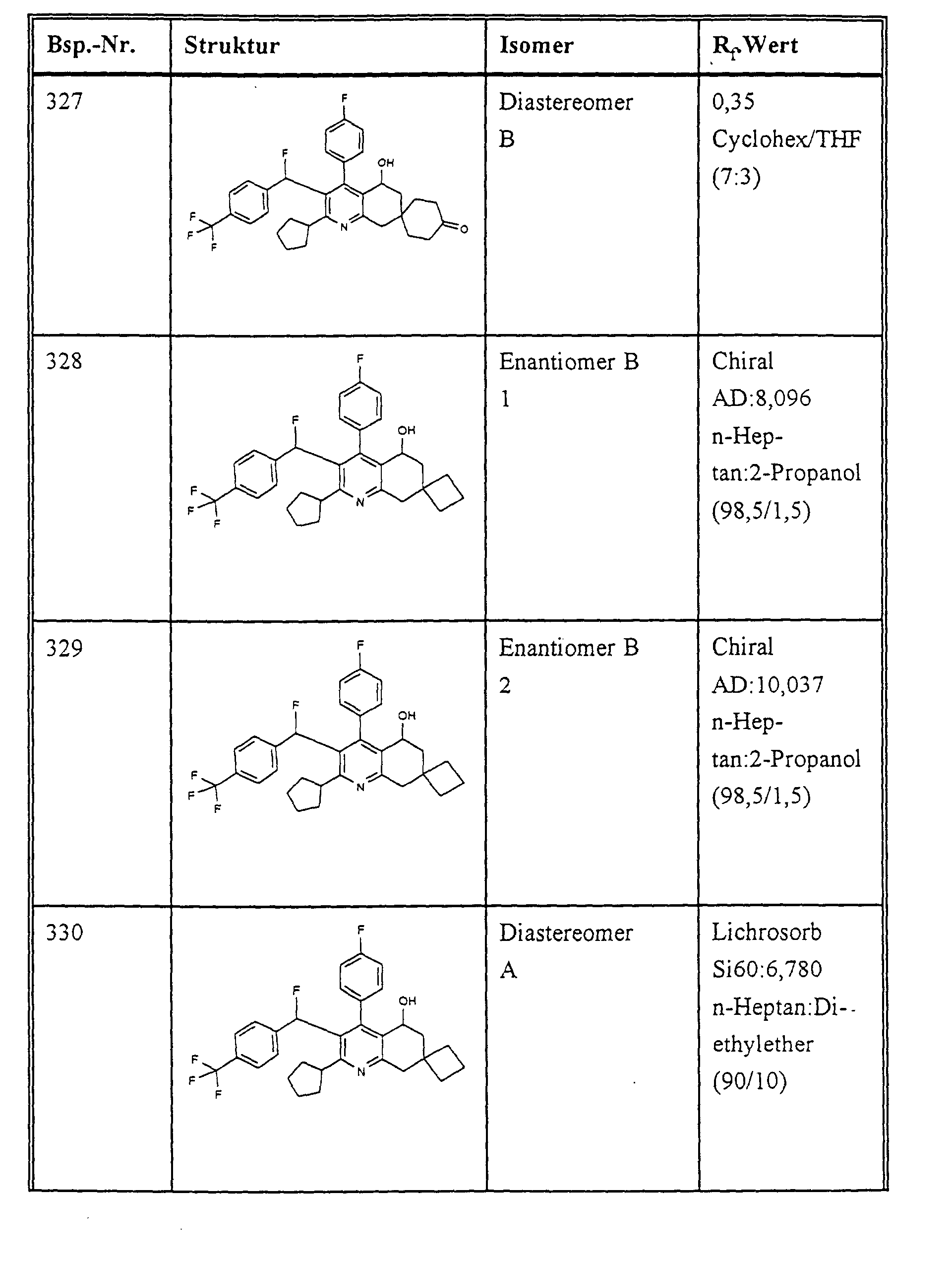

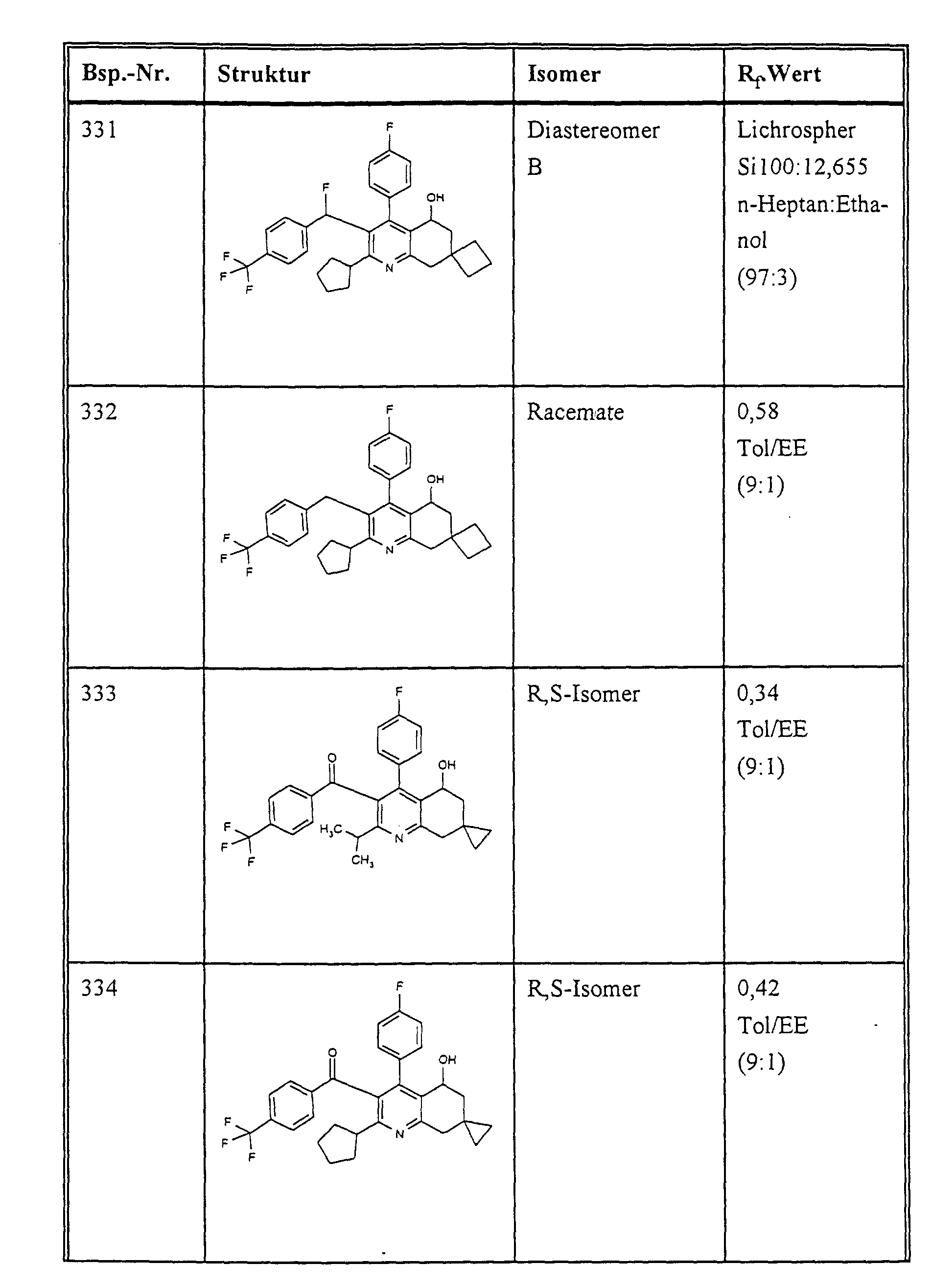

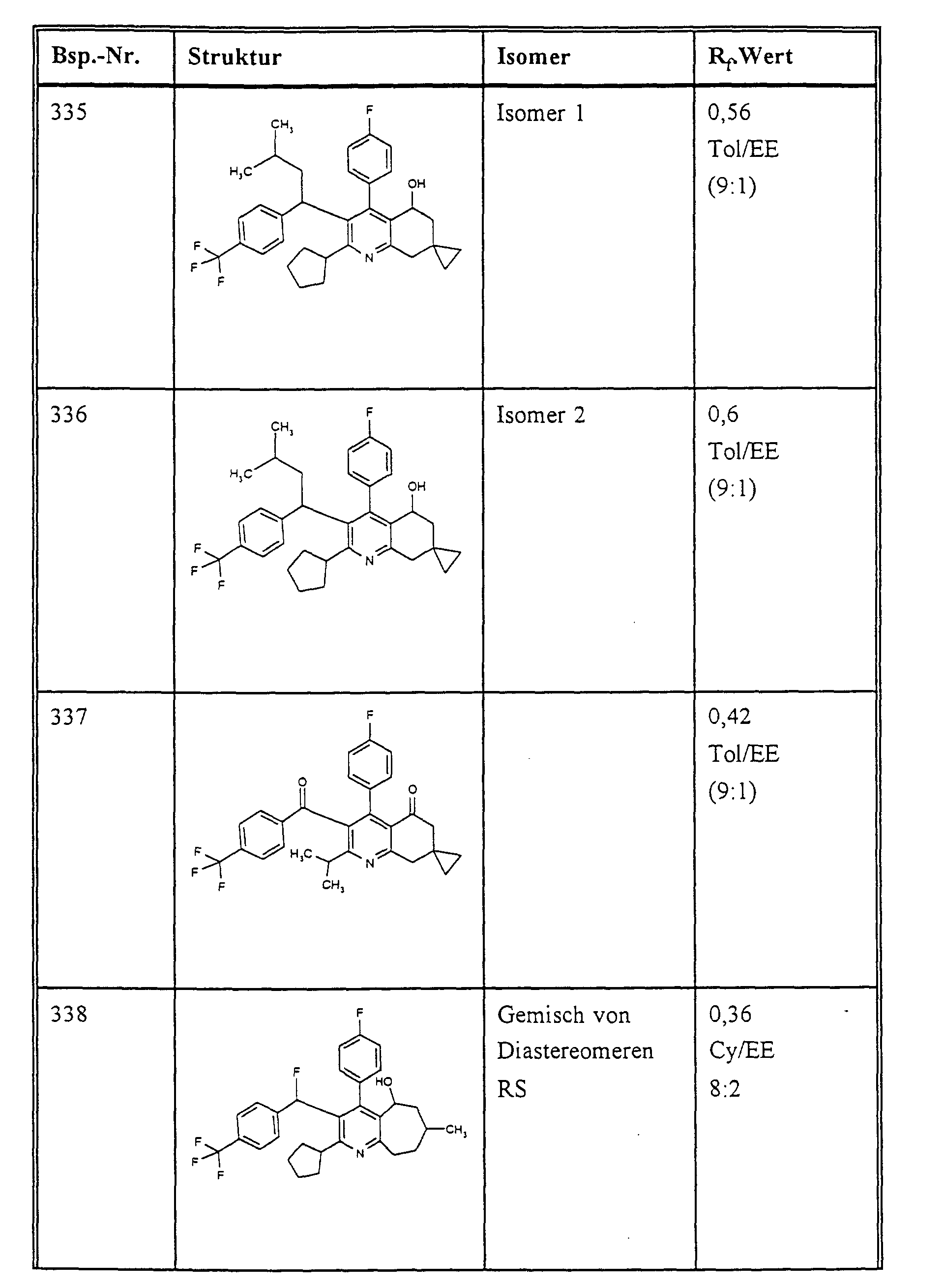

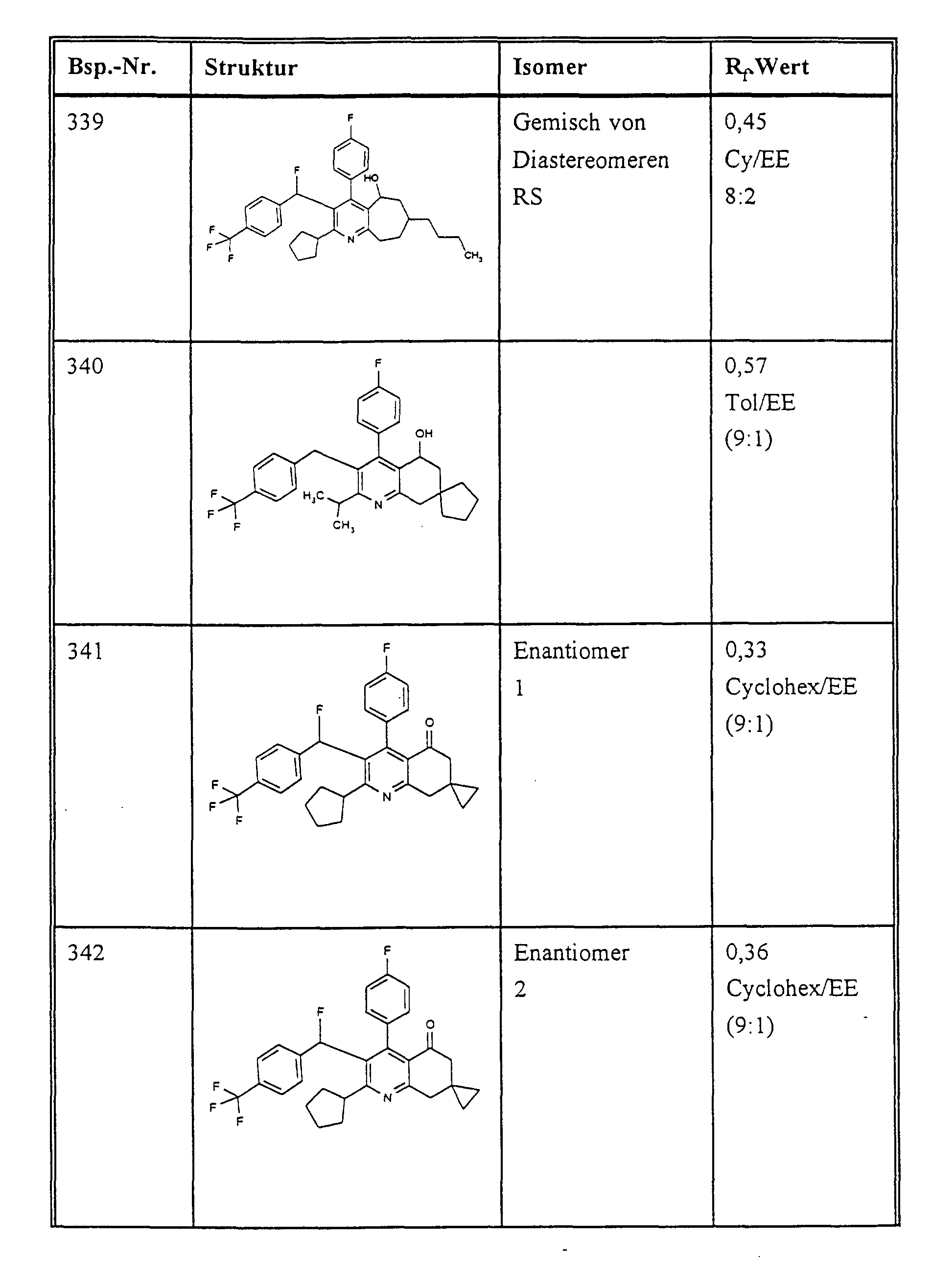

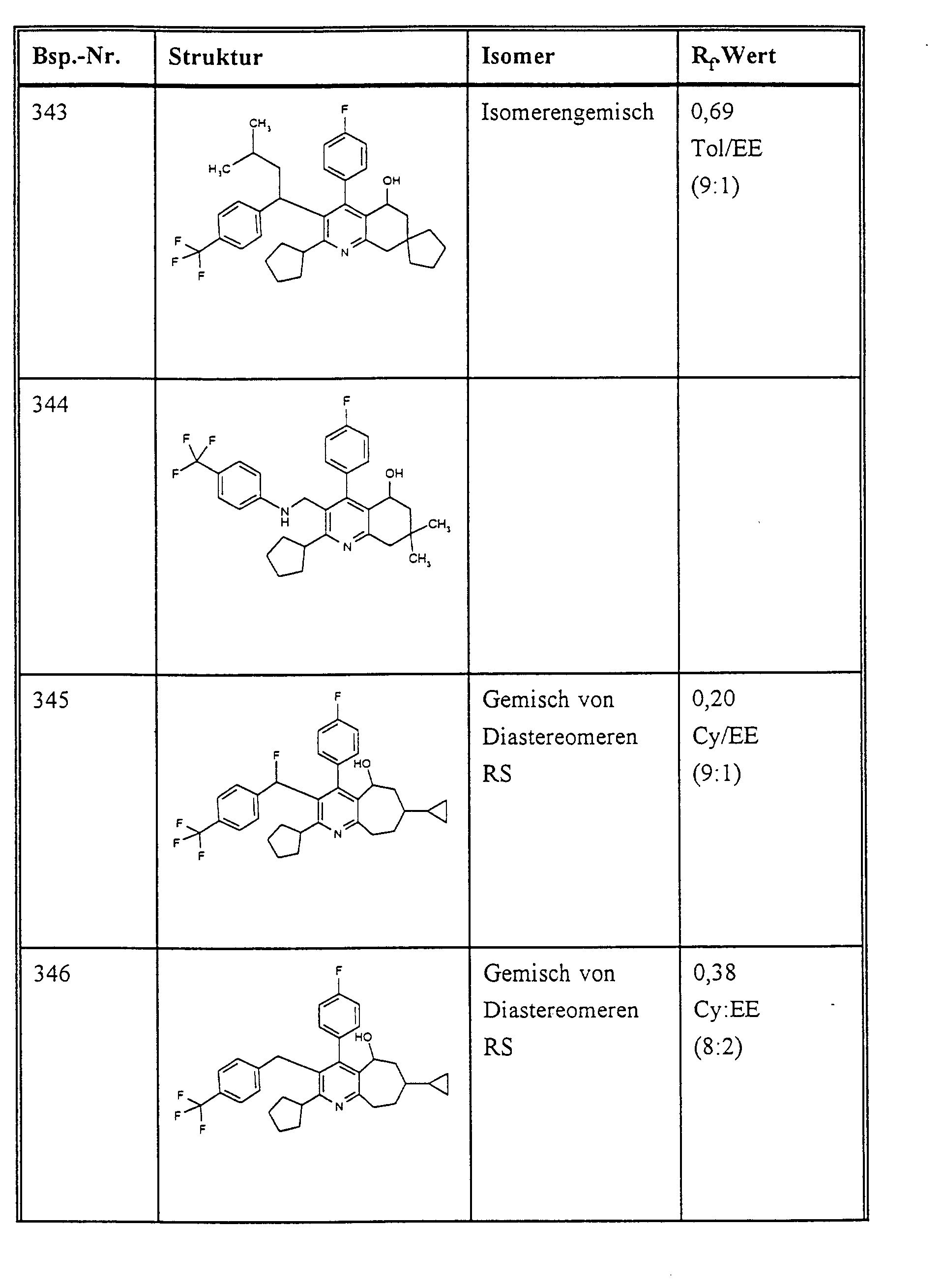

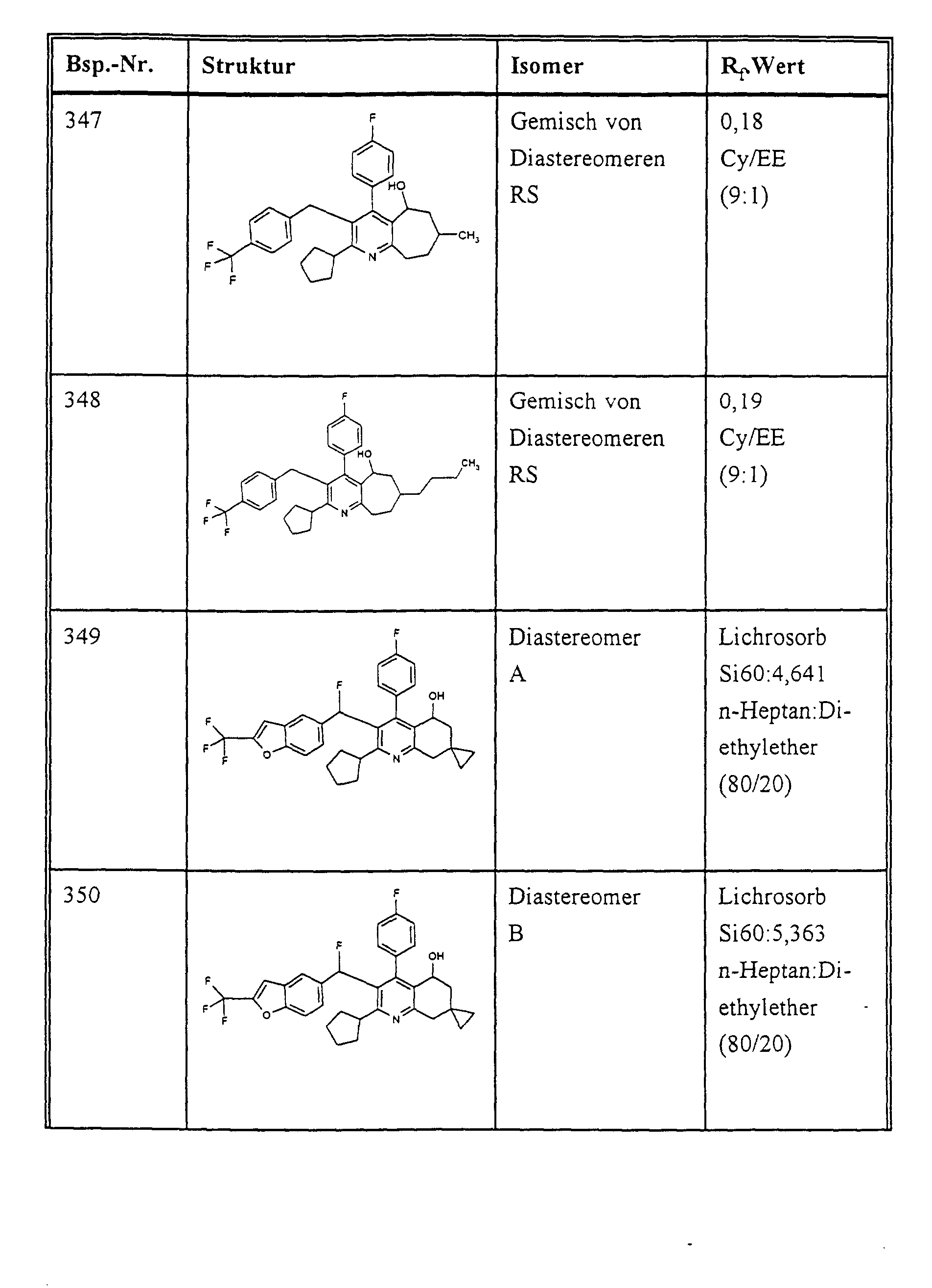

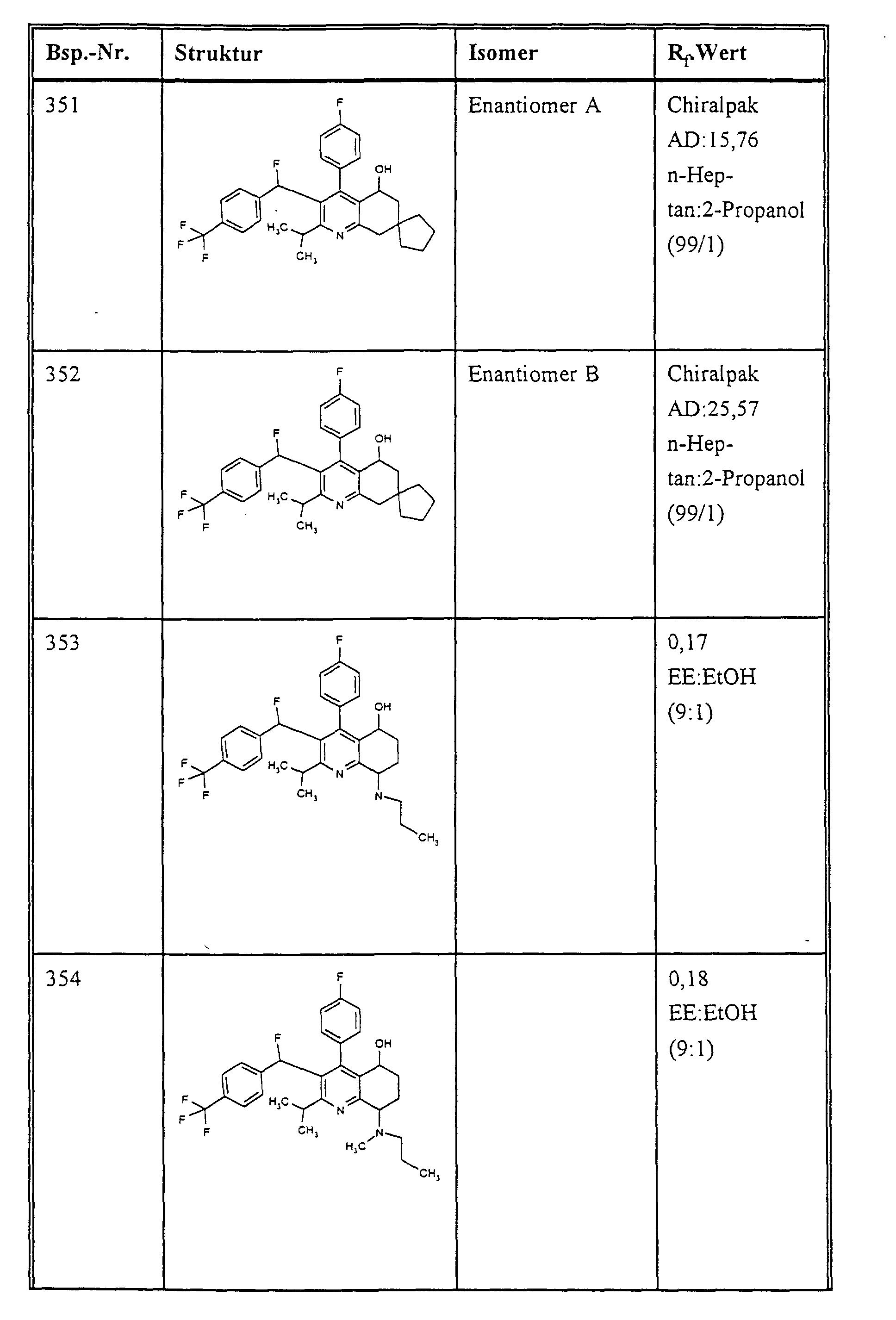

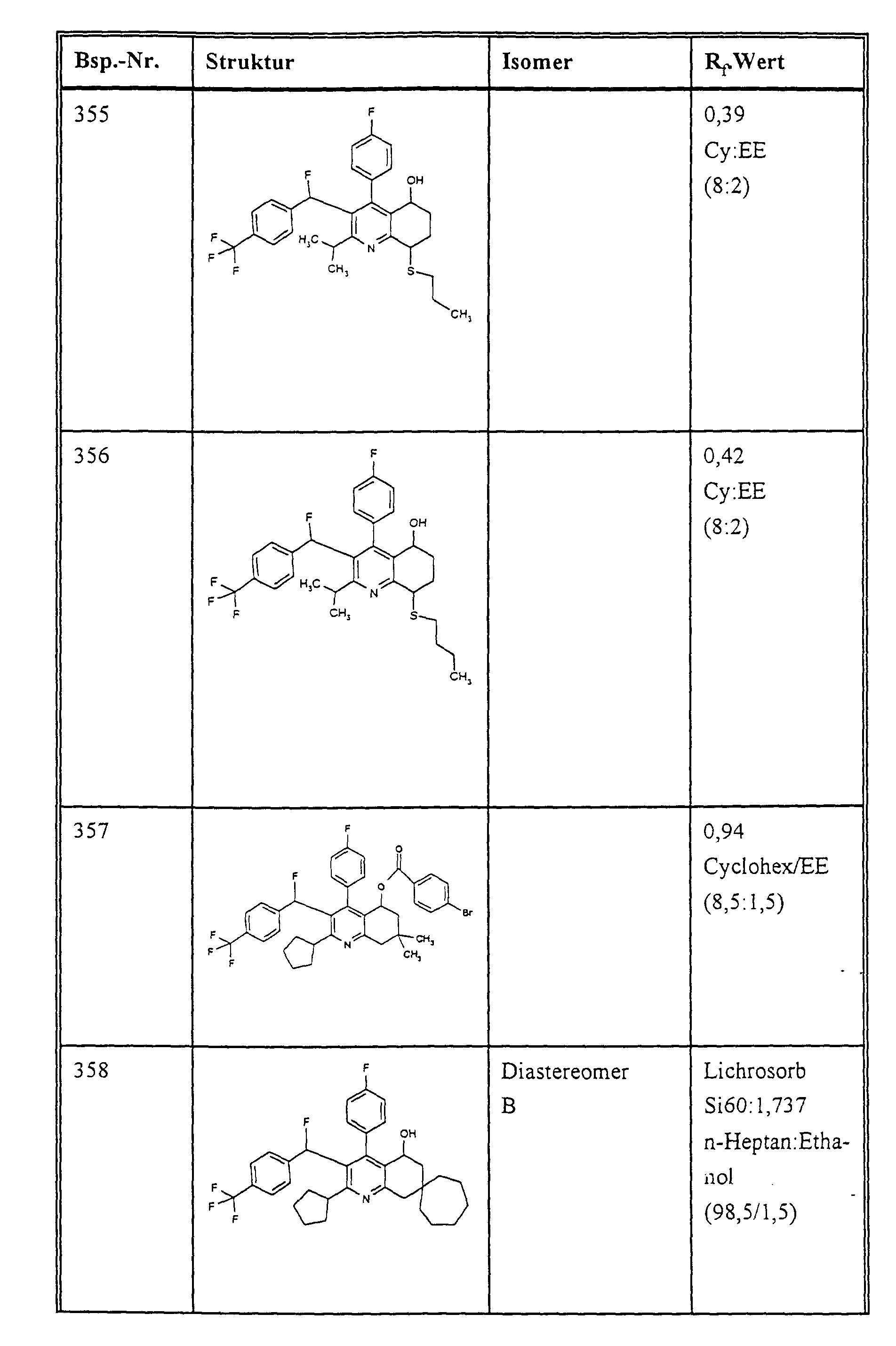

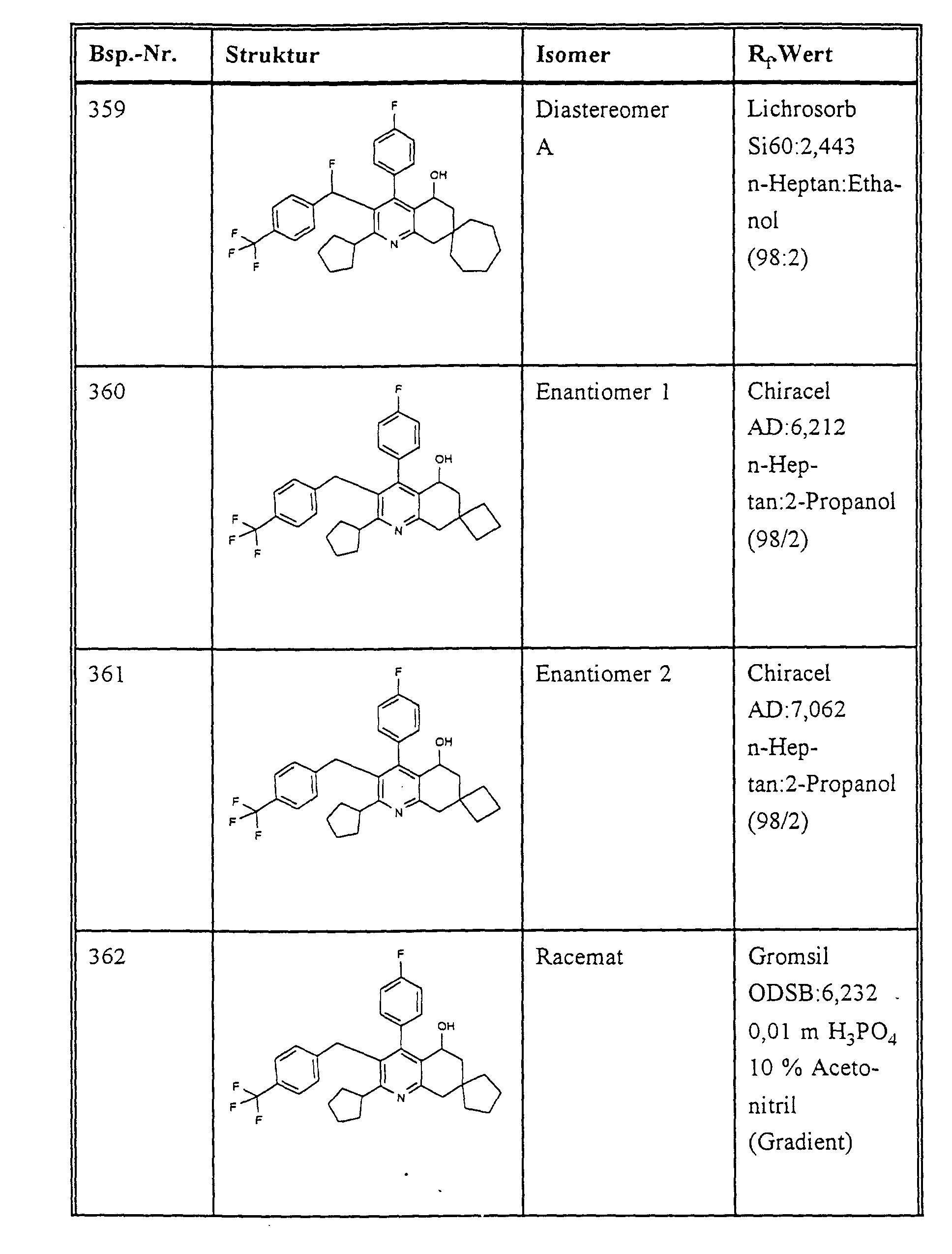

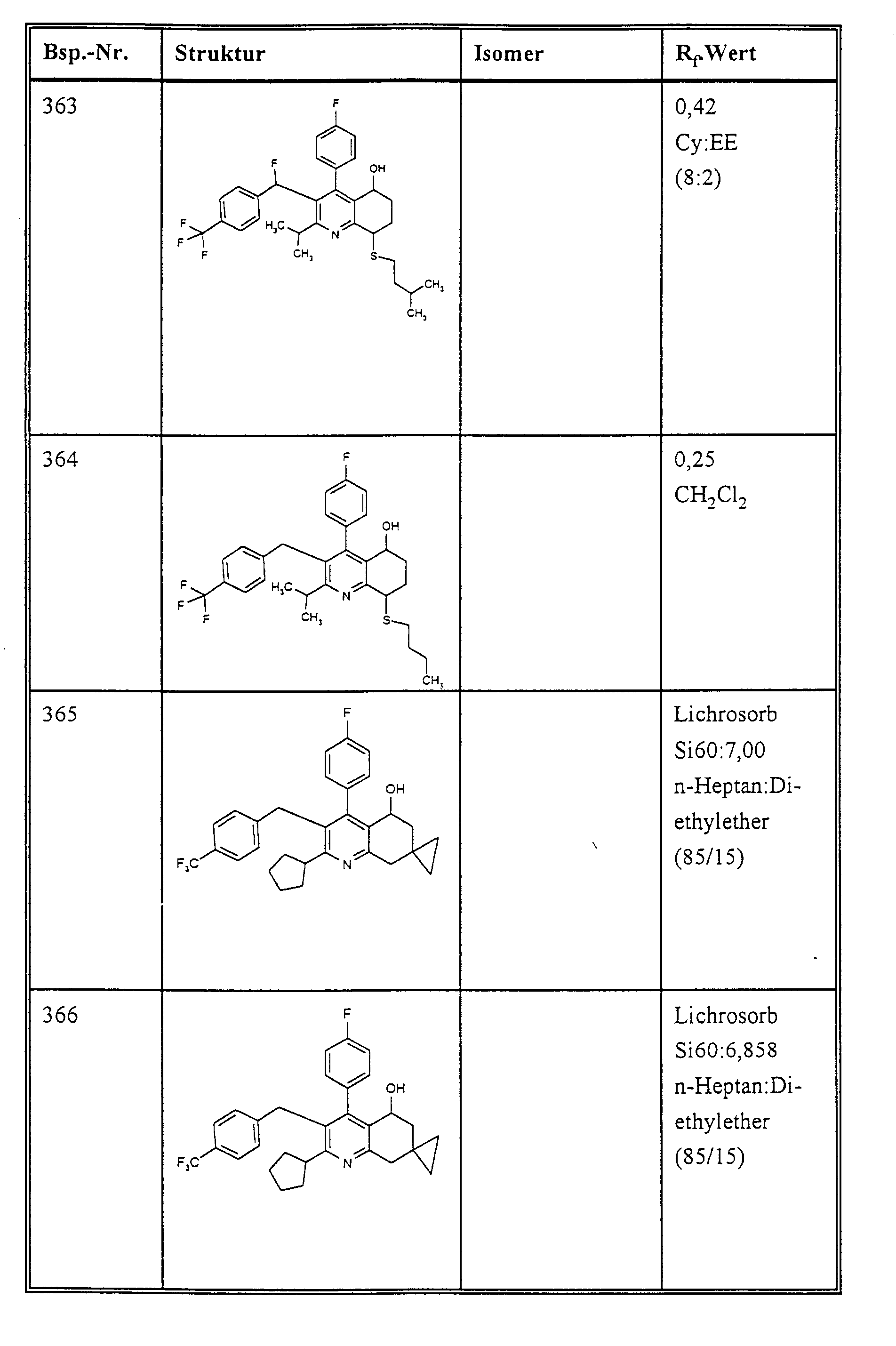

### Beispiel 367

### 2-Cyclopentyl-4-(4-fluorphenyl)-7,7-dimethyl-3-(4-trifluormethylbenzoyl)-7,8-dihydro-6H-chinolin-5-on

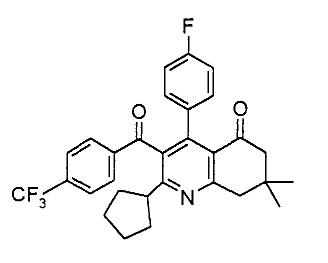

843 g 2-Cyclopentyl-4-(4-fluorphenyl)-7,7-dimethyl-3-(4-trifluormethylbenzoyl)-4,6,7,8-tetrahydro-*1H*-chinolin-5-on wurden mit 7021,3 g Braunstein (aktiviert) in 28 1 Methylenchlorid innerhalb einer Stunde bei Raumtemperatur zum Pyridin oxidiert. Nach dem Abtrennen des Braunsteins und Abdestillieren des Methylenchlorids wurde aus Petrolether umkristallisiert. Es resultierten 618 g Kristalle mit einer Reinheit von 99,3 % nach HPLC. Aus der Mutterlauge der Kristallisation konnten durch Chromatographie an Kieselgel, mit Toluol/Essigester 4:1, weitere 168 g mit gleicher Qualität erhalten werden.
Schmelzpunkt: 186°C.
DC: R_{f} = 0,8 (Toluol/Essigester 4:1)

### Beispiel 368

### [2-Cyclopentyl-4-(4-fluorphenyl)-5-hydroxy-7,7-dimethyl-5,6,7,8-tetrahydrochinolin-3-yl]-(4-trifluormethylphenyl)-methanon

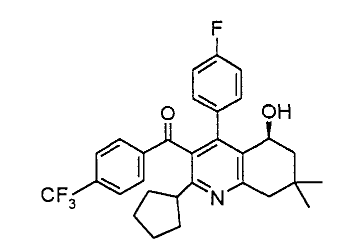

625,3 g 2-Cyclopentyl-4-(4-fluorphenyl)-7,7-dimethyl-3-(4-trifluormethylbenzoyl)-7,8-dihydro-6*H*-chinolin-5-on wurden in 6 l Tetrahydrofuran mit 56,4 g 1R,2S-Aminoindanol und 800 g Borandiethylanilin-Komplex bei 0°C bis -5°C reduziert. Quenchen der Reaktion erfolgte nach etwa 20 Stunden mit 500 ml 1,2-Ethandiol. Das Tetrahydrofuran wurde abdestilliert, das resultierende Öl in Essigester aufgenommen und die organische Phase nach dem Waschen mit 2N Salzsäure und gesättigter Natriumhydrogencarbonatlösung, getrocknet und eingeengt. Der Rückstand wurde heiß aus Cyclohexan umkristallisiert. Es resultierten insgesamt 574 g farblose Kristalle mit einer Reinheit von 99,4 % nach HPLC (e.e. 97,4 %).
Schmelzpunkt: 114°C.
DC: R_{f} = 0,2 (Petrolether/Essigester 9:1)

### Beispiel 369

### [5-(tert-Butyldimethylsilanyloxy)-2-cyclopentyl-4-(4-fluorophenyl)-7,7-dimethyl-5,6,7,8-tetrahydrochinolin-3-yl]-(4-trifluormethylphenyl)-methanon

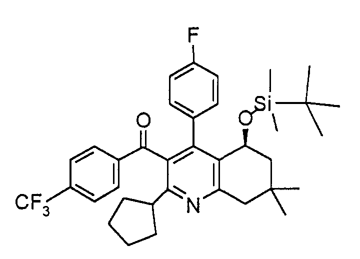

a) 574 g [2-Cyclopentyl-4-(4-fluorphenyl)-5-hydroxy-7,7-dimethyl-5,6,7,8-tetrahydro-chinolin-3-yl]-(4-trifluormethylphenyl)-methanon und 522 ml Lutidin gelöst in 5,4 l Toluol wurden bei -5°C bis Raumtemperatur mit einer Mischung aus 593,2 g tert-Butyldimethylsilyltrifluormethansulfonat und 1 l Toluol innerhalb von 2,5 Stunden umgesetzt. Die Reaktion wurde mit 10 %iger wäßriger Ammonchloridlösung gequencht, die organische Phase mit 0,1 N Salzsäure und gesättigter wäßriger Natriumhydrogencarbonatlösung gewaschen und getrocknet. Nach dem Abdestillieren des Lösungsmittels im Vakuum wurde der Rückstand aus Ethanol umkristallisiert. Es resultierten insgesamt 633 g farblose Kristalle mit einer Reinheit von 99,2 % nach HPLC (e.e. 98,7 %).
   Schmelzpunkt: 108°C.
   DC: R_{f}= 0,8 (Petrolether/Essigester 9:1)
b) Zu 50 mg syn- und 50 mg anti-[5(tert.Butyldimethylsilanyloxy-2-cyclopentyl-4-(4-fluorophenyl)-7,7-dimethyl-5,6,7,8-tetrahydrochinolin-3-yl]-(4-trifluoromethyl-phenyl)-methanol gelöst in 2 ml Dichlormethan wurden portionsweise 320 mg Mangandioxid (Merck Best.-Nr. 805958, 90 %, gefällt, aktiv) hinzugegeben und 7 h gerührt. Das Solvens wurde im Vakuum entfernt und der Rückstand direkt auf eine Flash-Kieselgelsäule aufgetragen. Die Chromatographie mit Petrolether / Essigester 15:1 ergab 93 mg des Produkts.
   DC: R_{f} = 0,6 (Petrolether / Essigester 9:1)

### Beispiel 370

### [5-(tert-Butyldimethylsilanyloxy)-2-cyclopentyl-4-(4-fluorphenyl)-7,7-dimethyl-5,6,7,8-tetrahydrochinolin-3-yl]-(4-trifluormethylphenyl)-methanol

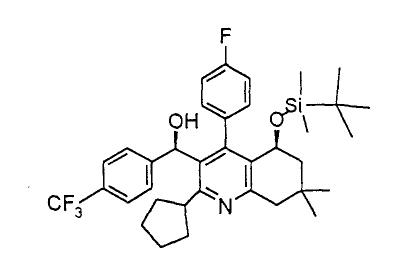

Zu 9 g [5-(tert-Butyldimethylsilanyloxy)-2-cyclopentyl-4-(4-fluorophenyl)-7,7-dimethyl-5,6,7,8-tetrahydrochinolin-3-yl]-(4-trifluormethylphenyl)-methanon gelöst in 60 ml Toluol wurden 16,5 ml Natriumbis-(2-methoxyethoxy)-dihydroaluminatlösung (65 %ig in Toluol) getropft. Die Reaktion wurde nach 3,5 Stunden mit Methanol gequencht, mit Essigester extrahiert und die organische Phase mit Kalium-Natriumtartratlösung und gesättigter wäßriger Natriumhydrogencarbonatlösung gewaschen und getrocknet. Nach dem Abdestillieren des Lösungsmittels im Vakuum wurde der Rückstand aus Petrolether umkristallisiert. Es resultierten insgesamt 4,8 g farblose Kristalle mit einer Reinheit von 99,4 % nach HPLC (e.e. 99,0 %). Aus der Mutterlauge der Kristallisation konnten durch Chromatographie an Kieselgel, mit Petrolether/Essigester 9:1, weitere 1,8 g mit gleicher Qualität erhalten werden.
Schmelzpunkt: 142°C.
DC: R_{f}= 0,5 (Petrolether/Essigester 9:1)

### Beispiel 371

### 5-(tert-Butyldimethylsilanyloxy)-2-cyclopentyl-4-(4-fluorphenyl)-3-[fluor-(4-trifluormethylphenyl)-methyl]-7,7-dimethyl-5,6,7,8-tetrahydrochinolin

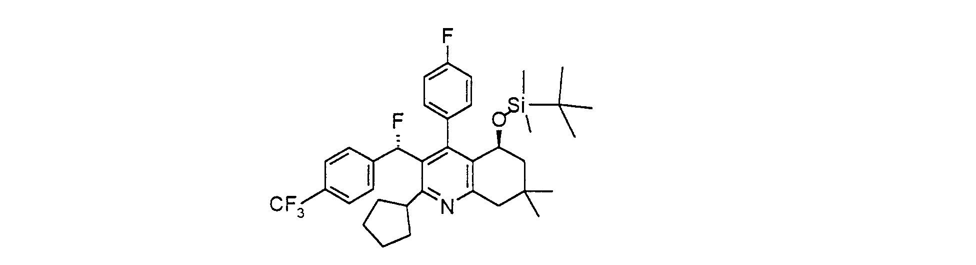

Zu 3,8 g [5-(tert-Butyldimethylsilanyloxy)-2-cyclopentyl-4-(4-fluorphenyl)-7,7-dimethyl-5,6,7,8-tetrahydrochinolin-3-yl]-(4-trifluormethylphenyl)-methanol gelöst in 37,8 ml Toluol wurden bei -5°C 1,46 g Diethylaminoschwefeltrifluorid gelöst in 10 ml Toluol getropft. Die Reaktion wurde nach 30 Minuten mit gesättigter wäßriger Natriumhydrogencarbonatlösung gequencht und die organische Phase nochmals mit gesättigter wäßriger Natriumhydrogencarbonatlösung gewaschen und getrocknet. Nach dem Abdestillieren des Lösungsmittels im Vakuum wurde der Rückstand aus Ethanol umkristallisiert. Es resultierten insgesamt 3,33 g farblose Kristalle mit einer Reinheit von 99,4 % nach HPLC. Aus der Mutterlauge der Kristallisation konnten durch Chromatographie an Kieselgel, mit Petrolether/ Essigester 10:1, weitere 0,26 g mit gleicher Qualität erhalten werden.
Schmelzpunkt: 128°C.
DC: R_{f} = 0,8 (Petrolether/Essigester 9:1)

### Beispiel 372

### 5-(tert-Butyldimethylsilanyloxy)-2-cyclopentyl-4-(4-fluorphenyl)-3-[fluor-(4-trifluormethylphenyl)-methyl]-7,7-dimethyl-5,6,7,8-tetrahydrochinolin

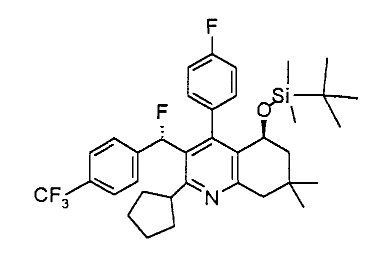

2 g [5-(tert-Butyldimethylsilanyloxy)-2-cyclopentyl-4-(4-fluorphenyl)-7,7-dimethyl-5,6,7,8-tetrahydrochinolin-3-yl]-(4-trifluormethylphenyl)-methanol und 2,14 ml N,N-Diethyl-1,1,2,3,3,3-hexafluorpropylamin wurden unter Argon in 25 ml Toluol 18 Stunden bei 60°C gerührt. Die Aufarbeitung erfolgte durch Eingießen in gesättigte Natriumhydrogencarbonatlösung, Abtrennen der organischen Phase, erneute Extraktion mit gesättigter Natriumhydrogencarbonatlösung, Trocknen und Einengen im Vakuum. Der Rückstand wurde aus heißem Ethanol umkristallisiert und ergab nach dem Trocknen 1,3 g farblose Kristalle mit einer Reinheit von 99,4 % nach HPLC. Aus der Mutterlauge der Kristallisation konnten durch Chromatographie an Kieselgel, mit Petrolether/Essigester 10:1, weitere 0,3 g mit gleicher Qualität erhalten werden.

### Beispiel 373

### 2-Cyclopentyl-4-(4-fluorophenyl)-3-[fluor-(4-trifluormethylphenyl)-methyl]-7,7-dimethyl-5,6,7,8-tetrahydrochinolin-5-ol

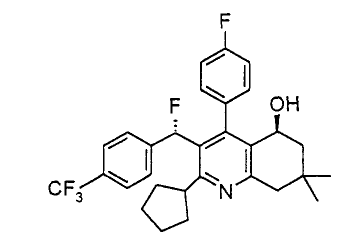

110 g 5-(tert-Butyldimethylsilanyloxy)-2-cyclopentyl-4-(4-fluorphenyl)-3-[fluor-(4-trifluormethylphenyl)-methyl]-7,7-dimethyl-5,6,7,8-tetrahydrochinolin wurden in einer Mischung aus 913 ml 5 N Salzsäure, 1364 ml Methanol und 902 ml Tetrahydrofuran 2 Stunden bei 40°C gerührt. Aufarbeitung erfolgte durch Eingießen in gesättigte wäßrige Natriumhydrogencarbonatlösung und Extraktion mit Essigester. Die organischen Phasen wurden nach erneutem Waschen mit gesättigter wäßriger Natriumhydrogencarbonatlösung getrocknet. Nach dem Abdestillieren des Lösungsmittels im Vakuum wurde der Rückstand aus Cyclohexan umkristallisiert. Nach dem Trocknen im Ölpumpenvakuum resultierten insgesamt 71,1 g farblose Kristalle mit einer Reinheit von 99,4 % nach HPLC (e.e. 99,5 %). Aus der Mutterlauge der Kristallisation konnten durch Chromatographie an Kieselgel, mit Petrolether/Essigester 7:1, weitere 10,8 g mit gleicher Qualität erhalten werden.
Schmelzpunkt: 140°C.
DC: R_{f}=0,2 (Petrolether/Essigester 9:1)

## Patentansprüche

1. Cycloalkanopyridinen der allgemeinen Formel (I) 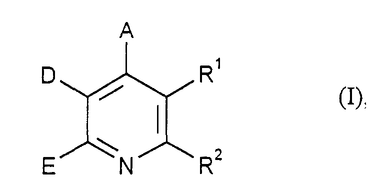 in welcher
A für Aryl mit 6 bis 10 Kohlenstoffatomen steht, das gegebenenfalls bis zu 5-fach gleich oder verschieden durch Halogen, Nitro, Hydroxy, Trifluormethyl, Trifluormethoxy oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert sind,
worin
R³ und R⁴ gleich oder verschieden sind und Wasserstoff, Phenyl oder geradkettiges oder verzweigtes Alkyl mit bis zu 6 Kohlenstoffatomen bedeuten,
D für Aryl mit 6 bis 10 Kohlenstoffatomen steht, das gegebenenfalls durch Phenyl, Nitro, Halogen, Trifluormethyl oder Trifluormethoxy substituiert ist, oder
für einen Rest der Formel
R⁵―L― 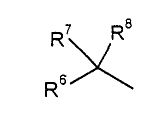
oder
R⁹―T―V―X―
steht,
worin
R⁵, R⁶ und R⁹ unabhängig voneinander Cycloalkyl mit 3 bis 6 Kohlenstoffatomen bedeuten, oder
Aryl mit 6 bis 10 Kohlenstoffatomen bedeuten oder einen 5- bis 7-gliedrigen, gegebenenfalls benzokondensierten, gesättigten oder ungesättigten, mono-, bi- oder tricyclischen Heterocyclus mit bis zu 4 Heteroatomen aus der Reihe S, N und/oder O bedeuten,
wobei die Cyclen, gegebenenfalls, im Fall der stickstoffhaltigen Ringe auch über die N-Funktion, bis zu 5-fach gleich oder verschieden durch Halogen, Trifluormethyl, Nitro, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 6 Kohlenstoffatomen, durch Aryl oder Trifluormethyl substituiertes Aryl mit 6 jeweils bis 10 Kohlenstoffatomen oder durch einen, gegebenenfalls benzokondensierten, aromatischen 5- bis 7- gliedrigen Heterocyclus mit bis zu 3 Heteroatomen aus der Reihe S, N und/oder O substituiert sind,
und/oder durch eine Gruppe der Formel -OR¹⁰, -SR¹¹, -SO₂R¹² oder -NR¹³R¹⁴ substituiert sind,
worin
R¹⁰, R¹¹ und R¹² unabhängig voneinander Aryl mit 6 bis 10 Kohlenstoffatomen bedeuten, das seinerseits gegebenenfalls bis zu 2- fach gleich oder verschieden durch Phenyl, Halogen oder durch geradkettiges oder verzweigtes Alkyl mit bis zu 6 Kohlenstoffatomen substituiert ist,
R¹³ und R¹⁴ gleich oder verschieden sind und die oben angegebene Bedeutung von R³ und R⁴ haben,
oder
R⁵ und/oder R⁶ einen Rest der Formel 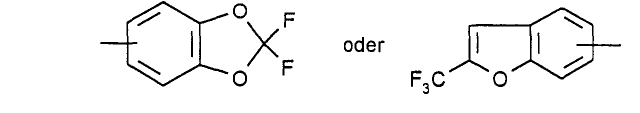 bedeuten,
R⁷ Wasserstoff oder Halogen bedeutet,
und
R⁸ Wasserstoff, Halogen, Azido, Trifluormethyl, Hydroxy, Trifluormethoxy, geradkettiges oder verzweigtes Alkoxy oder Alkyl mit jeweils bis zu 6 Kohlenstoffatomen oder einen Rest der Formel -NR¹⁵R¹⁶ bedeutet,
worin
R¹⁵ und R¹⁶ gleich oder verschieden sind und die oben angegebene Bedeutung von R³ und R⁴ haben,
oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 6 Kohlenstoffatomen bedeutet,
L eine geradkettige oder verzweigte Alkylen- oder Alkenylen-Kette mit jeweils bis zu 8 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 8 Kohlenstoffatomen bedeuten,
oder
T oder X eine Bindung bedeuten,
V für ein Sauerstoff- oder Schwefelatom oder für eine -NR¹⁸- Gruppe steht,
worin
R¹⁸ Wasserstoff oder geradkettiges oder verzweigtes Alkyl mit bis zu 6 Kohlenstoffatomen oder Phenyl bedeutet,
E für Cycloalkyl mit 3 bis 8 Kohlenstoffatomen steht, oder für geradkettiges oder verzweigtes Alkyl mit bis zu 8 Kohlenstoffatomen steht, das gegebenenfalls durch Cycloalkyl mit 3 bis 8 Kohlenstoffatomen oder Hydroxy substituiert ist, oder für Phenyl steht, das gegebenenfalls durch Halogen oder Trifluormethyl substituiert ist,
R¹ und R² gemeinsam eine geradkettige oder verzweigte Alkylenkette mit bis zu 7 Kohlenstoffatomen bilden, die durch eine oxogruppe und/oder durch einen Rest der Formel 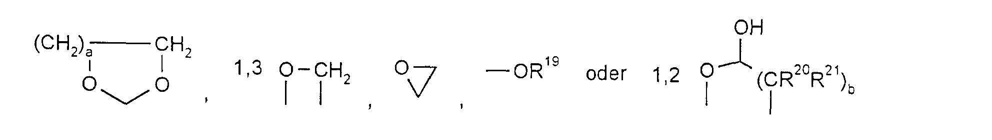 substituiert sein muß,
worin
a und b gleich oder verschieden sind und eine Zahl 1, 2 oder 3 bedeuten,
R¹⁹ Wasserstoff, Cycloalkyl mit 3 bis 7 Kohlenstoffatomen, geradkettiges oder verzweigtes Silylalkyl mit bis zu 8 Kohlenstoffatomen oder geradkettiges oder verzweigtes Alkyl mit bis zu 8 Kohlenstoffatomen bedeutet, das gegebenenfalls durch Hydroxy, geradkettiges oder verzweigtes Alkoxy mit bis zu 6 Kohlenstoffatomen oder durch Phenyl substituiert ist, das seinerseits durch Halogen, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 4 Kohlenstoffatomen oder Benzyl bedeutet,
oder
R¹⁹ geradkettiges oder verzweigtes Acyl mit bis zu 20 Kohlenstoffatomen oder Benzoyl bedeutet, das gegebenenfalls durch Halogen, Trifluormethyl, Nitro oder Trifluormethoxy substituiert ist, oder
geradkettiges oder verzweigtes Fluoracyl mit bis zu 8 Kohlenstoffatomen und 9 Fluoratomen bedeutet,
R²⁰ und R²¹ gleich oder verschieden sind, Wasserstoff, Phenyl oder geradkettiges oder verzweigtes Alkyl mit bis zu 6 Kohlenstoffatomen bedeuten,
oder
R²⁰ und R²¹ gemeinsam einen 3 bis 6-gliedrigen Carbocyclus bilden,
und, gegebenenfalls auch geminal, die gebildeten Carbocyclen gegebenenfalls bis zu 6-fach gleich oder verschieden durch Trifluormethyl, Hydroxy, Nitril, Halogen, Carboxyl, Nitro, Azido, Cyano, Cycloalkyl oder Cycloalkyloxy mit jeweils 3 bis 7 Kohlenstoffatomen, durch geradkettiges oder verzweigtes Alkoxycarbonyl, Alkoxy oder Alkylthio mit jeweils bis zu 6 Kohlenstoffatomen oder durch geradkettiges oder verzweigtes Alkyl mit bis zu 6 Kohlenstoffatomen substituiert sind, das seinerseits bis zu 2-fach gleich oder verschieden durch Hydroxyl, Benzyloxy, Trifluormethyl, Benzoyl, geradkettiges oder verzweigtes Alkoxy, Oxyacyl oder Carboxyl mit jeweils bis zu 4 Kohlenstoffatomen und/oder Phenyl substituiert ist, das seinerseits durch Halogen, Trifluormethyl oder Trifluormethoxy substituiert sein kann,
und/oder die gebildeten Carbocyclen, auch geminal, gegebenenfalls bis zu 5-fach gleich oder verschieden durch Phenyl, Benzoyl, Thiophenyl oder Sulfonylbenzyl substituiert sind, die ihrerseits gegebenenfalls durch Halogen, Trifluormethyl, Trifluormethoxy oder Nitro substituiert sind,
und/oder gegebenenfalls durch einen Rest der Formel 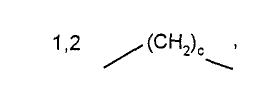
-SO₂-C₆H₅,
-(CO)_{d}-NR²³R²⁴
oder=O
substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cycloalkyl mit 3 bis 6 Kohlenstoffatomen, geradkettiges oder verzweigtes Alkyl mit bis zu 6 Kohlenstoffatomen, Benzyl oder Phenyl bedeuten, das gegebenenfalls bis zu 2-fach gleich oder verschieden durch Halogen, Trifluormethyl, Cyano, Phenyl oder Nitro substituiert ist,
und/oder die gebildeten Carbocyclen gegebenenfalls durch einen spiro-verknüpften Rest der Formel 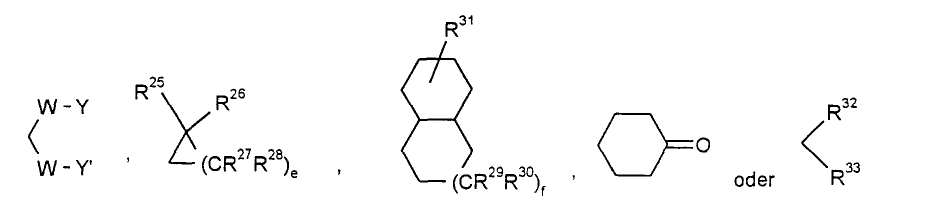 substituiert sind,
worin
W entweder ein Sauerstoff oder ein Schwefelatom bedeutet,
Y und Y' gemeinsam eine 2- bis 6-gliedrige geradkettige oder verzweigte Alkylenkette bilden,
e eine Zahl 1, 2, 3, 4, 5, 6 oder 7 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Halogen oder geradkettiges oder verzweigtes Alkyl oder Alkoxy mit jeweils bis zu 6 Kohlenstoffatomen bedeuten,
oder
R²⁵ und R²⁶ oder R²⁷ und R²⁸ jeweils gemeinsam eine geradkettige oder verzweigte Alkylkette mit bis zu 6 Kohlenstoffatomen bilden,
oder
R²⁵ und R²⁶ oder R²⁷ und R²⁸ jeweils gemeinsam einen Rest der Formel 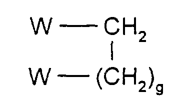 bilden,
worin
W die oben angegebene Bedeutung hat,
g eine Zahl 1, 2, 3, 4, 5, 6 oder 7 bedeutet,
R³² und R³³ gemeinsam einen 3- bis 7-gliedrigen Heterocyclus bilden, der ein Sauerstoff- oder Schwefelatom oder eine Gruppe der Formel SO, SO₂ oder -NR³⁴ enthält,
worin
R³⁴ Wasserstoff, Phenyl, Benzyl oder geradkettiges oder verzweigtes Alkyl mit bis zu 4 Kohlenstoffatomen bedeutet,
und deren Salze und N-Oxide, mit Ausnahme von 5(6H)-Quinolone,3-benzoyl-7,8-dihydro-2,7,7-trimethyl-4-phenyl.

2. Cycloalkano-pyridine der Formel nach Anspruch 1,
in welcher
A für Naphthyl oder Phenyl steht, die gegebenenfalls bis zu 2-fach gleich oder verschieden durch Fluor, Chlor, Brom, Amino, Hydroxy, Trifluormethyl, Trifluormethoxy oder durch geradkettiges oder verzweigtes Alkyl, oder Alkoxy mit jeweils bis zu 6 Kohlenstoffatomen substituiert sind,
D für Phenyl steht, das gegebenenfalls durch Nitro, Fluor, Chlor, Brom, Phenyl, Trifluormethyl oder Trifluormethoxy substituiert ist, oder
für einen Rest der Formel
R⁵―L― 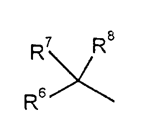
oder
R⁹―T―V―X―
steht,
worin
R⁵, R⁶ und R⁹ unabhängig voneinander Cyclopropyl, Cyclopentyl oder Cyclohexyl bedeuten, oder
Phenyl, Napthyl, Pyridyl, Tetrazolyl, Pyrimidyl, Pyrazinyl, Pyrrolidinyl, Indolyl, Morpholinyl, Imidazolyl, Benzothiazolyl, Phenoxathiin-2-yl, Benzoxazolyl, Furyl, Chinolyl oder Purin-8-yl bedeuten,
wobei die Cyclen, gegebenenfalls bis zu 3-fach im Fall der stickstoffhaltigen Ringe auch über die N-Funktion, gleich oder verschieden durch Fluor, Chlor, Brom, Trifluormethyl, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 4 Kohlenstoffatomen, Triazolyl, Tetrazolyl, Benzoxathiazolyl oder Trifluormethyl substituiertes Phenyl oder Phenyl substituiert sind,
und/oder durch eine Gruppe der Formel -OR¹⁰, -SR¹¹ oder -SO₂R¹² substituiert sind,
worin
R¹⁰, R¹¹ und R¹² gleich oder verschieden sind und Phenyl bedeuten, das seinerseits gegebenenfalls bis zu 2-fach gleich oder verschieden durch Phenyl, Fluor, Chlor oder durch geradkettiges oder verzweigtes Alkyl mit bis zu 4 Kohlenstoffatomen substituiert ist,
oder
R⁵ und/oder R⁶ einen Rest der Formel 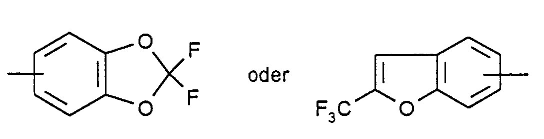 bedeuten,
R⁷ Wasserstoff, Fluor, Chlor oder Brom bedeutet,
und
R⁸ Wasserstoff, Fluor, Chlor, Brom, Azido, Trifluormethyl, Hydroxy, Trifluormethoxy, geradkettiges oder verzweigtes Alkoxy oder Alkyl mit bis zu jeweils 5 Kohlenstoffatomen oder einen Rest der Formel -NR¹⁵R¹⁶ bedeutet,
worin
R¹⁵ und R¹⁶ gleich oder verschieden sind und Wasserstoff, Phenyl oder geradkettiges oder verzweigtes Alkyl mit bis zu 4 Kohlenstoffatomen bedeuten,
oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 4 Kohlenstoffatomen bedeutet,
L eine geradkettige oder verzweigte Alkylen- oder Alkenylen-Kette mit jeweils bis zu 6 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 6 Kohlenstoffatomen bedeuten,
oder
T oder X eine Bindung bedeuten,
V ein Sauerstoff- oder Schwefelatom oder für eine Gruppe der Formel -NR¹⁸- steht,
worin
R¹⁸ Wasserstoff oder geradkettiges oder verzweigtes Alkyl mit bis zu 4 Kohlenstoffatomen oder Phenyl bedeutet,
E für Cyclopropyl, -butyl, -pentyl, -hexyl oder -heptyl steht, oder
für geradkettiges oder verzweigtes Alkyl mit bis zu 6 Kohlenstoffatomen steht, das gegebenenfalls durch Cyclopropyl,
-butyl, -hexyl, -pentyl, -heptyl oder durch Hydroxy substituiert ist, oder für Phenyl steht, das gegebenenfalls durch Fluor, Chlor oder Trifluormethyl substituiert ist,
R¹ und R² gemeinsam eine geradkettige oder verzweigte Alkylenkette mit bis zu 6 Kohlenstoffatomen bilden, die durch eine Oxogruppe und/oder durch einen Rest der Formel 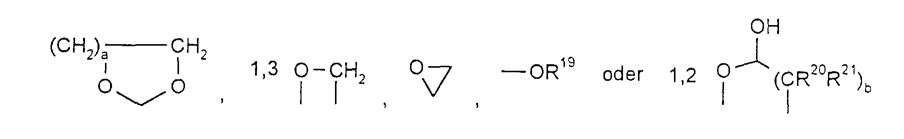 substituiert sein muß,
worin
a und b gleich oder verschieden sind und eine Zahl 1, 2 oder 3 bedeuten,
R¹⁹ Wasserstoff, Cyclopropyl, Cyclopentyl, Cyclohexyl, geradkettiges oder verzweigtes Silylalkyl mit bis zu 7 Kohlenstoffatomen oder geradkettiges oder verzweigtes Alkyl mit bis zu 6 Kohlenstoffatomen bedeutet, das gegebenenfalls durch Hydroxy, geradkettiges oder verzweigtes Alkoxy mit bis zu 4 Kohlenstoffatomen oder durch Phenyl substituiert ist, das seinerseits durch Fluor, Chlor, Brom, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 3 Kohlenstoffatomen oder Benzyl bedeutet,
oder
R¹⁹ geradkettiges oder verzweigtes Acyl mit bis zu 18 Kohlenstoffatomen oder Benzoyl bedeutet, das gegebenenfalls durch Fluor, Chlor, Brom, Trifluormethyl, Nitro oder Trifluormethoxy substituiert ist, oder
geradkettiges oder verzweigtes Fluoracyl mit bis zu 6 Kohlenstoffatomen bedeutet,
R²⁰ und R²¹ gleich oder verschieden sind, Wasserstoff, Phenyl oder geradkettiges oder verzweigtes Alkyl mit bis zu 4 Kohlenstoffatomen bedeuten,
oder
R²⁰ und R²¹ gemeinsam einen Cyclpropyl-, Cyclobutyl-, Cyclo- pentyl-, Cyclohexyl- oder Cycloheptylring bilden,
und die gebildeten Carbocyclen gegebenenfalls bis zu 5-fach gleich oder verschieden, gegebenenfalls auch germinal, durch Trifluormethyl, Hydroxy, Carboxyl, Azido, Fluor, Chlor, Brom, Nitro, Cyano, Cyclopropyl, Cyclobutyl, Cyclopentyl, Cyclohexyl, Cyclopropyloxy, Cyclopentyloxy, Cyclohexyloxy, durch geradkettiges oder verzweigtes Alkoxycarbonyl, Alkoxy oder Alkylthio mit jeweils bis ca. 5 Kohlenstoffatomen oder geradkettiges oder verzweigtes Alkyl mit bis zu 5 Kohlenstoffatomen substituiert sind, das seinerseits bis zu 2-fach gleich oder verschieden durch Hydroxyl, Benzyloxy, Benzoyl, geradkettiges oder verzweigtes Alkoxy oder Oxyacyl mit jeweils bis zu 3 Kohlenstoffatomen, Trifluormethyl und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden durch Phenyl, Benzoyl, Thiophenyl oder Sulfonylbenzyl substituiert sind, die ihrerseits gegebenenfalls durch Fluor, Chlor, Brom, Trifluormethyl, Trifluormethoxy oder Nitro substituiert sind,
und/oder gegebenenfalls durch einen Rest der Formel 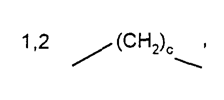
-SO₂-C₆H₅,
-(CO)_{d}-NR²³R²⁴
oder =O
substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cyclopropyl, Cyclobutyl, Cyclopentyl, Cyclohexyl, geradkettiges oder verzweigtes Alkyl mit bis zu 5 Kohlenstoffatomen, Benzyl oder Phenyl bedeuten, das gegebenenfalls durch Fluor, Chlor, Brom, Phenyl oder Trifluormethyl substituiert ist,
und/oder die gebildeten Carbocyclen gegebenenfalls durch einen spiro-verknüpften Rest der Formel 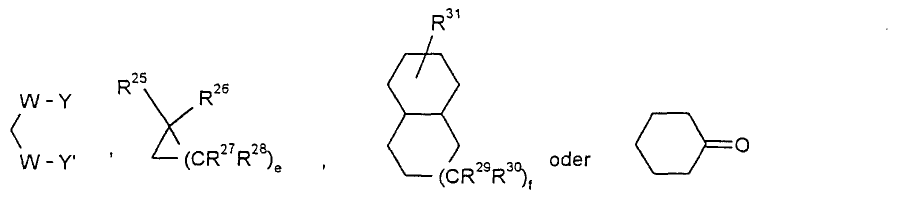 substituiert sind,
worin
W entweder ein Sauerstoff oder ein Schwefelatom bedeutet,
Y und Y' gemeinsam eine 2- bis 5-gliedrige geradkettige oder verzweigte Alkylkette bilden,
e eine Zahl 1, 2, 3, 4, 5 oder 6 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Fluor, Chlor, Brom oder geradkettiges oder verzweigtes Alkyl oder Alkoxy mit jeweils bis zu 5 Kohlenstoffatomen bedeuten,
oder
R²⁵ und R²⁶ oder R²⁷ und R²⁸ jeweils gemeinsam eine geradkettige oder verzweigte Alkylkette mit bis zu 5 Kohlenstoffatomen bilden oder
R²⁵ und R²⁶ oder R²⁷ und R²⁸ jeweils gemeinsam einen Rest der Formel 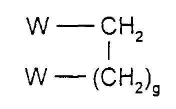 bilden,
worin
W die oben angegebene Bedeutung hat,
g eine Zahl 1, 2, 3, 4, 5 oder 6 bedeutet,
und deren Salze und N-Oxide mit Ausnahme von 5(6H)-Quinolone,3-benzoyl-7,8-dihydro-2,7,7-trimethyl-4-phenyl.

3. Cycloalkano-pyridine der Formel nach Anspruch 1,
in welcher
A für Phenyl steht, das gegebenenfalls bis zu 2-fach gleich oder verschieden durch Fluor, Chlor, Brom, Hydroxy, Trifluormethyl, Trifluormethoxy oder durch geradkettiges oder verzweigtes Alkyl oder Alkoxy mit jeweils bis zu 5 Kohlenstoffatomen substituiert ist,
D für Phenyl steht, das gegebenenfalls durch Nitro, Trifluormethyl, Phenyl, Fluor, Chlor oder Brom substituiert ist, oder
für einen Rest der Formel
R⁵―L― , 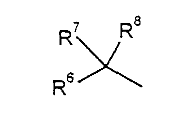
oder
R⁹―T―V―X―
steht,
worin
R⁵, R⁶ und R⁹ unabhängig voneinander Cyclopropyl, Cyclopentyl oder Cyclohexyl bedeutet, oder
Phenyl, Napthyl, Pyridyl, Tetrazolyl, Pyrimidyl, Pyrazinyl, Phenoxathiin-2-yl, Indolyl, Imidazolyl, Pyrrolidinyl, Morpholinyl, Benzothiazolyl, Benzoxazolyl, Furyl, Chinolyl oder Purin-8-yl bedeutet,
wobei die Cyclen, gegebenenfalls bis zu 3-fach, im Fall der stickstoffhaltigen Ringe auch über die N-Funktion, gleich oder verschieden durch Fluor, Chlor, Trifluormethyl, Hydroxy, Cyano, Carboxyl, Trifluormethoxy, geradkettiges oder verzweigtes Alkyl, Alkylthio, Alkylalkoxy, Alkoxy oder Alkoxycarbonyl mit jeweils bis zu 4 Kohlenstoffatomen, Triazolyl, Tetrazolyl, Benzothiazolyl, Trifluormethyl substituiertes Phenyl oder Phenyl substituiert sind
und/oder durch eine Gruppe der Formel -OR¹⁰, -SR¹¹ oder -SO₂R¹² substituiert sind,
worin
R¹⁰, R¹¹ und R¹² gleich oder verschieden sind und Phenyl bedeuten, das seinerseits gegebenenfalls zu 2-fach gleich oder verschieden durch Phenyl, Fluor, Chlor oder durch geradkettiges oder verzweigtes Alkyl mit bis zu 3 Kohlenstoffatomen substituiert ist,
oder
R⁵ und/oder R⁶ einen Rest der Formel 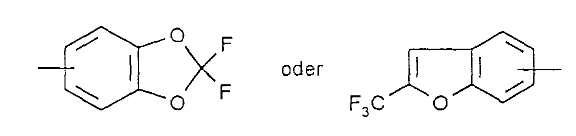 bedeuten,
R⁷ Wasserstoff oder Fluor bedeutet,
und
R⁸ Wasserstoff, Fluor, Chlor, Brom, Azido, Trifluormethyl, Hydroxy, Trifluormethoxy, oder geradkettiges oder verzweigtes Alkoxy oder Alkyl mit jeweils bis zu 4 Kohlenstoffatomen oder einen Rest der Formel -NR¹⁵R¹⁶ bedeutet,
worin
R¹⁵ und R¹⁶ gleich oder verschieden sind und Wasserstoff oder geradkettiges oder verzweigtes Alkyl mit bis zu 3 Kohlenstoffatomen bedeuten,
oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 4 Kohlenstoffatomen bedeutet,
L eine geradkettige oder verzweigte Alkylen- oder Alkenylen-Kette mit jeweils bis zu 5 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 3 Kohlenstoffatomen bedeuten,
oder
T oder X eine Bindung bedeuten,
V ein Sauerstoff- oder Schwefelatom oder für eine Gruppe der Formel -NR¹⁸ steht,
worin
R¹⁸ Wasserstoff oder geradkettiges oder verzweigtes Alkyl mit bis zu 3 Kohlenstoffatomen bedeutet,
E für Cyclopropyl, Cyclopentyl oder Cyclohexyl oder Phenyl steht, das gegebenenfalls durch Fluor oder Trifluormethyl substituiert ist, oder
für geradkettiges oder verzweigtes Alkyl mit bis zu 4 Kohlenstoffatomen steht, das gegebenenfalls durch Hydroxy substituiert ist,
R¹ und R² gemeinsam eine geradkettige oder verzweigte Alkylenkette mit bis zu 5 Kohlenstoffatomen bilden, die durch eine Oxogruppe und/oder einen Rest der Formel 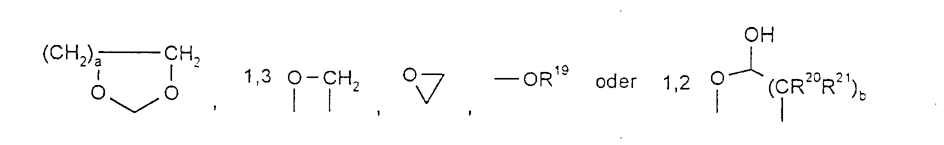 substituiert sein muß,
worin
a und b gleich oder verschieden sind und eine Zahl 1, 2 oder 3 bedeuten,
R¹⁹ Wasserstoff, Cyclopropyl, Cyclopentyl, Cyclohexyl, geradkettiges oder verzweigtes Silylalkyl mit bis zu 6 Kohlenstoffatomen oder geradkettiges oder verzweigtes Alkyl mit bis zu 4 Kohlenstoffatomen bedeutet, das gegebenenfalls durch Hydroxy, geradkettiges oder verzweigtes Alkoxy mit bis zu 3 Kohlenstoffatomen oder durch Phenyl substituiert ist, das seinerseits durch Fluor, Chlor, Brom, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 3 Kohlenstoffatomen oder Benzyl bedeutet,
oder
R¹⁹ geradkettiges oder verzweigtes Acyl mit bis zu 15 Kohlenstoffatomen oder Benzoyl bedeutet, das gegebenenfalls durch Fluor, Chlor, Brom, Trifluormethyl, Nitro oder Trifluormethoxy substituiert ist, oder
geradkettiges oder verzweigtes Fluoracyl mit bis zu 4 Kohlenstoffatomen bedeutet,
R²⁰ und R²¹ gleich oder verschieden sind, Wasserstoff, Phenyl oder geradkettiges oder verzweigtes Alkyl mit bis zu 3 Kohlenstoffatomen bedeuten,
oder
R²⁰ und R²¹ gemeinsam einen Cyclopropyl-, Cyclopentyl- oder Cyclohexylring bilden,
und die gebildeten Carbocyclen gegebenenfalls bis zu 4-fach gleich oder verschieden, gegebenenfalls auch geminal, durch Fluor, Hydroxyl, Trifluormethyl, Nitril, Carboxyl, Azido, Chlor, Brom, Nitro, Cyano, Cyclopropyl, Cyclobutyl, Cyclopentyl, Cyclohexyl, Cyclopropyloxy, Cyclopentyloxy, Cyclohexyloxy, durch geradkettiges oder verzweigtes Alkoxycarbonyl, Alkoxy oder Alkylthio mit jeweils bis zu 4 Kohlenstoffatomen oder geradkettiges oder verzweigtes Alkyl mit bis zu 4 Kohlenstoffatomen substituiert sind, das seinerseits bis zu 2-fach gleich oder verschieden durch Hydroxyl, Benzyloxy, Trifluormethyl, Benzoyl, Methoxy, Oxyacetyl und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden, durch Phenyl, Benzoyl, Thiophenyl oder Sulfonylbenzyl substituiert sind, die ihrerseits gegebenenfalls durch Fluor, Trifluormethyl, Trifluormethoxy oder Nitro substituiert sind,
und/oder gegebenenfalls durch einen Rest der Formel 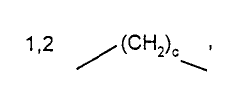
-SO₂-C₆H₅,
-(CO)_{d}-NR²³R²⁴
oder=O
substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cyclopropyl, Cyclopentyl, Benzyl, geradkettiges oder verzweigtes Alkyl mit bis zu 4 Kohlenstoffatomen oder Phenyl bedeuten, das gegebenenfalls durch Fluor, Chlor oder Brom substituiert ist,
und/oder die gebildeten Carbocyclen gegebenenfalls durch einen spiro-verknüpften Rest der Formel 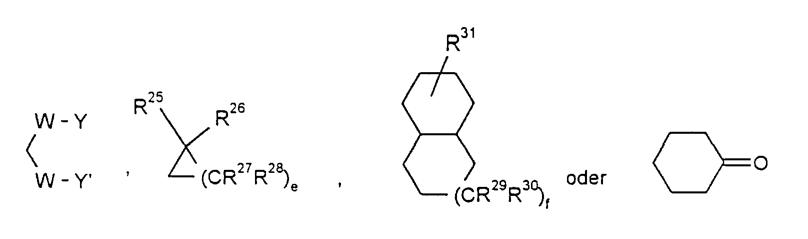 substituiert sind,
worin
W entweder ein Sauerstoff oder ein Schwefelatom bedeutet,
Y und Y' gemeinsam eine 2- bis 6-gliedrige geradkettige oder verzweigte Alkylkette bilden,
e eine Zahl 1, 2, 3, 4 oder 5 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Fluor, Chlor, Brom oder geradkettiges oder verzweigtes Alkyl oder Alkoxy mit jeweils bis zu 4 Kohlenstoffatomen bedeuten,
oder
R²⁵ und R²⁶ oder R²⁷ und R²⁸ jeweils gemeinsam eine geradkettige oder verzweigte Alkylkette mit bis zu 4 Kohlenstoffatomen bilden,
oder
R²⁵ und R²⁶ oder R²⁷ und R²⁸ jeweils gemeinsam einen Rest der Formel 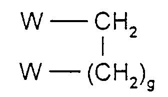 bilden,
worin
W die oben angegebene Bedeutung hat,
g eine Zahl 1, 2, 3, 4, 5, 6 oder 7 bedeutet,
und deren Salze und N-Oxide, mit Ausnahme von 5(6H)-Quinolone, 3-benzoyl-7,8-dihydro-2,7,7-trimethyl-4-phenyl.

4. Cycloalkano-pyridine nach Anspruch 1 bis 3 als Arzneimittel.

5. Verfahren zur Herstellung von Cycloalkano-pyridinen nach Anspruch 1 bis 3, **dadurch gekennzeichnet, daß** man
[A] im Fall D ≠ Aryl, Verbindungen der allgemeinen Formel (II) 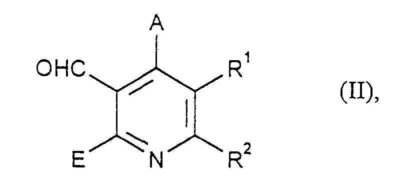 in welcher
A, E, R¹ und R² die oben angegebene Bedeutung haben,
mit metallorganischen Reagenzien im Sinne einer Grignard-, Wittig- oder Li-organische-Reaktion den Substituenten D in inerten Lösemitteln synthetisiert,
oder im Fall, daß D für den Rest der Formel R⁹-T-V-X steht, in welcher V ein Sauerstoffatom bedeutet,
[B] entweder Verbindungen der allgemeinen Formel (III) 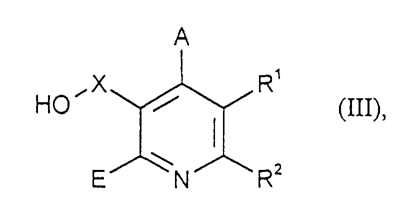 in welcher
A, E, X, R¹ und R² die oben angegebene Bedeutung haben,
mit Verbindungen der allgemeinen Formel (IV)
R⁹-T-Z (IV),
in welcher
R⁹ und T die oben angegebene Bedeutung haben,
und
Z für Halogen, vorzugsweise Chlor oder Brom, steht,
in inerten Lösemitteln, gegebenenfalls in Anwesenheit einer Base und/oder Hilfsstoffs umsetzt, oder
[C] Verbindungen der allgemeinen Formel (III) zunächst durch Umsetzungen mit Verbindungen der allgemeinen Formel (V) 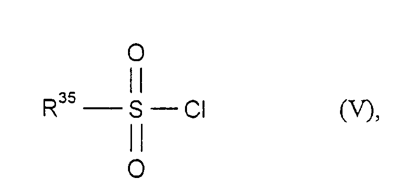 in welcher
R³⁵ für geradkettiges Alkyl mit bis zu 4 Kohlenstoffatomen steht,
in die Verbindungen der allgemeinen Formel (VI) 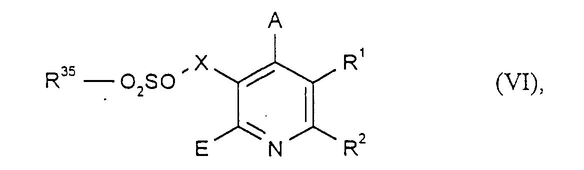 in welcher
A, E, X, R¹, R² und R³⁵ die oben angegebene Bedeutung haben,
überführt und anschließend mit Verbindungen der allgemeinen Formel (VII)
R⁹-T-V-H (VII),
in welcher
R⁹, T und V die oben angegebene Bedeutung haben,
umsetzt und gegebenenfalls Schutzgruppen abspaltet, oder
[D] im Fall der Verbindungen der allgemeinen Formel (Ia) 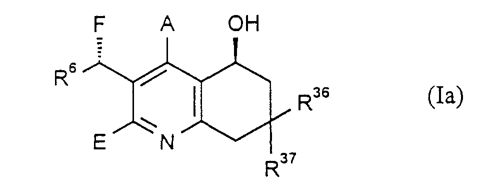
in welcher
A und R⁶ die oben angegebene Bedeutung haben,
R³⁶ und R³⁷ gleich oder verschieden sind und
für Trifluormethyl, Halogen, Nitro, Azido, Cyano, Cycloalkyl oder Cycloalkyloxy mit jeweils 3 bis 7 Kohlenstoffatomen stehen, oder für geradkettiges oder verzweigtes Alkoxycarbonyl, Alkoxy oder Alkylthio mit jeweils bis zu 6 Kohlenstoffatomen oder für geradkettiges oder verzweigtes Alkyl mit bis zu 6 Kohlenstoffatomen stehen, oder
für Phenyl, Benzoyl, Thiophenyl oder Sulfonylbenzyl stehen, die ihrerseits gegebenenfalls durch Halogen, Trifluormethyl, Trifluormethoxy oder Nitro substituiert sind, oder
R³⁵ und R³⁶ für einen der oben aufgeführten spiro-verknüpften Reste der Formel 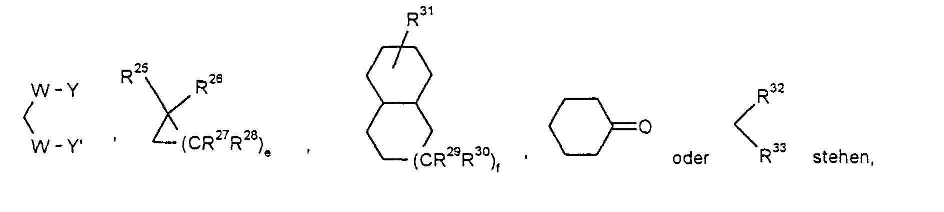
worin
W, Y, Y¹, R²⁵, R²⁶, R²⁷, R²⁸, e, R²⁹, R³⁰, R³¹, R³² und R³³ die oben angegebene Bedeutung haben,
Verbindungen der allgemeinen Formel (VIII) 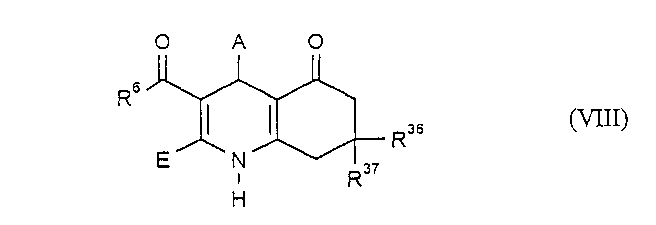 in welcher
R⁶, R³⁶, R³⁷, A und E die oben angegebene Bedeutung haben,
zunächst zu den Verbindungen der allgemeinen Formel (IX) 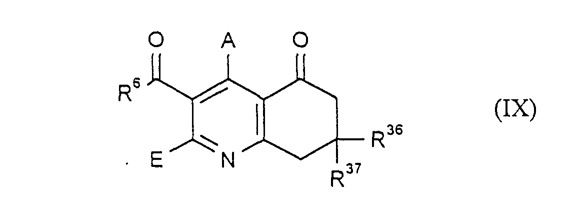 in welcher
R⁶, R³⁶, R³⁷, A und E die oben angegebene Bedeutung haben,
oxidiert,
diese in einem nächsten Schritt durch eine asymmetrische Reduktion zu den Verbindungen der allgemeinen Formel (X) 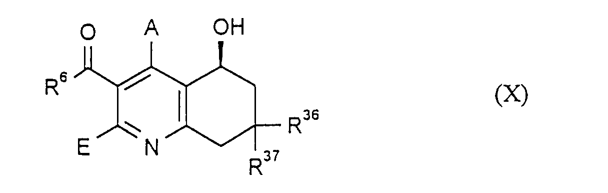 in welcher
R⁶, R³⁶, R³⁷, A und E die oben angegebene Bedeutung haben,
umsetzt,
diese dann durch die Einführung einer Hydroxyschutzgruppe die Verbindungen der allgemeinen Formel (XI) 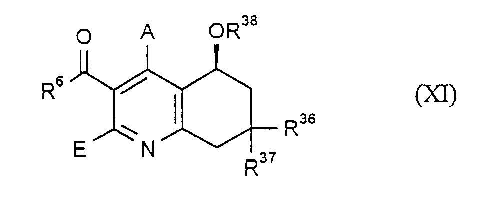 in welcher
R⁶, R³⁶, R³⁷, A und E die oben angegebene Bedeutung haben,
und
R³⁸ für eine Hydroxyschutzgruppe, vorzugsweise für einen Rest der Formel -SiR³⁹R⁴⁰R⁴¹ steht,
worin
R³⁹, R⁴⁰ und R⁴¹ gleich oder verschieden sind und C₁-C₄-Alkyl bedeuten,
überführt,
aus diesem in einem Folgeschritt durch diastereoselektive Reduktion die Verbindungen der allgemeinen Formel (XII) 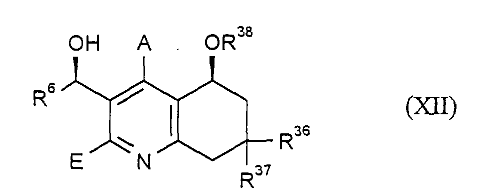 in welcher
R⁶, R³⁶, R³⁷, R³⁸, A und E die oben angegebene Bedeutung haben,
herstellt,
und anschließend durch Einführung des Fluorsubstituenten mit Fluorierungsreagentien und SF₄-Derivaten die Verbindungen der allgemeinen Formel (XIII) 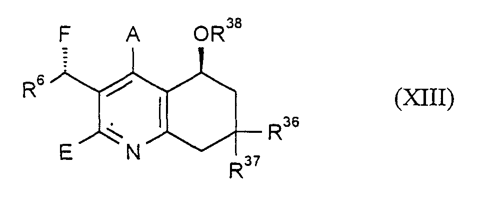 in welcher
R⁶, R³⁶, R³⁷, R³⁸, A und E die oben angegebene Bedeutung haben,
herstellt,
und anschließend die Hydroxyschutzgruppe nach üblichen Methoden abspaltet,
und gegebenenfalls die unter D, E und/oder R¹ und R² aufgeführten Substituenten nach üblichen Methoden, variiert oder einführt.

6. Arzneimittel enthaltend mindestens ein Cycloalkano-pyridin nach Anspruch 1 bis 3 sowie pharmakologisch verträgliche Formulierungshilfsmittel.

7. Arzneimittel nach Anspruch 6 zur Behandlung von Arteriosklerose und Dyslipidämie.

8. Verwendung von Cycloalkano-pyridinen nach Anspruch 1 bis 3 zur Herstellung von Arzneimitteln.

9. Verwendung nach Anspruch 8 zur Herstellung von Arzneimitteln zur Behandlung von Arteriosklerose, insbesondere Dyslipidämien.

10. Zwischenprodukte der Reihe:
a) 3-Amino-3-cyclopentyl-1-(4-trifluormethylphenyl)-propenon der Formel 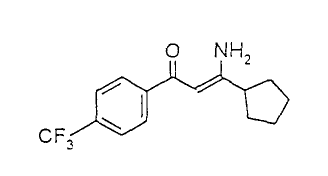
b) 2-Cyclopentyl-4-(4-fluorphenyl)-7,7-dimethyl-3-(4-trifluormethylbenzoyl)-4,6,7,8-tetrahydro-1*H*-chinolin-5-on der Formel 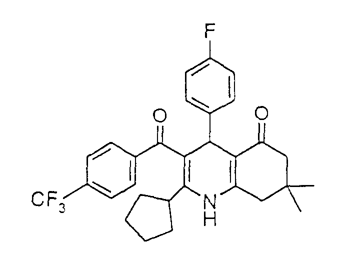
c) 2-Cyclopentyl-4-(4-fluorphenyl)-7,7-dimethyl-3-(4-trifluormethylbenzoyl)-7,8-dihydro-6*H*-chinolin-5-on der Formel 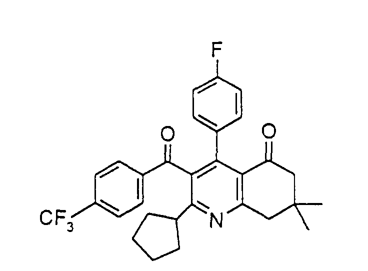
d) [2-Cyclopentyl-4-(4-fluorphenyl)-5-hydroxy-7,7-dimethyl-5,6,7,8-tetrahydrochinolin-3-yl]-(4-trifluormethylphenyl)-methanon der Formel 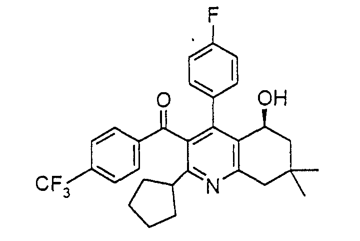
e) [5-(tert-Butyldimethylsilanyloxy)-2-cyclopentyl-4-(4-fluorophenyl)-7,7-dimethyl-5,6,7,8-tetrahydrochinolin-3-yl]-(4-trifluormethylphenyl)-methanon der Formel 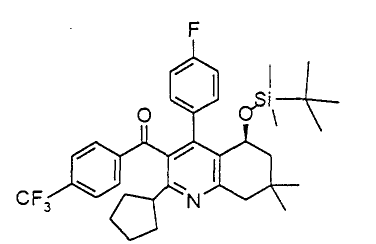
f) [5-(tert-Butyldimethylsilanyloxy)-2-cyclopentyl-4-(4-fluorophenyl)-7,7-dimethyl-5,6,7,8-tetrahydrochinolin-3-yl]-(4-trifluormethylphenyl)-methanol der Formel 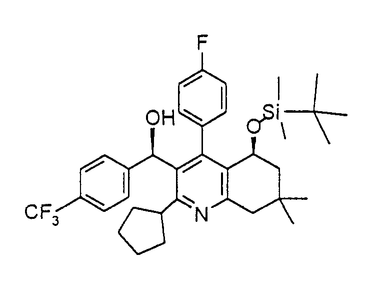
g) 5-(tert-Butyldimethylsilanyloxy)-2-cyclopentyl-4-(4-fluorphenyl)-3-[fluor-(4-trifluormethylphenyl)-methyl]-7,7-dimethyl-5,6,7,8-tetrahydrochinolin der Formel

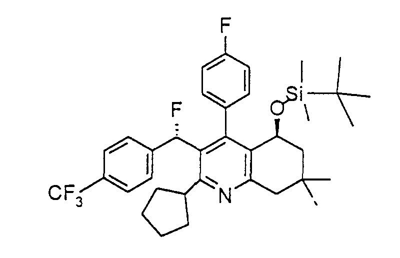

11. 2-Cyclopentyl-4-(4-fluorophenyl)-3-[fluor-(4-trifluormethylphenyl)-methyl]-7,7-dimethyl-5,6,7,8-tetrahydrochinolin-5-ol nach Anspruch 1 der Formel

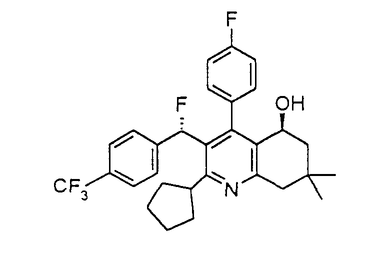

## Claims

1. Cycloalkano-pyridines of the general formula (I) 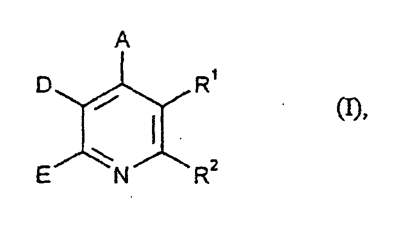 in which
A represents aryl having 6 to 10 carbon atoms, which is optionally substituted up to 5 times in an identical or different manner by halogen, nitro, hydroxyl, trifluoromethyl, trifluoromethoxy, or by straight-chain or branched alkyl, acyl, hydroxyalkyl or alkoxy each having up to 7 carbon atoms, or by a group of the formula -NR³R⁴,
in which
R³ and R⁴ are identical or different and denote hydrogen, phenyl or straight-chain or branched alkyl having up to 6 carbon atoms,
D represents aryl having 6 to 10 carbon atoms, which is optionally substituted by phenyl, nitro, halogen, trifluoromethyl or trifluoromethoxy, or represents a radical of the formula
R⁵―L― 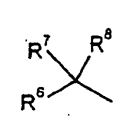
or
R⁹―T―V―X―,
in which
R⁵ and R⁶ and R⁹ independently of one another denote cycloalkyl having 3 to 6 carbon atoms, or
denote aryl having 6 to 10 carbon atoms, or a 5- to 7-membered, optionally benzofused, saturated or unsaturated, mono-, bi- or tricyclic heterocycle having up to 4 heteroatoms from the series S, N and/or O,
where the cycles are substituted, if appropriate, in the case of the nitrogen-containing rings also via the N function, up to 5 times in an identical or different manner by halogen, trifluoromethyl, nitro, hydroxyl, cyano, carboxyl, trifluoromethoxy, straight-chain or branched acyl, alkyl, alkylthio, alkylalkoxy, alkoxy or alkoxycarbonyl each having up to 6 carbon atoms, by aryl- or trifluoromethyl-substituted aryl each having 6 to 10 carbon atoms or by an optionally benzo-fused, aromatic 5- to 7-membered heterocycle having up to 3 heteroatoms from the series S, N and/or O,
and/or are substituted by a group of the formula -OR¹⁰, -SR¹¹, -S0₂R¹² or -NR¹³R¹⁴,
in which
R¹⁰, R¹¹ and R¹² independently of one another denote aryl having 6 to 10 carbon atoms, which for its part is optionally substituted up to 2 times in an identical or different manner by phenyl, halogen or by straight-chain or branched alkyl having up to 6 carbon atoms,
R¹³ and R¹⁴ are identical or different and have the meaning of R³ and R⁴ indicated above,
or
R⁵ and/or R⁶ denote a radical of the formula 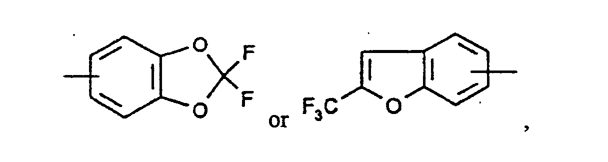 R⁷ denotes hydrogen or halogen
and
R⁸ denotes hydrogen, halogen, azido, trifluoromethyl, hydroxyl, trifluoromethoxy, straight-chain or branched alkoxy or alkyl each having up to 6 carbon atoms or a radical ofthe formula -NR¹⁵R¹⁶,
in which
R¹⁵ and R¹⁶ are identical or different and have the meaning of R³ and R⁴ indicated above,
or
R⁷ and R⁸ together form a radical of the formula =O or =NR¹⁷,
in which
R¹⁷ denotes hydrogen or straight-chain or branched alkyl, alkoxy or acyl each having up to 6 carbon atoms,
L denotes a straight-chain or branched alkylene or alkenylene chain each having up to 8 carbon atoms, each of which is optionally substituted up to 2 times by hydroxyl,
T and X are identical or different and denote a straight-chain or branched alkylene chain having up to 8 carbon atoms,
or
T or X denotes a bond,
V represents an oxygen or sulphur atom or an -NR¹⁸ group,
in which
R¹⁸ denotes hydrogen or straight-chain or branched alkyl having up to 6 carbon atoms or phenyl,
E represents cycloalkyl having 3 to 8 carbon atoms, or represents straight-chain or branched alkyl having up to 8 carbon atoms, which is optionally substituted by cycloalkyl having 3 to 8 carbon atoms or hydroxyl, or represents phenyl which is optionally substituted by halogen or trifluoromethyl,
R¹ and R² together form a straight-chain or branched alkylene chain having up to 7 carbon atoms, which must be substituted by an oxo group and/or by a radical of the formula 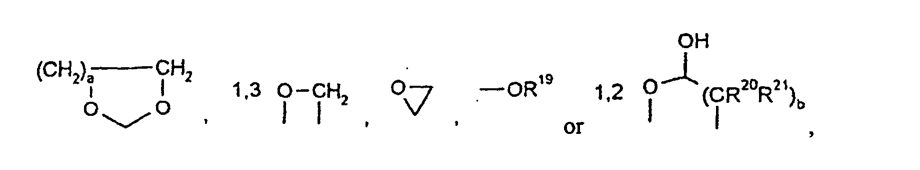
in which
a and b are identical or different and denote a number 1,2 or 3,
R¹⁹ denotes hydrogen, cycloalkyl having 3 to 7 carbon atoms, straight-chain or branched silylalkyl having up to 8 carbon atoms or straight-chain or branched alkyl having up to 8 carbon atoms, which is optionally substituted by hydroxyl, straight-chain or branched alkoxy having up to 6 carbon atoms or by phenyl, which for its part can be substituted by halogen, nitro, trifluoromethyl, trifluoromethoxy or by phenyl- or tetrazole-substituted phenyl,
and alkyl is optionally substituted by a group of the formula -OR²²,
in which
R²² denotes straight-chain or branched acyl having up to 4 carbon atoms or benzyl,
or
R¹⁹ denotes straight-chain or branched acyl having up to 20 carbon atoms or benzoyl which is optionally substituted by halogen, trifluoromethyl, nitro or trifluoromethoxy, or
denotes straight-chain or branched fluoroacyl having up to 8 carbon atoms and 9 fluorine atoms,
R²⁰ and R²¹ are identical or different, and denote hydrogen, phenyl or straight-chain or branched alkyl having up to 6 carbon atoms,
or
R²⁰ and R²¹ together form a 3 to 6-membered carbocycle, and, if appropriate also geminally, the carbocycles formed are optionally substituted up to 6 times in an identical or different manner by trifluoromethyl, hydroxyl, halogen, carboxyl, nitro, azido, cyano, cycloalkyl or cycloalkyloxy each having 3 to 7 carbon atoms, by straight-chain or branched alkoxycarbonyl, alkoxy or alkylthio each having up to 6 carbon atoms or by straight-chain or branched alkyl having up to 6 carbon atoms, which for its part is substituted up to 2 times in an identical or different manner by hydroxyl, benzyloxy, trifluoromethyl, benzoyl, straight-chain or branched alkoxy, oxyacyl or carboxyl each having up to 4 carbon atoms and/or phenyl, which for its part can be substituted by halogen, trifluoromethyl or trifluoromethoxy,
and/or the carbocycles formed, also geminally, are optionally substituted up to 5 times in an identical or different manner by phenyl, benzoyl, thiophenyl or sulphonylbenzyl, which for their part are optionally substituted by halogen, trifluoromethyl, trifluoromethoxy or nitro,
and/or are optionally substituted by a radical of the formula 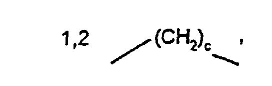
-SO₂-C₆H₅,
-(CO)_{d}-NR²³R²⁴
or =O,
in which
c denotes a number 1, 2, 3 or 4,
d denotes a number 0 or 1,
R²³ and R²⁴ are identical or different and denote hydrogen, cycloalkyl having 3 to 6 carbon atoms, straight-chain or branched alkyl having up to 6 carbon atoms, benzyl or phenyl, which is optionally substituted up to 2 times in an identical or different manner by halogen, trifluoromethyl, cyano, phenyl or nitro,
and/or the carbocycles formed are optionally substituted by a spiro-linked radical of the formula 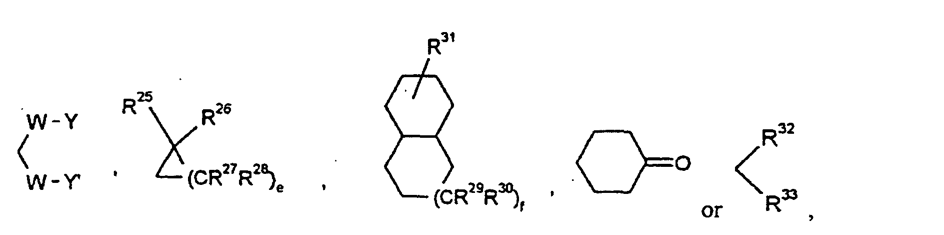
in which
W either denotes an oxygen or a sulphur atom,
Y and Y' together form a 2- to 6-membered straight-chain or branched alkylene chain,
e denotes a number 1, 2, 3, 4, 5,6 or 7,
f denotes a number 1 or 2,
R²⁵, R²⁶, R²⁷, R²⁸, R²⁹, R³⁰ and R³¹ are identical or different and denote hydrogen, trifluoromethyl, phenyl, halogen or straight-chain or branched alkyl or alkoxy each having up to 6 carbon atoms,
or
R²⁵ and R²⁶ or R²⁷ and R²⁸ in each case together form a straight-chain or branched alkyl chain having up to 6 carbon atoms,
or
R²⁵ and R²⁶ or R²⁷ and R²⁸ in each case together form a radical of the formula 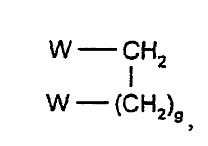
in which
W has the meaning indicated above,
g denotes a number 1, 2, 3, 4, 5, 6 or 7,
R³² and R³³ together form a 3- to 7-membered heterocycle which contains an oxygen or sulphur atom or a group of the formula SO, SO₂ or -NR³⁴,
in which
R³⁴ denotes hydrogen, phenyl, benzyl or straight-chain or branched alkyl having up to 4 carbon atoms,
and their salts and N-oxides, with the exception of 5(6H)-quinolone, 3-benzoyl-7,8-dihydro-2,7,7-trimethyl-4-phenyl.

2. Cycloalkano-pyridines of the formula according to Claim 1
in which
A represents naphthyl or phenyl, each of which is optionally substituted up to 2 times in an identical or different manner by fluorine, chlorine, bromine, amino, hydroxyl, trifluoromethyl, trifluoromethoxy or by straight-chain or branched alkyl or alkoxy each having up to 6 carbon atoms,
D represents phenyl which is optionally substituted by nitro, fluorine, chlorine, bromine, phenyl, trifluoromethyl or trifluoromethoxy, or represents a radical of the formula
R⁵―L― 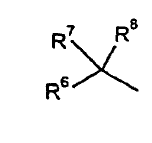
or
R⁹―T―V―X―
in which
R⁵, R⁶ and R⁹ independently of one another denote cyclopropyl, cyclopentyl or cyclohexyl, or
denote phenyl, naphthyl, pyridyl, tetrazolyl, pyrimidyl, pyrazinyl, pyrrolidinyl, indolyl, morpholinyl, imidazolyl, benzothiazolyl, phenoxathiin-2-yl, benzoxazolyl, furyl, quinolyl or purin-8-yl,
where the cycles are also substituted via the N function, optionally up to 3 times in the case of the nitrogen-containing rings, in an identical or different manner by fluorine, chlorine, bromine, trifluoromethyl, hydroxyl, cyano, carboxyl, trifluoromethoxy, straight-chain or branched acyl, alkyl, alkylthio, alkylalkoxy, alkoxy or alkoxycarbonyl each having up to 4 carbon atoms, triazolyl, tetrazolyl, benzoxathiazolyl, or trifluoromethyl-substituted phenyl or phenyl,
and/or are substitued by a group of the formula -OR¹⁰, -SR¹¹ or -SO₂R¹²,
in which
R¹⁰, R¹¹ and R¹² are identical or different and denote phenyl, which for its part is optionally substituted up to 2 times in an identical or different manner by phenyl, fluorine, chlorine or by straight-chain or branched alkyl having up to 4 carbon atoms,
or
R⁵ and/or R⁶ denotes a radical of the formula 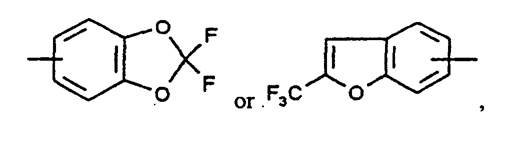 R⁷ denotes hydrogen, fluorine, chlorine or bromine
and
R⁸ denotes hydrogen, fluorine, chlorine, bromine, azido, trifluoromethyl, hydroxyl, trifluoromethoxy, straight-chain or branched alkoxy or alkyl having up to 5 carbon atoms in each case or a radical of the formula - NR¹⁵R¹⁶,
in which
R¹⁵ and R¹⁶ are identical or different and denote hydrogen, phenyl or straight-chain or branched alkyl having up to 4 carbon atoms,
or
R⁷ and R⁸ together form a radical of the formula =O or =NR¹⁷, in which
R¹⁷ denotes hydrogen or straight-chain or branched alkyl, alkoxy or acyl each having up to 4 carbon atoms,
L denotes a straight-chain or branched alkylene or alkenylene chain each having up to 6 carbon atoms, each of which is optionally substituted up to 2 times by hydroxyl,
T and X are identical or different and denote a straight-chain or branched alkylene chain having up to 6 carbon atoms,
or
T or X denotes a bond,
V represents an oxygen or sulphur atom or a group of the formula -NR¹⁸-,
in which
R¹⁸ denotes hydrogen or straight-chain or branched alkyl having up to 4 carbon atoms or phenyl,
E represents cyclopropyl, -butyl, -pentyl, -hexyl or -heptyl, or represents straight-chain or branched alkyl having up to 6 carbon atoms, which is optionally substituted by cyclopropyl, -butyl, -hexyl, -pentyl, -heptyl or by hydroxyl, or represents phenyl which is optionally substituted by fluorine, chlorine or trifluoromethyl,
R¹ and R² together form a straight-chain or branched alkylene chain having up to 6 carbon atoms, which must be substituted by an oxo group and/or by a radical of the formula 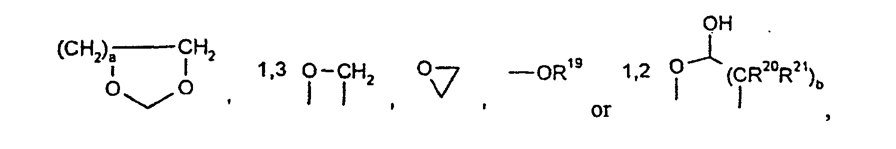 in which
a and b are identical or different and denote a number 1,2 or 3,
R¹⁹ denotes hydrogen, cyclopropyl, cyclopentyl, cyclohexyl, straight-chain or branched silylalkyl having up to 7 carbon atoms or straight-chain or branched alkyl having up to 6 carbon atoms, which is optionally substituted by hydroxyl, straight-chain or branched alkoxy having up to 4 carbon atoms or by phenyl which for its part can be substituted by fluorine, chlorine, bromine, nitro, trifluoromethyl, trifluoromethoxy or by phenyl- or tetrazole-substituted phenyl,
and alkyl is optionally substituted by a group of the formula -OR²²,
in which
R²² denotes straight-chain or branched acyl having up to 3 carbon atoms or benzyl,
or
R¹⁹ denotes straight-chain or branched acyl having up to 18 carbon atoms or benzoyl which is optionally substituted by fluorine, chlorine, bromine, trifluoromethyl, nitro or trifluoromethoxy, or denotes straight-chain or branched fluoroacyl having up to 6 carbon atoms,
R²⁰ and R²¹ are identical or different, and denote hydrogen, phenyl or straight-chain or branched alkyl having up to 4 carbon atoms,
or
R²⁰ and R²¹ together form a cyclopropyl, cyclobutyl, cyclopentyl, cyclohexyl or cycloheptyl ring,
and the carbocycles formed are optionally substituted up to 5 times in an identical or different manner, optionally also geminally, by trifluoromethyl, hydroxyl, carboxyl, azido, fluorine, chlorine, bromine, nitro, cyano, cyclopropyl, cyclobutyl, cyclopentyl, cyclohexyl, cyclopropyloxy, cyclopentyloxy, cyclohexyloxy, by straight-chain or branched alkoxycarbonyl, alkoxy or alkylthio each having up to about 5 carbon atoms or straight-chain or branched alkyl having up to 5 carbon atoms, which for its part is substituted up to 2 times in an identical or different manner by hydroxyl, benzyloxy, benzoyl, straight-chain or branched alkoxy or oxyacyl each having up to 3 carbon atoms, trifluoromethyl and/or phenyl, which for its part can be substituted by fluorine, chlorine, bromine, trifluoromethyl or trifluoromethoxy,
and/or the carbocycles formed, also geminally, are optionally substituted up to 4 times in an identical or different manner by phenyl, benzoyl, thiophenyl or sulphonylbenzyl, which for their part are optionally substituted by fluorine, chlorine, bromine, trifluoromethyl, trifluoromethoxy or nitro,
and/or are optionally substituted by a radical of the formula 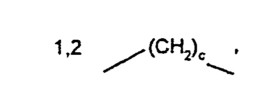
-SO₂-C₆H₅,
-(CO)_{d}-NR²³R²⁴
or=O,
in which
c denotes a number 1,2,3 or 4,
d denotes a number 0 or 1,
R²³ and R²⁴ are identical or different and denote hydrogen, cyclopropyl, cyclobutyl, cyclopentyl, cyclohexyl, straight-chain or branched alkyl having up to 5 carbon atoms, benzyl or phenyl, which is optionally substituted by fluorine, chlorine, bromine, phenyl or trifluoromethyl,
and/or the carbocycles formed are optionally substituted by a spiro-linked radical of the formula 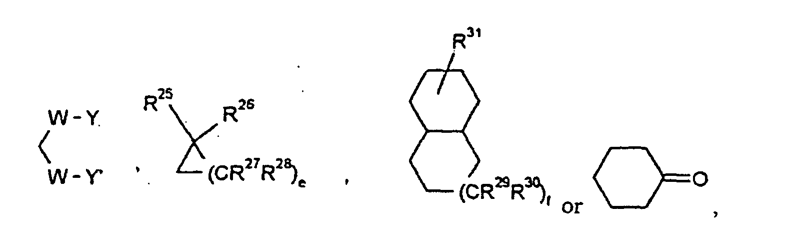 in which
W denotes either an oxygen or a sulphur atom,
Y and Y' together form a 2- to 5-membered straight-chain or branched alkyl chain,
e denotes a number 1, 2, 3, 4, 5 or 6,
f denotes a number 1 or 2,
R²⁵, R²⁶, R²⁷, R²⁸, R²⁹, R³⁰ and R³¹ are identical or different and denote hydrogen, trifluoromethyl, phenyl, fluorine, chlorine, bromine or straight-chain or branched alkyl or alkoxy each having up to 5 carbon atoms,
or
R²⁵ and R²⁶ or R²⁷ and R²⁸ in each case together form a straight-chain or branched alkyl chain having up to 5 carbon atoms or
R²⁵ and R²⁶ or R²⁷ and R²⁸ in each case together form a radical of the formula 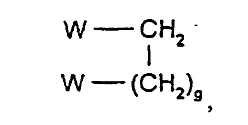
in which
W has the meaning indicated above,
g denotes a number 1, 2, 3, 4, 5 or 6,
and their salts and N-oxides with the exception of 5(6H)-quinolone, 3-benzoyl-7,8-dihydro-2,7,7-trimethyl-4-phenyl.

3. Cycloalkano-pyridines of the formula according to Claim 1
in which
A represents phenyl which is optionally substituted up to 2 times in an identical or different manner by fluorine, chlorine, bromine, hydroxyl, trifluoromethyl, trifluoromethoxy or by straight-chain or branched alkyl or alkoxy each having up to 5 carbon atoms,
D represents phenyl which is optionally substituted by nitro, trifluoromethyl, phenyl, fluorine, chlorine or bromine or
represents a radical of the formula
R⁵―L― 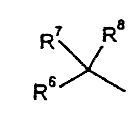
or
R⁹―T―V―X―,
in which
R⁵, R⁶ and R⁹ independently of one another denote cyclopropyl, cyclopentyl or cyclohexyl, or
denote phenyl, naphthyl, pyridyl, tetrazolyl, pyrimidyl, pyrazinyl, phenoxathiin-2-yl, indolyl, imidazolyl, pyrrolidinyl, morpholinyl, benzothiazolyl, benzoxazolyl, furyl, quinolyl or purin-8-yl,
where the cycles are substituted, optionally up to 3 times, in the case of the nitrogen-containing rings also via the N function in an identical or different manner by fluorine, chlorine, trifluoromethyl, hydroxyl, cyano, carboxyl, trifluoromethoxy, straight-chain or branched alkyl, alkylthio, alkylalkoxy, alkoxy or alkoxycarbonyl each having up to 4 carbon atoms, triazolyl, tetrazolyl, benzothiazolyl, trifluoromethyl-substituted phenyl or phenyl
and/or are substituted by a group of the formula -OR¹⁰, -SR¹¹ or -SO₂R¹²,
in which
R¹⁰, R¹¹ and R¹² are identical or different and denote phenyl, which for its part is optionally substituted up to 2 times in an identical or different manner by phenyl, fluorine, chlorine or by straight-chain or branched alkyl having up to 3 carbon atoms,
or
R⁵ and/or R⁶ denotes a radical of the formula 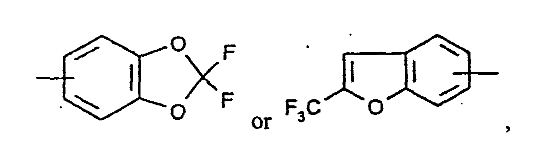
R⁷ denotes hydrogen or fluorine
and
R⁸ denotes hydrogen, fluorine, chlorine, bromine, azido, trifluoromethyl, hydroxyl, trifluoromethoxy, or straight-chain or branched alkoxy or alkyl each having up to 4 carbon atoms or a radical of the formula -NR¹⁵R¹⁶,
in which
R¹⁵ and R¹⁶ are identical or different and denote hydrogen or straight-chain or branched alkyl having up to 3 carbon atoms,
or
R⁷ and R⁸ together form a radical of the formula =O or =NR¹⁷,
in which
R¹⁷ denotes hydrogen or straight-chain or branched alkyl, alkoxy or acyl each having up to 4 carbon atoms,
L denotes a straight-chain or branched alkylene or alkenylene chain each having up to 5 carbon atoms, each of which is optionally substituted up to 2 times by hydroxyl,
T and X are identical or different and denote a straight-chain or branched alkylene chain having up to 3 carbon atoms,
or
T or X denotes a bond,
V represents an oxygen or sulphur atom or a group of the formula -NR¹⁸,
in which
R¹⁸ denotes hydrogen or straight-chain or branched alkyl having up to 3 carbon atoms,
E represents cyclopropyl, cyclopentyl or cyclohexyl or phenyl, which is optionally substituted by fluorine or trifluoromethyl, or
represents straight-chain or branched alkyl having up to 4 carbon atoms, which is optionally substituted by hydroxyl,
R¹ and R² together form a straight-chain or branched alkylene chain having up to 5 carbon atoms, which must be substituted by an oxo group and/or a radical of the formula 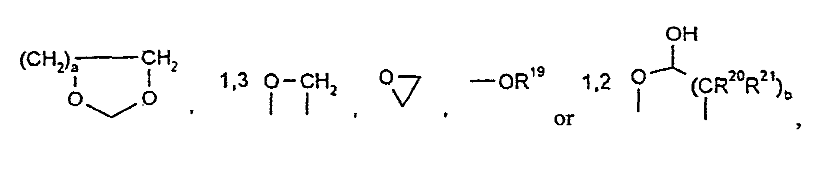 in which
a and b are identical or different and denote a number 1, 2 or 3,
R¹⁹ denotes hydrogen, cyclopropyl, cyclopentyl, cyclohexyl, straight-chain or branched silylalkyl having up to 6 carbon atoms or straight-chain or branched alkyl having up to 4 carbon atoms, which is optionally substituted by hydroxyl, straight-chain or branched alkoxy having up to 3 carbon atoms or by phenyl, which for its part can be substituted by fluorine, chlorine, bromine, nitro, trifluoromethyl, trifluoromethoxy or by phenyl- or tetrazole-substituted phenyl,
and alkyl is optionally substituted by a group of the formula -OR²²,
in which
R²² denotes straight-chain or branched acyl having up to 3 carbon atoms or benzyl,
or
R¹⁹ denotes straight-chain or branched acyl having up to 15 carbon atoms or benzoyl, which is optionally substituted by fluorine, chlorine, bromine, trifluoromethyl, nitro or trifluoromethoxy, or denotes straight-chain or branched fluoroacyl having up to 4 carbon atoms,
R²⁰ and R²¹ are identical or different, and-denote hydrogen, phenyl or straight-chain or branched alkyl having up to 3 carbon atoms
or
R²⁰ and R²¹ together form a cyclopropyl, cyclopentyl or cyclohexyl ring,
and the carbocycles formed are optionally substituted up to 4 times in an identical or different manner, optionally also geminally, by fluorine, hydroxyl, trifluoromethyl, carboxyl, azido, chlorine, bromine, nitro, cyano, cyclopropyl, cyclobutyl, cyclopentyl, cyclohexyl, cyclopropyloxy, cyclopentyloxy, cyclohexyloxy, by straight-chain or branched alkoxycarbonyl, alkoxy or alkylthio each having up to 4 carbon atoms or straight-chain or branched alkyl having up to 4 carbon atoms, which for its part is substituted up to 2 times in an identical or different manner by hydroxyl, benzyloxy, trifluoromethyl, benzoyl, methoxy, oxyacetyl and/or phenyl, which for its part can be substituted by fluorine, chlorine, bromine, trifluoromethyl or trifluoromethoxy,
and/or the carbocycles formed, are optionally substituted, also geminally, up to 4 times in an identical or different manner by phenyl, benzoyl, thiophenyl or sulphonylbenzoyl, which for their part are optionally substituted by fluorine, trifluoromethyl, trifluoromethoxy or nitro,
and/or are optionally substituted by a radical of the formula 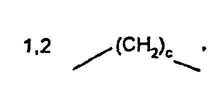
-SO₂-C₆H₅,
-(CO)_{d}-NR²³R²⁴
or=O,
in which
c denotes a number 1,2,3 or 4,
d denotes a number 0 or 1,
R²³ and R²⁴ are identical or different and denote hydrogen, cyclopropyl, cyclopentyl, benzyl, straight-chain or branched alkyl having up to 4 carbon atoms or phenyl which is optionally substituted by fluorine, chlorine or bromine,
and/or the carbocycles formed are optionally substituted by a spiro-linked radical of the formula 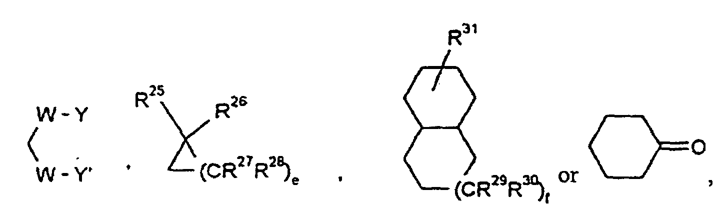 in which
W denotes either an oxygen or a sulphur atom,
Y and Y' together form a 2- to 6-membered straight-chain or branched alkyl chain,
e denotes a number 1,2,3,4 or 5,
f denotes a number 1 or 2,
R²⁵, R²⁶, R²⁷, R²⁸, R²⁹, R³⁰ and R³¹ are identical or different and denote hydrogen, trifluoromethyl, phenyl, fluorine, chlorine, bromine or straight-chain or branched alkyl or alkoxy each having up to 4 carbon atoms,
or
R²⁵ and R²⁶ or R²⁷ and R²⁸ each together form a straight-chain or branched alkylene chain having up to 4 carbon atoms,
or
R²⁵ and R²⁶ or R²⁷ and R²⁸ each together form a radical of the formula 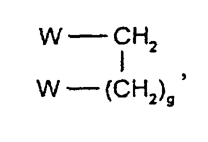 in which
W has the meaning indicated above,
g denotes a number 1, 2, 3, 4, 5, 6 or 7,
and their salts and N-oxides, with the exception of 5(6H)-quinolone, 3-benzoyl-7,8-dihydro-2, 7, 7-trimethyl-4-phenyl.

4. Cycloalkano-pyridines according to Claims 1 to 3 as medicaments.

5. Process for the preparation of cycloalkano-pyridines according to Claims 1 to 3, **characterized in that**
[A] if D ≠ aryl, in compounds of the general formula (II) 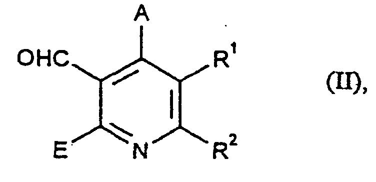 in which
A, E, R¹ and R² have the meaning indicated above,
the substituent D is synthesized in inert solvents using organometallic reagents in the sense of a Grignard, Wittig or organolithium reaction,
or if D represents the radical of the formula R⁹-T-V-X, in which V denotes an oxygen atom,
[B] either compounds of the general formula (III) 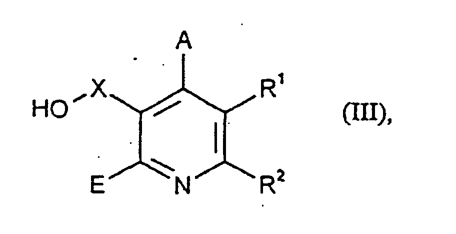 in which
A, E, X, R¹ and R² have the meaning indicated above,
R⁹-T-Z (IV),
in which
R⁹ and T have the meaning indicated above
and
Z represents halogen, preferably chlorine or bromine,
in inert solvents, if appropriate in the presence of a base and/or auxiliary,
or
[C] compounds of the general formula (III) are first converted by reaction with compounds of the general formula (V) 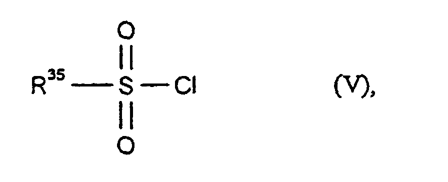 in which
R³⁵ represents straight-chain alkyl having up to 4 carbon atoms,
into the compounds of the general formula (VI) 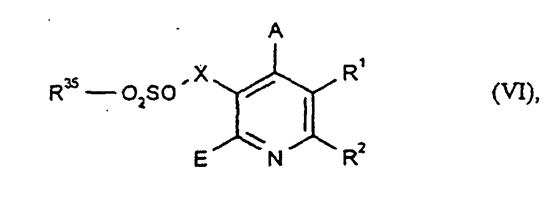 in which
A, E, X, R¹, R² and R³⁵ have the meaning indicated above,
and then reacted with compounds of the general formula (VII)
R⁹-T-V-H (VII),
in which
R⁹, T and V have the meaning indicated above,
and, if appropriate, protective groups are removed,
or
[D] in the case of the compounds of the general formula (Ia) 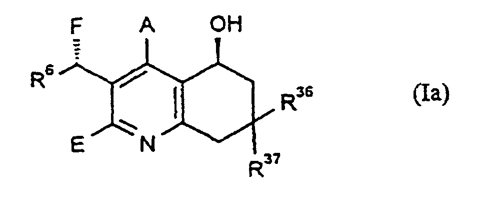 in which
A and R⁶ have the meaning indicated above,
R³⁶ and R³⁷ are identical or different and
represent trifluoromethyl, halogen, nitro, azido, cyano, cycloalkyl or cycloalkyloxy each having 3 to 7 carbon atoms, or
represent straight-chain or branched alkoxycarbonyl, alkoxy or alkylthio each having up to 6 carbon atoms or straight-chain or branched alkyl having up to 6 carbon atoms, or
represent phenyl, benzoyl, thiophenyl or sulphonylbenzyl, which for their part are optionally substituted by halogen, trifluoromethyl, trifluoromethoxy or nitro, or
R³⁶ and R³⁷ represent one of the abovementioned spiro-linked radicals of the formula 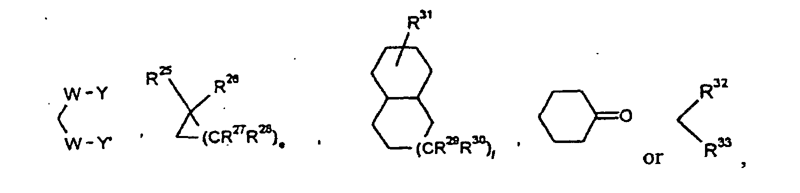
in which
W, Y, Y', R²⁵, R²⁶, R²⁷, R²⁸, e, R²⁹, R³⁰, R³¹, R³² and R³³ have the meaning indicated above
compounds of the general formula (VIII) 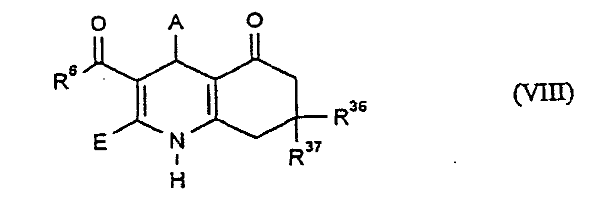 in which
R⁶, R³⁶, R³⁷, A and E have the meaning indicated above,
are first oxidized to the compounds of the general formula (IX) 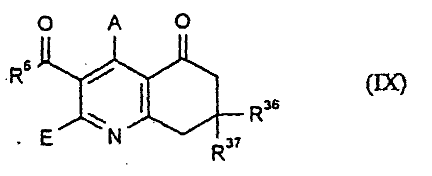 in which
R⁶, R³⁶, R³⁷, A and E have the meaning indicated above,
these are reacted in a next step by means of an asymmetric reduction to give the compounds of the general formula (X) 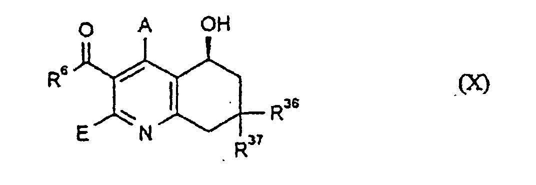 in which
R⁶, R³⁶, R³⁷, A and E have the meaning indicated above,
these are then converted by the introduction of a hydroxyl protective group into the compounds of the general formula (XI) 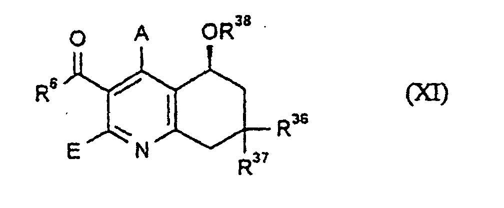 in which
R⁶, R³⁶, R³⁷, A and E have the meaning indicated above
and
R³⁸ represents a hydroxyl protective group, preferably a radical of the formula -SiR³⁹R⁴⁰R⁴¹,
in which
R³⁹, R⁴⁰ and R⁴¹ are identical or different and denote C₁-C₄-alkyl,
the compounds of the general formula (XII) 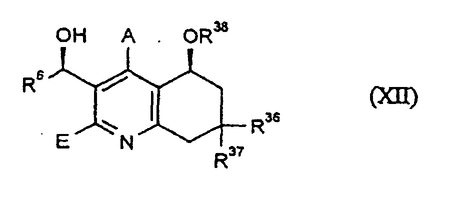
in which
R⁶, R³⁶, R³⁷, R³⁸, A and E have the meaning indicated above,
are prepared from these in a subsequent step by diastereoselective reduction,
and then by introduction of the fluorine substituent using fluorination reagents and SF₄ derivatives the compounds of the general formula (XIII) 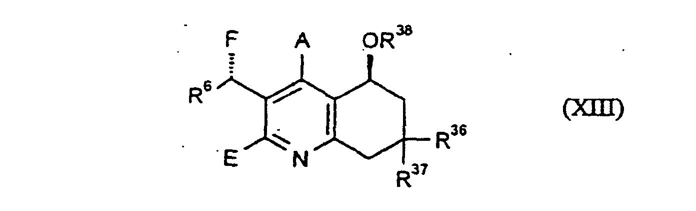 in which
R⁶, R³⁶, R³⁷, R³⁸, A and E have the meaning indicated above,
are prepared,
and then the hydroxyl protective group is removed according to customary methods,
and if appropriate the substituents mentioned under D, E and/or R¹ and R² are varied or introduced according to customary methods.

6. Medicaments comprising at least one cycloalkano-pyridine according to Claims 1 to 3, and pharmacologically tolerable formulation auxiliaries.

7. Medicaments according to Claim 6 for the treatment of arteriosclerosis and dyslipidaemia.

8. Use of cycloalkanopyridines according to Claims 1 to 3 for the production of medicaments.

9. Use according to Claim 8 for the production of medicaments for the treatment of arteriosclerosis, in particular dyslipidaemias.

10. Intermediates of the series:
a) 3-Amino-3-cyclopentyl-1-(4-trifluoromethylphenyl)-propenone of the formula 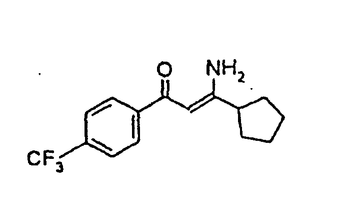
b) 2-Cyclopentyl-4-(4-fluorophenyl)-7,7-dimethyl-3-(4-trifluoromethylbenzoyl)-4,6, 7,8-tetrahydro-1*H*-quinoline-5-one of the formula 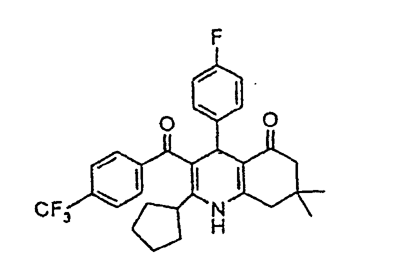
c) 2-Cyclopentyl-4-(4-fluorophenyl)-7,7-dimethyl-3-(4-trifluoromethylbenzoyl)-7,8-dihydro-6*H*-quinolin-5-one of the formula 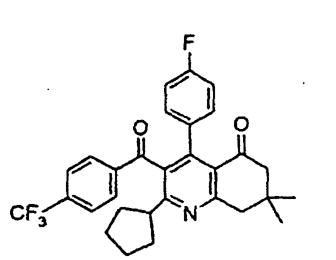
d) [2-Cyclopentyl-4-(4-fluorophenyl)-5-hydroxy-7,7-dimethyl-5,6,7,8-tetra-hydroquinolin-3-yl]-(4-trifluoromethylphenyl)-methanone of the formula 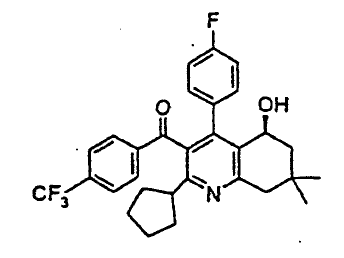
e) [5-tert-Butyldimethylsilanyloxy)-2-cyclopentyl-4-(4-fluorophenyl)-7,7-dimethyl-5,6,7,8-tetrahydroquinolin-3-yl]-(4-trifluoromethylphenyl)-methanone of the formula 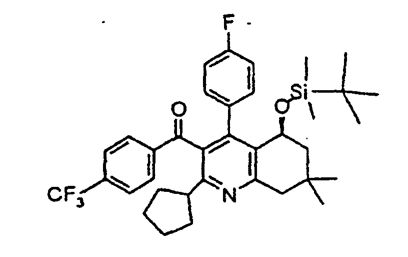
f) [5-(tert-Butyldimethylsilanyloxy)-2-cyclopentyl-4-(4-fluorophenyl)-7,7-dimethyl-5,6,7,8-tetrahydroquinolin-3-yl]-(4-trifluoromethylphenyl)-methanol of the formula 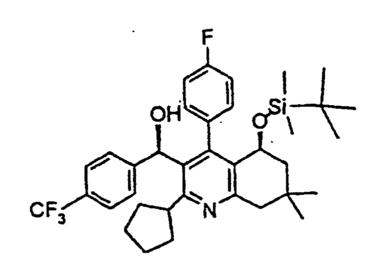
g) 5-(tert-Butyldimethylsilanyloxy)-2-cyclopentyl-4-(4-fluorophenyl)-3-[fluoro-(4-trifluoromethylphenyl)-methyl]-7,7-dimethyl-5,6,7,8-tetrahydro-quinoline of the formula

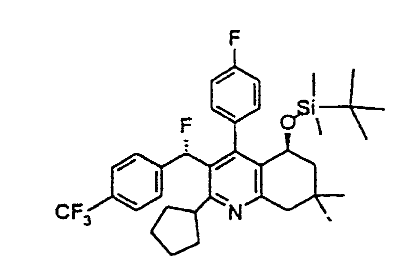

11. 2-Cyclopentyl-4-(4-fluorophenyl)-3-[fluoro-(4-trifluoromethylphenyl)methyl]-7,7-dimethyl-5,6,7,8-tetrahydroquinolin-5-ol according to Claim 1 of the formula

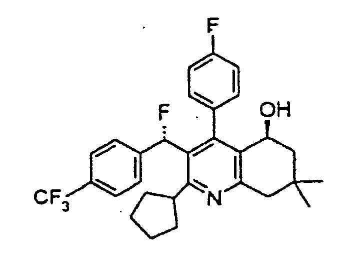

## Revendications

1. Cycloalcanopyridines de formule générale (I) 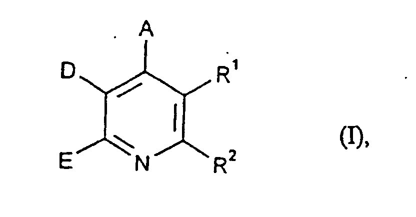 dans laquelle
A est un reste aryle ayant 6 à 10 atomes de carbone, qui est substitué jusqu'à 5 fois identiques ou différentes par un halogène, un groupe nitro, hydroxy, trifluorométhyle, trifluorométhoxy ou par un groupe alkyle, acyle, hydroxyalkyle ou alkoxy linéaire ou ramifié ayant dans chaque cas jusqu'à 7 atomes de carbone, ou par un groupe de formule -NR³R⁴,
où
R³ et R⁴ sont identiques ou différents et représentent l'hydrogène, un groupe phényle ou un groupe alkyle linéaire ou ramifié ayant jusqu'à 6 atomes de carbone,
D est un reste aryle ayant 6 à 10 atomes de carbone, qui est éventuellement substitué par un groupe phényle, nitro, un halogène, un groupe trifluorométhyle ou trifluorométhoxy, ou bien
un reste de formule
R⁵―L― 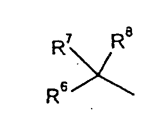
ou
R⁹―T―V―X―
dans laquelle
R⁵, R⁶ et R⁹ représentent, indépendamment l'un de l'autre, un reste cycloalkyle ayant 3 à 6 atomes de carbone, ou bien
un reste aryle ayant 6 à 10 atomes de carbone ou un hétérocycle pentagonal à heptagonal saturé ou non saturé, monocyclique, bicyclique ou tricyclique éventuellement condensé au benzène, ayant 4 hétéroatomes de la série S, N et/ou O,
les cycles étant éventuellement substitués jusqu'à 5 fois identiques ou différentes, le cas échéant, aussi par l'intermédiaire de la fonction N dans le cas des noyaux contenant de l'azote, par un halogène, un groupe trifluorométhyle, nitro, hydroxy, cyano, carboxyle, trifluorométhoxy, par un groupe acyle, alkyle, alkylthio, alkylalkoxy, alkoxy ou alkoxycarbonyle linéaire
ou ramifié ayant dans chaque cas jusqu'à 6 atomes de carbone, par un reste aryle à substituant aryle ou trifluorométhyle ayant dans chaque cas 6 à 10 atomes de carbone ou par un hétérocycle aromatique pentagonal à heptagonal éventuellement condensé au benzène, ayant jusqu'à 3 hétéroatomes de la série S, N et/ou O,
et/ou subsitués par un groupe de formule -OR¹⁰, -SR¹¹, -SO₂R¹² ou -NR¹³R¹⁴,
où
R¹⁰, R¹¹ et R¹² représentent, indépendamment l'un de l'autre, un groupe aryle ayant 6 à 10 atomes de carbone, qui est lui-même éventuellement substitué jusqu'à 2 fois identiques ou différentes par un groupe phényle, un halogène, ou par un groupe alkyle linéaire ou ramifié ayant jusqu'à 6 atomes de carbone,
R¹³ et R¹⁴ sont identiques ou différents et ont la définition indiquée ci-dessus pour R³ et R⁴,
ou bien
R⁵ et/ou R⁶ représentent un reste de formule 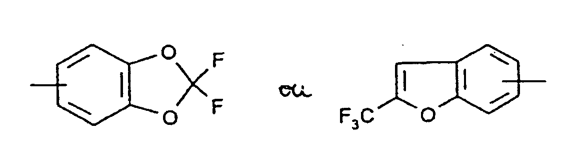
R⁷ est l'hydrogène ou un halogène,
et
R⁸ est l'hydrogène, un halogène, un groupe azido, trifluorométhyle, hydroxy, trifluorométhoxy, un groupe alkoxy ou alkyle linéaire ou ramifié ayant chacun jusqu'à 6 atomes de carbone ou un reste de formule -NR¹⁵R¹⁶,
dans laquelle
R¹⁵ et R¹⁶ sont identiques ou différents et ont la définition indiquées ci-dessus pour R³ et R⁴,
ou bien
R⁷ et R⁸ forment ensemble un reste de formule =O ou =NR¹⁷,
où
R¹⁷ désigne l'hydrogène ou un reste alkyle, alkoxy ou acyle linéaire ou ramifié ayant dans chaque cas jusqu'à 6 atomes de carbone,
L est une chaîne alkylénique ou alcénylénique linéaire ou ramifiée ayant dans chaque cas jusqu'à 8 atomes de carbone et chacune étant éventuellement substituée jusqu'à 2 fois par un groupe hydroxy,
T et X sont identiques ou différents et représentent une chaîne alkylénique linéaire ou ramifiée ayant jusqu'à 8 atomes de carbone,
ou bien
T ou X désigne une liaison,
V est un atome d'oxygène ou de soufre ou un groupe -NR¹⁸-
dans lequel
R¹⁸ représente l'hydrogène ou un groupe alkyle linéaire ou ramifié ayant jusqu'à 6 atomes de carbone ou un groupe phényle,
E est un reste cycloalkyle ayant 3 à 8 atomes de carbone ou un reste alkyle linéaire ou ramifié ayant jusqu'à 8 atomes de carbone, qui est éventuellement substitué par un groupe cycloalkyle ayant 3 à 8 atomes de carbone ou un groupe hydroxy, ou représente un groupe phényle qui est éventuellement substitué par un halogène ou un groupe trifluorométhyle,
R¹ et R² forment ensemble une chaîne alkylénique linéaire ou ramifiée ayant jusqu'à 7 atomes de carbone, qui doit être substituée par un groupe oxo et/ou par un reste de formule 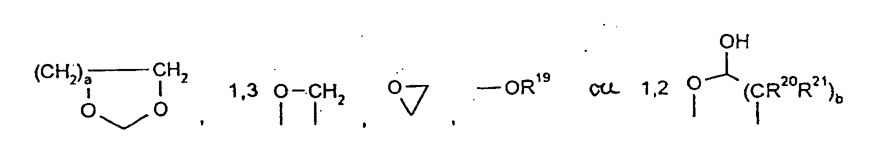 où
a et b sont identiques ou différents et représentent le nombre 1, 2 ou 3,
R¹⁹ est l'hydrogène, un reste cycloalkyle ayant 3 à 7 atomes de cabone, un reste silylalkyle linéaire ou ramifié ayant jusqu'à 8 atomes de carbone ou un reste alkyle linéaire ou ramifié ayant jusqu'à 8 atomes de carbone, qui est éventuellement substitué par un groupe hydroxy, un groupe alkoxy linéaire ou ramifié ayant jusqu'à 6 atomes de carbone ou par un groupe phényle qui peut lui-même être substitué par un halogène, un groupe nitro, trifluorométhyle, trifluorométhoxy ou par un groupe phényle ou tétrazole,
et le groupe alkyle est éventuellement substitué par un groupe de formule -OR²²
dans laquelle
R²² est un groupe acyle linéaire ou ramifié ayant jusqu'à 4 atomes de carbone ou un groupe benzyle,
ou bien
R¹⁹ est un reste acyle linéaire ou ramifié ayant jusqu'à 20 atomes de carbone ou un reste benzoyle, qui est éventuellement substitué par un halogène, un groupe trifluorométhyle, nitro ou trifluorométhoxy, ou bien
un reste fluoracyle linéaire ou ramifié ayant jusqu'à 8 atomes de carbone et jusqu'à 9 atomes de fluor,
R²⁰ et R²¹ sont identiques ou différents et désignent l'hydrogène, un groupe phényle ou un groupe alkyle linéaire ou ramifié ayant jusqu'à 6 atomes de carbone,
ou bien
R²⁰ et R²¹ forment ensemble un carbocycle de 3 à 6 chaînons,
et les carbocycles formés portent, le cas échéant, jusqu'à 6 substituants identiques ou différents, éventuellement géminés, trifluorométhyle, hydroxy, nitrile, halogène, carboxyle, nitro, azido, cyano, cycloalkyle ou cycloalkyloxy ayant chacun jusqu'à 3 à 7 atomes de carbone, alkoxycarbonyle, alkoxy ou alkylthio linéaire ou ramifié ayant chacun jusqu'à 6 atomes de carbone, ou bien alkyle linéaire ou ramifié ayant jusqu'à 6 atomes de carbone, lui-même substitué jusqu'à 2 fois identiques ou différentes par un groupe hydroxyle, benzyloxy, trifluorométhyle, benzoyle, un groupe alkoxy, oxyacyle ou carboxyle linéaire ou ramifié ayant chacun jusqu'à 4 atomes de carbone, et/ou phényle qui peut lui-même porter un substituant halogéno, trifluorométhyle ou trifluorométhoxy,
et/ou les carbocycles formés portent éventuellement jusqu'à 5 substituants identiques ou différents, également géminés, phényle, benzoyle, thiophényle ou sulfonylbenzyle qui sont eux-mêmes éventuellement substitués par un halogène, un groupe trifluorométhyle, trifluorométhoxy ou nitro, et/ou sont substitués, le cas échéant, par un reste de formule 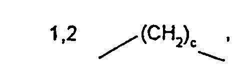
-SO₂-C₆H₅,
-(CO)_{d}-NR²³R²⁴
ou =O
dans laquelle
c représente le nombre 1, 2, 3 ou 4,
d représente le nombre 0 ou 1,
R²³ et R²⁴ sont identiques ou différents et représentent l'hydrogène, un reste cycloalkyle ayant 3 à 6 atomes de carbone, un reste alkyle linéaire ou ramifié ayant jusqu'à 6 atomes de carbone, un reste benzyle ou phényle qui porte, le cas échéant, jusqu'à 2 substituants, identiques ou différents, halogène, trifluorométhyle, cyano, phényle ou nitro,
et/ou les carbocycles formés sont éventuellement substitués par un reste à liaison spiro de formule 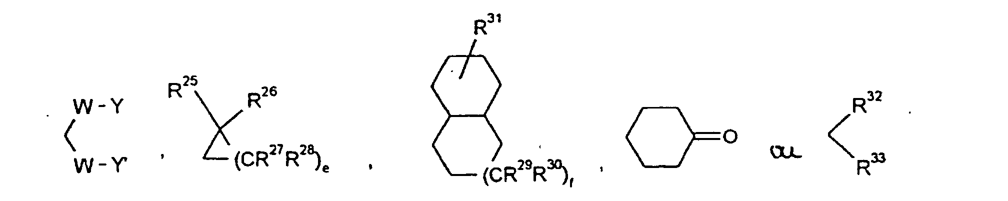 dans laquelle
W désigne un atome d'oxygène ou un atome de soufre,
Y et Y' forment ensemble une chaîne alkylénique linéaire ou ramifiée de 2 à 6 chaînons,
e représente le nombre 1, 2, 3, 4, 5, 6 ou 7,
f représente le nombre 1 ou 2,
R²⁵, R²⁶, R²⁷, R²⁸, R²⁹, R³⁰ et R³¹ sont identiques ou différents et représentent l'hydrogène, un groupe trifluorométhyle, phényle, un halogène ou un groupe alkyle ou alkoxy linéaire ou ramifié ayant dans chaque cas jusqu'à 6 atomes de carbone,
ou bien
R²⁵ et R²⁶ ou R²⁷ et R²⁸ forment ensemble dans chaque cas une chaîne alkyle linéaire ou ramifiée ayant jusqu'à 6 atomes de carbone,
ou bien
R²⁵ et R²⁶ ou R²⁷ et R²⁸ forment ensemble dans chaque cas un reste de formule 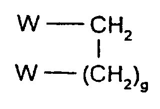 dans laquelle
W a la définition indiquée ci-dessus,
g est le nombre 1, 2, 3, 4, 5, 6 ou 7,
R³² et R³³ forment ensemble un hétérocycle de 3 à 7 chaînons qui contient un atome d'oxygène ou de soufre ou un groupe de formule SO, SO₂ ou -NR³⁴,
où
R³⁴ est l'hydrogène, un groupe phényle, benzyle ou un groupe alkyle linéaire ou ramifié ayant jusqu'à 4 atomes de carbone,
et leurs sels et leurs N-oxydes, à l'exception de la 5(6H)-quinolone à substituant 3-benzoyl-7,8-dihydro-2,7,7-triméthyl-4-phényle.

2. Cycloalcanopyridines de formule suivant la revendication 1,
dans laquelle
A est un reste naphtyle ou un reste phényle qui porte éventuellement jusqu'à 2 subtituants, identiques ou différents, fluor, chlore, brome, amino, hydroxy, trifluorométhyle, trifluorométhoxy, alkyle ou alkoxy linéaire ou ramifié ayant chacun jusqu'à 6 atomes de carbone,
D est un reste phényle qui porte éventuellement un substituant nitro, fluor, chlore, brome, phényle, trifluorométhyle ou trifluorométhoxy,
ou
représente un reste de formule
R⁵―L― 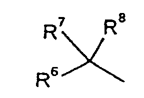
ou
R⁹―T―V―X―
dans laquelle
R⁵, R⁶ et R⁹ représentent, indépendamment l'un de l'autre, un reste cyclopropyle, cyclopentyle ou cyclohexyle, ou bien
un reste phényle, naphtyle, pyridyle, tétrazolyle, pyrimidyle, pyrazinyle, pyrrolidinyle, indolyle, morpholinyle, imidazolyle, benzothiazolyle, phénoxathiine-2-yle, benzoxazolyle, furyle, quinolyle ou purine-8-yle,
les cycles étant substitués éventuellement jusqu'à 3 fois identiques ou différentes, également par l'intermédiaire de la fonction N dans le cas des noyaux contenant de l'azote, par du fluor, du chlore, du brome, un groupe trifluorométhyle, hyroxy, cyano, carboxyle, trifluorométhoxy, acyle, alkyle, alkylthio, alkylalkoxy, alkoxy ou alkoxycarbonyle linéaire ou ramifié ayant chacun jusqu'à 4 atomes de carbone, triazolyle, tétrazolyle, benzoxathiazolyle, ou bien phényle à substituant trifluorométhyle ou phényle,
et/ou substitués par un groupe de formule -OR¹⁰, -SR¹¹ ou -SO₂R¹²,
où
R¹⁰, R¹¹ et R¹² sont identiques ou différents et désignent un groupe phényle qui est lui-même éventuellement substitué jusqu'à 2 fois identiques ou différentes par un groupe phényle, le fluor, le chlore ou par un groupe alkyle linéaire ou ramifié ayant jusqu'à 4 atomes de carbone,
ou bien
R⁵ et/ou R⁶ représentent un reste de formule 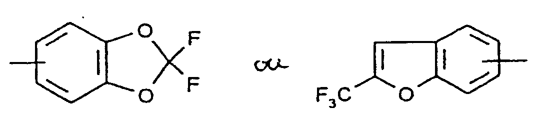
R⁷ est l'hydrogène, le fluor, le chlore ou le brome,
et
R⁸ est l'hydrogène, le fluor, le chlore, le brome, un groupe azido, trifluorométhyle, hydroxy, trifluorométhoxy, un groupe alkoxy ou alkyle linéaire ou ramifié ayant chacun jusqu'à 5 atomes de carbone ou un reste de formule -NR¹⁵R¹⁶,
dans laquelle
R¹⁵ et R¹⁶ sont identiques ou différents et représentent l'hydrogène, un groupe phényle ou un groupe alkyle linéaire ou ramifié ayant jusqu'à 4 atomes de carbone,
ou bien
R⁷ et R⁸ forment ensemble un reste de formule =O ou =NR¹⁷,
dans laquelle
R¹⁷ est l'hydrogène ou un groupe alkyle, alkoxy ou acyle linéaire ou ramifié ayant chacun jusqu'à 4 atomes de carbone,
L désigne une chaîne alkylénique ou alcénylénique linéaire ou ramifiée ayant chacune jusqu'à 6 atomes de carbone et chacune étant éventuellement substituée jusqu'à 2 fois par un groupe hydroxy,
T et X sont identiques ou différents et désignent une chaîne alkylénique linéaire ou ramifiée ayant jusqu'à 6 atomes de carbone,
ou bien
T ou X désigne une liaison,
V est un atome d'oxygène ou de soufre ou un groupe de formule -NR¹⁸-
dans laquelle
R¹⁸ désigne l'hydrogène ou un groupe alkyle linéaire ou ramifié ayant jusqu'à 4 atomes de carbone ou un groupe phényle,
E est un reste cyclopropyle, cyclobutyle, cyclopentyle, cyclohexyle ou cycloheptyle,
ou bien
un reste alkyle linéaire ou ramifié ayant jusqu'à 6 atomes de carbone, qui porte éventuellement un substituant cyclopropyle, cyclobutyle, cyclohexyle, cyclopentyle, cycloheptyle ou un substituant hydroxy, ou représente un reste phényle qui est éventuellement substitué par du fluor, du chlore ou un groupe trifluorométhyle,
R¹ et R² forment ensemble une chaîne alkylénique linéaire ou ramifiée ayant jusqu'à 6 atomes de carbone, qui doit être substituée par un groupe oxo et/ou par un reste de formule 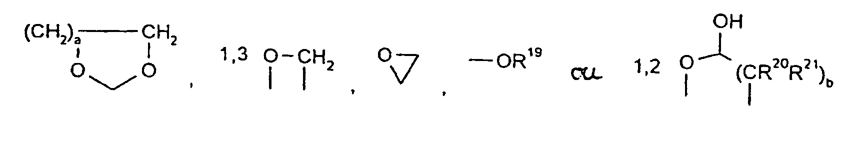 où
a et b sont égaux ou différents et désignent le nombre 1, 2 ou 3,
R¹⁹ représente l'hydrogène, un reste cyclopropyle, cyclopentyle, cyclohexyle, un reste silylalkyle linéaire ou ramifié ayant jusqu'à 7 atomes de carbone ou un reste alkyle linéaire ou ramifié ayant jusqu'à 6 atomes de carbone, qui est éventuellement substitué par un groupe hydroxy, un groupe alkoxy linéaire ou ramifié ayant jusqu'à 4 atomes de carbone ou par un groupe phényle qui peut lui-même être substitué par du fluor, du chlore, du brome, un groupe nitro, trifluorométhyle, trifluorométhoxy ou par un groupe phényle à substituant phényle ou tétrazole,
et le reste alkyle est éventuellement substitué par un groupe de formule -OR²²
dans laquelle
R²² désigne un groupe acyle linéaire ou ramifié ayant jusqu'à 3 atomes de carbone ou un groupe benzyle,
ou bien
R¹⁹ est un reste acyle linéaire ou ramifié ayant jusqu'à 18 atomes de carbone ou un reste benzoyle, qui est éventuellement substitué par du fluor, du chlore, du brome, un groupe trifluorométhyle, nitro ou trifluorométhoxy, ou bien un reste fluoracyle linéaire ou ramifié ayant jusqu'à 6 atomes de carbone,
R²⁰ et R²¹ identiques ou différents, représentent l'hydrogène, un reste phényle ou un reste alkyle linéaire ou ramifié ayant jusqu'à 4 atomes de carbone,
ou bien
R²⁰ et R²¹ forment ensemble un noyau cyclopropyle, cyclobutyle, cyclopentyle, cyclohexyle ou cycloheptyle,
et les carbocycles formés portent éventuellement jusqu'à 5 substituants identiques ou différents, éventuellement géminés, trifluorométhyle, hydroxy, carboxyle, azido, fluor, chlore, brome, nitro, cyano, cyclopropyle, cyclobutyle, cyclopentyle, cyclohexyle, cyclopropyloxy, cyclopentyloxy, cyclohexyloxy, alkoxycarbonyle, alkoxy ou alkylthio linéaire ou ramifié ayant chacun jusqu'à environ 5 atomes de carbone, ou alkyle linéaire ou ramifié ayant jusqu'à 5 atomes de carbone, qui est lui-même substitué jusqu'à 2 fois identiques ou différentes par un groupe hydroxyle, benzyloxy, benzoyle, un groupe alkoxy, ou oxyacyle linéaire ou ramifié ayant jusqu'à 3 atomes de carbone, un groupe trifluorométhyle et/ou un groupe phényle qui peut lui-même être substitué par du fluor, du chlore, du brome, un groupe trifluorométhyle ou trifluorométhoxy,
et/ou les carbocycles formés portent éventuellement jusqu'à 4 substituants identiques ou différents, également géminés, phényle, benzoyle, thiophényle ou sulfonylbenzyle qui sont eux-mêmes éventuellement substitués par du fluor, du chlore, du brome, un groupe trifluorométhyle, trifluorométhoxy ou nitro,
et/ou sont substitués, le cas échéant, par un reste de formule 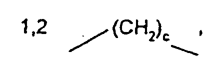
-SO₂-C₆H₅,
-(CO)_{d}-NR²³R²⁴
ou
=O
dans laquelle
c désigne le nombre 1, 2, 3 ou 4,
d représente le nombre 0 ou 1,
R²³ et R²⁴ sont identiques ou différents et représentent l'hydrogène, un reste cyclopropyle, cyclobutyle, cyclopentyle, cyclohexyle, un reste alkyle linéaire ou ramifié ayant jusqu'à 5 atomes de carbone, un reste benzyle ou phényle qui porte éventuellement un substituant fluoro, chloro, bromo, phényle ou trifluorométhyle,
et/ou les carbocycles formés sont éventuellement substitués par un reste à liaison spiro de formule 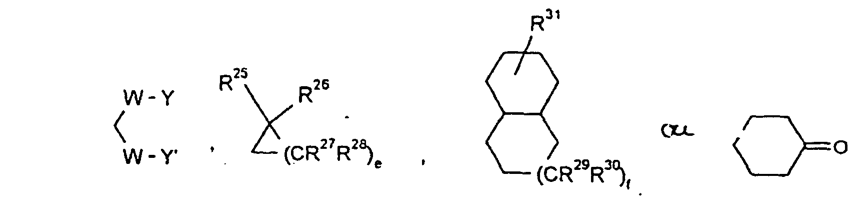 dans laquelle
W désigne un atome d'oxygène ou un atome de soufre,
Y et Y' forment ensemble une chaîne alkylique linéaire ou ramifiée de 2 à 5 chaînons,
e représente le nombre 1, 2, 3, 4, 5 ou 6,
f représente le nombre 1 ou 2,
R²⁵, R²⁶, R²⁷, R²⁸, R²⁹, R³⁰ et R³¹ sont identiques ou différents et représentent l'hydrogène, un groupe trifluorométhyle, phényle, le fluor, le chlore, le brome ou un groupe alkyle ou alkoxy linéaire ou ramifié ayant chacun jusqu'à 5 atomes de carbone,
ou bien
R²⁵ et R²⁶ ou R²⁷ et R²⁸ forment ensemble dans chaque cas une chaîne alkylique linéaire ou ramifiée ayant jusqu'à 5 atomes de carbone, ou bien
R²⁵ et R²⁶ ou R²⁷ et R²⁸ forment ensemble dans chaque cas un reste de formule 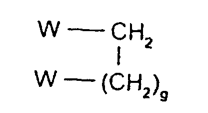 dans laquelle
W a la définition indiquée ci-dessus,
g est le nombre 1, 2, 3, 4, 5 ou 6,
et leurs sels et leurs N-oxydes, à l'exception de la 5(6H)-quinolone à substituant 3-benzoyl-7,8-dihydro-2,7,7-triméthyl-4-phényle.

3. Cycloalcanopyridines de formule suivant la revendication 1,
dans laquelle
A est un reste phényle qui est éventuellement substitué jusqu'à 2 fois identiques ou différentes par du fluor, du chlore, du brome, un groupe hydroxy, trifluorométhyle, trifluorométhoxy ou par un groupe alkyle ou alkoxy linéaire ou ramifié ayant chacun jusqu'à 5 atomes de carbone,
D est un reste phényle qui est éventuellement substitué par un groupe nitro, trifluorométhyle, phényle, du fluor, du chlore ou du brome, ou
représente un reste de formule 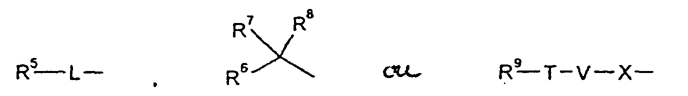 dans laquelle
R⁵, R⁶ et R⁹ représentent, indépendamment l'un de l'autre, un groupe cyclopropyle, cyclopentyle ou cyclohexyle, ou bien
un reste phényle, naphtyle, pyridyle, tétrazolyle, pyrimidyle, pyrazinyle, phénoxathiine-2-yle, indolyle, imidazolyle, pyrrolidinyle, morpholinyle, benzothiazolyle, benzoxazolyle, furyle, quinolyle ou purine-8-yle, les cycles étant éventuellement substitués jusqu'à 3 fois identiques ou différentes, par l'intermédiaire de la fonction N dans le cas des noyaux contenant de l'azote, par du fluor, du chlore, un groupe trifluorométhyle, hydroxy, cyano, carboxyle, trifluorométhoxy, un groupe alkyle, alkylthio, alkylalkoxy, alkoxy ou alkoxycarbonyle linéaire ou ramifié ayant chacun jusqu'à 4 atomes de carbone, triazolyle, tétrazolyle, benzothiazolyle, phényle à substituant trifluorométhyle ou phényle,
et/ou par un groupe de formule -OR¹⁰, -SR¹¹ ou -SO₂R¹²,
où
R¹⁰, R¹¹ et R¹² sont identiques ou différents et désignent un groupe phényle qui est lui-même éventuellement substitué jusqu'à 2 fois identiques ou différentes par un groupe phényle, le fluor, le chlore ou par un groupe alkyle linéaire ou ramifié ayant jusqu'à 3 atomes de carbone,
ou bien
R⁵ et/ou R⁶ représentent un reste de formule 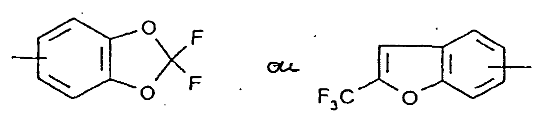
R⁷ est l'hydrogène ou le fluor,
et
R⁸ est l'hydrogène, le fluor, le chlore, le brome, un groupe azido, trifluorométhyle, hydroxy, trifluorométhoxy ou un groupe alkoxy ou alkyle linéaire ou ramifié ayant chacun jusqu'à 4 atomes de carbone ou un reste de formule -NR¹⁵R¹⁶,
dans laquelle
R¹⁵ et R¹⁶ sont identiques ou différents et représentent l'hydrogène ou un reste alkyle linéaire ou ramifié ayant jusqu'à 3 atomes de carbone,
ou bien
R⁷ et R⁸ forment ensemble un reste de formule =O ou =NR¹⁷,
où
R¹⁷ désigne l'hydrogène ou un reste alkyle, alkoxy ou acyle linéaire ou ramifié ayant dans chaque cas jusqu'à 4 atomes de carbone,
L est une chaîne alkylénique ou une chaîne alcénylénique linéaire ou ramifiée ayant chacune jusqu'à 5 atomes de carbone et chacune portant éventuellement jusqu'à 2 substituants hydroxy,
T et X sont identiques ou différents et représentent chacun une chaîne alkylénique linéaire ou ramifiée ayant jusqu'à 3 atomes de carbone,
ou bien
T ou X représente une liaison,
V est un atome d'oxygène ou de soufre ou un groupe de formule -NR¹⁸,
dans laquelle
R¹⁸ est l'hydrogène ou un groupe alkyle linéaire ou ramifié ayant jusqu'à 3 atomes de carbone,
E est un reste cyclopropyle, cyclopentyle ou cyclohexyle ou un reste phényle qui est éventuellement substitué par du fluor ou par un radical trifluorométhyle, ou bien
un reste alkyle linéaire ou ramifié ayant jusqu'à 4 atomes de carbone, qui est éventuellement substitué par un groupe hydroxy,
R¹ et R² forment ensemble une chaîne alkylénique linéaire ou ramifiée ayant jusqu'à 5 atomes de carbone, qui doit être substituée par un groupe oxo et/ou par un reste de formule 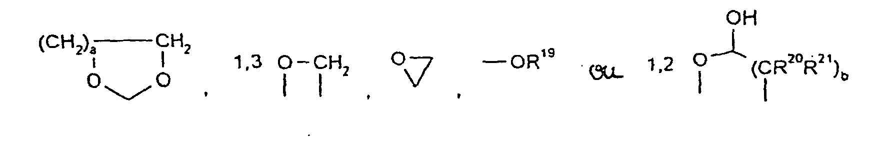 où
a et b sont égaux ou différents et représentent le nombre 1, 2 ou 3,
R¹⁹ représente l'hydrogène, un reste cyclopropyle, cyclopentyle, cyclohexyle, un reste silylalkyle linéaire ou ramifié ayant jusqu'à 6 atomes de carbone ou un reste alkyle linéaire ou ramifié ayant jusqu'à 4 atomes de carbone, qui est éventuellement substitué par un groupe hydroxy, un groupe alkoxy linéaire ou ramifié ayant jusqu'à 3 atomes de carbone ou par un groupe phényle qui peut lui-même être substitué par du fluor, du chlore, du brome, un groupe nitro, trifluorométhyle, trifluorométhoxy ou par un groupe phényle ou phényle à substituant tétrazole,
et le groupe alkyle est éventuellement substitué par un groupe de formule -OR²²
dans laquelle
R²² est un groupe acyle linéaire ou ramifié ayant jusqu'à 3 atomes de carbone ou un groupe benzyle,
ou bien
R¹⁹ est un reste acyle linéaire ou ramifié ayant jusqu'à 15 atomes de carbone ou un reste benzoyle qui est éventuellement substitué par du fluor, du chlore, du brome, un groupe trifluorométhyle, nitro ou trifluorométhoxy, ou bien
un groupe fluoracyle linéaire ou ramifié ayant jusqu'à 4 atomes de carbone,
R²⁰ et R²¹ sont identiques ou différents et représentent l'hydrogène, un reste phényle ou un reste alkyle linéaire ou ramifié ayant jusqu'à 3 atomes de carbone,
ou bien
R²⁰ et R²¹ forment ensemble un noyau cyclopropyle, cyclopentyle ou cyclohexyle,
et les carbocycles formés portent éventuellement jusqu'à 4 substituants identiques ou différents, éventuellement géminés, fluor, hydroxyle, trifluorométhyle, nitrile, carboxyle, azido, chlore, brome, nitro, cyano, cyclopropyle, cyclobutyle, cyclopentyle, cyclohexyle, cyclopropyloxy, cyclopentyloxy, cyclohexyloxy, alkoxycarbonyle, alkoxy ou alkylthio linéaire ou ramifié ayant chacun jusqu'à 4 atomes de carbone, ou bien alkyle linéaire ou ramifié ayant jusqu'à 4 atomes de carbone, qui porte lui-même jusqu'à 2 substituants, identiques ou différents, hydroxyle, benzyloxy, trifluorométhyle, benzoyle, méthoxy, oxyacétyle et/ou phényle qui peut lui-même être substitué par du fluor, du chlore, du brome, un groupe trifluorométhyle ou trifluorométhoxy, et/ou les carbocycles formés portent éventuellement jusqu'à 4 substituants identiques ou différents, également géminés, phényle, benzoyle, thiophényle ou sulfonylbenzyle qui sont eux-mêmes éventuellement substitués par du fluor, un groupe trifluorométhyle, trifluorométhoxy ou nitro,
et/ou sont substitués par un reste de formule 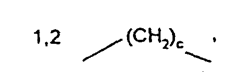 -SO₂-C₆H₅, -(CO)_{d}-NR²³R²⁴ ou =O
dans laquelle
c représente le nombre 1, 2, 3 ou 4,
d représente le nombre 0 ou 1,
R²³ et R²⁴ sont identiques ou différents et représentent l'hydrogène, un groupe cyclopropyle, cyclopentyle, benzyle, un groupe alkyle linéaire ou ramifié ayant jusqu'à 4 atomes de carbone ou un groupe phényle qui est éventuellement substitué par du fluor, du chlore ou du brome,
et/ou les carbocycles formés sont éventuellement substitués par un reste à liaison spiro de formule 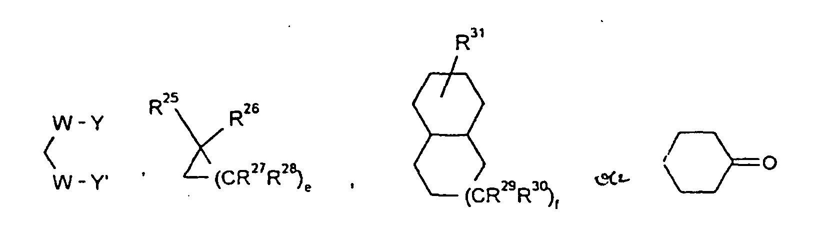 dans laquelle
W représente un atome d'oxygène ou un atome de soufre,
Y et Y' forment ensemble une chaîne alkyle linéaire ou ramifiée de 2 à 6 chaînons,
e représente le nombre 1, 2, 3, 4 ou 5,
f représente le nombre 1 ou 2,
R²⁵, R²⁶, R²⁷, R²⁸, R²⁹, R³⁰ et R³¹ sont identiques ou différents et représentent l'hydrogène, un groupe trifluorométhyle, phényle, le fluor, le chlore, le brome ou un groupe alkyle ou alkoxy linéaire ou ramifié ayant chacun jusqu'à 4 atomes de carbone,
ou bien
R²⁵ et R²⁶ ou R²⁷ et R²⁸ forment ensemble dans chaque cas une chaîne alkyle linéaire ou ramifiée ayant jusqu'à 4 atomes de carbone,
ou bien
R²⁵ et R²⁶ ou R²⁷ et R²⁸ forment ensemble dans chaque cas un reste de formule 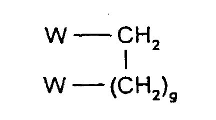 dans laquelle
W a la définition indiquée ci-dessus,
g est le nombre 1, 2, 3, 4, 5, 6 ou 7,
et leurs sels et leurs N-oxydes, à l'exception de la 5(6H)-quinolone à substituant 3-benzoyl-7,8-dihydro-2,7,7-triméthyl-4-phényle.

4. Cycloalcanopyridines suivant les revendications 1 à 3, utilisées comme médicament.

5. Procédé de production de cycloalcanopyridines suivant les revendications 1 à 3, **caractérisé en ce que** :
[A] au cas où D n'est pas un groupe aryle, on synthétise dans des composés de formule générale (II) 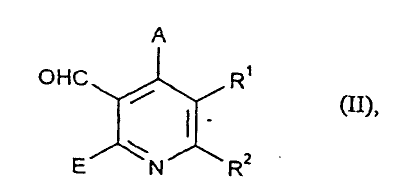 dans laquelle
A, E, R¹ et R² ont la définition indiquée ci-dessus,
avec des réactifs organométalliques au sens d'une réaction de Grignard, d'une réaction de Wittig ou d'une réaction avec un composé organique de lithium, le substituant D dans des solvants inertes,
ou bien au cas où D représente le reste de formule R⁹-T-V-X dans laquelle V est un atome d'oxygène,
[B] on fait réagir des composés de formule générale (III) 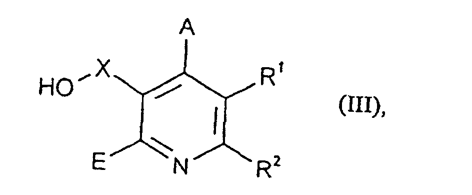 dans laquelle
A, E, X, R¹ et R² ont la définition indiquée ci-dessus,
avec des composés de formule générale (IV)
R⁹-T-Z (IV)
dans laquelle
R⁹ et T ont la définition indiquée ci-dessus,
et
Z est un halogène, de préférence le chlore ou le brome,
dans des solvants inertes, éventuellement en présence d'une base et/ou d'une substance auxiliaire,
ou bien
[C] on transforme des composés. de formule générale (III) tout d'abord par réaction avec des composés de formule générale (V) 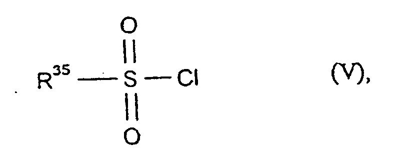 dans laquelle
R³⁵ est un reste alkyle linéaire ayant jusqu'à 4 atomes de carbone,
en composés de formule générale (VI) 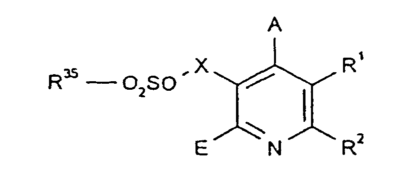 dans laquelle
A, E, X, R¹, R² et R³⁵ ont les définitions indiquées ci-dessus,
puis on fait réagir ces composés avec des composés de formule générale (VII)
R⁹-T-V-H (VII)
dans laquelle
R⁹, T et V ont la définition indiquée ci-dessus,
et on élimine éventuellement les groupes protecteurs,
ou bien
[D] dans le cas des composés de formule générale (Ia) 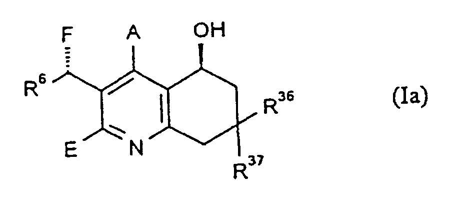
dans laquelle
A et R⁶ ont la définition indiquée ci-dessus,
R³⁶ et R³⁷ sont identiques ou différents et représentent un groupe trifluorométhyle, un halogène, un groupe nitro, azido, cyano, cycloalkyle ou cycloalkyloxy ayant chacun 3 à 7 atomes de carbone, ou un groupe alkoxycarbonyle, alkoxy ou alkylthio linéaire ou ramifié ayant dans chaque cas jusqu'à 6 atomes de carbone ou un groupe alkyle linéaire ou ramifié ayant jusqu'à 6 atomes de carbone, ou bien
un groupe phényle, benzoyle, thiophényle ou sulfonylbenzyle dont chacun est éventuellement substitué par un halogène, un radical trifluorométhyle, trifluorométhoxy ou nitro, ou bien
R³⁵ et R³⁶ représentent l'un des restes à liaison spiro mentionnés ci-dessus, de formule 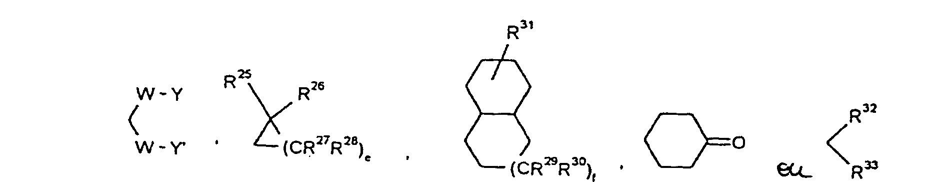
dans laquelle
W, Y, Y', R²⁵, R²⁶, R²⁷, R²⁸, e, R²⁹, R³⁰, R³¹, R³² et R³³ ont la définition indiquée ci-dessus,
on oxyde tout d'abord des composés de formule générale (VIII) 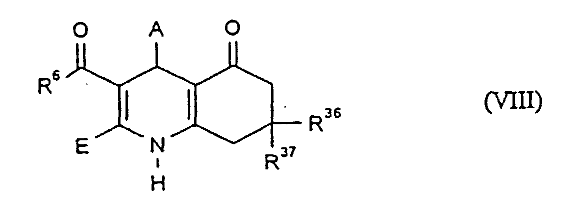 dans laquelle
R⁶, R³⁶, R³⁷, A et E ont la définition indiquée ci-dessus, tout d'abord en composés de formule générale (IX) 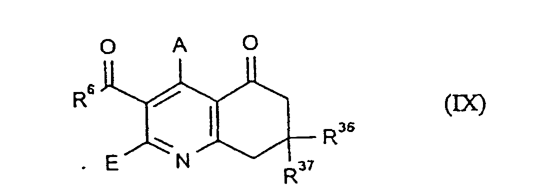 dans laquelle
R⁶, R³⁶, R³⁷, A et E ont la définition indiquée ci-dessus,
on fait réagir ces composés dans une étape subséquente par une réduction asymétrique en composés de formule générale (X) 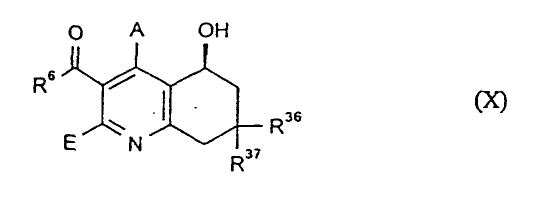 dans laquelle
R⁶, R³⁶, R³⁷, A et E ont la définition indiquée ci-dessus,
on transforme ensuite ces composés par l'introduction d'un groupe protégeant la fonction hydroxy en composés de formule générale (XI) 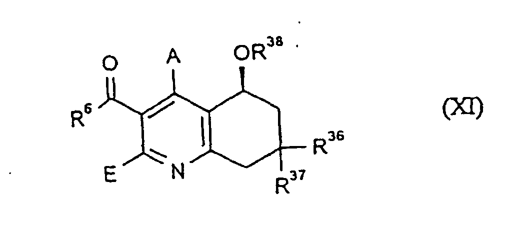 dans laquelle
R⁶, R³⁶, R³⁷, A et E ont la définition indiquée ci-dessus,
et
R³⁸ est un groupe protégeant la fonction hydroxy, de préférence un reste de formule -SiR³⁹R⁴⁰R⁴¹,
dans laquelle
R³⁹, R⁴⁰ et R⁴¹ sont identiques ou différents et désignent des restes alkyle en Ci à C₄,
on prépare à partir de ces composés dans une étape suivante, par réduction diastéréosélective, les composés de formule générale (XII) 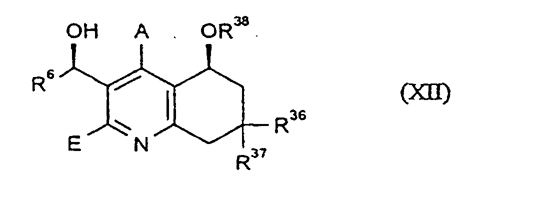 dans laquelle
R⁶, R³⁶, R³⁷, R³⁸, A et E ont la définition indiquée ci-dessus,
puis on prépare par introduction du substituant fluoro avec des réactifs de fluoration et des dérivés SF₄ les composés de formule générale (XIII) 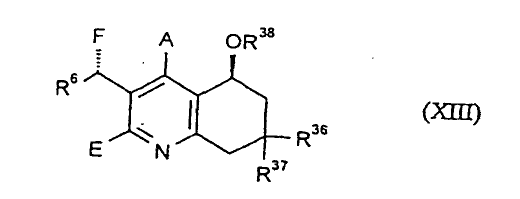 dans laquelle
R⁶, R³⁶, R³⁷, R³⁸, A et E ont la définition indiquée ci-dessus,
puis on élimine le groupe protégeant la fonction hydroxy selon des modes opératoires classiques,
et, le cas échéant, on fait varier ou on introduit selon des modes opératoires classiques les substituants énumérés en D, E et/ou R¹ et R².

6. Médicament contenant au moins une cycloalcanopyridine suivant les revendications 1 à 3, de même que des auxiliaires de formulation acceptables du point de vue pharmacologique.

7. Médicament suivant la revendication 6, destiné au traitement de l'artériosclérose et d'une dyslipidémie.

8. Utilisation de cycloalcanopyridines suivant les revendications 1 à 3 pour la préparation de médicaments.

9. Utilisation suivant la revendication 8, pour la préparation de médicaments destinés au traitement de l'artériosclérose, en particulier de dyslipidémies.

10. Produits intermédiaires de la série :
a) 3-amino-3-cyclopentyl-1-(4-trifluorométhylphényl)-propénone de formule 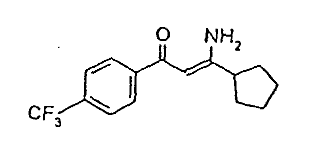
b) 2-cyclopentyl-4-(4-fluorophényl)-7,7-diméthyl-3-(4-trifluorométhylbenzoyl)-4,6,7,8-tétrahydro-lH-quinoléine-5-one de formule 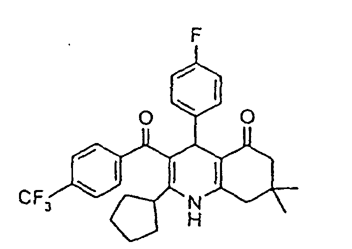
c) 2-cyclopentyl-4-(4-fluorophényl)-7,7-diméthyl-3-(4-trifluorométhylbenzoyl)-7,8-dihydro-6H-quinoléine-5-one de formule 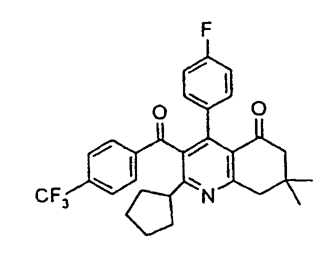
d) [2-cyclopentyl-4-(4-fluorophényl)-5-hydroxy-7,7-diméthyl-5,6,7,8-tétrahydroquinoléine-3-yl]-(4-trifluorométhylphényl)-méthanone de formule 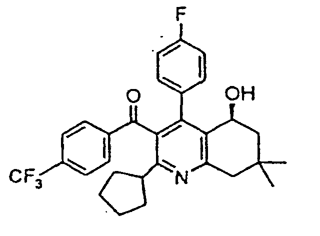
e) [5-(tertio-butyldiméthylsilanyloxy)-2-cyclopentyl-4-(4-fluorophényl)-7,7-diméthyl-5,6,7,8-tétrahydroquinoléine-3-yl]-(4-trifluorométhylphényl)-méthanone de formule 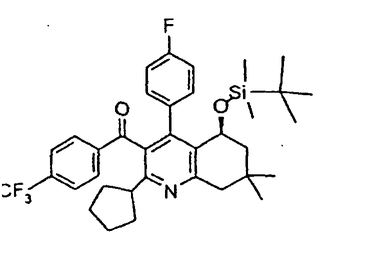
f) [5-(tertio-butyldiméthylsilanyloxy)-2-cyclopentyl-4-(4-fluorophényl)-7,7-diméthyl-5,6,7,8-tétrahydroquinoléine-3-yl]-(4-trifluorométhylphényl)-méthanol de formule 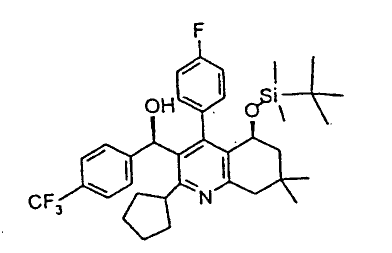
g) 5-(tertio-butyldiméthylsilanyloxy)-2-cyclopentyl-4-(4-fluorophényl)-3-[fluoro-(4-trifluorométhylphényl)-méthyl]-7,7-diméthyl-5,6,7,8-tétrahydroquinoléine de formule

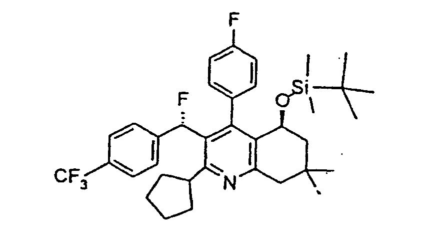

11. Le 2-cyclopentyl-4-(4-fluorophényl)-3-[fluoro-(4-trifluorométhylphényl)-méthyl]-7,7-diméthyl-5,6,7,8-tétrahydroquinoléine-5-ol suivant la revendication 1, de formule
